# FASCICULE DE BREVET EUROPEEN

(11) **EP 0 964 870 B1**
(45) Date de publication et mention de la délivrance du brevet: **10.12.2003**
(21) Numéro de dépôt: 98936611.7
(22) Date de dépôt: 06.08.1998
(51) Int. Cl.: C07K 14/00

(54) **RECEPTEUR LSR, CLONAGE ET APPLICATIONS**
KLONIERUNG UND VERWENDUNG DES LSR-REZEPTORS
LSR RECEPTOR, ACTIVITY, CLONING, AND USES FOR DIAGNOSING, PREVENTING AND/OR TREATING OBESITY AND RELATED RISKS OR COMPLICATIONS

(30) Priorité: 06.08.1997 FR 9710088; 22.04.1998 FR 9805032
(43) Date de publication de la demande: 22.12.1999
(60) Demande divisionnaire: 03102647.9
(73) Titulaire: Genset, 91000 Evry (FR); INSTITUT NATIONAL DE LA SANTE ET DE LA RECHERCHE MEDICALE (INSERM), 75013 Paris (FR)
(72) Inventeur: BIHAIN, Bernard, Encinitas, California 92024 (US); BOUGUELERET, Lydie, F-92170 Vanves (FR); YEN-POTIN, Frances, San Diego, California 92126 (FR)
(74) Mandataire: Warcoin, Jacques
(86) Numéro de dépôt international: IB9801257
(87) Numéro de publication internationale: WO99007737

(56) Documents cités:
- WO-A-96/30400
- WO-A-97/27286
- MANN C J ET AL.: "ApoCIII inhibits the binding of triglyceride-rich lipoproteins to the lipolysis stimulated receptor" CIRCULATION, vol. 94, no. 8,supp., 15 octobre 1996, page I-698 XP002067462 US
- WOODWARD F P ET AL (EDS): "Atherosclerosis X" 1995 , ELSEVIER SCIENCE , AMSTERDAM NL XP002067469 page 465-470: "Characterization and purification of the lipolysis-stimulated receptor"
- GHEBREHIWET ET AL.: "Isolation, cDNA cloning, and overexpression of a 33-kD cell surface glycoprotein that binds to the globular "heads" of C1Q" THE JOURNAL OF EXPERIMENTAL MEDICINE, vol. 179, juin 1994, pages 1809-1821, XP002067463 NEW YORK US cité dans la demande
- HENRION A A ET AL: "Structure, sequence, and chromosomal location of the gene for USF2 transcription factors in mouse" GENOMICS, vol. 25, 1995, pages 36-43, XP002067465 SAN DIEGO US
- GROENEN P M A ET AL: "Structure, sequence and chromosome 19 location of human USF2 ..." EMBL SEQUENCE DATABASE,24 janvier 1997, XP002067467 HEIDELBERG DE
- LIN Q ET AL.: "Lisch7, a liver-specific gene immediately upstream of the USF2 gene on chromosome 7" EMBL SEQUENCE DATABASE,20 mai 1996, XP002096565 HEIDELBERG DE
- LIN Q ET AL.: "Liver-specific protein Lisch7" EMBL SEQUENCE DATABASE,1 novembre 1996, XP002096566 HEIDELBERG DE
- LAMERDIN J E: "Homo sapiens DNA from chromosome 19-cosmid R30879 containing USF2, genomic sequence" EMBL SEQUENCE DATABASE,26 mars 1997, XP002096567 HEIDELBERG DE
- LAMERDIN J E ET AL.: "Sequence analysis of a 1 Mb region of human 19q13.1" EMBL SEQUENCE DATABASE,28 mai 1997, XP002096568 HEIDELBERG DE
- LAMERDIN J E ET AL.: "Human Lisch7" EMBL SEQUENCE DATABASE,1 juillet 1997, XP002096569 HEIDELBERG DE
- LAMERDIN J E : "Liver-specific BHLP-ZIP transcription factor, Lisch7" EMBL SEQUENCE DATABASE,1 juillet 1997, XP002096570 HEIDELBERG DE
- MANN C J ET AL: "Mechanism of activation and functional significance of the lipolysis-stimulated receptor. Evidence for a role as chylomicron remnant receptor" BIOCHEMISTRY., vol. 34, 1995, pages 10421-10431, XP002067468 EASTON, PA US cité dans la demande

## Description

### INTRODUCTION

L'invention concerne un nouveau polypeptide récepteur complexe LSR (Lipolysis Stimulated Receptor), caractérisé par ses activités fonctionnelles, le clonage des ADNc complémentaires des ARNs messagers codant pour chacune des sous-unités du complexe multimérique, des vecteurs et cellules transformées, des méthodes de diagnostic et de sélection de composés utilisables à titre de médicament pour la prévention et/ou le traitement de pathologies et/ou de pathogénies telles que l'obésité et l'anorexie, les hyperlipidémies, l'athérosclérose, le diabète, l'hypertension, et plus généralement les diverses pathologies associées à des anomalies du métabolisme des cytokines.

L'obésité est un problème de santé publique à la fois grave et répandu : dans les pays industrialisés, le tiers de la population présente un excès de poids d'au moins 20 % par rapport au poids idéal. Le phénomène ne fait que s'accentuer, dans les régions du globe dont les économies se modernisent, comme les îles du Pacifique, et de façon générale. Aux Etats-Unis, le taux de personnes obèses est passé de 25 % à la fin des années 70 à 33 % au début des années 90.

L'obésité augmente de façon considérable le risque de développer des maladies cardio-vasculaires ou métaboliques. On estime que si toute la population avait un poids idéal, le risque d'insuffisance coronarienne serait diminué de 25 % et celui d'insuffisance cardiaque et d'accidents vasculaires cérébraux de 35%. L'insuffisance coronarienne, la maladie athéromateuse et l'insuffisance cardiaque sont au premier rang des complications cardio-vasculaires induites par l'obésité. Pour une surcharge pondérale supérieure à 30 %, l'incidence des affections coronariennes se trouve doublée, chez des sujets de moins de 50 ans. Les études menées pour d'autres maladies sont tout aussi éloquentes. Pour un surpoids de 20 %, le risque d'hypertension artérielle est doublé. Pour un surpoids de 30 %, le risque de développer un diabète non insulino-dépendant est triplé. Celui des hyperlipidémies est multiplié par 6.

La liste des maladies dont l'apparition est favorisée par l'obésité est longue : hyperuricémie (11,4 % chez les sujets obèses, contre 3,4 % dans la population générale), pathologies digestives, anomalies des fonctions hépatiques et même, certains cancers.

Que les altérations physiologiques de l'obésité se caractérisent par une augmentation du nombre des cellules adipeuses, ou par une augmentation de la quantité de triglycérides stockés dans chaque cellule adipeuse, ou par les deux, cette surcharge résulte principalement d'un déséquilibre entre les quantités de calories absorbées et celles des calories dépensées par l'organisme. Les recherches sur les causes de ce déséquilibre ont pris plusieurs directions. Certains se sont attachés à étudier le mécanisme d'absorption des aliments, et donc, les molécules qui contrôlent la prise alimentaire et la sensation de satiété. D'autres études ont porté sur le métabolisme de base, c'est-à-dire la façon dont l'organisme utilise les calories absorbées.

Les traitements de l'obésité qui sont proposés sont de quatre types. La restriction alimentaire est le plus fréquemment utilisé. Les obèses se voient conseiller de changer leurs habitudes alimentaires, de façon à absorber moins de calories. Ce type de traitement est efficace à court terme. Toutefois le taux de récidive est très important. L'augmentation des dépenses caloriques par l'exercice physique est également proposée. Ce traitement appliqué seul est inefficace mais il améliore toutefois la perte de poids chez les sujets suivant un régime hypocalorique. La chirurgie gastro-intestinale, qui diminue l'absorption des calories ingérées, est efficace mais a été virtuellement abandonnée en raison des effets secondaires qu'elle entraîne. L'approche médicamenteuse met en jeu, soit l'action anorexigène de molécules intervenant au niveau du système nerveux central, soit l'effet de molécules qui augmentent les dépenses énergétiques en augmentant la production de chaleur. Le prototype de ce type de molécule sont les hormones thyroïdiennes qui découplent les phosphorylations oxydatives de la chaîne respiratoire mitochondriale. Les effets secondaires et la toxicité de ce type de traitement rendent leur utilisation dangereuse. Une approche qui vise à diminuer l'absorption des lipides alimentaires en les séquestrant dans la lumière du tube digestif est également mise en place. Toutefois, elle induit des déséquilibres physiologiques difficilement tolérables : déficit d'absorption des vitamines liposolubles, flatulence et stéatorrhée. Quelle que soit l'approche thérapeutique envisagée, les traitements de l'obésité se caractérisent tous par un taux de récidive extrêmement important.

Les mécanismes moléculaires responsables de l'obésité chez l'homme sont complexes et font appel à des facteurs génétiques et environnementaux. En raison de la faible efficacité des traitements connus jusqu'à présent, il est urgent de définir les mécanismes génétiques qui déterminent l'obésité, de façon à pouvoir développer des médicaments mieux ciblés.

Plus de 20 gènes ont été étudiés en tant que candidats possibles, soit qu'ils aient été impliqués dans des maladies dont l'obésité est l'une des manifestations cliniques, soit qu'ils soient des homologues de gènes intervenant dans l'obésité chez les modèles animaux. Situé dans la région chromosomique 7q31, le gène OB est l'un des plus étudiés. Son produit, la leptine, intervient dans les mécanismes de la satiété. La leptine est une protéine plasmatique de 16 kDa produite par les adipocytes sous l'action de différents stimuli. Les souris obèses de type *ob*/*ob* présentent un déficit du gène de la leptine ; cette protéine est indétectable dans le plasma de ces animaux. L'administration de leptine obtenue par génie génétique, à des souris *ob*/*ob* corrige leur hyperphagie relative et permet la normalisation de leur poids. Cet effet anorexigène de la leptine met en jeu un récepteur du système nerveux central : le récepteur ob qui appartient à la famille des récepteurs aux cytokines de classe 1. Le récepteur ob est déficient chez les souris obèses de la race *db*/*db*. L'administration de leptine à ces souris est sans effet sur leur prise alimentaire et ne permet pas de réduction importante de leur poids. Les mécanismes par lesquels les récepteurs ob transmettent le signal de satiété ne sont pas connus avec précision. Il est vraisemblable que le neuropeptide Y soit impliqué dans cette voie de signalisation. Il est important de préciser à ce stade que les récepteurs ob ne sont pas les seuls régulateurs de l'appétit. Le récepteur Melanocortin 4 intervient également, puisque les souris rendues déficientes pour ce récepteur sont obèses (Gura, 1997).

La découverte de la leptine et la caractérisation du récepteur de la leptine au niveau du système nerveux central ont fait naître une nouvelle voie de recherche de médicaments contre l'obésité. Ce modèle s'est cependant rapidement révélé décevant. En effet, à une seule exception près (Montague et al., 1997), les gènes codant pour la leptine ou pour son récepteur ob se sont révélés normaux chez les sujets humains obèses. De plus, et de façon paradoxale, les concentrations plasmatiques de leptine, hormone de satiété, sont anormalement élevées chez la plupart des sujets humains obèses. L'essentiel des efforts de recherche thérapeutique dans ce sens a porté sur la caractérisation de l'effet de la leptine au niveau du système nerveux central.

### RESUME DE L'INVENTION

La présente invention résulte d'une focalisation de l'effort de recherche sur la découverte des mécanismes d'élimination de la leptine. L'hypothèse de travail la plus généralement admise est que les taux plasmatiques de leptine sont élevés chez les sujets obèses parce que cette hormone est produite par le tissu adipeux et que la masse grasse est augmentée chez les sujets obèses. Les inventeurs ont formulé une hypothèse différente et postulé que les concentrations de leptine sont augmentées chez les obèses car la clairance de cette hormone est réduite. Ce déficit entraîne un syndrome de résistance à la leptine et l'individu obèse développe une réponse adaptée aux concentrations élevées de leptine. Dans cette perspective, le traitement des sujets obèses devrait consister non pas en une augmentation des taux de leptine mais en une normalisation de ceux-ci. A ce stade, il est essentiel de rappeler que les récepteurs de type ob sont des récepteurs de type signalisation. Ces récepteurs peuvent fixer la leptine au niveau de la membrane plasmique mais ne peuvent faire entrer la protéine à l'intérieur de la cellule pour qu'elle y soit dégradée. Les récepteurs ob ne sont pas des récepteurs d'endocytose.

### Récepteur LSR

Les inventeurs ont caractérisé un récepteur, notamment hépatique, dénommé récepteur LSR, dont l'activité est double. Le récepteur LSR permet d'une part l'endocytose de lipoprotéines, lorsqu'il est activé par les acides gras libres, servant ainsi de voie de clairance des lipoprotéines. Cette voie sert principalement, mais non exclusivement pour la clairance des particules riches en triglycérides d'origine intestinale (Mann et al., 1995). Cette activité, exprimée tout particulièrement au niveau hépatique, est dépendante de la présence d'acides gras libres qui, en se fixant au récepteur, induisent un changement réversible de la conformation de ce complexe et lui permettent de fixer avec une haute affinité différentes classes de lipoprotéines, comme celles contenant de l'apoprotéine B ou de l'apoprotéine E.

D'autre part, dans des conditions normales, en l'absence d'acides gras libres, le récepteur complexe LSR ne fixe pas les lipoprotéines, mais est capable de fixer une cytokine, en particulier la leptine, puis de l'internaliser et de la dégrader.

Il doit être compris que l'invention ne concerne pas les récepteurs LSR sous forme naturelle, c'est-à-dire qu'ils ne sont pas pris dans leur environnement naturel mais obtenus par purification à partir de sources naturelles, ou bien obtenus par recombinaison génétique, ou encore par synthèse chimique et pouvant alors comporter des acides aminés non naturels, comme cela sera décrit ci-après. La production d'un récepteur LSR recombinant, qui peut être réalisée en utilisant l'une des séquences nucléotidiques selon l'invention, est particulièrement avantageuse car elle permet d'obtenir un niveau de pureté accrue du récepteur.

Plus particulièrement, l'invention concerne un récepteur LSR de rat purifié, caractérisé en ce qu'il comprend au moins une sous-unité ayant un poids moléculaire d'environ 66 kDa et une sous-unité ayant un poids moléculaire d'environ 58 kDa.

Le récepteur LSR de rat purifié de la présente invention est caractérisé en ce qu'il contient une sous-unité α comprenant la séquence d'acides aminés de SEQ ID 2 ou une séquence homologue de celle-ci ou une sous-unité α' comprenant la séquence d'acides aminés de SEQ ID 4 ou une séquence homologue de celle-ci, et une, de préférence trois, sous-unités β comprenant la séquence d'acides aminés de SEQ ID 6 ou une séquence homologue de celle-ci.

L'invention concerne également un récepteur LSR de souris purifié, caractérisé en ce qu'il comprend au moins une sous-unité ayant un poids moléculaire d'environ 66 kDa et une sous-unité ayant un poids moléculaire d'environ 58 kDa.

Le récepteur LSR de souris purifié de la présente invention est caractérisé en ce qu'il contient une sous-unité α comprenant la séquence d'acides aminés de SEQ ID 16 ou une séquence homologue de celle-ci ou une sous-unité α' comprenant la séquence d'acides aminés de SEQ ID 17 ou une séquence homologue de celle-ci et une, de préférence trois, sous-unités β comprenant la séquence d'acides aminés de SEQ ID 18 ou une séquence homologue de celle-ci.

L'invention concerne également un récepteur LSR humain purifié, caractérisé en ce qu'il comprend au moins une sous-unité ayant un poids moléculaire d'environ 72 kDa et une sous-unité ayant un poids moléculaire d'environ 64 kDa.

Le récepteur LSR humain purifié de la présente invention est caractérisé en ce qu'il contient une sous-unité α comprenant la séquence d'acides aminés de SEQ ID 8 ou une séquence homologue de celle-ci ou une sous-unité α' comprenant la séquence d'acides aminés de SEQ ID 10 ou une séquence homologue de celle-ci et une, de préférence trois, sous-unités β comprenant la séquence d'acides aminés de SEQ ID 12 ou une séquence homologue de celle-ci.

Une réalisation particulièrement préférée des récepteurs LSR de la présente invention est un récepteur LSR recombinant obtenu par l'expression dans un hôte recombinant d'une ou plusieurs des séquences nucléotidiques selon l'invention. Ce récepteur recombinant préféré est constitué d'une sous-unité α ou α' et d'une, de préférence trois, sous-unités β, notamment une sous-unité α ou α' et trois sous-unités β d'un récepteur LSR humain.

### Séquences polypeptidiques de LSR

L'invention concerne des polypeptides, caractérisés en ce qu'ils sont un constituant d'un récepteur LSR selon l'invention.

Il doit être compris que l'invention ne concerne pas les polypeptides sous forme naturelle, c'est-à-dire qu'ils ne sont pas pris dans leur environnement. En effet, l'invention concerne les peptides obtenus par purification à partir de sources naturelles, ou bien obtenus par recombinaison génétique, ou encore par synthèse chimique et pouvant alors comporter des amino-acides non naturels, comme cela sera décrit ci-après. La production d'un polypeptide recombinant, qui peut être réalisée en utilisant l'une des séquences nucléotidiques selon l'invention ou un fragment d'un de ces séquences, est particulièrement avantageuse car elle permet d'obtenir un niveau de pureté accrue du polypeptide désiré.

L'invention concerne donc un polypeptide purifié, isolé ou recombinant, comprenant une séquence d'au moins 5, de préférence d'au moins 10 à 15 acides aminés consécutifs d'un récepteur LSR, ainsi que les homologues, équivalents ou variants dudit polypeptide, ou un de leurs fragments. De préférence, la séquence d'au moins 10 à 15 acides aminés du récepteur LSR est un fragment biologiquement actif d'un récepteur LSR.

Plus particulièrement, l'invention concerne des polypeptides purifiés, isolés ou recombinants, comprenant une séquence d'au moins 10 à 15 acides aminés d'un récepteur LSR de rat, d'un récepteur LSR de souris ou d'un récepteur LSR humain.

Dans la présente description, on utilisera le terme polypeptide pour désigner également une protéine ou un peptide.

### Séquences nucléotidiques de LSR

La présente invention a également pour objet des séquences d'acide nucléique purifiées, caractérisées en ce qu'elles codent pour un récepteur LSR ou un polypeptide selon l'invention.

L'invention concerne un acide nucléique purifié, caractérisé en ce qu'il comprend au moins 8, de préférence au moins 10 et plus particulièrement au moins 15 nucléotides consécutifs du polynucléotide d'une séquence génomique, d'ADNc ou d'ARN, de récepteur LSR, ainsi que les séquences d'acides nucléiques complémentaires de cet acide nucléique.

Plus particulièrement, l'invention concerne des acides nucléiques purifiés, isolés ou recombinants, comprenant une séquence d'au moins 8, de préférence au moins 10 et plus particulièrement au moins 15 nucléotides consécutifs du polynucléotide d'une séquence nucléique d'un récepteur LSR de souris ou d'un récepteur LSR humain.

L'invention concerne également les séquences nucléiques variantes, mutées, équivalentes ou homologues des séquences nucléiques selon l'invention, ou un de leurs fragments. Elle concerne enfin les séquences capables de s'hybrider spécifiquement avec les séquences nucléiques selon l'invention.

L'invention concerne donc également les séquences d'acides nucléiques contenues dans le gène codant pour le récepteur LSR, notamment chacun des exons dudit gène ou une combinaison d'exons dudit gène, ou encore un polynucléotide s'étendant sur une portion d'un ou plusieurs exons. De façon préférée, ces acides nucléiques codent pour un ou plusieurs fragments biologiquement actifs du récepteur LSR humain.

La présente invention concerne également les séquences d'acides nucléiques purifiées codant pour un ou plusieurs éléments de régulation de l'expression du gène LSR. Sont également comprises dans l'invention, les séquences d'acide nucléique de promoteur et/ou de régulateur de gène codant pour le récepteur selon l'invention, ou un de leurs variants alléliques, les séquences mutées, équivalentes ou homologues, ou un de leurs fragments.

L'invention concerne également des séquences nucléiques d'hybridation purifiées comprenant au moins 8 nucléotides, caractérisées en ce qu'elles peuvent s'hybrider spécifiquement avec une séquence nucléique selon l'invention.

De préférence, des fragments d'acide nucléiques ou des oligonucléotides, ayant pour séquences les séquences nucléotidiques selon l'invention pourront être utilisés comme sondes ou amorces.

L'invention comprend aussi des méthodes pour le criblage de banques d'ADNc et d'ADN génomique, pour le clonage des ADNc isolés, et/ou des gènes codant pour le récepteur selon l'invention, et pour leurs promoteurs et/ou régulateurs, caractérisées en ce qu'elles mettent en oeuvre une séquence nucléique selon l'invention.

Les séquences nucléiques caractérisées en ce qu'elles sont susceptibles d'être obtenues par une des méthodes précédentes selon l'invention, ou les séquences capables de s'hybrider avec lesdites séquences, font partie de l'invention.

### Vecteurs, cellules hôtes et animaux transgéniques

L'invention comprend également les vecteurs de clonage et/ou d'expression contenant une séquence d'acide nucléique selon l'invention.

Les vecteurs selon l'invention, caractérisés en ce qu'ils comportent les éléments permettant l'expression et/ou la sécrétion desdites séquences dans une cellule hôte, font également partie de l'invention.

L'invention comprend en outre les cellules hôtes, notamment les cellules eucaryotes et procaryotes, transformées par les vecteurs selon l'invention ainsi que les mammifères, excepté l'Homme, comprenant une desdites cellules transformées selon l'invention.

Parmi les mammifères selon l'invention, on préférera des animaux tels que les souris, les rats ou les lapins, exprimant un polypeptide selon l'invention, le phénotype correspondant au récepteur LSR normal ou variant, notamment muté d'origine humaine.

Ces cellules et animaux sont utilisables dans une méthode de production d'un polypeptide recombinant selon l'invention et peuvent également servir à titre de modèle d'analyse et de criblage.

L'invention concerne également l'utilisation de cellule, de mammifère ou de polypeptide selon l'invention, pour l'étude de l'expression et de l'activité du récepteur selon l'invention, et des interactions directes ou indirectes entre ledit récepteur et des composés chimiques ou biochimiques pouvant être impliqués dans l'activité dudit récepteur.

L'invention concerne également l'utilisation de cellule, de mammifère ou de polypeptide selon l'invention pour le criblage de composé chimique ou biochimique pouvant interagir directement ou indirectement avec le récepteur selon l'invention, et/ou capable de moduler l'expression ou l'activité dudit récepteur.

### Production de polypeptides issus du récepteur LSR

L'invention concerne également la synthèse de polypeptides synthétiques ou recombinants de l'invention, notamment par synthèse chimique ou par l'utilisation d'une séquence d'acide nucléique selon l'invention.

Les polypeptides obtenus par synthèse chimique et pouvant comporter des acides aminés non naturels correspondant auxdits polypeptides recombinants, sont également compris dans l'invention.

La méthode de production d'un polypeptide de l'invention sous forme recombinante est elle-même comprise dans la présente invention, et se caractérise en ce que l'on cultive les cellules transformées dans des conditions permettant l'expression d'un polypeptide recombinant de séquence polypeptidique selon l'invention, et que l'on récupère ledit polypeptide recombinant.

Les polypeptides recombinants, caractérisés en ce qu'ils sont susceptibles d'être obtenus par ladite méthode de production, font également partie de l'invention.

### Anticorps

Les anticorps mono ou polyclonaux ou leurs fragments, anticorps chimériques ou immunoconjugués, caractérisés en ce qu'ils sont capables de reconnaître spécifiquement un polypeptide ou un récepteur selon l'invention, font partie de l'invention.

On notera notamment l'intérêt d'anticorps reconnaissant de façon spécifique certains polypeptides, variants, ou fragments, notamment biologiquement actifs, selon l'invention.

L'invention concerne également des méthodes pour la détection et/ou la purification d'un polypeptide selon l'invention, caractérisées en ce qu'elles mettent en oeuvre un anticorps selon l'invention.

L'invention comprend en outre des polypeptides purifiés, caractérisés en ce qu'ils sont obtenus par une méthode selon l'invention.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Plus généralement, les anticorps de l'invention peuvent être avantageusement mis en oeuvre dans toute situation où l'expression, normale ou anormale, d'un polypeptide de récepteur LSR, normal ou muté, doit être observée.

### Détection de variabilité allélique et diagnostic

Font également partie de l'invention, les méthodes de détermination d'une variabilité allélique, d'une mutation, d'une délétion, d'une perte d'hétérozygotie ou d'une anomalie génétique, caractérisées en ce qu'elles mettent en oeuvre une séquence d'acide nucléique ou un anticorps selon l'invention.

Ces méthodes visent par exemple les méthodes de diagnostic de prédisposition à l'obésité, aux risques associés, ou aux pathologies associées à des anomalies du métabolisme des cytokines, en déterminant à partir d'un prélèvement biologique du patient la présence de mutations dans au moins une des séquences décrites précédemment. Les séquences d'acides nucléiques analysées pourront aussi bien être de l'ADN génomique, de l'ADNc, ou de l'ARNm.

Des acides nucléiques ou anticorps basés sur la présente invention pourront également être utilisés pour permettre un diagnostic positif et différentiel chez un malade pris isolément, ou un diagnostic pré-symptomatique chez un sujet à risque, notamment avec antécédent familial.

En outre, la mise en évidence d'une mutation spécifique peut permettre un diagnostic évolutif, notamment quant à l'intensité de la pathologie ou à l'époque probable de son apparition.

### Criblage de composés d'intérêt

Sont également comprises dans l'invention, les méthodes de sélection de composés chimiques ou biochimiques capables d'interagir directement ou indirectement avec le récepteur ou les séquences polypeptidiques ou nucléotidiques selon l'invention, et/ou permettant de moduler l'expression ou l'activité du récepteur LSR.

L'invention concerne notamment une méthode de sélection de composés chimiques ou biochimiques capables d'interagir avec une séquence d'acides nucléiques comprise dans un gène codant pour un récepteur LSR, ladite méthode étant caractérisée en ce qu'elle comprend la mise en contact d'une cellule hôte exprimant un récepteur LSR ou un fragment dudit récepteur avec un composé candidat susceptible de modifier l'expression ou la régulation de l'expression de ladite séquence nucléique, et, la détection directe ou indirecte d'une modification de l'expression ou de l'activité du récepteur LSR.

L'invention concerne aussi une méthode de sélection de composés chimiques ou biochimiques capables d'interagir avec le récepteur LSR, ladite méthode étant caractérisée en ce qu'elle comprend la mise en contact d'un récepteur LSR ou d'un fragment dudit récepteur, ou d'une cellule hôte exprimant un récepteur LSR ou un fragment dudit récepteur, avec un composé candidat susceptible de modifier l'activité LSR, et, la détection directe ou indirecte d'une modification de l'activité du récepteur LSR ou de la formation d'un complexe entre le composé candidat et ledit récepteur LSR ou ledit polypeptide.

L'invention comprend les composés capables d'interagir directement ou indirectement avec un récepteur LSR ainsi que les composés susceptibles d'interagir avec une ou plusieurs séquences nucléiques du récepteur LSR. Elle comprend également les composés chimiques ou biochimiques permettant de moduler l'expression ou l'activité du récepteur selon l'invention. Les composés, caractérisés en ce qu'ils ont été sélectionnés par une des méthodes selon la présente invention, font également partie de l'invention.

En particulier, parmi ces composés selon l'invention, on préfère les anticorps selon l'invention, les polypeptides selon l'invention, les acides nucléiques, oligonucléotides et vecteurs selon l'invention, ou une leptine ou un de ses composés dérivés, de préférence un de ses variants protéiques, ou des leptines modifiées chimiquement ou obtenues par recombinaison génétique, ou la protéine gC1qR ou un de ses analogues, ou un de leurs fragments.

L'invention comprend enfin des composés capables de moduler l'expression ou l'activité du récepteur selon l'invention, à titre de médicament pour la prévention de pathologies et/ou de pathogénies telles que l'obésité et l'anorexie, les hyperlipidémies, l'athérosclérose, le diabète, l'hypertension, et plus généralement les diverses pathologies associées à des anomalies du métabolisme des cytokines.

### DESCRIPTION DETAILLEE

### Le récepteur LSR

L'invention concerne un récepteur LSR (« Lipolysis Stimulated Receptor ») purifié, de préférence hépatique, constitué d'au moins une sous-unité α ou α' et d'au moins une sous-unité β. La sous-unité α a un poids moléculaire d'environ 66 kDa chez le rat et chez la souris et d'environ 72 kDa chez l'homme. La sous-unité α' a un poids moléculaire d'environ 64 kDa chez le rat et chez la souris et d'environ 70 kDa chez l'homme. La sous-unité β a un poids moléculaire d'environ 58 kDa chez le rat et chez la souris et d'environ 64 kDa chez l'homme.

Les inventeurs ont formulé l'hypothèse selon laquelle la forme la plus abondante, et vraisemblablement la plus active du récepteur LSR est celle dans laquelle on retrouve une sous-unité α ou α' et trois sous-unités β. Il semble cependant possible que les sous-unités α et α' d'une part et β d'autre part aient des fonctions biologiques distinctes et que ces fonctions puissent être exécutées dans une cellule indépendamment de leur assemblage sous forme de récepteur.

Les inventeurs ont également observé qu'un complexe peut se former entre le récepteur LSR et le récepteur gC1qR d'un poids moléculaire d'environ 33 kDa, ou une protéine homologue. Il semble que le récepteur gC1qR soit associé de façon temporaire au récepteur LSR et que la présence de la protéine C1q ou de protéines homologues permette non seulement de dissocier gC1qR du récepteur LSR mais également d'activer le récepteur LSR, y compris en l'absence d'acides gras.

### Activité du récepteur LSR et applications

La présente invention est donc relative à un récepteur, en particulier de cellule hépatique, caractérisé en ce qu'il est capable, en présence d'acides gras libres, de fixer des lipoprotéines, et en absence d'acides gras libres, de fixer une cytokine, de préférence la leptine, les lipoprotéines et la cytokine fixées étant incorporées puis dégradées par la cellule, ledit récepteur pouvant en outre fixer la protéine gC1qR ou une de ses protéines analogues.

### Clairance des lipoprotéines

Le récepteur LSR représente la voie principale de l'élimination des lipoprotéines d'origine intestinale et des particules riches en triglycérides, notamment les VLDL et les chylomicrons. Le récepteur LSR peut aussi servir de voie pour l'élimination des LDL, particules riches en cholestérol, qui sont pour la plus grande part épurées par la voie du LDL récepteur, mais dont environ 30% sont éliminées au niveau hépatique par des voies différentes du LDL récepteur.

Les inventeurs ont en fait démontré que le récepteur LSR est capable de fixer les lipoprotéines, notamment les lipoprotéines riches en triglycérides, puis de les internaliser et de les dégrader. Cette activité de clairance des lipoprotéines par le récepteur nécessite la présence d'acides gras libres, par exemple l'oléate, et est inhibée en présence d'anticorps dirigés contre le LSR ou contre des peptides issus du LSR.

### Clairance des cytokines

Les inventeurs ont également démontré, qu'en absence d'acide gras libre, par exemple l'oléate, le récepteur LSR est capable de fixer des cytokines, de préférence la leptine. La fonction de clairance de la leptine n'est cependant possible que si le récepteur n'a pas fixé d'acides gras produits par la lipase hépatique ou par la lipase hormono-sensible du tissus adipeux. Une fois les cytokines fixées, le récepteur LSR les internalise et les dégrade. Cette activité de dégradation des cytokines, de préférence de la leptine, est inhibée par des anticorps dirigés contre le LSR ou contre des peptides issus du LSR.

Les inventeurs ont montré que c'est la sous-unité α du récepteur LSR qui est plus particulièrement impliquée dans la fixation des cytokines, et préférentiellement de la leptine.

De plus, les inventeurs ont montré à l'aide de souris que, *in vivo*, les récepteurs LSR réalisent la captation hépatique de cytokines, de préférence de la leptine.

Les taux élevés de leptine chez les sujets humains obèses peuvent s'expliquer par plusieurs mécanismes moléculaires qui sont susceptibles de diminuer la clairance hépatique de la leptine, dont notamment :
a) une altération d'un (des) gène(s) du LSR, et/ou de leur(s) promoteur(s) ;
b) une facilitation, par des modifications post-transcriptionnelles, du remaniement allostérique permettant le passage de la conformation cytokine-compétente à la conformation de récepteur aux lipoprotéines ;
c) un déficit de transport des vésicules contenant le LSR de, ou vers, la membrane plasmique (cette fonction dépend de l'intégrité du cytosquelette) ;
d) une augmentation de la dégradation du LSR ;
e) une augmentation de la ration calorique lipidique qui, en détournant le récepteur vers la clairance des lipoprotéines, réduit en partie sa capacité de dégrader la leptine.

### Contrôle de l'activité LSR par les cytokines

Enfin, les inventeurs ont démontré que des cytokines, de préférence la leptine, modulent l'activité du récepteur LSR en présence d'acide gras libre. Plus particulièrement, les cytokines augmentent l'activité de clairance des lipoprotéines du récepteur LSR et plus précisément, la fixation, l'internalisation et la dégradation des VLDL et des LDL. Cette augmentation de l'activité LSR pourrait être le résultat de l'augmentation du nombre apparent de récepteur LSR à la surface des cellules suite à une augmentation de la synthèse protéique et à une mobilisation des vésicules d'endocytose. En outre, les inventeurs ont montré à l'aide de souris, que, *in vivo*, les cytokines, de préférence la leptine, sont capables de diminuer la réponse lipémique post-prandiale.

La leptine et vraisemblablement d'autres cytokines sont donc régulateurs de l'activité du LSR. Un syndrome de résistance à la leptine, ou à d'autres cytokines, peut entraîner une hypertriglycéridémie, soit permanente, soit limitée à la phase post-prandiale.

### Traitements de l'obésité

Le rôle joué par le LSR dans la clairance de la leptine permet de formuler un modèle physiopathologique qui impose une révision des stratégies mises en oeuvre pour traiter l'obésité. Il est en effet essentiel de réduire les concentrations de leptine chez les sujets humains obèses, afin de restaurer les fluctuations physiologiques de cette hormone.

C'est pourquoi il est possible d'envisager d'utiliser des composés pour le traitement de l'obésité permettant la modulation du nombre de récepteurs LSR, de leur vitesse de recyclage, ou du changement de leur conformation, et/ou permettant notamment:
1. de contrôler la leptinémie, et donc les sensations de satiété et de faim ;
2. de restaurer des concentrations de leptine normales et de permettre la régulation normale du comportement alimentaire par la perception normale des sensations de faim et de satiété ;
3. de contrôler la triglycéridémie ;
4. de réguler les concentrations plasmatiques de résidus de chylomicrons, particules très athérogènes.

Le rôle joué par le récepteur LSR dans la clairance hépatique des lipoprotéines d'origine intestinale permet d'envisager d'utiliser des composés capables de moduler l'expression et/ou l'acitivité du LSR afin de moduler la répartition des lipides d'origine alimentaire entre les tissus périphériques, notamment les tissus adipeux, et le foie. Un traitement de l'obésité consistera à favoriser la dégradation hépatique des lipoprotéines, et par là-même de diminuer leur stockage dans le tissu adipeux, et de réguler leurs concentrations plasmatiques. Ce dernier effet permet d'envisager l'utilisation de tels composés afin de réduire les risques associés à l'obésité, notamment les risques athérogènes.

### Traitements de l'anorexie et de la cachexie

Il est possible d'envisager d'utiliser des méthodes de régulation des activités du LSR pour mettre en place des traitements permettant de vaincre le cercle vicieux qui caractérise l'anorexie nerveuse. En réduisant le nombre de récepteurs, on devrait favoriser la prise de poids chez les sujets anorexiques ou dénutris.

Dans ces conditions, il est intéressant d'inhiber sélectivement la clairance de la leptine en utilisant des peptides synthétiques ou molécules pharmacologiques qui, soit diminuent la synthèse du LSR, soit bloquent sa capacité à fixer la leptine et/ou les lipoprotéines, soit encore, augmentent le catabolisme du récepteur.

### Traitements des anomalies du métabolisme des cytokines

L'analyse de la structure primaire de la sous-unité α du LSR, telle que décrite ci-dessous, met en évidence un site homologue aux sites de fixation des cytokines présents sur leurs récepteurs, ainsi que deux signaux de routage qui permettent l'endocytose et la dégradation rapide des ligands dans les lysozomes. Cette observation est originale dans le sens où les récepteurs aux cytokines ne permettent pas l'internalisation et la dégradation des ligands. Ces récepteurs ont été caractérisés sur la base de leur propriété de signalisation intracellulaire.

Ainsi, outre qu'il présente la propriété de permettre la dégradation protéolytique des lipoprotéines et de la leptine, il est hautement probable que le récepteur LSR réalise également la dégradation d'autres cytokines. Cette fonction pourra être étudiée grâce aux anticorps anti-LSR et à des cellules CHO transfectées exprimant la sous-unité α du LSR telles que celles décrites à l'exemple 4. L'implication du LSR dans la clairance des cytokines est essentielle car ces moléules jouent un rôle important dans la régulation du métabolisme des lipides, du métabolisme du glucose, et dans la régulation de la prise alimentaire et de la prise de poids.

Les mécanismes moléculaires par lesquels les cytokines modulent les fonctions physiologiques impliquées dans l'obésité et ses complications sont nombreux et complexes. On retiendra cependant le fait que les anomalies du métabolisme des cytokines sont associées à l'hypertriglycéridémie qui accompagne fréquemment les infections virales, bactériennes ou à protozoaires. Par ailleurs, les cytokines et plus particulièrement le Tumor Necrosis Factor (TNF), induisent une hypertriglycéridémie transitoire semblable à celle observée dans certaines formes de diabètes associés à l'obésité.

La réduction du nombre de récepteurs LSR exprimés au niveau du foie des souris obèses pourrait expliquer un déficit de l'élimination de certaines cytokines, ce déficit entraînant des perturbations métaboliques telles que celles retrouvées dans l'obésité. L'utilisation de cellules hépatiques en culture, et des différents modèles d'animaux obèses cités ci-dessous, permettra de déterminer, parmi toutes les cytokines et plus particulièrement celles qui induisent une perte de poids (IL-6, LIF, OSM, CNTF, IL-11, IL-12α, ainsi que TNFα et TNFβ), celles qui modulent l'expression et/ou l'activité du LSR. La détermination de telles cytokines peut par exemple être conduite en utilisant des méthodes telles que celles présentées aux Exemples 4 à 6.

Enfin, l'analyse de la structure primaire du LSR α met en évidence des sites potentiels de phosphorylation. Ceci ouvre la perspective d'une régulation de l'activité cellulaire par le récepteur LSR. Un exemple particulièrement important serait l'implication du LSR dans la régulation de la production de « Acute Phase Proteins » sous l'impulsion de différents stimuli, dont les cytokines.

L'implication du LSR dans la clairance et la dégradation des cytokines pourrait en outre ne pas se limiter au foie. En effet, s'il est démontré que l'expression du LSR est de façon prédominante hépatique, il est également certain que l'expression de ce récepteur n'est pas limitée à cet organe. Une analyse préliminaire en Northern blot sur différents tissus humains a pu mettre en évidence, outre les produits hépatiques, des produits d'expression au niveau du rein et du testicule. Une analyse plus approfondie permettra de mettre en évidence les différents tissus exprimant le LSR chez l'homme. Dans cette perspective, le LSR pourrait intervenir dans la dégradation de cytokines non seulement au niveau hépatique, mais aussi au niveau des tissus périphériques. Un déficit de cette activité pourrait être impliqué dans la pathogénie de maladies auto-immunes, de scléroses an plaques, de l'arthrite rhumatoïde. Une accumulation de cytokines est fréquemment retrouvée dans la pathogénie de ces maladies.

### Les séquences polypeptidiques du récepteur LSR

L'invention concerne des polypeptides, caractérisés en ce qu'ils sont un constituant d'un récepteur LSR selon l'invention. L'invention concerne plus particulièrement les polypeptides caractérisés en ce qu'ils constituent les sous-unités α, α' ou β du récepteur LSR.

L'invention concerne plus particulièrement un polypeptide purifié, isolé ou recombinant, comprenant une séquence d'au moins 5, de préférence d'au moins 10 à 15 acides aminés consécutifs d'un récepteur LSR, ainsi que les homologues, équivalents ou variants dudit polypeptide, ou un de leurs fragments. De préférence, la séquence d'au moins 10 à 15 acides aminés du récepteur LSR est un fragment biologiquement actif d'un récepteur LSR.

De façon préférée, l'invention concerne des polypeptides purifiés, isolés ou recombinants, comprenant une séquence d'au moins 10 à 15 acides aminés d'un récepteur LSR de rat, d'un récepteur LSR de souris ou d'un récepteur LSR humain.

Dans une première réalisation préférée de l'invention, le polypeptide est caractérisé en ce qu'il comprend une séquence d'au moins 10 à 15 acides aminés consécutifs d'une séquence choisie parmi le groupe comprenant les séquences de SEQ ID 2, SEQ ID 4 et SEQ ID 6 ainsi que les variants, équivalents ou homologues de ce polypeptide, ou un de leurs fragments. De préférence, le polypeptide est un homologue ou un fragment biologiquement actif d'une des séquences citées précédemment.

Dans une deuxième réalisation préférée de l'invention, le polypeptide est caractérisé en ce qu'il comprend une séquence d'au moins 10 à 15 acides aminés consécutifs d'une séquence choisie parmi le groupe comprenant les séquences de SEQ ID 16, SEQ ID 17 et SEQ ID 18 ainsi que les variants, équivalents ou homologues de ce polypeptide, ou un de leurs fragments. De préférence, le polypeptide est un homologue ou un fragment biologiquement actif d'une des séquences citées précédemment.

Dans une troisième réalisation préférée de l'invention, le polypeptide est caractérisé en ce qu'il comprend une séquence d'au moins 10 à 15 acides aminés consécutifs d'une séquence choisie parmi le groupe comprenant les séquences de SEQ ID 8, SEQ ID 10 et SEQ ID 12 ainsi que les variants, équivalents ou homologues de ce polypeptide, ou un de leurs fragments. De préférence, le polypeptide est un homologue ou un fragment biologiquement actif d'un des séquences citées précédemment.

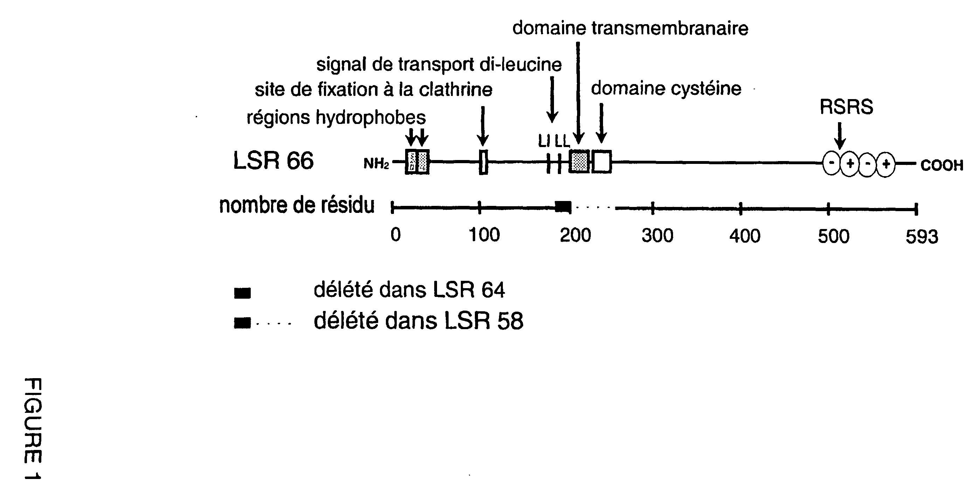

Parmi les polypeptides préférés de l'invention, on notera particulièrement les polypeptides de séquence humaine SEQ ID 8, SEQ ID 10, ou SEQ ID 12, ainsi que ceux de séquence de rat SEQ ID 2, SEQ ID 4, ou SEQ ID 6, ou ceux de séquence de souris SEQ ID 16, SEQ ID 17, SEQ ID 18. Les fragments correspondant aux domaines représentés dans les figures 1 à 6, dont les positions sur les séquences correspondant aux SEQ ID 2, 8 ou 16 sont indiquées dans les tableaux 1, 3 et 4.

Enfin, l'invention concerne également les polypeptides de SEQ ID 29 et SEQ ID 30.

La présente invention concerne également les polypeptides comprenant les polypeptides décrits ci-dessus, ainsi que leurs polypeptides homologues, équivalents ou variants, ainsi que les fragments, de préférence biologiquement actifs, desdits polypeptides.

Parmi les polypeptides selon l'invention, on préfère également les polypeptides comprenant ou constitués par une séquence d'acides aminés choisie parmi les séquences d'acides aminés telles que définies précédemment, caractérisés en ce qu'ils sont un constituant du récepteur selon l'invention.

### Analyse des séquences polypeptidiques des sous-unités α, α' et β du récepteur LSR

L'analyse systématique des produits des 3 ADNc de rat décrits dans la présente demande est représentée schématiquement Figure 1. La sous-unité α du récepteur LSR de rat, protéine codée par l'ADNc de plus longue taille (LSR-Rn-2097), présente les caractéristiques suivantes.

Des sites potentiels de glycosylation sont trouvés en positions 12-14 et 577-579. Un site potentiel d'attachement des glycosaminoglycanes est trouvé en position 14-17.

Plusieurs sites de phosphorylation sont localisés au niveau de l'extrémité NH₂ terminale (en positions 193-196, 597-600, 169-171, 172-174, 401-403, 424-426, 464-466, 467-469, 185-188, 222-225, 436-439, 396-399, 504-507, 530-533, 624-627, 608-615), suggérant que celle-ci est orientée vers la région intracellulaire.

Par ailleurs, la protéine présente, du côté NH₂ terminal, une séquence d'acides aminés hydrophobes, séparée en deux parties par 2 acides aminés induisant une structure en épingle à cheveux dont les deux bras seraient constitués d'acides aminés hydrophobes. Il est raisonnable de supposer que cette région représente le site de fixation des acides gras du LSR. La structure en doigt de gant ainsi réalisée peut accommoder une chaîne hydrocarbonée aliphatique. Les deux acides aminés, sont plus précisément dans le cas du LSR de rat, deux Prolines, situées en positions 31 et 33 de la séquence polypeptidique de la sous-unité α.

Du côté NH₂ terminal encore, se trouve une séquence consensus de fixation à la clathrine, protéine qui tapisse la face interne des « coated pits » (Chen et al., 1990). Ces régions spécifiques de la membrane plasmique permettent l'endocytose rapide des protéines membranaires. Une telle séquence consensus est retrouvée au niveau de la LRP-α₂-macroglobuline récepteur, de la CRAM et du LDL-récepteur (Herz et al., 1988 ; Lee et al., 1990 ; Goldstein et al., 1995). Une mutation à ce niveau a comme conséquence un retard important d'internalisation des LDL, et induit une hypercholestérolémie familiale (Davis et al., 1986).

Le récepteur possède ensuite une séquence d'acides aminés hydrophobes qui constitue un domaine transmembranaire potentiel. La longueur de ce segment ne permet qu'un seul passage à travers la bicouche phospholipidique (Brendel et al., 1992).

Entre ce signal de fixation à la clathrine et la chaîne hydrophobe correspondant au segment transmembranaire unique, se trouvent 2 motifs LI et LL (Letourneur et al., 1992). Ces deux motifs sont retrouvés au niveau des protéines suivantes : glut 4 transporteur de glucose (Verhey et al., 1994); la chaîne invariante et le complexe d'histocompatibilité de classe II (Zhong et al., 1997 ; Parra-Lopez et al., 1997). Ces signaux contrôlent l'endocytose et l'adressage intracellulaire des protéines dans le système membranaire périphérique.

Du côté C-terminal, on trouve ensuite une région riche en cystéine qui présente une homologie avec les récepteurs des cytokines et plus particulièrement : les récepteurs aux TNF 1 et 2 (Tumor Necrosis Factor 1 et 2) ; le récepteur au NGF de faible affinité (Nerve Growth factor) ; le récepteur soluble au TNF du virus de fibrome de Shope ; CD40,CD27 et CD30, récepteurs pour les cytokines CD40L, CD27L et CD30L ; la protéine de cellule T 4-1 BB, récepteur pour la cytokine putative 4-1 BBL, l'antigène FAS (APO 1), récepteur pour la protéine FASL impliquée dans l'apoptose, l'antigène 0X40 de cellule T, récepteur pour la cytokine 0X40L, et la protéine A53 du virus de la vaccine (Cytokines and their receptors, 1996 ; Banner et al., 1993).

Outre ce segment cystéine riche, on trouve une région d'acides aminés chargés alternativement + et - (Brendel et al., 1992). Cette région fournit un site de fixation potentiel pour les ligands apoprotéiques Apo B et Apo E.

Cette région contient en outre un motif RSRS retrouvé au niveau de la lamine (Simos et al., 1994) et du SF2' (Krainer et al., 1991).

La forme LSR α' codée par l'ADNc LSR-Rn-2040 possède tous les domaines décrits précédemment d'après la séquence de LSR α codée par l'ADNc LSR-Rn-2097, à l'exception de l'élément LI/LL, dont le doublet Leucine se trouve éliminé par épissage alternatif. Bien que possédant des séquences très proches, les sous-unités α codée par LSR-Rn-2097 et α' codée par LSR-Rn-2040 pourraient donc différer quant à leur vitesse de recyclage et leur adressage. La forme β codée par LSR-Rn-1893 ne possède pas de domaine transmembranaire, ni de région riche en cystéines et homologue des récepteurs à cytokines. Toutefois, elle possède au niveau NH₂-terminal la région hydrophobe séparée par une répétition de prolines, la région riche en acides aminés chargés, et le motif RSRS. Ce constituant est vraisemblablement positionné entièrement à l'extérieur de la cellule où il est fixé par l'intermédiaire de ponts disulfures, soit au produit de LSR-Rn-2040, soit à celui de LSR-Rn-2097.

Le tableau 1 ci-dessous répertorie les différents domaines ou motifs décrits ci-dessus, indique leur appartenance ou non à chacune des sous-unités du récepteur LSR, ainsi que les positions de début et fin desdits domaines ou motifs, ou de régions portant lesdits domaines ou motifs, telles qu'indiquées dans la séquence de SEQ ID 2.

**Tableau 1**

| **Domaine ou motif** | | **Position sur SEQ ID 2** | | **Présence sur :** | | |
|---|---|---|---|---|---|---|
| | | **Début** | **Fin** | α | α' | β |
| Potentiel site de fixation des acides gras | | 23 | 41 | X | X | X |
| Potentiel site de fixation à la clathrine | | 104 | 107 | X | X | X |
| Signal de transport : | LI | 183 | 184 | X | X | X |
| | LL | 195 | 196 | X | | |
| Domaine transmembranaire | | 204 | 213 | X | X | |
| Potentiel site récepteur aux cytokines | | 214 | 249 | X | X | |
| Motif RSRS | | 470 | 473 | X | X | X |
| Potentiel site de fixation des ligands lipoprotéiques | | 544 | 557 | X | X | X |

### Comparaison des séquences polypeptidiques des récepteurs LSR chez le rat, la souris et l'homme

Les longueurs des séquences polypeptidiques, ainsi que les SEQ ID de leurs séquences respectives dans le listing inclus, des trois types de sous-unités des récepteurs LSR selon l'invention, chez le rat, la souris et l'homme, sont indiquées dans le tableau 2a ci-dessous.

**Tableau 2a**

| **Polypeptide** | **Rat** | **Souris** | **Homme** |
|---|---|---|---|
| Sous-unité α | 593 aa (SEO ID 2) | 594 aa (SEQ ID 16) | 649 aa (SEQ ID 8) |
| Sous-unité α' | 574 aa (SEQ ID 4) | 575 aa (SEQ ID 17) | 630 aa (SEQ ID 10) |
| Sous-unité β | 525 aa (SEQ ID 6) | 526 aa (SEQ ID 18) | 581 aa (SEQ ID 12) |

Ces séquences polypeptidiques ont été obtenues à partir de chacune des trois séquences d'ADNc correspondantes, chez le rat, la souris et l'homme, qui seront décrites de façon détaillée plus loin. La nomenclature utilisée pour désigner ces séquences d'ADNc, qui reflète leur longueur en nucléotides, ainsi que les SEQ ID de leurs séquences respectives dans le listing inclus, sont présentées dans le tableau 2b ci-dessous.

**Tableau 2b**

| **ADNc** | **Rat** | **Souris** | **Homme** |
|---|---|---|---|
| Sous-unité α | LSR-Rn-2097 (SEQ ID 1) | LSR-Mm-1886 (SEQ ID 13) | LSR-Hs-2062 (SEQ ID 7) |
| Sous unité α' | LSR-Rn-2040 (SEQ ID 3) | LSR-Mm-1829 (SEQ ID 14) | LSR-Hs-2005 (SEQ ID 9) |
| Sous-unité β | LSR-Rn-1893 (SEQ ID 5) | LSR-Mm-1682 (SEQ ID 15) | LSR-Hs-1858 (SEQ ID 11) |

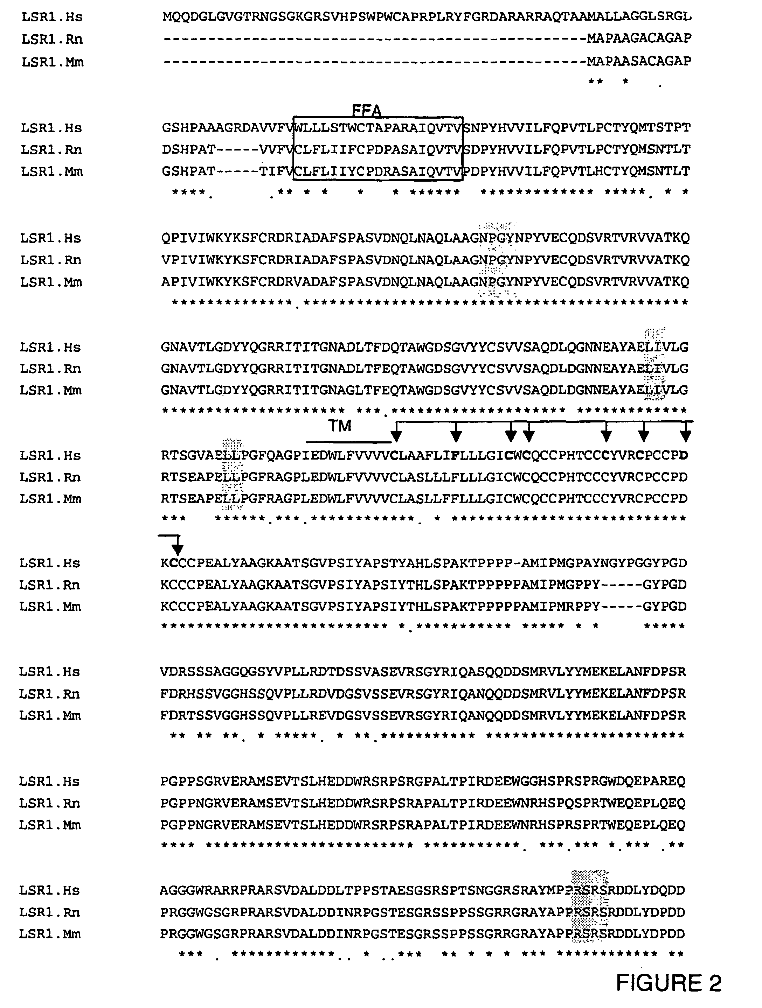

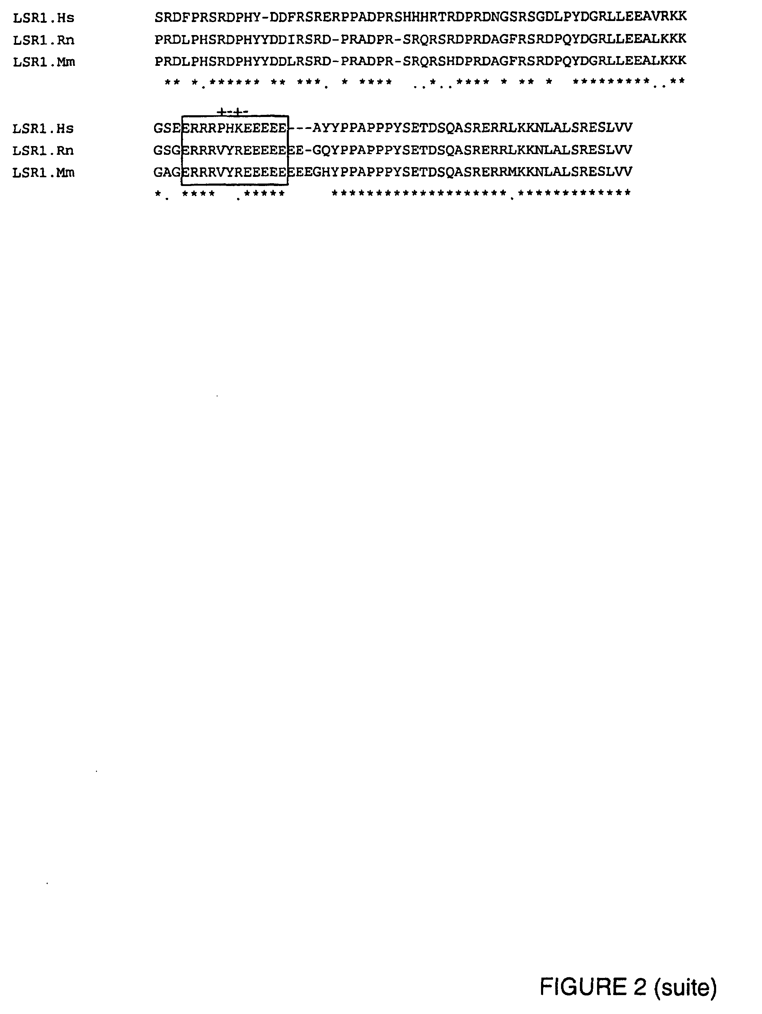

La séquence protéique, correspondant à la sous-unité α du récepteur LSR, déduite de la séquence de LSR-Hs-2062 a une longueur de 649 acides aminés. Elle est alignée avec les séquences protéiques déduites de LSR-Mm-1886, de longueur 594 acides aminés et de LSR-Rn-2097, de longueur 593 acides aminés (figure 2). La conservation des séquences protéiques est très grande (respectivement 80,2 % et 82,2 % d'identité pour 591 et 590 acides aminés chevauchants). Les domaines fonctionnels mis en évidence dans la séquence protéique du LSR α de rat se retrouvent dans la séquence LSR α humaine ainsi que dans celle de LSR α murin (figure 2).

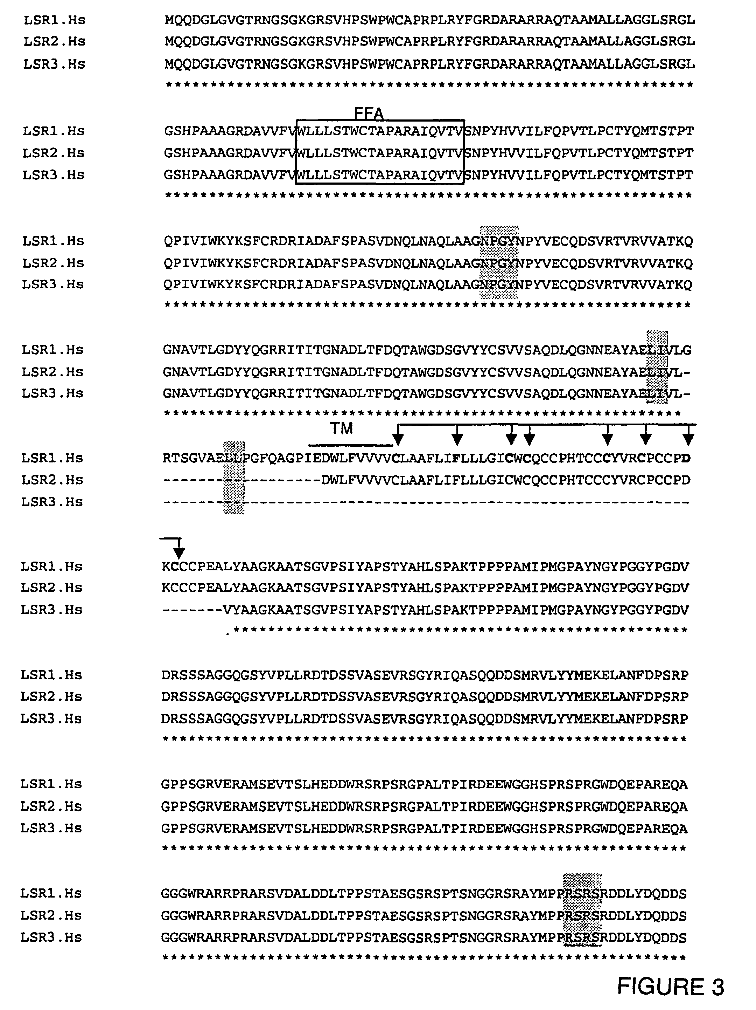

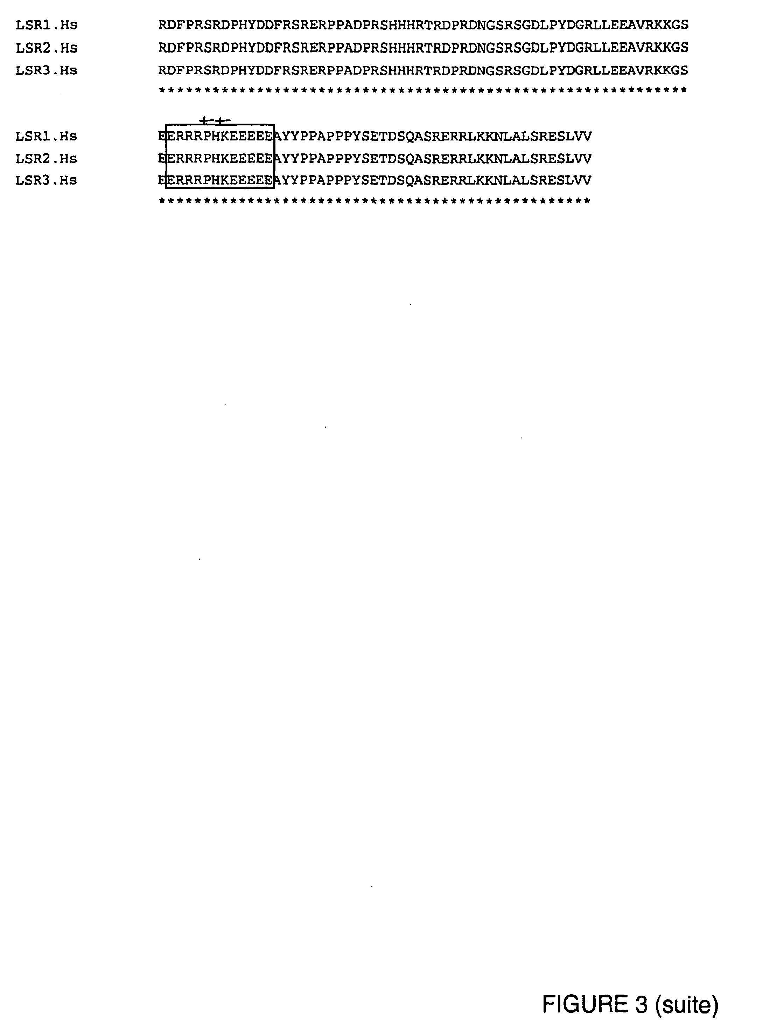

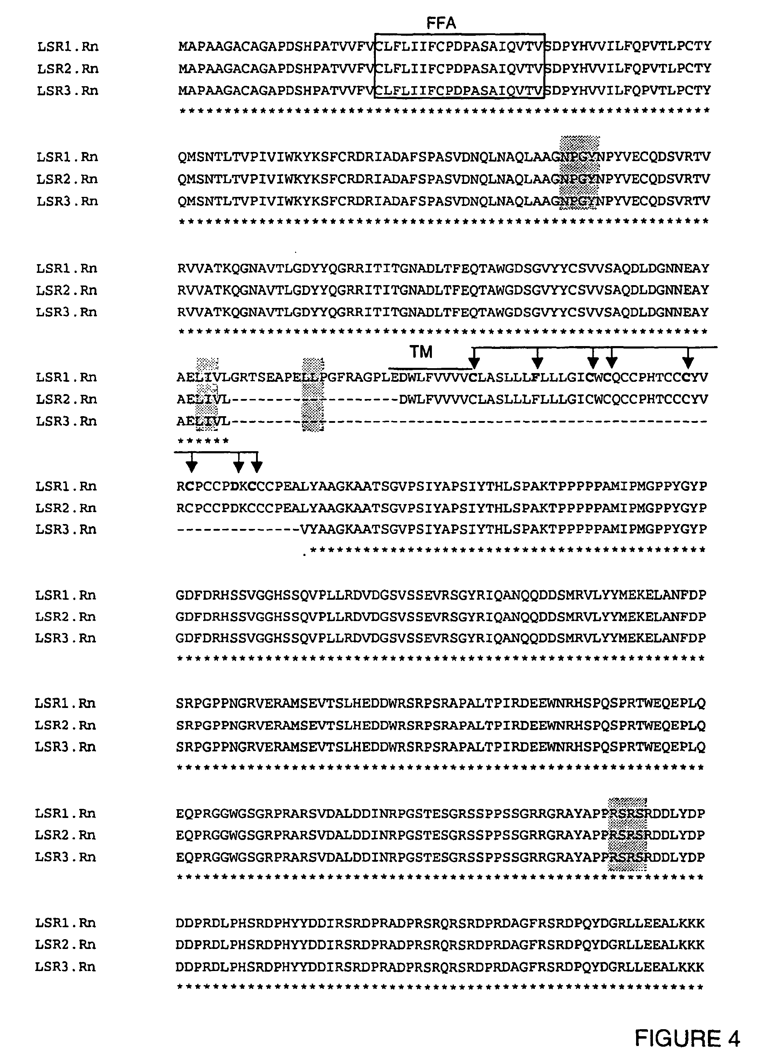

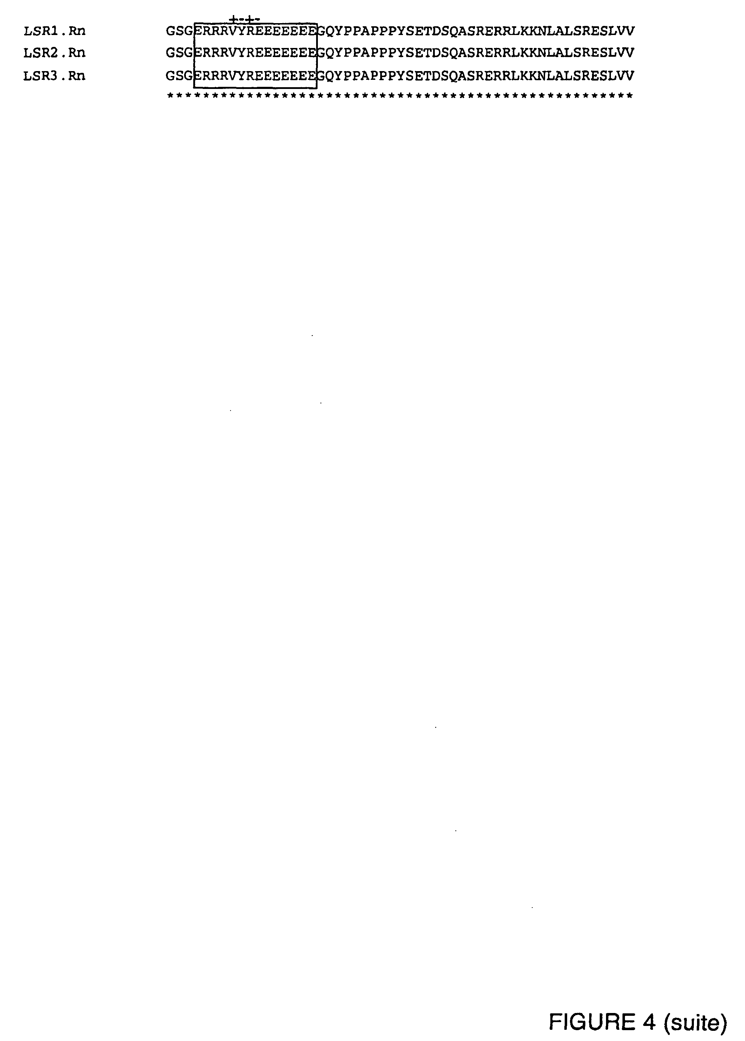

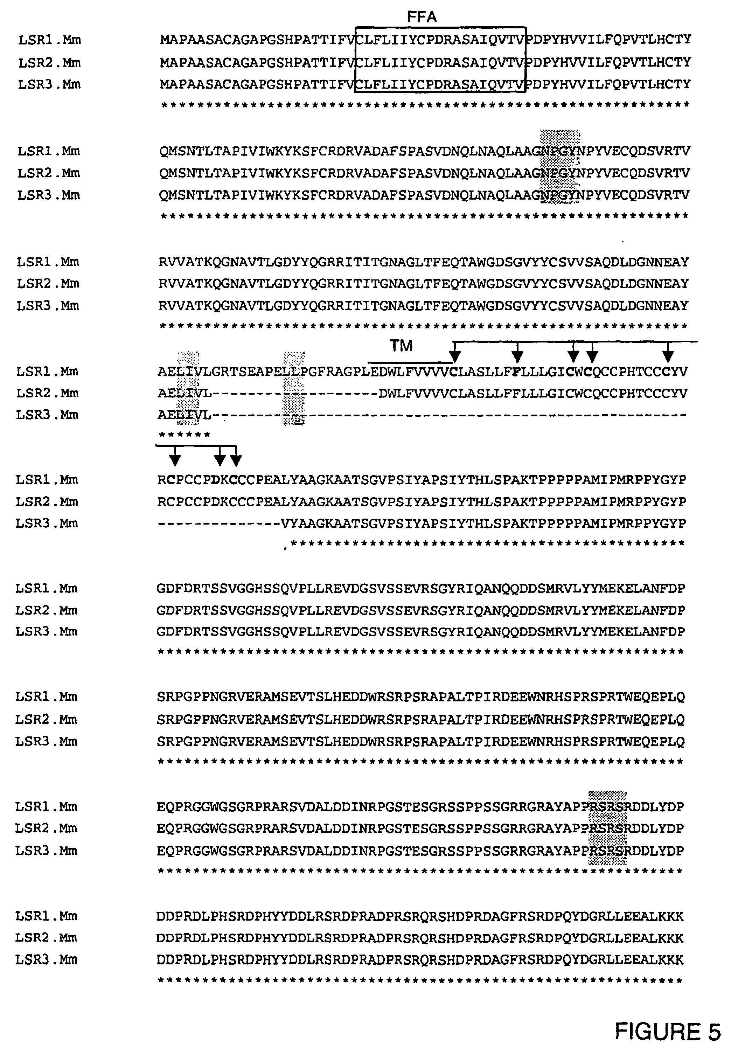

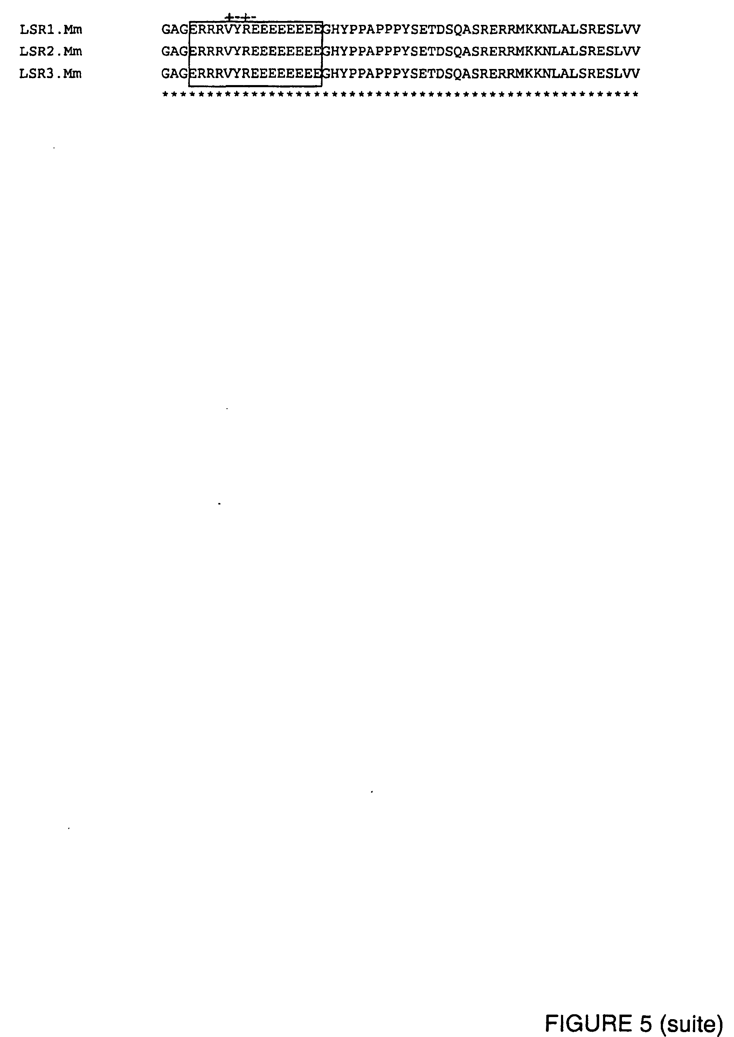

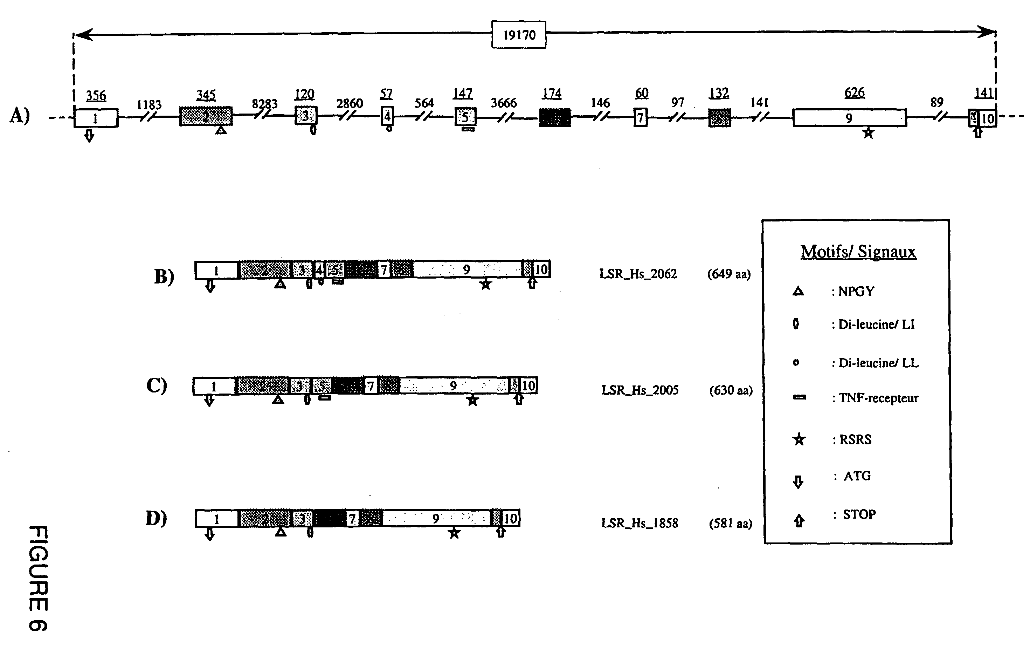

Les protéines humaines correspondant aux formes LSR-Hs-2005 (α') et LSR-Hs-1858 (β) ont une taille prédite de 630 et 581 acides aminés respectivement. Les protéines de rat correspondant aux formes LSR-Rn-2040 (α') et LSR-Rn-1893 (β) ont une taille prédite de 574 et 525 acides aminés respectivement. Les protéines de souris correspondant aux formes LSR-Mm-1829 (α') et LSR-Mm-1682 (β) ont une taille prédite de 575 et 526 acides aminés respectivement. L'alignement des trois formes humaines (figure 3), des trois formes décrites chez le rat (figure 4) et des trois formes décrites chez la souris (figure 5) montre que chez les trois espèces toutes les formes protéiques conservent le signal NPGY de fixation à la clathrine et le motif RSRS. Les formes longues (α) humaines (produit de LSR-Hs-2062), de rat (produit de LSR-Rn-2097) et de souris (produit de LSR(Mm-1886) présentent l'ensemble des caractéristiques fonctionnelles du LSR. Les trois formes courtes (β) (produits respectifs de LSR-Hs-1817, LSR-Rn-1893 et LSR-Mm-1682) perdent le domaine di-leucine d'adressage lysosomal, le domaine transmembranaire et la signature de récepteur à cytokine. On peut également remarquer que les trois formes intermédiaires (α') (produit de LSR-Hs-2005, de LSR-Rn-2040 et LSR-Mn-1829) perdent le domaine di-leucine, le domaine transmembranaire et le domaine correspondant à la signature du récepteur à cytokine étant conservés (figures 3, 4 et 5). La figure 6 représente enfin les protéines issues des trois formes d'ADNc mises en évidence chez l'homme, et les motifs que porte chacune d'elle en conséquence de l'épissage dont elle est issue.

Le tableau 3 ci-dessous répertorie les différents domaines ou motifs décrits ci-dessus, ainsi que les positions de début et fin desdits domaines ou motifs, ou de régions portant lesdits domaines ou motifs, telles qu'indiquées dans la séquence de SE4 ID 16 de souris.

**Tableau 3**

| **Domaine ou motif** | | **Position sur SEQ ID 16** | | **Présence sur :** | | |
|---|---|---|---|---|---|---|
| | | **Début** | **Fin** | α | α' | p |
| Potentiel site de fixation des acides gras | | 23 | 41 | X | X | X |
| Potentiel site de fixation à la clathrine | | 104 | 107 | X | X | X |
| Signal de transport : | LI | 183 | 184 | X | X | X |
| | LL | 195 | 196 | X | | |
| Domaine transmembranaire | | 204 | 213 | X | X | |
| Potentiel site récepteur aux cytokines | | 214 | 249 | X | X | |
| Motif RSRS | | 470 | 473 | X | X | X |
| Potentiel site de fixation des ligands lipoprotéiques | | 544 | 558 | X | X | X |

Le tableau 4 ci-dessous répertorie les différents domaines ou motifs décrits ci-dessus, ainsi que les positions de début et fin desdits domaines ou motifs, ou de régions portant lesdits domaines ou motifs, telles qu'indiquées dans la séquence de SEQ ID 8 humaine.

**Tableau 4**

| **Domaine ou motif** | | **Position sur SEQ ID 8** | | **Présence sur :** | | |
|---|---|---|---|---|---|---|
| | | **Début** | **Fin** | α | α' | β |
| Potentiel site de fixation des acides gras | | 76 | 94 | X | X | X |
| Potentiel site de fixation à la clathrine | | 157 | 160 | X | X | X |
| Signal de transport : | LI | 236 | 237 | X | X | X |
| | LL | 248 | 249 | X | | |
| Domaine transmembranaire | | 257 | 266 | X | X | |
| Potentiel site récepteur aux cytokines | | 267 | 302 | X | X | |
| Motif RSRS | | 527 | 530 | X | X | X |
| Potentiel site de fixation des ligands lipoprotéiques | | 601 | 613 | X | X | X |

En conclusion, la similarité de séquence et de structure de LSR décrite ci-dessus permet d'extrapoler à l'homme les observations réalisées chez le rat et/ou la souris.

Par polypeptide homologue, on entendra désigner les polypeptides présentant, par rapport au polypeptide naturel, certaines modifications comme en particulier une délétion, une troncation, un allongement, une fusion chimérique, et/ou une mutation, notamment ponctuelle. Parmi les polypeptides homologues, on préfère ceux dont la séquence d'acides aminés présente au moins 80 %, de préférence 90 %, d'identité avec les séquences d'acides aminés des polypeptides selon l'invention.

Par polypeptide équivalent, on entendra désigner un polypeptide ayant au moins une des activités du récepteur LSR, notamment l'activité de récepteur de lipoprotéines ou de chylomicrons, l'activité de récepteur de cytokine, notamment de la leptine, ou l'activité de récepteur de la protéine gC1q-R ou une de ses protéines analogues. Par polypeptide équivalent, on entendra désigner également tout polypeptide résultant d'épissage alternatif de la séquence nucléique génomique codant pour les polypeptides selon l'invention.

On entendra par polypeptide variant (ou variant protéique) l'ensemble des polypeptides mutés pouvant exister, en particulier chez l'être humain, et qui correspondent notamment à des troncatures, délétions et/ou additions de résidus d'acides aminés, substitutions ou mutations, notamment ponctuelles, ainsi que les polypeptides variants artificiels qui seront néanmoins appelés polypeptides variants. Dans le cas présent, les polypeptides variants seront notamment en partie associés à la survenue et au développement de l'obésité ou de l'anorexie. Ils pourront aussi être associés à la survenue et/ou au développement des risques ou complications associés à l'obésité, notamment au niveau cardio-vasculaire, et/ou de pathologies associées à des anomalies du métabolisme des cytokines.

Par fragment de polypeptide, on entend désigner un polypeptide ou peptide codé par une séquence nucléique comportant au minimum 15 nucléotides ou bases, de préférence 20 bases et 30 bases. Ces fragments pourront notamment comporter une mutation ponctuelle, par comparaison à la séquence de polypeptide normal, ou correspondre à des séquences d'acides aminés spécifiques de polypeptides variants, artificiels ou existant chez l'homme, tels que ceux liés à un polymorphisme lié en particulier à l'obésité ou aux pathologies citées précédemment.

Par fragment biologiquement actif, on entendra désigner en particulier un fragment de séquence d'acides aminés de polypeptide :
- présentant au moins une des activités du récepteur LSR, notamment l'activité de récepteur de lipoprotéines, ou de récepteur à cytokine, particulièrement à la leptine et/ou de signalisation cellulaire, et/ou
- capable d'être reconnu par un anticorps spécifique du récepteur selon l'invention, et/ou
- capable d'être reconnu par un composé pouvant, par exemple, en neutralisant la fixation d'un ligand spécifique dudit récepteur, moduler l'activité du récepteur LSR, et/ou
- capable de moduler l'adressage et/ou la localisation cellulaire du récepteur LSR, et/ou
- plus généralement, constituant un domaine ou motif biologiquement actif du récepteur LSR.
Parmi les fragments biologiquement actifs préférés selon l'invention, on compte notamment :
- les fragments comprenant un site de fixation à la clathrine,
- les fragments comprenant un site de fixation des acides gras, notamment un site de fixation des acides gras comprenant une séquence d'acides aminés hydrophobes, séparée en deux parties par deux prolines contiguës, qui induisent une structure en épingle à cheveux dont les bras sont constitués d'acides aminés hydrophobes,
- les fragments comprenant une région hydrophobe constituant un domaine transmembranaire,
- les fragments comprenant une région capable de controler l'endocytose et l'adressage intracellulaire des protéines dans le système membranaire périphérique, notamment un fragment comprenant un site comportant les motifs LI et LL,
- les fragments comprenant un site de fixation des cytokines, notamment un site incluant une région riche en cystéine,
- les fragments comprenant une région définissant un site de fixation potentiel de ligands lipoprotéiques tels que l'ApoB et l'ApoE, notamment une région comprenant une séquence d'acides aminés chargés alternativement + et -, et
- les fragments comprenant un motif RSRS.

On compte notamment parmi ces fragments les polypeptides tels que définis dans les tableaux 1, 2 et 4, ou tous fragments des nucléotides de SEQ ID 2, 8 ou 16, comportant lesdits polypeptides, et tous fragments équivalents, homologues ou variants.

D'autres fragments préférés incluent des peptides antigéniques tels que ceux de séquences SEQ ID 29 et 30.

### Les séquences nucléotidiques du récepteur LSR

La présente invention a pour objet des séquences d'acide nucléique isolées, caractérisées en ce qu'elles codent pour un récepteur LSR ou un polypeptide selon l'invention.

Plus particulièrement, l'invention concerne un acide nucléique purifié, caractérisé en ce qu'il comprend au moins 8, de préférence au moins 10 et plus particulièrement au moins 15 nucléotides consécutifs du polynucléotide de SEQ ID 19, ainsi que les séquences d'acides nucléiques complémentaires de cet acide nucléique.

L'invention concerne également un acide nucléique purifié, caractérisé en ce qu'il comprend au moins 8, de préférence au moins 10 et plus particulièrement au moins 15 nucléotides consécutifs du polynucléotide de SEQ ID 41, ainsi que les séquences d'acides nucléiques complémentaires de cet acide nucléique.

L'invention concerne également un acide nucléique purifié codant pour le récepteur LSR humain, caractérisé en ce qu'il comprend une séquence nucléotidique correspondant aux nucléotides 1898 à 21094, particulièrement aux nucléotides 2001 à 20979, plus particulièrement aux nucléotides 2145 à 20979 de SEQ ID 19, ainsi que les séquences d'acides nucléiques complémentaires de cet acide nucléique.

L'invention concerne également les séquences d'acides nucléiques contenues dans le gène codant pour le récepteur LSR humain, notamment chacun des exons dudit gène ou une combinaison d'exons dudit gène, ou encore un polynucléotide s'étendant sur une portion d'un ou plusieurs exons. De façon préférée, ces acides nucléiques codent pour un ou plusieurs fragments biologiquement actifs du récepteur LSR humain.

L'invention concerne également un acide nucléique purifié, caractérisé en ce qu'il comprend une séquence nucléotidique correspondant aux nucléotides 1 à 1897 de SEQ ID 19, ainsi que les séquences d'acides nucléiques complémentaires de cet acide nucléique.

L'invention concerne également un acide nucléique purifié, caractérisé en ce qu'il comprend une séquence nucléotidique correspondant aux nucléotides 21095 à 22976 de SEQ ID 19, ainsi que les séquences d'acides nucléiques complémentaires de cet acide nucléique.

L'invention concerne également un acide nucléique purifié, caractérisé en ce qu'il comprend une séquence nucléotidique choisie parmi le groupe comportant les séquences de SEQ ID 7, SEQ ID 9 et SEQ ID 11, ainsi que les séquences d'acides nucléiques complémentaires de cet acide nucléique.

L'invention concerne également un acide nucléique purifié, caractérisé en ce qu'il comprend une séquence nucléotidique choisie parmi le groupe comportant les séquences de SEQ ID 1, SEQ ID 3 et SEQ ID 5, ainsi que les séquences d'acides nucléiques complémentaires de cet acide nucléique.

L'invention concerne également un acide nucléique purifié, caractérisé en ce qu'il comprend une séquence nucléotidique choisie parmi le groupe comportant les séquences de SEQ ID 13, SEQ ID 14 et SEQ ID 15, ainsi que les séquences d'acides nucléiques complémentaires de cet acide nucléique.

L'invention concerne également un acide nucléique purifié caractérisé en ce qu'il comprend une séquence nucléotidique correspondant aux nucléotides 1898 à 2001 de SEQ ID 19 ou de manière préférée aux nucléotides 1898 à 2144 de SEQ ID 19, ainsi que les séquences d'acides nucléiques complémentaires de cet acide nucléique.

L'invention concerne également un acide nucléique purifié caractérisé en ce qu'il comprend une séquence nucléotidique correspondant aux nucléotides 20980 à 21094 de SEQ ID 19, ainsi que les séquences d'acides nucléiques complémentaires de cet acide nucléique.

Parmi les acides nucléiques selon l'invention, on préfère les acides nucléiques de séquence nucléotidique choisie parmi le groupe comportant les séquences de SEQ ID 7, SEQ ID 9 et SEQ ID 11, les séquences de SEQ ID 1, SEQ ID 3 et SEQ ID 5, ainsi que les séquences de SEQ ID 13, SEQ ID 14 et SEQ ID 15, ainsi que leurs séquences complémentaires.

Font également partie de l'invention, les séquences variantes, mutées, équivalentes, ou homologues des séquences selon l'invention, ainsi que leurs fragments et les séquences nucléiques capables de s'hybrider spécifiquement avec les séquences selon l'invention.

### Séquence génomique humaine

L'invention concerne donc la séquence génomique du récepteur LSR humain, de préférence la séquence de SEQ ID 19, ainsi que leurs séquences complémentaires, ou un de leurs variants alléliques, les séquences mutées, équivalentes ou homologues, ou un de leurs fragments.

Le gène du LSR humain (SEQ ID 19) comprend 10 exons répartis sur 21 094 pb. La taille des exons est respectivement de : 356, 345, 120, 57, 147, 174, 60, 132, 626 et 141 pb (Tableau 5).

**Tableau 5**

| **EXON** | **DEBUT** | **FIN** | **5' EPIS.** | **BL 5'** | **3' EPIS.** | **BL 3'** |
|---|---|---|---|---|---|---|
| Ex1 | 1898 | 2253 | --- | --- | GTACGG | + 2 |
| Ex2 | 3437 | 3781 | CAG | + 1 | GTATGT | + 1 |
| Ex3 | 12067 | 12186 | CAG | + 2 | GTGAGT | + 1 |
| Ex4 | 15047 | 15103 | CAG | + 2 | GTACGG | + 1 |
| Ex5 | 15668 | 15814 | CAG | + 2 | GTAAGT | + 1 |
| Ex6 | 19481 | 19654 | CAG | + 2 | GTGAGG | + 1 |
| Ex7 | 19801 | 19860 | CAG | + 2 | GTGAGA | + 1 |
| Ex8 | 19958 | 20089 | TAG | + 2 | GTAAGC | + 1 |
| Ex9 | 20231 | 20856 | CAG | + 2 | GTGAGG | 0 |
| Ex10 | 20946 | 21094 | CAG | 0 | --- --- | |

La colonne EXON indique les exons numérotés de 1 à 10 dans l'ordre 5'-3' de leur position sur la séquence génomique. Les colonnes DEBUT et FIN indiquent respectivement la position du premier et du dernier nucléotide de l'exon considéré. Dans les colonnes 5'EPIS. et 3'EPIS. sont indiquées les séquences du site d'épissage bordant l'exon en 5' et 3'. Les colonnes BL 5' et BL 3' indiquent le nombre de bases respectivement en 5' et en 3' d'un exon qui ne seront engagées dans la phase de lecture du messager qu'après l'épissage. Par exemple : l'exon 7 ayant une base libre en 3', cet exon peut être joint à l'extrémité 5' de l'exon 8 qui a 2 bases libres en 5'. L'ensemble 1 base + 2 bases reconstitue le codon qui était détruit par l'intron dans la séquence génomique. L'exon 7 peut être rabouté par son extrémité 3' à n'importe quel exon ayant deux bases libres en 5' ; si le nouveau codon créé ne correspond pas à un codon stop, la phase ouverte de lecture sera conservée.

Les exons 1 et 2 ainsi que 9 et 10 sont nécessairement co-épissés, formant ainsi un bloc 5' correspondant aux exons 1 et 2 et un bloc 3' correspondant aux exons 9 et 10. Le messager minimal fonctionnel, correspondant au produit de ces quatre exons, aurait ainsi une taille d'environ 1 331 pb. Pour les autres exons, toutes les combinaisons possibles permettent de conserver la phase ouverte de lecture.

La taille des exons non codants en 5' n'a pas pu être déterminée avec précision. En effet, les séquences 5' UTR du rat sont trop divergentes de celles de l'homme pour finaliser l'analyse de ces séquences et identifier la réelle extrémité 5' de l'ADNc de LSR humain. Celle-ci peut être menée grâce à l'isolement de l'extrémité 5' des messagers LSR humains par les méthodes de capture d'extrémité 5' développées par les inventeurs (WO 96/34981 ). Le site de polyadénylation décrit ci-dessous est le seul qui soit présent avant le gène USF2, situé en 3' du gène LSR humain. Il est donc très vraisemblable que la région 3' non traduite de ce gène soit très courte (de taille estimée d'environ 100pb). Toutes les tailles données, concernant les molécules d'ADNc de LSR humain devront donc être ajustées en fonction de la taille de l'extrémité 5'- non traduite. La séquence d'ADNc humaine obtenue en tenant compte de l'ensemble des exons déduits de l'analyse de la séquence génomique a une taille de 2 158 pb. Cette forme correspondrait à la forme LSR-Rn-2097.

La localisation de certains des signaux d'expression de la séquence nucléotidique de SEQ ID 19 est présentée dans le tableau 6 qui suit.

**Tableau 6**

| **Signal** | **Début** | **Fin** |
|---|---|---|
| ATG préféré | 2145 | 2147 |
| Autre ATG possible | 2001 | 2003 |
| STOP | 20977 | 20979 |
| POLYAd | 21065 | 21070 |

Dans la colonne Signal sont décrits les éléments caractéristiques de la molécule d'ARN messager : Initiation de la traduction (ATG), fin de la traduction (STOP) et signal de polyadénylation (POLY Ad). Les colonnes Début et Fin indiquent la position en nucléotide de début et de fin de ces signaux sur la séquence génomique SEQ ID 19. Un signal ATG d'initiation de la traduction est préféré à l'autre car celui-ci présente un environnement plus propice à l'initiation.

La présente invention concerne également les séquences d'acides nucléiques purifiées codant pour un ou plusieurs éléments de régulation de l'expression du gène LSR humain. Sont également comprises dans l'invention, les séquences d'acide nucléique de promoteur et/ou de régulateur de gène codant pour le récepteur selon l'invention, ou un de leurs variants alléliques, les séquences mutées, équivalentes ou homologues, ou un de leurs fragments.

L'invention concerne plus particulièrement un acide nucléique purifié situé en 5' de la séquence codante du gène LSR. Cet acide nucléique est caractérisé en ce qu'il comprend une séquence nucléotidique correspondant aux nucléotides 1 à 1897 de SEQ ID 19, ainsi que les séquences d'acides nucléiques complémentaires de cet acide nucléique. Des fragments plus courts de cet acide nucléique peuvent également être utilisés à titre de promoteurs d'expression du gène LSR ou de toute autre séquence codant pour un polypeptide hétérologue.

L'invention concerne également un acide nucléique purifié situé en 3' de la séquence transcrite du gène LSR. Cet acide nucléique est caractérisé en ce qu'il comprend une séquence nucléotidique correspondant aux nucléotides 21095 à 22976 de SEQ ID 19, ainsi que les séquences d'acides nucléiques complémentaires de cet acide nucléique. Des fragments plus courts de cet acide nucléique peuvent également être utilisés à titre d'éléments régulateurs d'expression de gènes.

Enfin l'invention concerne aussi la séquence génomique du récepteur LSR humain, de préférence la séquence de SEQ ID 41, ainsi que leurs séquences complémentaires, ou un de leurs variants alléliques, les séquences mutées, équivalentes ou homologues, ou un de leurs fragments.

### Comparaison des organisations génomiques chez l'homme, le rat et la souris

Il est intéressant de noter qu'une synthénie (conservation de l'organisation de certaines régions chromosomiques entre espèces) entre la région du chromosome 7 de la souris où est localisé le gène de Lisch7, à proximité immédiate de USF2, et la région 19q13 du chromosome 19 humain, portant le LSR, est bien décrite. L'organisation des deux gènes Lisch7/LSR et USF2 est conservée entre espèces. De même, de localisation plus centromérique par rapport à ces gènes, Apo E se trouve aussi bien chez la souris que chez l'homme. Il est remarquable que le récepteur aux lipoprotéines LSR et un de leurs ligands ApoE soient localisés dans une même région chromosomique. En effet, il est fréquent que le récepteur et le ligand soient co-régulés. Une telle situation permettrait d'envisager que des phénomènes observés chez la souris soient transposables chez l'homme.

### Séquences d'ADNc humaines, de rat et de souris

L'invention concerne en outre 3 ADNc différents issus du gène du récepteur LSR par épissage alternatif. Ces 3 ADNc ont été identifiés chez l'homme, le rat et la souris (Tableau 2b). Ils codent pour les trois types de sous-unités du récepteur LSR, α (longue), α' (intermédiaire) et β (courte). L'ADNc le plus long contient la totalité des 10 exons du gène. L'ADNc intermédiaire ne comprend pas l'exon 4. Enfin, l'ADNc le plus court ne contient pas les exons 4 et 5.

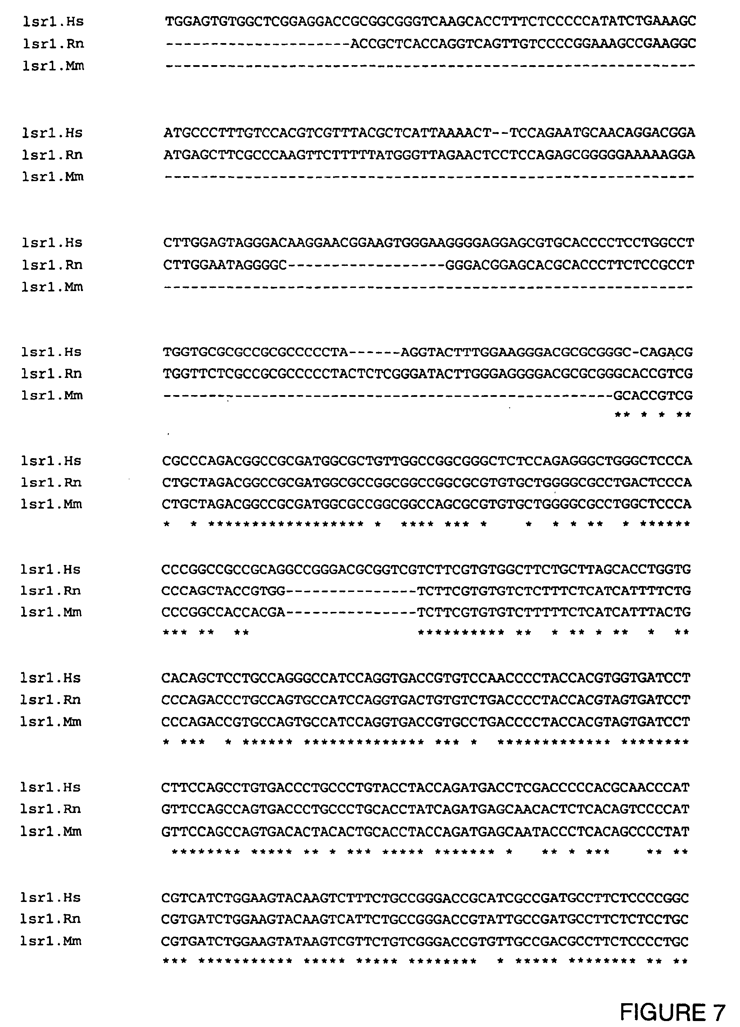

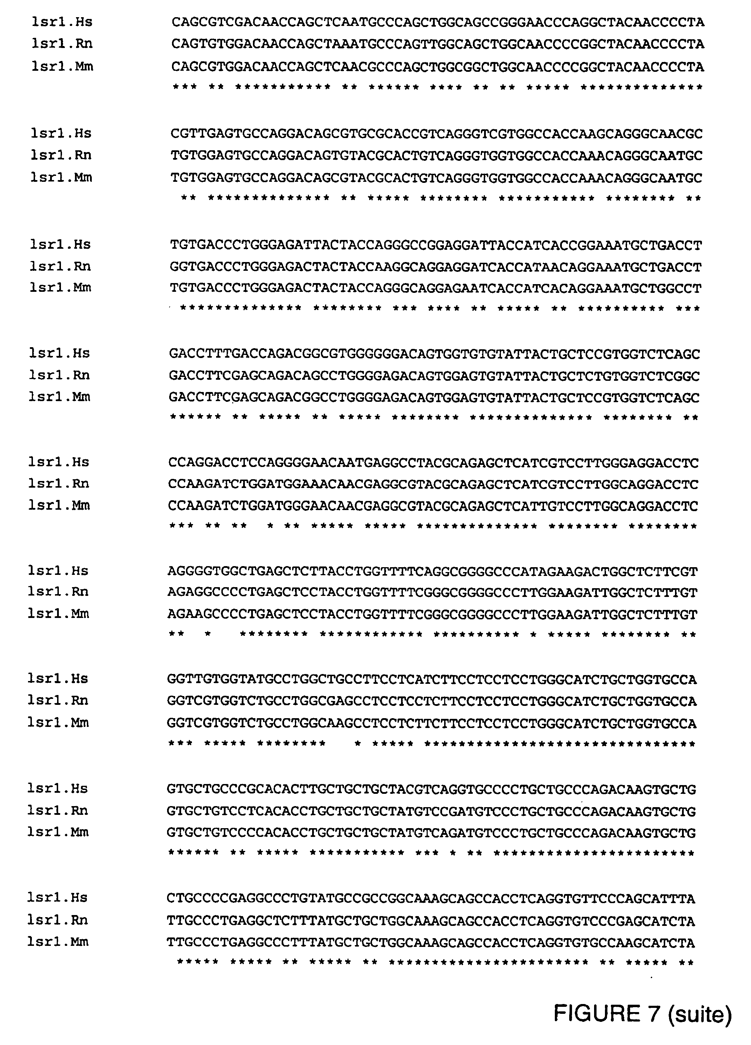

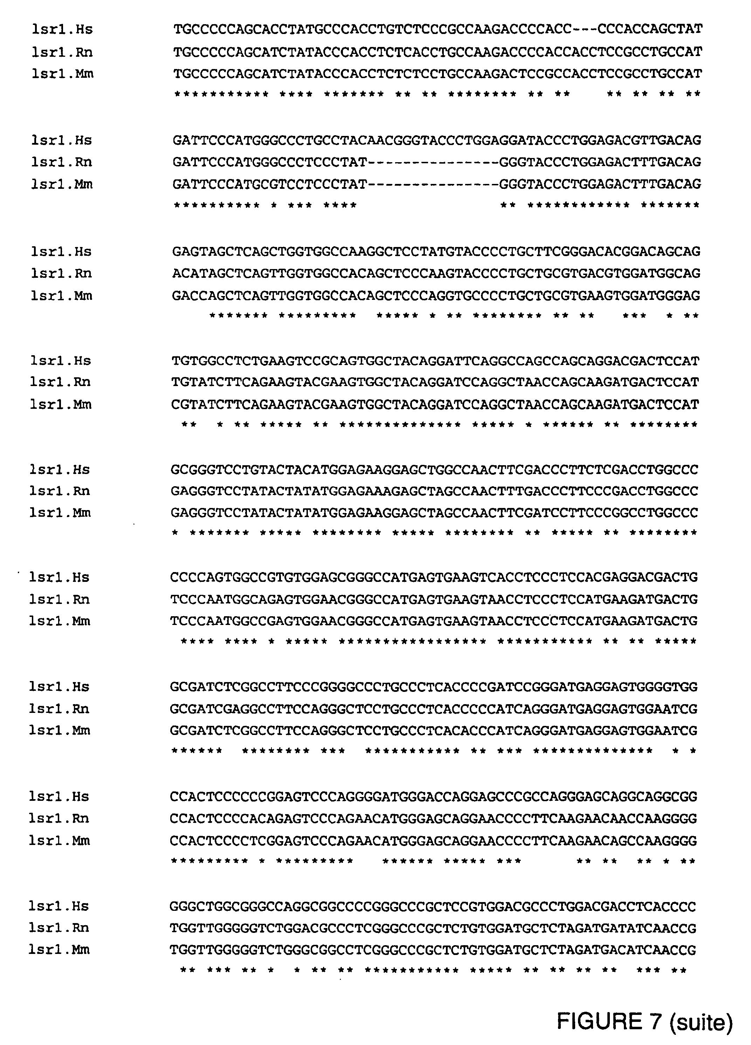

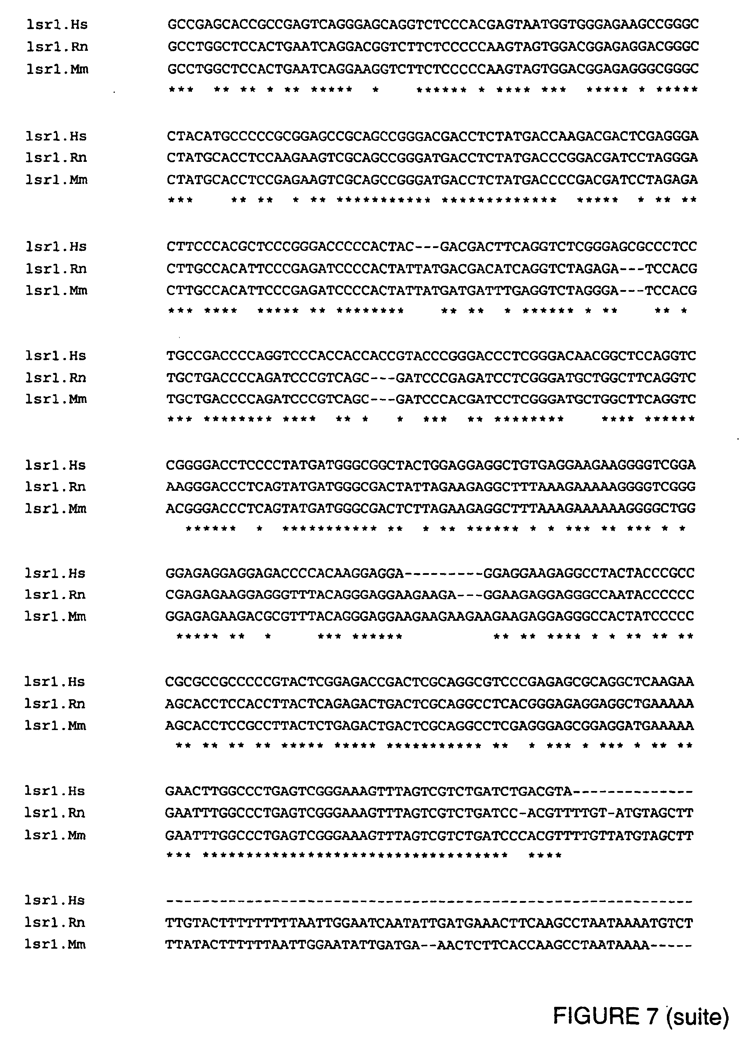

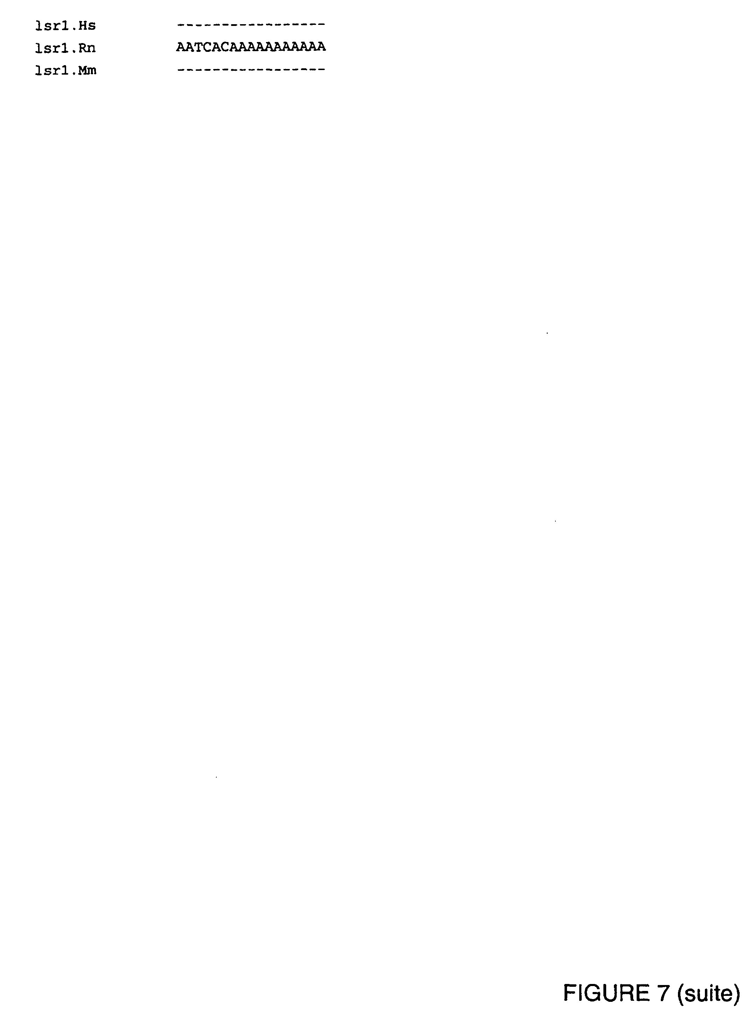

La séquence nucléotidique d'ADNc LSR-Hs-2062 humaine, codant pour la sous-unité α du récepteur LSR, et la séquence nucléotidique d'ADNc LSR-Rn-2097 de rat sont identiques à 78,6 % sur 1 955 pb chevauchantes. Ces chiffres sont respectivement de 78,8 % et 1 851 pb lorsque la séquence de LSR-Mm-1886 murine (forme longue) est alignée avec la séquence humaine. Cela traduit une très grande conservation des séquences nucléiques entre espèces. Les plus grandes divergences sont observées dans l'extrémité 5' non traduite (quand la séquence est disponible), dans le premier exon codant et dans l'extrémité 3' non traduite (Figure 7).

L'invention concerne donc également un acide nucléique purifié, caractérisé en ce qu'il est choisi parmi le groupe comportant les séquences de SEQ ID 7, SEQ ID 9 et SEQ ID 11, les séquences SEQ ID 1, SEQ ID 3 et SEQ ID 5, et les séquences SEQ ID 13, SEQ ID 14 et SEQ ID 15, ainsi que les séquences d'acides nucléiques complémentaires de cet acide nucléique, ou un de leurs variants alléliques, les séquences mutées, équivalentes ou homologues, ou un de leurs fragments.

Les acides nucléiques constituant les phases codantes des acides nucléiques précités, entre les codons d'initiation et de fin de traduction, font aussi partie de l'invention.

Les acides nucléiques codant pour les fragments polypeptidiques selon l'invention sont aussi partie de l'invention. On notera particulièrement les acides nucléiques codent pour les fragments décrits aux Tableaux 1, 3 et 4.

Ainsi le tableau 7 décrit le positionnement de tels fragments d'acide nucléique sur la séquence humaine de SEQ ID 7.

**Tableau 7**

| **Domaine ou motif** | | **Position sur l'ADNc de SEQ 7** | |
|---|---|---|---|
| | | **Début** | **Fin** |
| Potentiel site de fixation des acides gras | | 329 | 385 |
| Potentiel site de fixation à la clathrine | | 572 | 583 |
| Signal de transport : | LI | 809 | 814 |
| | LL | 845 | 850 |
| Domaine transmembranaire | | 872 | 901 |
| Potentiel site récepteur aux cytokines | | 902 | 1009 |
| Motif RSRS | | 1682 | 1693 |
| Potentiel site de fixation des ligands lipoprotéiques | | 1904 | 1942 |

L'invention concerne également un acide nucléique purifié correspondant à la séquence du 5'UTR des ADNc codant pour le récepteur LSR humain. Cet acide nucléique est caractérisé en ce qu'il comprend une séquence nucléotidique correspondant aux nucléotides 1898 à 2001 de SEQ ID 19 ou de manière préférée aux nucléotides 1898 à 2144 de SEQ ID 19, ainsi que les séquences d'acides nucléiques complémentaires de cet acide nucléique. Des fragments plus courts de cet acide nucléique peuvent également être utilisés.

L'invention concerne également un acide nucléique purifié correpondant à la séquence du 3'UTR des ADNc codant pour le récepteur LSR. Cet acide nucléique est caractérisé en ce qu'il comprend une séquence nucléotidique correspondant aux nucléotides 20980 à 21094 de SEQ ID 19, ainsi que les séquences d'acides nucléiques complémentaires de cet acide nucléique. Des fragments plus courts de cet acide nucléique peuvent également être utilisés.

L'invention concerne également les acides nucléiques purifiés correspondant respectivement aux séquences des 5'UTR ou des 3'UTR des ADNc codant pour le récepteur LSR de rat ou de souris. Des fragments plus courts de cet acide nucléique peuvent également être utilisés.

Les 5'UTR et 3'UTR peuvent contenir des éléments (« responsive elements » et « enhancers ») qui sont impliqués dans la régulation de la transcription et de la traduction. Ces régions ont notamment un rôle dans la stabilité des ARNm. De plus, le 5'UTR comprend le motif Shine-Delgarno qui est indispensable à la traduction de l'ARNm.

Par acide nucléique, séquence nucléique ou d'acide nucléique, on entend un fragment d'ADN et/ou d'ARN naturel isolé, ou de synthèse, comportant ou non des nucléotides non naturels, désignant un enchaînement précis de nucléotides, modifiés ou non, permettant de définir un fragment, un segment ou une région d'un acide nucléique.

Par séquences nucléiques équivalentes, on entend désigner les séquences nucléiques codant pour les polypeptides selon l'invention, compte tenu de la dégénérescence du code génétique, les séquences d'ADN complémentaire et les séquences d'ARN correspondant, ainsi que les séquences nucléiques codant pour les polypeptides équivalents.

Par séquences nucléiques homologues, on entend désigner les séquences nucléiques codant pour les polypeptides homologues et/ou les séquences nucléiques présentant un taux d'identité d'au moins 80 %, de préférence 90 %. Selon l'invention, l'identité est uniquement de type statistique, elle signifie que les séquences présentent au minimum 80 %, et préférentiellement 90 %, de nucléotides en commun. Il s'agit de préférence de séquences susceptibles de s'hybrider spécifiquement avec une des séquences de l'invention. De préférence, les conditions d'hybridation spécifiques seront telles que celles définies dans les exemples, ou telles qu'elles assurent au moins 95 % d'homologie.

La longueur de ces séquences nucléiques d'hybridation peut varier de 8, 10, 15, 20 ou 30 à 200 nucléotides, particulièrement de 20 à 50 nucléotides, plus particulièrement de 20 à 30 nucléotides.

On entendra par allèle ou variant allélique les séquences mutées naturelles correspondant à des polymorphismes présents chez l'être humain et, notamment, à des polymorphismes pouvant conduire à la survenue et/ou au développement de l'obésité ou de l'anorexie. Ces polymorphismes pourront aussi conduire à la survenue et/ou au développement des risques ou complications associés à l'obésité, notamment au niveau cardio-vasculaire, et/ou de pathologies associées à des anomalies du métabolisme des cytokines.

On entend par séquences nucléiques mutées, les séquences nucléiques comportant au moins une mutation ponctuelle par comparaison à la séquence normale.

Si les séquences selon l'invention sont en général les séquences normales, ce sont également les séquences mutées dans la mesure où elles comportent au moins une mutation ponctuelle et de préférence au plus 10 % de mutations par rapport à la séquence normale.

De préférence, la présente invention concerne des séquences nucléiques mutées dans lesquelles les mutations ponctuelles sont non muettes, c'est-à-dire qu'elles conduisent à une modification de l'acide aminé codé par rapport à la séquence normale. De façon encore préférée, ces mutations portent sur des acides aminés qui structurent le récepteur et/ou le complexe LSR ou les domaines et fragments correspondants de celui-ci. Ces mutations peuvent encore porter sur des acides aminés portés par les régions correspondant aux sites récepteurs, aux lipoprotéines ou aux cytokines, notamment à la leptine, ou aux sites de fixation des cofacteurs, notamment des acides gras libres, ou encore aux sites de phosphorylation. Ces mutations peuvent également porter sur les séquences impliquées dans le transport, l'adressage et l'ancrage membranaire du LSR.

De façon générale, la présente invention s'intéresse aussi bien aux polypeptides LSR normaux qu'aux polypeptides LSR mutés, ainsi qu'à leurs fragments et aux séquences d'ADN et d'ARN correspondantes, les polypeptides LSR désignant les polypeptides du récepteur selon l'invention.

Selon l'invention, les fragments de séquences nucléiques peuvent notamment coder pour des domaines des récepteurs et polypeptides possédant une fonction ou une activité biologique tel que défini précédemment, comporter des domaines ou des régions situés en amont ou en aval de la séquence codante et contenant des éléments régulateurs de l'expression du gène LSR ou bien possédant une séquence permettant leur utilisation comme sonde ou comme amorce dans des procédés de détection, d'identification ou d'amplification de séquence nucléique. Ces fragments présentent de préférence une taille minimale de 8, de 10 bases et on préférera des fragments de 20 bases, et de préférence de 30 bases.

Parmi les fragments nucléiques pouvant être intéressants, notamment pour le diagnostic, il faut citer par exemple les séquences génomiques introniques du gène du complexe LSR, comme notamment les séquences jonctions entre les introns et les exons, normales ou mutées.

Les séquences d'acide nucléique utilisables comme oligonucléotides sens ou anti-sens, caractérisées en ce que leurs séquences sont choisies parmi les séquences selon l'invention, font partie de l'invention.

Parmi les fragments d'acides nucléiques intéressants, il faut ainsi citer en particulier les oligonucléotides anti-sens, c'est-à-dire dont la structure assure, par hybridation avec la séquence cible, une inhibition de l'expression du produit correspondant. Il faut également citer les oligonucléotides sens qui, par interaction avec des protéines impliquées dans la régulation de l'expression du produit correspondant, induiront soit une inhibition, soit une activation de cette expression.

Les séquences portant des mutations pouvant intervenir dans les séquences promotrices et/ou régulatrices des gènes du complexe LSR, lesquelles peuvent avoir des effets sur l'expression des protéines correspondantes, notamment sur leur niveau d'expression, font également partie des séquences précédentes selon l'invention.

Les séquences nucléiques utilisables comme amorce ou sonde, caractérisées en ce que leur séquence nucléique est une séquence de l'invention, font également partie de l'invention.

La présente invention concerne l'ensemble des amorces qui peuvent être déduites des séquences nucléotidiques de l'invention et qui peuvent permettre de mettre en évidence lesdites séquences nucléotidiques de l'invention, en particulier les séquences mutées, en utilisant notamment une méthode d'amplification telle que la méthode PCR, ou une méthode apparentée.

La présente invention concerne l'ensemble des sondes qui peuvent être déduites des séquences nucléotidiques de l'invention, notamment des séquences capables d'hybrider avec elles, et qui peuvent permettre de mettre en évidence lesdites séquences nucléotidiques de l'invention, en particulier de discriminer les séquences normales des séquences mutées.

L'invention concerne également l'utilisation d'une séquence d'acide nucléique selon l'invention comme sonde ou amorce, pour la détection et/ou l'amplification de séquence d'acide nucléique selon l'invention.

L'ensemble des sondes et amorces selon l'invention pourront être marquées par des méthodes bien connues de l'homme du métier, afin d'obtenir un signal détectable et/ou quantifiable.

La présente invention concerne également les séquences nucléotidiques qui peuvent comporter des nucléotides non naturels, notamment des nucléotides soufrés par exemple ou de structure α ou β.

La présente invention concerne, bien entendu, aussi bien les séquences ADN qu'ARN ainsi que les séquences qui s'hybrident avec elles, de même que les ADNs double brin correspondants.

Dans ce qui va suivre, on nommera les séquences d'ADN précédentes gènes du complexe LSR, qu'il s'agisse de séquences normales ou pathologiques.

Il doit être compris que la présente invention ne concerne pas les séquences nucléotidiques génomiques dans leur environnement chromosomique naturel, c'est-à-dire à l'état naturel. Il s'agit de séquences qui ont été isolées, c'est-à-dire qu'elles ont été prélevées directement ou indirectement, par exemple par copie (ADNc), leur environnement ayant été au moins partiellement modifié.

Ainsi, il peut s'agir aussi bien d'ADNc que d'ADN génomique partiellement modifié ou porté par des séquences au moins partiellement différentes des séquences les portant naturellement.

Ces séquences pourront également être qualifiées de non naturelles.

L'invention comprend aussi des méthodes pour le criblage de banques d'ADNc et d'ADN génomique, pour le clonage des ADNc isolés, et/ou des gènes codant pour le récepteur selon l'invention, et pour leurs promoteurs et/ou régulateurs, caractérisées en ce qu'elles mettent en oeuvre une séquence nucléique selon l'invention. Parmi ces méthodes, on peut citer notamment :
- le criblage de banques d'ADNc et le clonage des ADNc isolés (Sambrook et al., 1989 ; Suggs et al., 1981 ; Woo et al., 1979), à l'aide des séquences nucléiques selon l'invention ;
- le criblage de banques d'étiquettes d'extrémités 5' (WO 96/34981) pour des séquences nucléiques selon l'invention, et ainsi l'isolement d'étiquettes permettant le clonage d'ADNc complets et des promoteurs correspondants à partir de banques d'ADN génomique ;
- le criblage de banques génomiques, par exemple de BACs (Chumakov et al., 1992 ; Chumakov et al., 1995) et éventuellement une analyse génétique en FISH (Cherif et al., 1990) à l'aide de séquences selon l'invention, permettant l'isolement et la localisation chromosomique, puis le séquençage complet des gènes codant pour le récepteur LSR.

Est également comprise dans l'invention, une séquence, notamment une séquence génomique codant pour un récepteur ou un polypeptide selon l'invention, ou une séquence d'acide nucléique de promoteur et/ou de régulateur de gène codant pour un récepteur ou un polypeptide selon l'invention, ou un de leurs variants alléliques, une séquence mutée, équivalente ou homologue, ou un de leurs fragments, caractérisée en ce qu 'elle est susceptible d'être obtenue par les méthodes précédentes selon l'invention, ou une séquence capable de s'hybrider avec lesdites séquences.

### Vecteurs, cellules hôtes et animaux transgéniques

L'invention comprend également les vecteurs de clonage et/ou d'expression contenant une séquence d'acide nucléique selon l'invention.

Les vecteurs selon l'invention, caractérisés en ce qu'ils comportent les éléments permettant l'expression et/ou la sécrétion desdites séquences une cellule hôte, font également partie de l'invention.

Les vecteurs caractérisés en ce qu'ils comportent une séquence de promoteur et/ou de régulateur selon l'invention, ou une séquence d'adressage cellulaire selon l'invention, ou l'un de leurs fragments, font également partie de l'invention.

Lesdits vecteurs comporteront de préférence un promoteur, des signaux d'initiation et de terminaison de la traduction, ainsi que des régions appropriées de régulation de la transcription. Ils doivent pouvoir être maintenus de façon stable dans la cellule et peuvent éventuellement posséder des signaux particuliers spécifiant la sécrétion de la protéine traduite.

Ces différents signaux de contrôle sont choisis en fonction de l'hôte cellulaire utilisé. A cet effet, les séquences d'acide nucléique selon l'invention peuvent être insérées dans des vecteurs à réplication autonome au sein de l'hôte choisi, ou des vecteurs intégratifs de l'hôte choisi.

Parmi les systèmes à réplication autonome, on utilisera de préférence en fonction de la cellule hôte, des systèmes de type plasmidique ou viral, les virus vecteurs pouvant notamment être des adénovirus (Perricaudet et al., 1992), des rétrovirus, des poxvirus ou des virus herpétiques (Epstein et al., 1992). L'homme du métier connaît les technologies utilisables pour chacun de ces systèmes.

Lorsque l'on souhaitera l'intégration de la séquence dans les chromosomes de la cellule hôte, on pourra utiliser par exemple des systèmes de type plasmidique ou viral ; de tels virus seront, par exemple, les rétrovirus (Temin, 1986), ou les AAV (Carter, 1993).

De tels vecteurs seront préparés selon les méthodes couramment utilisées par l'homme du métier, et les clones en résultant peuvent être introduits dans un hôte approprié par des méthodes standard, telles que par exemple la lipofection, l'électroporation, le choc thermique.

L'invention comprend en outre les cellules hôtes, notamment les cellules eucaryotes et procaryotes, transformées par les vecteurs selon l'invention ainsi que les animaux transgéniques, excepté l'Homme, comprenant une desdites cellules transformées selon l'invention.

Parmi les cellules utilisables à ces fins, on peut citer bien entendu les cellules bactériennes (Olins et Lee, 1993), mais également les cellules de levure (Buckholz, 1993), de même que les cellules animales, en particulier les cultures de cellules de mammifères (Edwards et Aruffo, 1993), et notamment les cellules d'ovaire de hamster chinois (CHO), mais également les cellules d'insectes dans lesquelles on peut utiliser des procédés mettant en oeuvre des baculovirus par exemple (Luckow, 1993). Un hôte cellulaire préféré pour l'expression des protéines de l'invention est constitué par les cellules CHO.

Parmi les mammifères selon l'invention, on préférera des animaux tels que les souris, les rats ou les lapins, exprimant un polypeptide selon l'invention, le phénotype correspondant au récepteur LSR normal ou variant, notamment muté d'origine humaine.

Parmi les modèles animaux plus particulièrement intéressants ici, on trouve notamment :
- les animaux transgéniques présentant un déficit d'un des composants du LSR. Ils sont obtenus par recombinaison homologue sur cellules souches embryonnaires, transfert de ces cellules souches à des embryons, sélection des chimères affectées au niveau des lignées reproductrices, et croissance desdites chimères ;
- les souris transgéniques surexprimant l'un ou plusieurs des gènes du complexe LSR d'origine murin et/ou humain. Les souris sont obtenues par transfection de copies multiples des gènes du complexe LSR sous contrôle d'un promoteur puissant de nature ubiquitaire, ou sélectif d'un type de tissu, de préférence le foie ;
- les animaux (de préférence souris) transgéniques rendus déficients pour l'un ou plusieurs des gènes du complexe LSR, par inactivation à l'aide du système LOXP/CRE recombinase (Rohlmann et al., 1996) ou de tout autre système d'inactivation de l'expression d'un gène à un âge précis de l'animal ;
- les animaux (de préférence rats, lapins, souris) surexprimant l'un ou plusieurs des gènes du complexe LSR, après transcription virale ou thérapie génique ;
- les croisements d'animaux déficients pour le LSR (notamment souris), avec des animaux déficients pour, ou surexprimant :
   > le LDL récepteur (Herz et al., 1995 ; Ishibashi et al., 1993),
   > la lipase hépatique (Homanics et al., 1995 ; Kobayashi et al., 1996),
   > l'apoprotéine B (Purcellhuynh et al., 1995 ; Fan et al., 1995),
   > l'apoprotéine E (Plump et al., 1992 ; Zhang et al., 1992 ; Huang et al., 1996),
   > l'apoCIII (Aalto-Setälä et al., 1992 ; Ito et al., 1990 ; Maeda et al., 1994).

L'obtention d'animaux transgéniques, et les transfections, virales ou non virales, seront menés de façon préférée sur les lignées de rats et souris suivantes :
- rat Zucker (fa/fa) (lida et al., 1996),
- souris AKR/J (West et al., 1992),
- souris ob/ob (Zhang et al., 1994),
- souris ob2j/ob2j (ibid),
- souris tubby (Kleyn et al., 1996 ; Nobben-Trauth et al., 1996),
- fat/fat (Heldin et al., 1995),
- souris agouti (Lu et al., 1994 ; Manne et al., 1995),
- souris db/db (Chen et al., 1996).

Les cellules et mammifères selon l'invention sont utilisables dans une méthode de production d'un polypeptide selon l'invention, comme décrit ci-dessous, et peuvent également servir à titre de modèle d'analyse et de criblage.

Les cellules ou mammifères transformés tels que décrits précédemment peuvent être ainsi utilisés à titre de modèles afin d'étudier les interactions entre les polypeptides du complexe LSR, entre ceux-ci et leurs partenaires, composés chimiques ou protéiques, impliqués directement ou indirectement dans les activités de récepteur aux lipoprotéines ou de récepteur aux cytokines, et notamment à la leptine, et afin d'étudier les différents mécanismes et interactions mis en jeu selon le type d'activité, ou selon qu'il s'agit d'un complexe normal, ou dont l'un au moins des domaines est un variant.

Surtout, ils peuvent être utilisés pour la sélection de produits interagissant avec le complexe LSR, ou l'un de ses domaines, normal(aux) ou variant(s), à titre de cofacteur, ou d'inhibiteur, notamment compétitif, ou encore ayant une activité agoniste ou antagoniste sur les changements conformationnels du complexe LSR. De préférence, on utilisera lesdites cellules transformées à titre de modèle permettant, notamment, la sélection de produits permettant de lutter contre l'obésité ou les pathologies mentionnées ci-dessus. Lesdites cellules pourront encore servir pour la mise en évidence des risques potentiels présentés par certains composés.

### Production de polypeptides issus du récepteur LSR

L'invention concerne également la synthèse de polypeptides synthétiques ou recombinants de l'invention, notamment par synthèse chimique ou par l'utilisation d'une séquence d'acide nucléique selon l'invention.

Les polypeptides selon la présente invention peuvent être obtenus par synthèse chimique et ce en utilisant l'une quelconque des nombreuses synthèses peptidiques connues, par exemple les techniques mettant en oeuvre des phases solides ou des techniques utilisant des phases solides partielles, par condensation de fragments ou par une synthèse en solution classique.

Lorsque les composés selon la présente invention sont synthétisés par la méthode en phase solide, l'acide aminé C-terminal est fixé sur un support solide inerte et comporte des groupes protecteurs de son groupement amino en alpha (et si cela est nécessaire, des protections sur ses groupes fonctionnels latéraux).

A la fin de cette étape, le groupe protecteur du groupement amino terminal est éliminé et on fixe le second acide aminé comportant lui aussi les protections nécessaires.

Les groupes protecteurs N-terminaux sont éliminés après que chaque acide aminé a été fixé, par contre on maintient, bien entendu, la protection sur les chaînes latérales. Lorsque la chaîne polypeptidique est complète, on clive le peptide de son support et on élimine les groupes de protection latéraux.

La technique de synthèse en phase solide est bien connue de l'homme du métier. Voir notamment Stewart et al. (1984) et Bodansky (1984).

Les polypeptides obtenus par synthèse chimique et pouvant comporter des acides aminés non naturels correspondant sont également compris dans l'invention.

La méthode de production d'un polypeptide de l'invention sous forme recombinante est elle-même comprise dans la présente invention, et se caractérise en ce que l'on cultive les cellules transformées, notamment les cellules ou mammifères de la présente invention, dans des conditions permettant l'expression d'un polypeptide recombinant codé par une séquence d'acide nucléique selon l'invention, et que l'on récupère ledit polypeptide recombinant.

Fait également partie de l'invention, une méthode de production de polypeptide hétérologue, caratérisée en ce qu'elle met en oeuvre un vecteur ou une cellule hôte comportant une au moins des séquences promotrices et/ou régulatrices selon l'invention, ou l'une au moins des séquences d'adressage cellulaire selon l'invention, ou l'un de leurs fragments.

Les polypeptides recombinants, caractérisés en ce qu'ils sont susceptibles d'être obtenus par ladite méthode de production, font également partie de l'invention.

Les polypeptides recombinants obtenus comme indiqué ci-dessus, peuvent aussi bien se présenter sous forme glycosylée que non glycosylée et peuvent présenter ou non la structure tertiaire naturelle.

Ces polypeptides peuvent être produits à partir des séquences d'acide nucléique définies ci-dessus, selon les techniques de production de polypeptides recombinants connues de l'homme du métier. Dans ce cas, la séquence d'acide nucléique utilisée est placée sous le contrôle de signaux permettant son expression dans un hôte cellulaire.

Un système efficace de production d'un polypeptide recombinant nécessite de disposer d'un vecteur et d'une cellule hôte selon l'invention.

Ces cellules peuvent être obtenues par l'introduction dans des cellules hôtes d'une séquence nucléotidique insérée dans un vecteur tel que défini ci-dessus, puis la mise en culture desdites cellules dans des conditions permettant la réplication et/ou l'expression de la séquence nucléotidique transfectée.

Les procédés de purification de polypeptide recombinant utilisés sont connus de l'homme du métier. Le polypeptide recombinant peut être purifié à partir de lysats et extraits cellulaires, du sumageant du milieu de culture, par des méthodes utilisées individuellement ou en combinaison, telles que le fractionnement, les méthodes de chromatographie, les techniques d'immunoaffinité à l'aide d'anticorps mono ou polyclonaux spécifiques, etc...

Une variante préférée consiste à produire un polypeptide recombinant fusionné à une protéine «porteuse» (protéine chimère). L'avantage de ce système est qu'il permet une stabilisation et une diminution de la protéolyse du produit recombinant, une augmentation de la solubilité au cours de la renaturation *in vitro* et/ou une simplification de la purification lorsque le partenaire de fusion possède une affinité pour un ligand spécifique.

### Anticorps

Les anticorps mono ou polyclonaux ou leurs fragments, anticorps chimériques ou immunoconjugués, caractérisés en ce qu'ils sont capables de reconnaître spécifiquement un polypeptide ou un récepteur selon l'invention, font partie de l'invention.

Des anticorps polyclonaux spécifiques peuvent être obtenus à partir d'un sérum d'un animal immunisé contre, par exemple :
- le récepteur LSR purifié à partir de membranes de cellules présentant ledit récepteur LSR, par des méthodes bien connues de l'homme de l'art comme la chromatographie d'affinité en utilisant par exemple de la leptine recombinante comme ligand spécifique, ou
- un polypeptide selon l'invention, notamment produit par recombinaison génétique ou par synthèse peptidique, selon les modes opératoires usuels, à partir d'une séquence d'acide nucléique selon l'invention.

On notera notamment l'intérêt d'anticorps reconnaissant de façon spécifique certains polypeptides, variants, ou fragments, notamment biologiquement actifs, selon l'invention.

Les anticorps monoclonaux spécifiques peuvent être obtenus selon la méthode classique de culture d'hybridomes décrite par Köhler et Milstein, 1975.

Les anticorps selon l'invention sont, par exemple, des anticorps chimériques, des anticorps humanisés, des fragments Fab ou F(ab')₂. Ils peuvent également se présenter sous forme d'immunoconjugués ou d'anticorps marqués afin d'obtenir un signal détectable et/ou quantifiable.

L'invention concerne également des méthodes pour la détection et/ou la purification d'un polypeptide selon l'invention, caractérisées en ce qu'elles mettent en oeuvre un anticorps selon l'invention.

L'invention comprend en outre des polypeptides purifiés, caractérisés en ce qu'ils sont obtenus par une méthode selon l'invention.

Par ailleurs, outre leur utilisation pour la purification des polypeptides, les anticorps de l'invention, en particulier les anticorps monoclonaux, peuvent également être utilisés pour la détection de ces polypeptides dans un échantillon biologique.

Ils constituent ainsi un moyen d'analyse immunocytochimique ou immunohistochimique de l'expression de polypeptide du récepteur LSR sur des coupes de tissus spécifiques, par exemple par immunofluorescence, marquage à l'or, immunoconjugués enzymatiques.

Ils permettent notamment de mettre en évidence une expression anormale de ces polypeptides dans les tissus ou prélèvements biologiques, ce qui les rend utiles pour la détection d'expression anormale du récepteur LSR ou pour le suivi de l'évolution de méthode de prévention ou de traitement.

Plus généralement, les anticorps de l'invention peuvent être avantageusement mis en oeuvre dans toute situation où l'expression d'un polypeptide de récepteur LSR, normal ou muté, doit être observée.

### Détection de variabilité allélique et diagnostic

Font également partie de l'invention, les méthodes de détermination d'une variabilité allélique, d'une mutation, d'une délétion, d'une perte d'hétérozygotie ou d'une anomalie génétique, caractérisées en ce qu'elles mettent en oeuvre une séquence d'acide nucléique ou un anticorps selon l'invention.

Ces méthodes visent par exemple les méthodes de diagnostic de prédisposition à l'obésité, aux risques associés, ou aux pathologies associées à des anomalies du métabolisme des cytokines, en déterminant à partir d'un prélèvement biologique du patient la présence de mutations dans au moins une des séquences décrites précédemment. Les séquences d'acides nucléiques analysées pourront aussi bien être de l'ADN génomique, de l'ADNc, ou de l'ARNm.

Des acides nucléiques ou anticorps basés sur la présente invention pourront également être utilisés pour permettre un diagnostic positif et différentiel chez un malade pris isolément. Les séquences nucléiques seront de préférence utilisées pour un diagnostic pré-symptomatique chez un sujet à risque, notamment avec antécédent familial. Il est également possible de prévoir un diagnostic anté-natal.

En outre, la mise en évidence d'une mutation spécifique peut permettre un diagnostic évolutif, notamment quant à l'intensité de la pathologie ou à l'époque probable de son apparition.

Les méthodes permettant de mettre en évidence une mutation dans un gène par rapport au gène naturel sont, bien entendu, très nombreuses. On peut essentiellement les diviser en deux grandes catégories. Le premier type de méthode est celui dans lequel la présence d'une mutation est détectée par comparaison de la séquence mutée avec la séquence correspondante naturelle non mutée, et le second type est celui dans lequel la présence de la mutation est détectée de façon indirecte, par exemple par évidence de mésappariements dus à la présence de la mutation.

Ces méthodes peuvent mettre en oeuvre les sondes et amorces de la présente invention décrites. Il s'agit généralement de séquences nucléiques d'hybridation purifiées comprenant au moins 8 nucléotides, caractérisées en ce qu'elles peuvent s'hybrider spécifiquement avec une séquence nucléique choisie parmi le groupe comportant SEQ ID 1, SEQ ID 3, SEQ ID 5, SEQ ID 7, SEQ ID 9, SEQ ID 11, SEQ ID 13, SEQ ID 14 SEQ ID 15, SEQ ID 19 et SEQ ID 41. De préférence, les conditions d'hybridation spécifiques sont telles que celles définies dans les exemples, ou telles qu'elles assurent au moins 95 % d'homologie. La longueur de ces séquences nucléiques d'hybridation peut varier de 8, 10, 15, 20 ou 30 à 200 nucléotides, particulièrement de 20 à 50 nucléotides, plus particulièrement de 20 à 30 nucléotides.

Parmi les méthodes de détermination d'une variabilité allélique, d'une mutation, d'une délétion, d'une perte d'hétérozygotie ou d'une anomalie génétique, on préfère les méthodes comprenant au moins une étape d'amplification dite par PCR (réaction en chaîne par la polymérase) ou par PCR-like de la séquence cible selon l'invention susceptible de présenter une anomalie à l'aide de couple d'amorces de séquences nucléotidiques selon l'invention. Les produits amplifiés pourront être traités à l'aide d'enzyme de restriction approprié avant de procéder à la détection ou au dosage du produit ciblé.

Par PCR-like on entendra désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues, en général il s'agit de l'amplification de l'ADN par une polymérase ; lorsque l'échantillon d'origine est un ARN il convient préalablement d'effectuer une transcription réverse. II existe actuellement de très nombreux procédés permettant cette amplification, par exemple les méthodes dites NASBA «Nucleic Acid Sequence Based Amplification» (Compton, 1991), TAS «Transcription based Amplification System» (Guatelli et al., 1990), LCR «Ligase Chain Reaction» (Landegren et al., 1988), «Endo Run Amplification» (ERA), «Cycling Probe Reaction» (CPR), et SDA «Strand Displacement Amplification» (Walker et al., 1992), bien connues de l'homme du métier.

L'invention comprend en outre les méthodes de diagnostic de pathologies et/ou de pathogénies corrélées à une expression anormale de polypeptide et/ou de récepteur selon l'invention, caractérisées en ce que l'on met en contact un anticorps selon l'invention avec le matériel biologique à tester, dans des conditions permettant la formation éventuelle de complexes immunologiques spécifiques entre ledit polypeptide et ledit anticorps et en ce que l'on détecte les complexes immunologiques éventuellement formés.

Les mutations d'un ou des gène(s) du complexe LSR peuvent être responsables de différentes modifications de leur(s) produit(s), modifications utilisables pour une approche diagnostique. En effet, les modifications d'antigénicité peuvent permettre la mise au point d'anticorps spécifiques. La discrimination des différentes conformations du LSR peut être réalisée par ces méthodes. Toutes ces modifications peuvent être utilisées dans une approche diagnostique grâce à plusieurs méthodes bien connues basées sur l'utilisation d'anticorps mono ou polyclonaux reconnaissant le polypeptide normal ou des variants mutés, comme par exemple par RIA ou par ELISA.

Ces méthodes de diagnostic visent également les méthodes de diagnostic par imagerie *in vivo* ou *ex vivo* en utilisant les anticorps monoclonaux ou polyclonaux selon l'invention, particulièrement ceux marqués et correspondant à tout ou partie des polypeptides mutés (imagerie à l'aide d'anticorps couplés à une molécule détectable en imagerie de type PET-scan, par exemple).

### Criblage de composés d'intérêt

Sont également comprises dans l'invention, les méthodes de sélection de composé chimique ou biochimique capable d'interagir, directement ou indirectement avec le récepteur selon l'invention, et/ou permettant de moduler l'expression ou l'activité dudit récepteur, caractérisées en ce qu'elles mettent en oeuvre un récepteur, un acide nucléique, un polypeptide, un vecteur, une cellule ou un mammifère selon l'invention.

### Criblage de composés modifiant l'activité du récepteur LSR

L'invention concerne une méthode de criblage de composés modifiant l'activité du récepteur LSR consistant en la mesure de l'effet de composés candidats sur différents paramètres reflétant, directement ou indirectement, pris indépendamment ou en association, une activité du récepteur LSR.

Pour le criblage de composés capables de moduler l'activité LSR de clairance des lipoprotéines, l'effet principal préféré est l'effet du composé sur l'activité de fixation, d'internalisation et de dégradation des lipoprotéines par le récepteur LSR.

Cet effet pourra être analysé en l'absence ou en la présence d'acides gras libres, ou de tout autre agent connu pour induire ou inhiber l'activité du LSR sur la clairance des lipoprotéines, ou en l'absence ou la présence de leptine, ou de tout autre agent capable d'induire ou d'inhiber la fonction LSR de clairance des cytokines. Il pourra en outre être mesuré en l'absence ou en présence d'agents capables de favoriser ou de réduire les activités lipases, soit intracellulaires, soit extracellulaires, ainsi qu'en la présence ou en l'absence de voies connues alternatives de dégradation des lipoprotéines.

Différents paramètres indirects peuvent également être mesurés, dont les suivants :
- le changement de poids induit par l'administration du composé
- la prise de nourriture induite par l'administration du composé
- la réponse lipémique post-prandiale induite par l'administration du composé avant, pendant ou après ingestion d'un repas, par exemple riche en graisses.

On préférera la sélection de composés capables d'influer sur les concentrations plasmatiques en triglycérides, et/ou sur la fixation, l'intemalisation et la dégradation hépatiques de lipoprotéines ou particules riches en triglycérides.

Pour le criblage de composés capables de moduler l'activité LSR de lcirance des cytokines, notamment de la leptine, l'effet principal préféré est l'effet du composé sur l'activité de fixation, d'intemalisation et de dégradation hépatique des cytokines par le récepteur LSR, en l'absence ou en la présence d'acides gras libres.

La mesure de la fixation, de l'intemalisation et/ou de la dégradation de lipides ou de cytokines peut être réaliséee par exemple sur des hépatocytes ou des fibroblastes en culture, ou sur toute autre cellule exprimant à sa surface le récepteur LSR. Les cellules seront de préférence des cellules exprimant un récepteur LSR recombinant, plus particulièrement des cellules exprimant un récepteur LSR recombinant et dont le récepteur LSR endogène serait inactivé ou absent. Ces cellules pourront exprimer ou non le LDL récepteur.

Le criblage de composé modulant l'activité LSR utilise de préférence des cellules ou des animaux modèles selon l'invention, notamment de souris, de rat, ou d'homme, plus particulièrement ceux décrits précédemment ou dans les exemples qui suivent.

### Criblage de composés modifiant l'expression du récepteur LSR

Le criblage peut être utilisé pour tester des composés capables de modifier le niveau et/ou la spécificité d'expression du récepteur LSR soit en se liant compétitivement aux sites de liaison des facteurs de transcription situés dans le promoteur de LSR soit en se liant directement aux facteurs de transcription.

Le niveau d'expression du récepteur LSR et sa localisation peuvent être analysés par hybridation en solution avec de grandes sondes comme indiqué dans le brevet PCT WO 97/05277, l'enseignement de ce document étant incorporé par référence. Brièvement, un ADNc ou l'ADN génomique du récepteur LSR ou encore un fragment de ceux-ci est inséré à un site de clonage situé immédiatement en aval d'un promoteur de RNA polymérase de bactériophage (T3, T7 ou SP6) pour produire un ARN antisens. De préférence, l'insert comprend au moins 100 nucléotides consécutifs de la séquence génomique du récepteur LSR ou d'un des ADNc de la présente invention, plus particulièrement un ou plusieurs des ADNc de SEQ ID 9, SEQ ID 11 ou SEQ ID 13. Le plasmide est linéarisé et transcrit en présence de ribonucléotides comprenant des ribonucléotides modifiés tels que Biotine-UTP et Digoxygénine-UTP. Un excès de cet ARN marqué est hybridé en solution avec les ARNm isolés de cellules ou de tissus d'intérêt. Les hybridations sont réalisées sous des conditions stringentes (40-50°C pendant 16 h dans une solution 80 % formamide, 0,4 M NaCl, pH 7-8). La sonde non-hybridée est éliminée par digestion avec des ribonucléases spécifiques des ARN simple-brin (Rnases CL3, T1, PhyM, U2 ou A). La présence de nucléotides modifiés biotine-UTP permet la capture des hybrides sur des plaques de microtitration portant de la streptavidine. La présence de la modification DIG permet la détection et la quantification des hybrides par ELISA en utilisant des anticorps anti-DIG couplés à la phosphatase alkaline.

Une analyse quantitative de l'expression du gène du récepteur LSR peut aussi être réalisée en utilisant des matrices à ADN, le terme de matrice à ADN désignant un arrangement monodimensionnel, bidimensionnel ou multidimensionnel d'une pluralité d'acides nucléiques présentant une longueur suffisante pour permettre une détection spécifique de l'expression des ARNm capables de s'y hybrider. Par exemple, les matrices à ADN peuvent contenir une pluralité d'acides nucléiques dérivés de gènes dont l'on veut estimer le niveau d'expression. Les matrices à ADN peuvent inclure les séquences génomiques de LSR, celle d'un ADNc de la présente invention, plus particulièrement un ou plusieurs des ADNc de SEQ ID 9, SEQ ID 11 ou SEQ ID 13, toutes séquences complémentaires de celles ci ou tous fragments de celles-ci. De préférence, les fragments comprennent au moins 15, au moins 25, au moins 50, au moins 100 ou au moins 500 nucléotides consécutifs des séquences nucléiques dont ils sont issus.

Par exemple, une analyse quantitative de l'expression du récepteur LSR peut être réalisée avec une matrice à ADN présentant l'ADNc du récepteur LSR comme décrit dans Schena et al. (1995 et 1996). Des ADNc du récepteur LSR ou des fragments de ceux-ci sont amplifiés par PCR et fixés sous forme de matrice à partir d'une microplaque à 96 puits sur une lame de microscope sylatée en utilisant une robotique très rapide. La matrice à ADN ainsi réalisée est incubée dans une chambre humide pour permettre sa réhydratation. Elle est ensuite rinsée une fois dans 0,2 % SDS pendant 1 min, deux fois dans l'eau pendant 1 min et une fois pendant 5 min dans une solution de borohydrure de sodium. La matrice est alors submergée dans l'eau pendant 2 min à 95°C, transférée dans 0,2 % SDS pendant 1 min, rincée deux fois avec de l'eau, séchée et stockée dans l'obscurité à 25°C.

Les ARNm de cellules ou de tissus sont isolés ou obtenus d'une source commerciale, par exemple de la société Clontech. Les sondes sont préparées par un cycle de reverse transcription. Les sondes sont ensuite hybridées à la matrice à ADN de 1 cm² sous une lamelle de 14x14 mm pendant 6-12 heures à 60°C. La matrice est lavée pendant 5 min à 25°C dans un tampon de lavage à basse stringence (1 x SSC/0,2% SDS) puis pendant 10 min à température ambiante dans un tampon hautement stringent (0,1 x SSC/0,2% SDS). La matrice est analysée dans 0,1 x SSC en utilisant un microscope à fluorescence laser avec un jeu de filtres adéquat. Des mesures d'expression différentielle précises sont obtenues en prenant la moyenne des rapports de deux hybridations indépendantes.

Une analyse quantitative de l'expression du récepteur LSR peut également être réalisée avec des ADNc du récepteur LSR ou des fragments de ceux-ci sur des matrices à ADN selon la description de Pietu et al. (1996). Les ADNc du récepteur LSR ou des fragments de ceux ci sont amplifiés par PCR et fixés sur des membranes. Les ARNm provenant de différents tissus ou cellules sont marqués avec des nucléotides radioactifs. Après hybridation et lavage dans des conditions contrôlées, les ARNm hybridés sont détectés avec un Phosphor Imager ou par autoradiographie. Les expériences sont effectuées en double et une analyse quantitative des ARNm différentiellement exprimés peut être effectuée.

De manière alternative, l'analyse de l'expression du récepteur LSR peut être faite avec des matrices à ADN à haute densité comme l'ont décrit Lockhart et al. (1996) et Sosnowski et al. (1997). Des oligonucléotides de 15 à 50 nucléotides, de préférence environ 20 nucléotides, extraits des séquences d'ADN génomique ou d'ADNc du récepteur LSR ou de leurs séquences complémentaires sont synthétisés directement sur une puce ou synthétisés puis adressés sur la puce.

Des sondes d'ADNc de LSR marquées avec un composé approprié tel que la biotine, la digoxigénine ou une molécule fluorescente sont synthétisées à partir d'une population d'ARNm et sont fragmentées en oligonucléotides de 50 à 100 nucléotides en moyenne. Les sondes ainsi obtenues sont alors hybridées à la puce. Après un lavage comme décrit dans Lockhart et al (1996) et une application de différents champs électriques (Sosnowski et al. 1997), les composés marqués sont détectés et quantifiés. Les hybridations sont dupliquées. Une analyse comparative de l'intensité des signaux générés par les sondes sur un même oligonucléotide cible dans différents échantillons d'ADNc indique une expression différentielle des ARNm du récepteur LSR.

Les techniques mentionnées ci-dessus permettent l'analyse des niveaux d'expression du récepteur LSR, dans une même cellule ou un même tissu selon des conditions différentes, par exemple d'induction ou de non induction, mais aussi l'analyse de la spécificité tissulaire de cette expression, dans des conditions qui peuvent également varier. On pourra grâce à ces techniques analyser l'expression de l'une ou l'autre des sous-unités du récepteur LSR, et plus généralement de différentes formes issues d'épissage alternatif, en définissant les sondes de façon adéquate.

L'effet de composés candidats à la modulation du niveau ou de la spécificité de l'expression, ou de l'épissage des différentes formes du récepteur LSR, pourra ainsi être analysé à large échelle en exposant les cellules sources d'ARN messager, notamment les cellules modèles selon l'invention, qu'elles expriment le LSR naturellement ou que ce soit des cellules recombinantes, auxdits composés candidats.

### Criblage de composés interagissant avec le récepteur LSR

Un autre aspect de la présente invention consiste en des méthodes d'identification de molécules capables de se lier au récepteur LSR. De telles molécules peuvent être utilisées pour moduler l'activité du récepteur LSR. Par exemple, de telles molécules peuvent être utilisées pour stimuler ou réduire la dégradation des lipoprotéines, de préférence de lipoprotéines riches en triglycérides, ou de cytokines, de préférence de la leptine. De telles molécules peuvent également être utilisées pour inhiber l'activation par la leptine ou l'activation par les acides gras libres de l'activité LSR.

Il existe de nombreuses méthodes pour identifier des ligands du récepteur LSR. L'une de ces méthodes est décrite dans le brevet US 5,270,170, dont l'enseignement est incorporé par référence. Brièvement, on construit une banque de peptides aléatoires, comprenant une pluralité de vecteurs codant chacun pour une fusion entre un peptide candidat pour une fixation au récepteur LSR, et une protéine se fixant à l'ADN telle que le répresseur Lac codé par le gène lacl. Les vecteurs de la banque de peptides aléatoires contiennent aussi des sites de fixation pour les protéines se fixant à l'ADN tels que le site LacO lorsque la protéine est le répresseur Lac. La banque de peptides aléatoires est introduite dans une cellule hôte dans laquelle la protéine de fusion est exprimée. La cellule hôte est ensuite lysée dans des conditions permettant la fixation de la protéine de fusion aux sites du vecteur.

Les vecteurs ayant fixé la protéine de fusion sont mis en contact avec le récepteur LSR immobilisé, une sous-unité du récepteur LSR immobilisée ou un fragment du récepteur LSR immobilisé dans des conditions permettant aux peptides de se fixer spécifiquement. Par exemple, le récepteur LSR, une sous-unité de celui-ci ou un fragment de celui-ci comprenant au moins 10, au moins 20, au moins 30, ou plus de 30 acides aminés consécutifs peut être immobilisé par fixation à une surface telle qu'une plaque ou une particule plastique.

Les vecteurs qui codent pour les peptides capables de se fixer au récepteur LSR seront spécifiquement retenus sur la surface par des interactions entre le peptide et le récepteur LSR, une sous-unité du récepteur ou un fragment de celui-ci.

De manière alternative, des molécules capables d'interagir avec le récepteur LSR peuvent être identifiées en utilisant un système de double hybrides tel que le Matchmaker Two Hybrid System 2. Selon les instructions du manuel accompagnant le Matchmaker Two Hybrid System 2 (Catalogue N° K1604-1, Clontech), dont l'enseignement est incorporé par référence, les acides nucléiques codant pour le récepteur LSR, une sous-unité de celui-ci ou un fragment de celui-ci comprenant au moins 10, au moins 20, au moins 30, ou plus de 30 acides aminés consécutifs sont insérés dans un vecteur d'expression de manière à ce qu'ils soient en phase avec l'ADN codant pour le domaine de liaison à l'ADN de l'activateur de transcription de levure GAL4. Les acides nucléiques d'une banque codant pour des protéines ou des peptides susceptibles d'interagir avec le récepteur LSR sont insérés dans un deuxième vecteur d'expression de manière à ce qu'ils soient en phase avec l'ADN codant pour le domaine d'activation de l'activateur GAL4. Les levures sont transformées par les deux plasmides d'expression et elles sont placées dans un milieu permettant de sélectionner les cellules exprimant des marqueurs contenus dans chacun des vecteurs ainsi que celles exprimant le gène HIS3 dont l'expression est dépendante de GAL4. Les cellules transformées capables de pousser sur un milieu dénué d'histidine sont analysées pour l'expression de LacZ sous dépendance de GAL4. Les cellules qui poussent en l'absence d'histidine et expriment LacZ contiennent un plasmide qui codent pour des protéines ou des peptides qui interagissent avec le récepteur LSR, une sous-unité de celui-ci ou un fragment de celui-ci comprenant au moins 10, au moins 20, au moins 30, ou plus de 30 acides aminés consécutifs de ceux-ci.

Pour étudier l'interaction du récepteur LSR, d'une sous-unité de celui-ci ou d'un fragment de celui-ci comprenant au moins 10, au moins 20, au moins 30, ou plus de 30 acides aminés consécutifs avec des petites molécules telles que celles générées par chimie combinatoire, il est possible d'utiliser une microdialyse couplée à une HPLC comme décrit dans Wang et al. (1997), ou une électrophorèse capillaire d'affinité comme décrit dans Busch et al. (1997), l'enseignement des ces documents étant incorporé par référence.

Dans d'autres méthodes, les peptides ou petites molécules susceptibles d'interagir avec le récepteur LSR, une sous-unité de celui-ci ou un fragment de celui-ci comprenant au moins 10, au moins 20, au moins 30, ou plus de 30 acides aminés consécutifs peuvent être liés à des marqueurs détectables telles que des marqueurs radioactifs, fluorescents ou enzymatiques. Ces molécules marquées sont mises en contact avec le récepteur LSR immobilisé, une sous-unité de celui-ci immobilisée ou un fragment de celui-ci immobilisé comprenant au moins 10, au moins 20, au moins 30, ou plus de 30 acides aminés consécutifs dans des conditions permettant une interaction spécifique. Après élimination des molécules non-fixées spécifiquement, les molécules liées sont détectées par des moyens appropriés.

Ces méthodes pourront notamment permettre l'identification d'acides gras ou analogues capables de se fixer au site de fixation des acides gras sur le LSR, de lipoprotéines ou analogues, capables de se fixer au site de fixation des lipoprotéines sur le récepteur LSR, de dérivés de la leptine ou analogues capables de se lier au site de fixation de la leptine sur le LSR, et de dérivés du récepteur gC1qR ou analogues capables de se fixer au site de fixation de gC1qR sur le LSR.

En outre, les peptides ou les petites molécules qui se lient au LSR, de préférence aux sites de fixation sur le récepteur LSR des acides gras, des lipoprotéines, des cytokines, notamment de la leptine, ou de gC1qR ou une de ses protéines analogues, peuvent être identifiés par des expériences de compétition. Dans de telles expériences, le récepteur LSR, une sous-unité de celui-ci ou un fragment de celui-ci comprenant au moins 10, au moins 20, au moins 30, ou plus de 30 acides aminés consécutifs est immobilisé sur une surface telle qu'un support plastique. Des quantités croissantes de peptides ou de petites molécules sont mises en contact avec le récepteur LSR immobilisé, une sous-unité de celui-ci immobilisée ou un fragment de celui-ci immobilisé comprenant au moins 10, au moins 20, au moins 30, ou plus de 30 acides aminés consécutifs en présence du ligand marqué du récepteur, ce ligand pouvant être par exemple la leptine, l'oléate, les LDL ou gC1qR. Le ligand du récepteur LSR peut être marqué avec un marqueur radioactif, fluorescent ou enzymatique. La capacité de la molécule testée à interagir avec le récepteur LSR, une sous-unité de celui-ci ou un fragment de celui-ci comprenant au moins 10, au moins 20, au moins 30, ou plus de 30 acides aminés consécutifs est déterminée par mesure de la quantité de ligand marqué liée en présence de la molécule testée. Une diminution de la quantité de ligand liée quand la molécule testée est présente indique que celle-ci est capable d'interagir avec le récepteur LSR, une sous-unité de celui-ci ou un fragment de celui-ci comprenant au moins 10, au moins 20, au moins 30, ou plus de 30 acides aminés consécutifs.

Ces méthodes pourront notamment permettre l'identification d'acides gras ou analogues capables de se fixer au site de fixation des acides gras sur le LSR, de lipoprotéines ou analogues, capables de se fixer au site de fixation des lipoprotéines sur le récepteur LSR, de dérivés de la leptine ou analogues capables de se lier au site de fixation de la leptine sur le LSR, et de dérivés du récepteur gC1qR ou analogues capables de se fixer au site de fixation de gC1qR sur le LSR. On pourra particulièrement mesurer la capacité de tels composés, ou de tout autre composé candidat, à concurrencer la liaison d'oléates, de lipoprotéines, de leptine, ou de gC1qR au LSR.

La technologie du BIACORE peut également être utilisée pour effectuer le criblage de composés capables d'interagir avec le récepteur LSR. Cette technologie est décrite dans Szabo et al. (1995) et dans Edwards et Leartherbarrow (1997), dont l'enseignement est incorporé par référence, et permet de détecter des interactions entre molécules en temps réel sans utilisation de marquage. Elle est basée sur le phénomène de SPR (surface plasmon resonance). Brièvement, la molécule à analyser est fixée sur une surface (utilisant typiquement une matrice de carboxymethyl dextran). Un rayon lumineux est dirigé sur la face de la surface qui ne contient pas l'échantillon et est réfléchi par celle-ci. Le phénomène de SPR provoque une réduction de l'intensité de la lumière réfléchie avec une combinaison spécifique d'angle et de longueur d'onde. Les événements de fixation de molécules provoquent un changement de l'indice de réfraction à la surface qui est détecté comme une modification du signal SPR. Pour faire un criblage de composés capables d'interagir avec le récepteur LSR, on immobilise sur une surface le récepteur LSR, une sous-unité de celui-ci ou un fragment de celui-ci comprenant au moins 10, au moins 20, au moins 30, ou plus de 30 acides aminés consécutifs. Cette surface constitue une face d'une cellule dans laquelle passe la molécule à tester. La fixation de la molécule sur le récepteur LSR, une sous-unité de celui-ci ou un fragment de celui-ci comprenant au moins 10, au moins 20, au moins 30, ou plus de 30 acides aminés consécutifs est détectée par un changement du signal SPR. Les molécules testées peuvent être des protéines, des peptides, des hydrates de carbones, des lipides ou des petites molécules générées, par exemple, par chimie combinatoire. Les protéines candidates peuvent être extraites de tous tissus, provenant de toutes espèces. La technologie du BIACORE peut également être utilisée en immobilisant des cellules eucaryotes ou procaryotes ou des vésicules lipidiques présentant un récepteur LSR endogène ou recombinant à leur surface.

Un des principaux avantages de cette méthode est qu'elle permet la détermination des constantes d'association entre le récepteur LSR et les molécules interagissant. Ainsi, il est possible de sélectionner spécifiquement les molécules interagissant avec de fortes ou de faibles constantes d'association.

Les protéines ou autres molécules interagissant avec le récepteur LSR, une sous-unité de celui-ci ou un fragment de celui-ci comprenant au moins 10, au moins 20, au moins 30, ou plus de 30 acides aminés consécutifs peuvent être identifiées en utilisant des colonnes d'affinité qui contiennent le récepteur LSR, une sous-unité de celui-ci ou un fragment de celui-ci comprenant au moins 10, au moins 20, au moins 30, ou plus de 30 acides aminés consécutifs. Le récepteur LSR, une sous-unité de celui-ci ou un fragment de celui-ci comprenant au moins 10, au moins 20, au moins 30, ou plus de 30 acides aminés consécutifs peut être attaché à la colonne en utilisant des techniques conventionnelles incluant le couplage chimique à une matrice de colonne appropriée telle que l'agarose, Affi Gel, ou d'autres matrices connues de l'homme de l'art. Dans un autre aspect de l'invention, la colonne d'affinité peut contenir des protéines chimériques dans lesquelles le récepteur LSR, une sous-unité de celui-ci ou un fragment de celui-ci comprenant au moins 10, au moins 20, au moins 30, ou plus de 30 acides aminés consécutifs serait fusionné par exemple avec la gluthation S-transférase. Les molécules à tester décrites ci-dessus sont ensuite déposées sur la colonne. Les molécules interagissant avec le récepteur LSR, une sous-unité de celui-ci ou un fragment de celui-ci comprenant au moins 10, au moins 20, au moins 30, ou plus de 30 acides aminés consécutifs sont retenues par la colonne et peuvent être isolées par élution. Dans le cas où les molécules testées sont des protéines, elles peuvent ensuite être analysées sur un gel d'électrophorèse 2-D comme décrit dans Ramunsen et al. (1997), dont l'enseignement est incorporée par référence. De manière alternative, les protéines ou les autres molécules retenues par la colonne d'affinité peuvent être purifiées par électrophorèse et séquencées. Une méthode similaire peut être utilisée pour isoler des anticorps, pour cribler des produits de « phage display » ou des anticorps humains issus de « phage display ».

### Criblage de composés interagissant avec les séquences promotrices et/ou régulatrices de récepteur LSR

L'invention concerne également une méthode de criblage de composés interagissant avec les séquences promotrices et/ou régulatrices du récepteur LSR.

Les acides nucléiques codant pour des protéines interagissant avec les séquences promotrices et/ou régulatrices du gène du récepteur LSR, plus particulièrement une séquence nucléotidique correspondant aux nucléotides 1 à 1897 de SEQ ID 19 ou un fragment de celle-ci, peuvent être identifiés en utilisant un système de simple hybride tel que celui décrit dans le manuel accompagnant le kit Matchmaker One-Hybrid System de Clontech (Catalog N° K1603-1), l'enseignement duquel est incorporé par référence. Brièvement, la séquence nucléotidique cible est clonée en amont d'un gène marqueur sélectionnable et intégrée dans un génome de levure. Les levures contenant le gène marqueur intégré sont transformées par une banque contenant des fusions entre des ADNc codant pour des protéines candidates à la fixation sur les régions promotrices et/ou régulatrices du gène du récepteur LSR et le domaine activateur d'un facteur de transcription de levure tel que GAL4. Les levures sont placées dans un milieu permettant de sélectionner les cellules exprimant le gène marqueur. Les levures sélectionnées contiennent une protéine de fusion capable de se fixer sur la région cible promotrice et/ou régulatrice. Les ADNc des gènes codant pour les protéines de fusion sont ensuite séquences. Les inserts correspondants peuvent ensuite être clonées dans des vecteurs d'expression ou de transcription in vitro. La liaison des polypeptides ainsi codés aux séquences cibles de promoteur peut être confirmée par des techniques familères de l'homme de l'art, dont les expériences de retard sur gel ou de protection à la DNAse.

Le criblage de composés capables de modifier l'expression du récepteur LSR en se fixant à ses séquences régulatrices et /ou promotrices peut aussi être réalisé à l'aide de gènes « reporter ». Par exemple, une région génomique située en 5' de la séquence codante du récepteur LSR, plus particulièrement une séquence nucléotidique correspondant aux nucléotides 1 à 1897 de SEQ ID 19 ou un fragment de celle-ci, peut être clonée dans un vecteur tel que pSEAP-Basic, pSEAP-Enhancer, pβgal-Basic, pβgal-Enhancer, ou pEGFP-1 disponibles chez Clontech. Brièvement, chacun de ces vecteurs contient des sites multiples de clonage situés en amont d'un gène marqueur codant pour une protéine facilement détectable telle que la phosphatase alkaline, la β galactosidase, ou la GFP (green fluorescent protein). Après insertion de la région génomique située en 5' de la séquence codante du récepteur LSR, plus particulièrement une séquence nucléotidique correspondant aux nucléotides 1 à 1897 de SEQ ID 19 ou un fragment de celle-ci, le niveau d'expression des protéines marqueurs est mesuré et comparé avec un vecteur ne contenant pas d'insert. L'effet de composés candidats sur l'expression issue des séquences régulatrices et/ou promotrices du LSR peut ainsi être évalué.

Le criblage des composés capables de se fixer sur les régions régulatrices et/ou promotrices du gène du récepteur LSR peut également être effectué par des expériences de retard sur gel bien-connues de l'homme de l'art et décrites dans Fried et Crothers (1981), Garner et Revzin (1981) et Dent et Latchman (1993), dont l'enseignement est incorporé par référence. Ces expériences sont basées sur le principe qu'un fragment ADN lié à une protéine migre plus lentement que le même fragment sans protéine. Brièvement, la séquence nucléotidique cible est marquée. Puis elle est mise en présence soit d'un extrait nucléaire ou total de cellules préparé de manière à contenir les facteurs de transcription, soit de différents composés à tester. L'interaction entre la région régulatrice et/ou promotrice du gène du récepteur LSR et le composé ou facteur de transcription est détectée après électrophorèse par un retard de migration.

### Composés

Les composés chimiques ou biochimiques, caractérisés en ce qu'ils permettent de moduler l'expression ou l'activité du récepteur selon l'invention, font également partie de l'invention.

Les composés chimiques ou biochimiques, caractérisés en ce qu'il sont capables d'interagir, directement ou indirectement, avec le récepteur selon l'invention, font partie de l'invention.
Les composés chimiques ou biochimiques caractérisés en ce qu'ils sont sélectionnés par lesdites méthodes définies ci-dessus font également partie de l'invention.

En particulier, parmi ces composés selon l'invention, on préfère une leptine ou un de ses composés dérivés, de préférence un de ses variants protéiques, ou des leptines modifiées chimiquement ou obtenues par recombinaison génétique, ou un de leurs fragments.

On entend désigner par composés permettant de moduler l'expression ou l'activité du récepteur, les composés qui permettent notamment de réduire, de stabiliser ou d'augmenter le nombre, la vitesse de recyclage et/ou le changement de conformation du récepteur selon l'invention, ou, de favoriser ou d'inhiber l'activité globale ou l'activité d'un des domaines dudit récepteur ou encore de rétablir l'expression normale dudit récepteur dans le cas, par exemple, où une anomalie génétique est constatée. Ces composés pourront par exemple interagir en tant que ligands spécifiques dudit récepteur ou d'un de ses domaines à titre de cofacteur, ou d'inhibiteur, notamment compétitif, ou encore ayant une activité agoniste ou antagoniste sur les changements conformationnels du complexe. Ces composés pourront également interagir en neutralisant les ligands spécifiques naturels dudit récepteur et en inhibant ainsi l'activité du récepteur induite par ces ligands.

Parmi ces composés, on préfère les composés permettant de moduler le nombre de polypeptides dudit récepteur, sa vitesse de recyclage et/ou la sélectivité de leur activité.

Sont également préférés les composés selon l'invention, caractérisés en ce qu'ils permettent une augmentation de l'activité totale ou de l'expression du récepteur selon l'invention, et/ou une augmentation spécifique de l'activité de clairance aux cytokines, notamment à la leptine, dudit récepteur, et/ou une augmentation spécifique de l'activité de clairance aux lipoprotéines, dudit récepteur.

On préfère également les composés caractérisés en ce qu'ils permettent une diminution de l'activité totale ou de l'expression du récepteur selon l'invention, et/ou une diminution spécifique de l'activité de clairance aux cytokines, notamment à la leptine, dudit récepteur, et/ou une diminution spécifique de l'activité de clairance aux lipoprotéines, dudit récepteur.

Egalement préférés sont les composés caractérisés en ce qu'ils permettent une modulation de l'élimination des cytokines, notamment de la leptine, et/ou une modulation de l'élimination des lipoprotéines, des résidus de chylomicrons, et/ou des triglycérides.

L'invention comprend également les composés selon l'invention, caractérisés en ce qu'ils permettent une modulation du taux de cytokines, notamment de la leptinémie, et/ou une modulation du taux des lipoprotéines, des résidus de chylomicrons, et/ou des triglycérides.

On préfère plus particulièrement les composés selon l'invention, caractérisés en ce qu'ils permettent un contrôle du taux de cytokines, notamment de la leptinémie.

De façon également préférée, l'invention comprend les composés selon l'invention, caractérisés en ce qu'ils permettent un contrôle, de préférence une diminution, du taux de lipoprotéines, une diminution de la concentration plasmatique de résidus de chylomicrons, et/ou une diminution de la triglycéridémie.

Parmi les composés les plus préférés, on préfère ceux, caractérisés en ce qu'ils sont choisis parmi :
a) un anticorps selon l'invention ;
b) un polypeptide selon l'invention ;
c) un polypeptide selon l'invention, caractérisé en ce qu'il correspond à une forme soluble du récepteur selon l'invention ;
d) un vecteur selon l'invention ;
e) un vecteur selon l'invention, caractérisé en ce qu'il présente à sa surface extérieure un site de reconnaissance spécifique des cellules hépatiques ;
f) un vecteur selon l'invention, caractérisé en ce que le produit d'expression de l'acide nucléique inséré par le vecteur dans la cellule cible, est, soit ancré dans ou soit excrété par ladite cellule cible transformée ;
g) un oligonucléotide sens ou anti-sens selon l'invention ;
h) une leptine, ou un de ses variants protéiques, ou une leptine modifiée chimiquement ou par recombinaison génétique, ou un de leurs fragments.

L'invention concerne enfin les composés selon l'invention à titre de médicament.

On préfère notamment les composés selon l'invention à titre de médicament pour la prévention et/ou le traitement de pathologies et/ou de pathogénies liées aux troubles du comportement alimentaire.

On préfère également les composés selon l'invention à titre de médicament pour la prévention et/ou le traitement de pathologies et/ou de pathogénies liées aux troubles du métabolisme des cytokines.

De façon préférée, l'invention concerne également les composés selon l'invention à titre de médicament pour la prévention ou le traitement de l'obésité ou de l'anorexie.

Les composés selon l'invention, à titre de médicament pour la prévention et/ou le traitement de pathologies et/ou de pathogénies associées à, ou induites par l'obésité, sont les composés préférés.

En particulier, on préfère les composés selon l'invention, à titre de médicament pour la prévention et/ou le traitement de l'insuffisance cardiaque, de l'insuffisance coronarienne, des accidents vasculaires cérébraux, de la maladie athéromateuse, de l'athérosclérose, de l'hypertension artérielle, du diabète non insuline-dépendant, de l'hyperlipidémie et/ou de l'hyperuricémie.

Les plus préférés sont les composés selon l'invention, à titre de médicament pour la prévention et/ou le traitement de la maladie athéromateuse et/ou de l'athérosclérose.

Enfin, l'invention comprend des composés selon l'invention pour la prévention et/ou le traitement par thérapie génique, de pathologies et/ou de pathogénies liées aux troubles du comportement alimentaire, de l'obésité et/ou de pathologies et/ou de pathogénies associées à, ou induites par, l'obésité.

Les composés de l'invention en tant que principes actifs de médicament, seront préférentiellement sous forme soluble, associés à un véhicule pharmaceutiquement acceptable.

De tels composés utilisables à titre de médicament offrent une nouvelle approche pour prévenir et/ou traiter les pathologies et/ou pathogénies liées aux troubles du comportement alimentaire telles que l'obésité ou l'anorexie, et les risques et/ou complications associés.

De préférence, ces composés seront administrés par voie systémique, en particulier par voie intraveineuse, par voie intramusculaire, intradermique ou par voie orale.

Leurs modes d'administration, posologies et formes galéniques optimaux peuvent être déterminés selon les critères généralement pris en compte dans l'établissement d'un traitement adapté à un patient comme par exemple l'âge ou le poids corporel du patient, la gravité de son état général, la tolérance au traitement et les effets secondaires constatés, etc...

Comme mentionné précédemment, selon les cas, il pourra convenir d'amplifier l'activité du LSR, en promouvant par exemple l'expression de ses gènes ou en augmentant l'activité de leurs produits d'expression, dans les cas pathologiques provenant du fait que l'un au moins de ces gènes n'est pas exprimé, insuffisamment exprimé ou exprimé sous une forme anormale qui ne permet pas au produit d'expression de remplir ses fonctions, ou au contraire de réprimer une surexpression, ou une expression anormale de ces gènes. Il convient donc de pallier de façon générale la carence ou la surexpression de produits d'expression de ce gène par une thérapie dite «de remplacement » permettant l'amplification ou la diminution des activités du complexe LSR.

La thérapie de remplacement pourra être effectuée par thérapie génique, c'est-à-dire en introduisant les séquences d'acide nucléique selon l'invention et/ou les gènes correspondants avec les éléments qui permettent leur expression in vivo, dans le cas où l'un des gènes est insuffisamment exprimé par exemple, ou bien lorsqu'il est exprimé sous une forme anormale.

Les principes de la thérapie génique sont connus. On peut utiliser des vecteurs viraux selon l'invention ; il est également possible de prévoir des vecteurs non viraux, c'est-à-dire synthétiques, qui miment des séquences virales ou bien qui sont constitués par de l'ADN ou de l'ARN nu selon la technique développée notamment par la société VICAL.

Dans la plupart des cas, il faudra prévoir des éléments de ciblage assurant une expression spécifique du foie de façon à pouvoir limiter les zones d'expression des protéines qui restent impliquées dans la clairance de la leptine et celle des lipoprotéines. Il est même intéressant, dans certains cas, d'avoir des vecteurs d'expression transitoire ou au moins d'expression contrôlée que l'on pourra bloquer lorsque cela sera nécessaire.

D'autres caractéristiques et avantages de l'invention apparaissent dans la suite de la description avec les exemples et les figures dont les légendes sont représentées ci-après.

### LEGENDE DES FIGURES

**FIGURE 1** : Représentation schématique des trois formes de la protéine LSR de rat : LSR 66 (sous-unité α), LSR 64 (sous-unité α'), et LSR 58 (sous-unité β).
**FIGURE 2** : Alignement des séquences protéiques des formes longues (sous-unités α) du LSR humain (LSR1.Hs), de rat (LSR1.Rn) et de souris (LSR1.Mm). Les symboles (*) placés sous les alignements indiquent les acides aminés conservés, les symboles (.) indiquent les substitutions conservatives d'acides aminés. Encadré, de l'extrémité NH2-terminale vers l'extrémité COOH-terminale, le site potentiel de fixation des acides gras (FFA) encadré, le site de liaison à la clathrine [NPGY], le consensus d'adressage lysosomal : di-leucine LI-X10-LL, le domaine transmembranaire TM surligné, le motif [RSRS], le site de fixation potentiel des lipoprotéines (+-+-) encadré. Surligné, la signature du récepteur du TNF avec (flèche) ; indiqués, les acides aminés conservés dans la signature. Le domaine transmembranaire est situé entre le dernier di-leucine et la signature du TNF.
**FIGURE 3** : Alignement des séquences protéiques des trois types de sous-unités du LSR humain(α : LSR1.Hs ; α' : LSR2.Hs ; β : LSR3.Hs). La signification des symboles, des encadrés et des surlignés est la même que celle de la figure 2.
**FIGURE 4** : Alignement des séquences protéiques des trois types de sous-unités du LSR de rat. La signification des symboles, des encadrés et des surlignés est la même que celle de la figure 2.
**FIGURE 5** : Alignement des séquences protéiques des trois types de sous-unités du LSR de souris. La signification des symboles, des encadrés et des surlignés est la même que celle de la figure 2.
**FIGURE 6 :** Représentation schématique des trois formes de LSR mises en évidence chez l'homme, indiquant les motifs conservés sur chacune d'elle.
   **A** : Représentation schématique de l'organisation génomique du LSR humain à partir du premier exon codant. Les exons sont indiqués par des boîtes, les introns par des barres interrompues. La taille en nucléotides des exons et des introns est indiquée au dessus de ceux-ci. Les éléments caractérisant le messager et la protéine codée sont reportés sur cette figure. La cartouche sur la droite donne la signification des symboles utilisés.
   **B** : Structure de la forme LSR-Hs-2062 de LSR humain. Cette forme encode une protéine de 649 acides aminés.
   **C** : Structure de la forme LSR-Hs-2005 de LSR humain. Cette forme encode une protéine de 630 acides aminés.
   **D** : Structure de la forme LSR-Hs-1858 de LSR humain. Cette forme encode une protéine de 581 acides aminés.
**FIGURE 7** : Alignement des séquences nucléotidiques des formes longues de cDNA (codant pour la sous-unité α) du LSR humain (Isr1.Hs), de rat (Isr1.rRn) et de souris (Isr1.Mm). Les nucléotides conservés dans les trois séquences sont repérés par un signe * placé sous les séquences. Des tirets sont ajoutés à l'intérieur des séquences lorsque l'alignement optimal des séquences ne peut se faire sans créer des microdélétions.

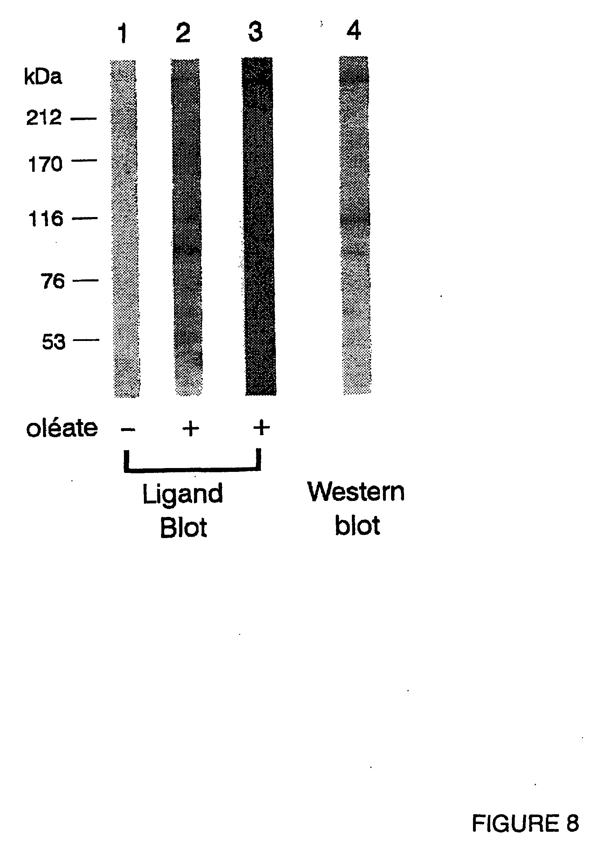

**FIGURE 8** : Identification du récepteur LSR par ligand et Western blotting sur des protéines solubilisées de membranes de foie de rat (lignes 1, 2 et 4), ou sur la protéine partiellement purifiée de 240 kD (ligne 3).
   Lignes 1, 2 et 3 : Ligand blotting. Ligne 1 : en absence d'oléate et de ¹²⁵I-LDL; ligne 2 : en présence d'oléate et de ¹²⁵I-LDL; ligne 3 : en présence d'oléate et de ¹²⁵I-LDL.
   Ligne 4 : Western blotting avec des anticorps anti-LSR.

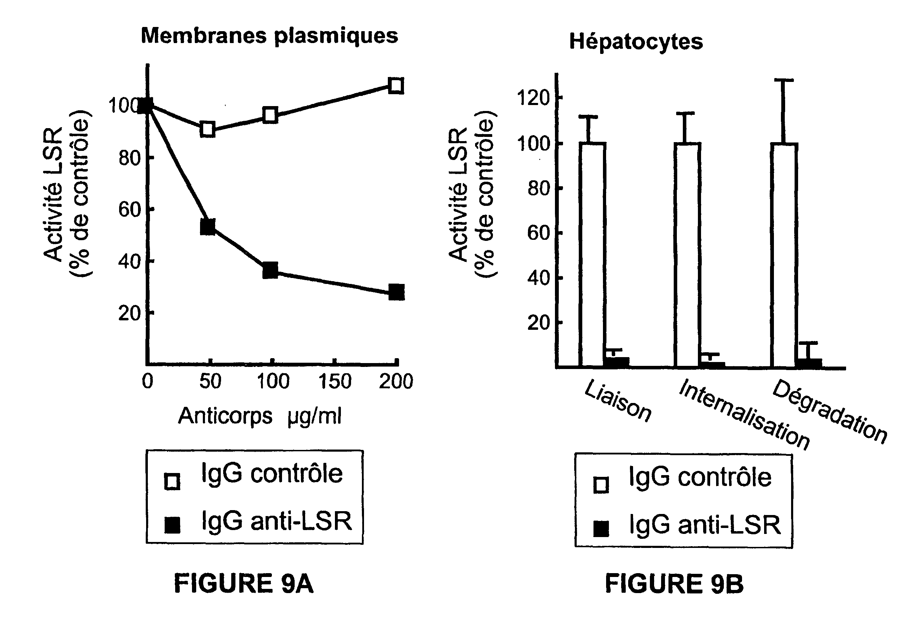

**FIGURE 9** : Effet d'anticorps anti-LSR sur l'activité LSR.
   **A.** Fixation de ¹²⁵I-LDL sur des membranes plasmiques d'hépatocytes de rat en présence d'oléate et de concentrations croissantes d'anticorps anti-LSR (■) ou d'anticorps contrôle (□), exprimée en % de la quantité totale de ¹²⁵I-LDL fixée en absence d'anticorps.
   **B.** Fixation, incorporation et dégradation de ¹²⁵I-LDL dans des hépatocytes de rat en culture primaire en présence d'oléate et d'anticorps anti-LSR (■) ou d'anticorps contrôle (Q), exprimées respectivement en % de la fixation, de l'incorporation et de la dégradation totale de ¹²⁵I-LDL en présence d'anticorps non spécifiques.

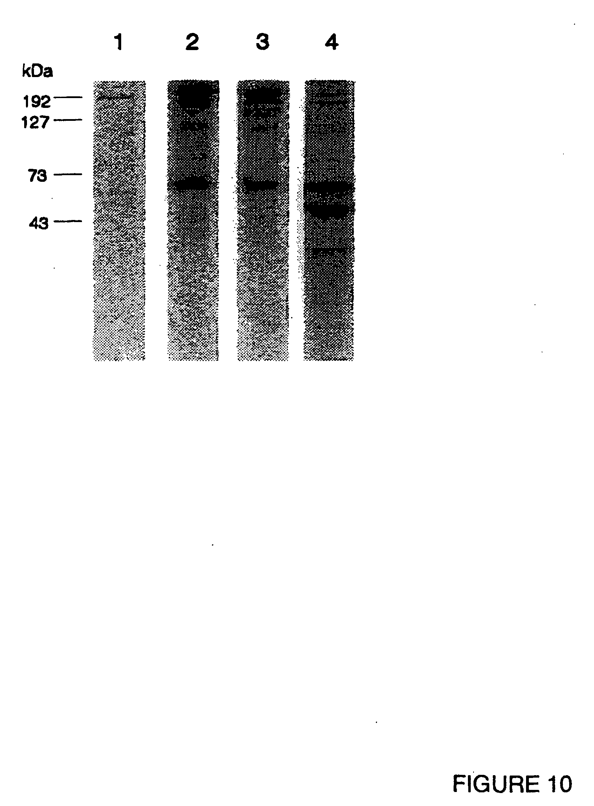

**FIGURE 10** : Identification du récepteur LSR par immuno-précipitation de lysats d'hépatocytes marqués à la ³⁵S méthionine et ³⁵S cystéine, en présence d'anticorps contrôle (ligne 1), ou d'anticorps anti-LSR (lignes 2 à 4), après séparation par élecrophorèse en conditions non-réductrices (lignes 2 et 3) ou réductrices (ligne 1 et 3).

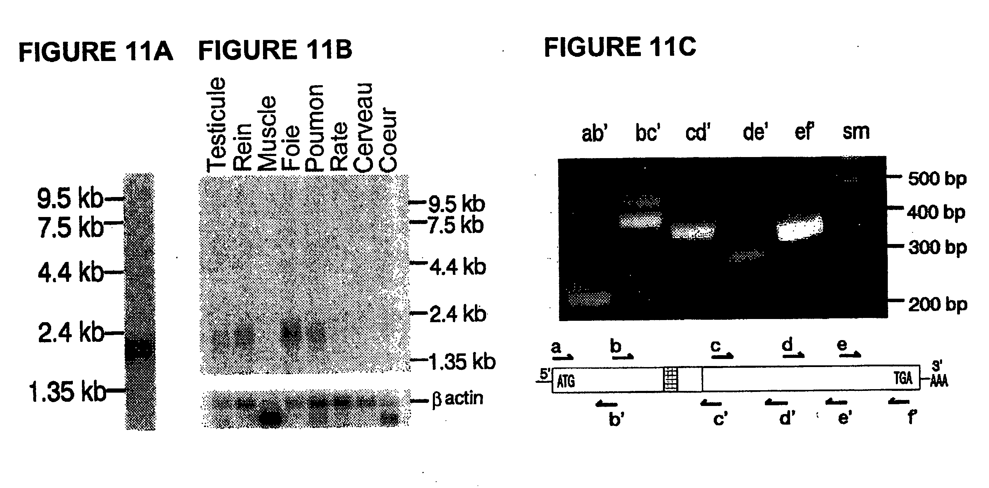

**FIGURE 11** : Clonage du cDNA codant pour le LSR α et β.
   **A.** Analyse en Northem blot montrant plusieurs tailles de l'ARN messager de LSR.
   **B.** Analyse en Northern blot multi-tissulaire de l'ARNm de LSR avec une sonde spécifique du LSR et une sonde contrôle spécifique de la β-actine.
   **C.** Analyse de l'ARNm du LSR en RT-PCR utilisant 5 couples d'amorces couvrant la séquence entière et mise en évidence de trois formes issues d'épissage alternatif dans le fragment d'amplification obtenu grâce au couple d'amorces bc'. Le schéma représente les résultats d'analyse de séquence des trois formes correspondantes d'ADNc de LSR : la région quadrillée est absente des deux formes courtes, la région hachurée est absente uniquement de la forme la plus courte.

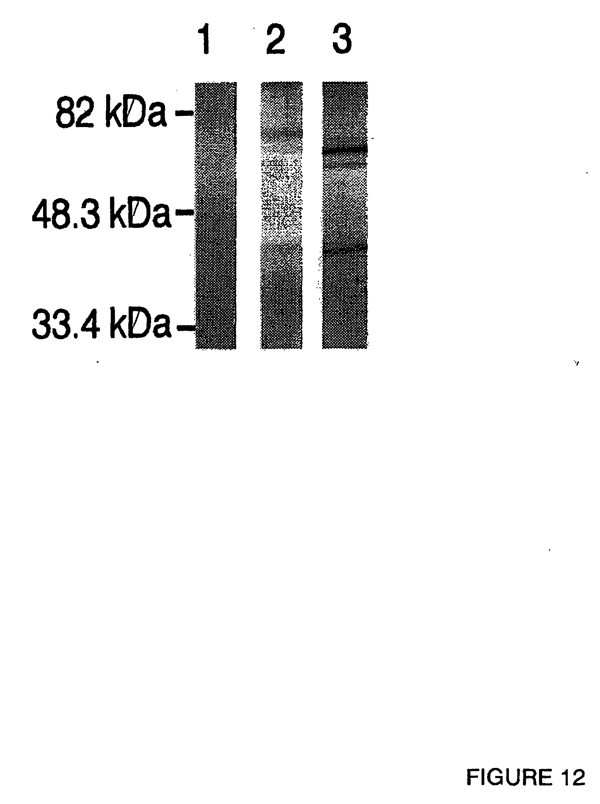

**FIGURE 12** : Traduction *in vitro* des deux cDNAs complets codant pour les formes la plus longue (66 kDa, ligne 2) et la plus courte (58 kDa, ligne 3) du LSR de rat, et d'un cDNA contrôle, anti-sens du cDNA codant pour la forme la plus longue du LSR (ligne 1).
   Les produits de traduction *in vitro,* marqués à la ³⁵S-méthionine, sont analysés après électrophorèse en conditions non réduites.

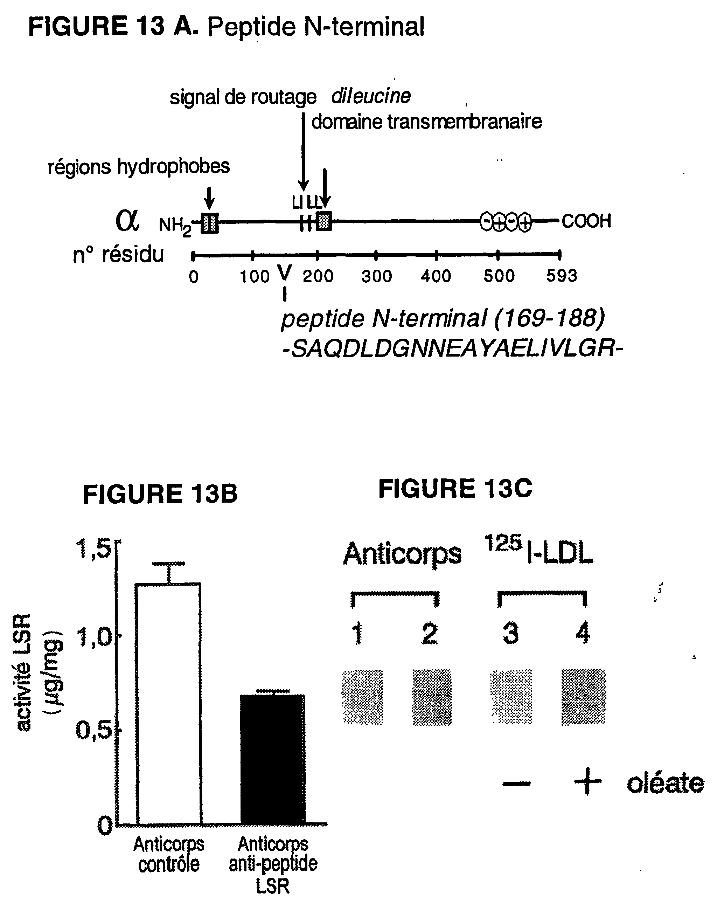

**FIGURE 13** : identification des sous-unités LSR α et β comme responsables de l'activité LSR.
   **A.** Schéma montrant la localisation et la séquence du peptide N-terminal de LSR employé pour générer des anticorps anti-peptide LSR.
   **B.** Western et Ligand blotting des sous-unités α et β du LSR. Le western blotting est conduit en utilisant l'anticorps anti-LSR (ligne 1) ou anti-peptide LSR (ligne 2). Le ligand blotting est conduit en présence de ¹²⁵I-LDL, avec (ligne 4) ou sans (ligne 3) oléate.
   **C.** Effet d'anticorps dirigés contre un peptide LSR synthétique sur l'activité LSR de membranes plasmiques de foie de rat. L'activité LSR est mesurée en présence d'un anticorps contrôle (□) ou de l'anticorps anti-peptide LSR (■).

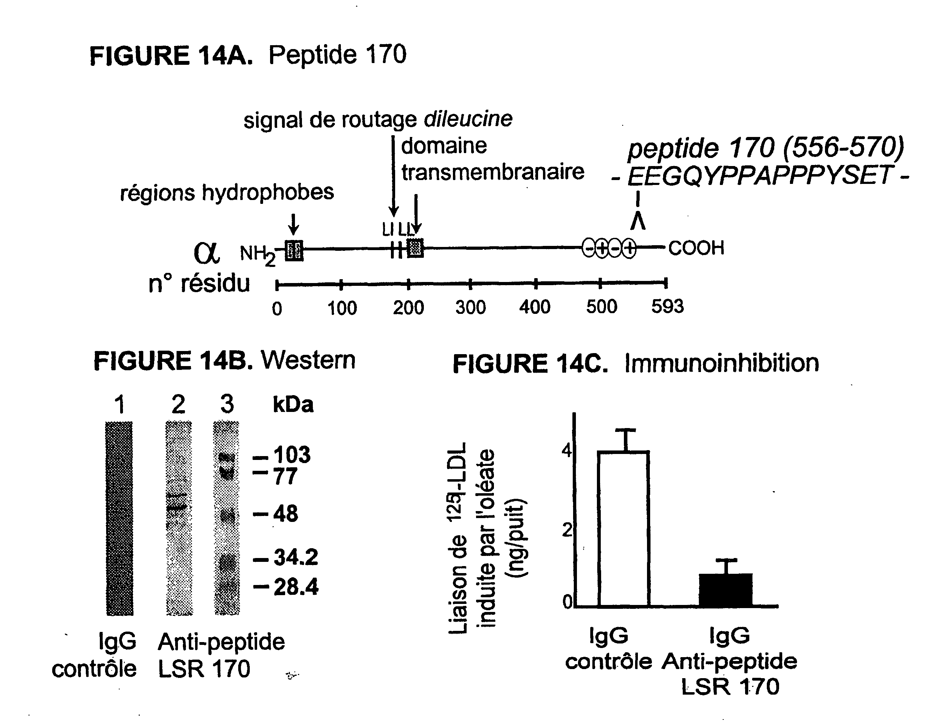

**FIGURE 14** : Identification des sous-unités du récepteur LSR et effet inhibiteur d'anticorps dirigés contre un peptide synthétique C-terminal issu de LSR.
   **A-** Schéma montrant la localisation et la séquence du peptide synthétique 170.
   **B-** Western blotting de lysats d'hépatocytes de rat utilisant les anticorps dirigés contre le peptide synthétique 170 (ligne 2), ou un anticorps contrôle (ligne 1); Ligne 3 : marqueurs de poids moléculaire.
   **C-** Fixation de ¹²⁵ I-LDL par le récepteur LSR en présence d'oléate et d'anticorps contrôle ou dirigés contre le peptide 170 de LSR.

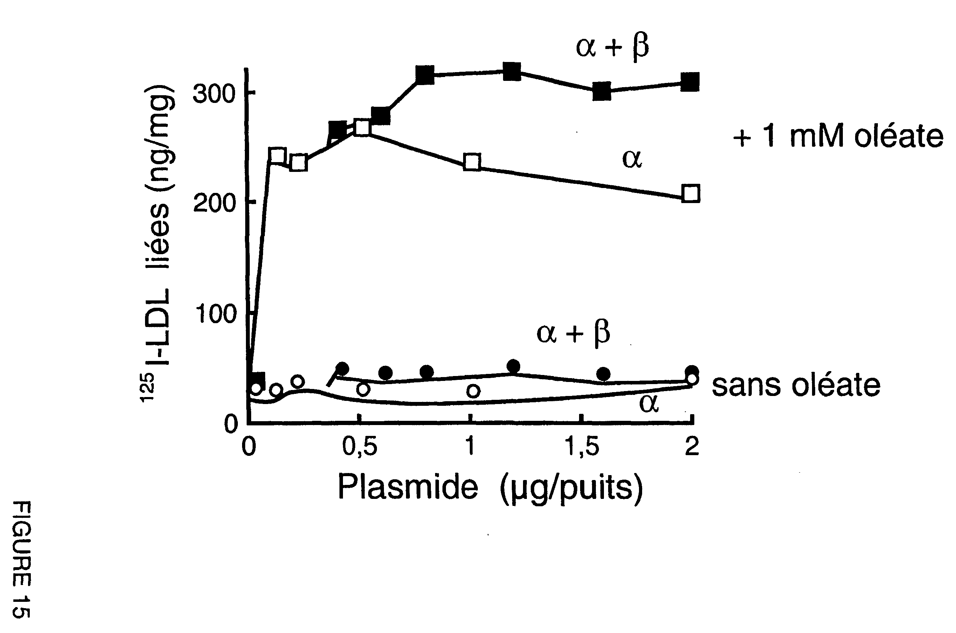

**FIGURE 15** : Effet d'une transfection transitoire de cellules CHO-K1 avec les plasmides exprimant les sous-unités α et β du récepteur LSR sur la fixation des LDL en présence ou absence d'oléate. Concentration croissante de plasmide α seulement (○□) ; concentration fixe de plasmide α et concentration croissante de plasmide β (●■).

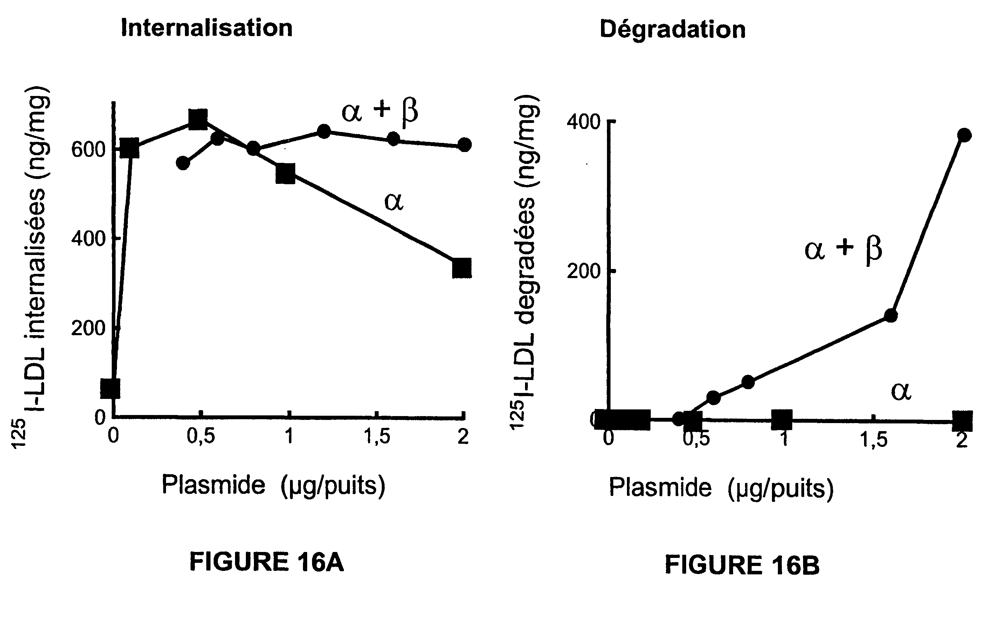

**FIGURE 16** : Effet d'une transfection transitoire de cellules CHO-K1 avec les plasmides exprimant les sous-unités α et β du récepteur LSR sur l'internalisation et la dégradation des LDL. Concentration croissante de plasmide α seulement (■) ; concentration fixe de plasmide α et concentration croissante de plasmide β (●). Les résultats sont exprimés comme la différence des mesures en présence et en absence d'oléate.

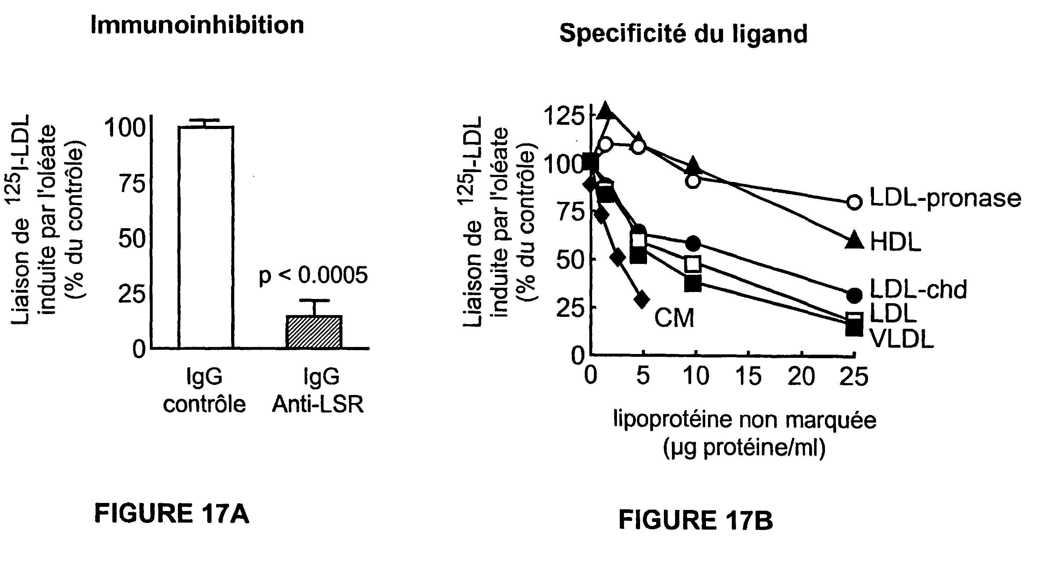

**FIGURE 17** : Caractérisation de l'activité LSR obtenue dans des cellules CHO-K1 transitoirement transfectées avec les séquences nucléiques codant pour les sous-unités α et β du récepteur LSR, comparée à l'activité LSR obtenue dans les mêmes cellules non transfectées (contrôle).
   **A-** Fixation de ¹²⁵I-LDL en présence d'un anticorps contrôle ou d'un anticorps anti-LSR.
   **B-** Fixation de ¹²⁵I-LDL en présence de concentrations croissantes de lipoprotéines non-marquées ; chylomicrons de rat (◆),VLDL humain (■), LDL (□), HDL (A ), LDL traités par la pronase (○), ou LDL modifiés par le cyclohexanedione (LDL-chd, ● ).

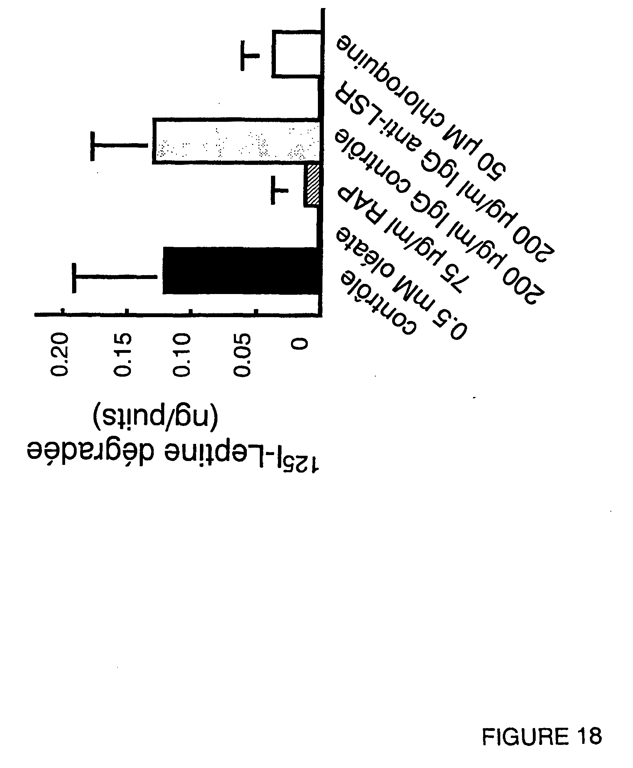

**FIGURE 18** : Effet de l'oléate, de RAP-39, des anticorps anti-LSR et de la chloroquine sur la dégradation spécifique de la leptine dans des cultures primaires d'hépatocytes de rat.

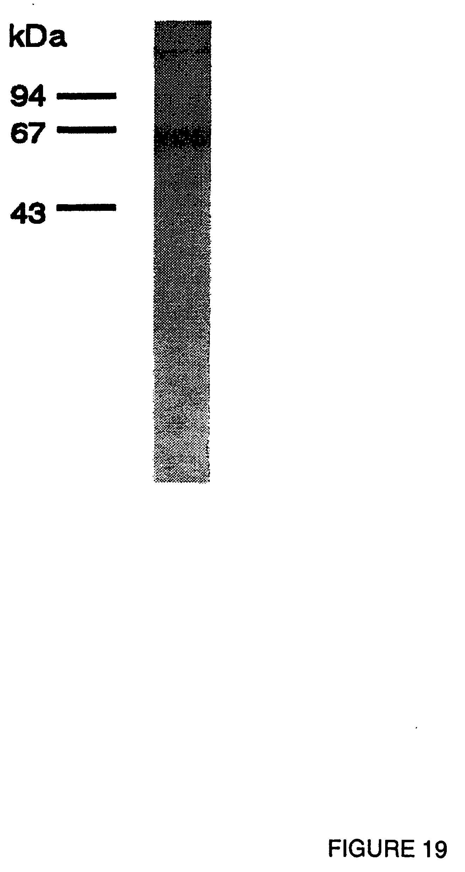

**FIGURE 19** : Analyse en Western blotting avec des anticorps anti-LSR, de la fraction des protéines de membrane plasmique de foie de rat retenue sur colonne de chromatographie d'affinité contenant de la leptine.

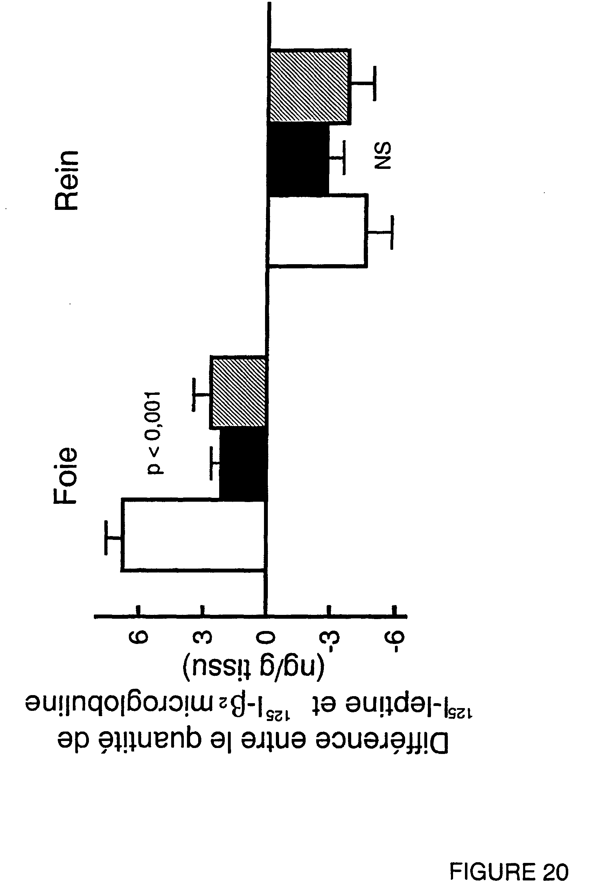

**FIGURE 20** : Clairance de la ¹²⁵I-leptine sur des souris témoins (□), *ob*/*ob* (■) et *db*/*db* ( 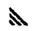 ) dans le foie et le rein. Les résultats sont exprimés comme la différence entre les quantités de ¹²⁵I-leptine et de ¹²⁵I-β2-microglobuline retrouvées dans le foie et dans le rein.

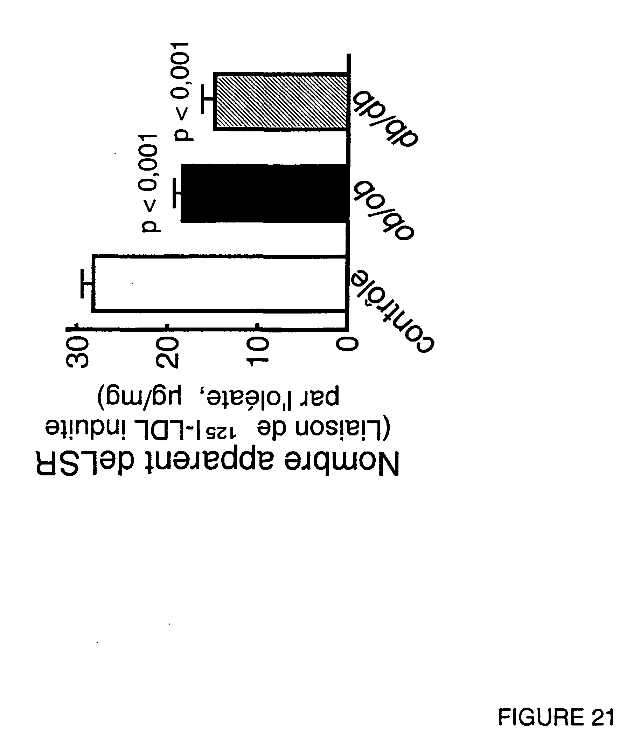

**FIGURE 21** : Nombre apparent de récepteurs LSR exprimés dans le foie de souris témoins, *ob*/*ob* et *db*/*db*.

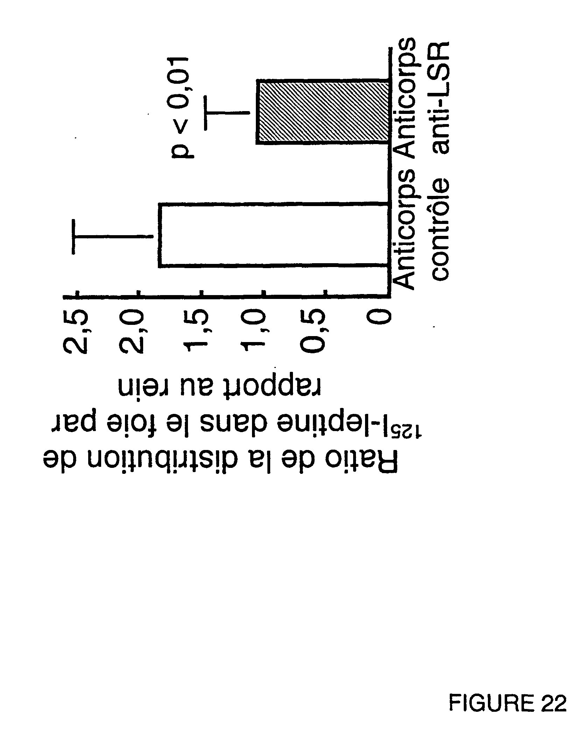

**FIGURE 22** : Effet des anticorps anti-LSR sur la proportion entre les quantités de ¹²⁵I-leptine distribuées dans le foie et dans le rein.

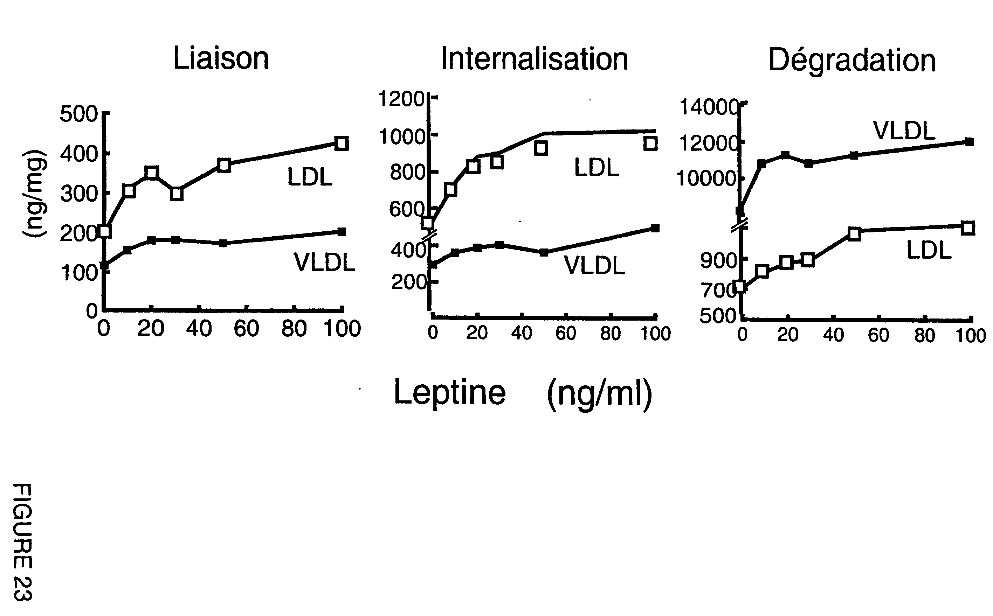

**FIGURE 23** : Effet de concentrations croissantes de leptine sur l'activité LSR d'hépatocytes de rat en culture primaire. Les résultats représentent les différences d'activité obtenues entre les cellules incubées avec et sans oléate en présence, soit de ¹²⁵I-LDL, soit de ¹²⁵I-VLDL.

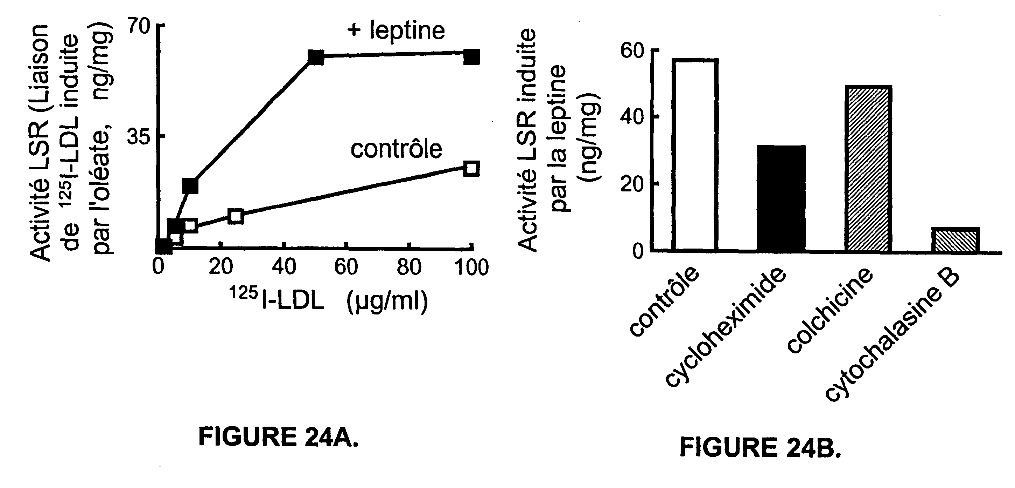

**FIGURE 24** : Capacité d'induction par la leptine de l'activité LSR d'hépatocytes de rat en culture primaire.
   **A.** Nombre apparent de récepteurs exprimés à la surface des hépatocytes en présence ou en absence de leptine, estimé par la mesure de la quantité de ¹²⁵I-LDL fixée en présence d'oléate.
   **B.** Effet de la cycloheximide, de la colchicine et de la cytochalasine B sur l'induction par la leptine de l'activité LSR.

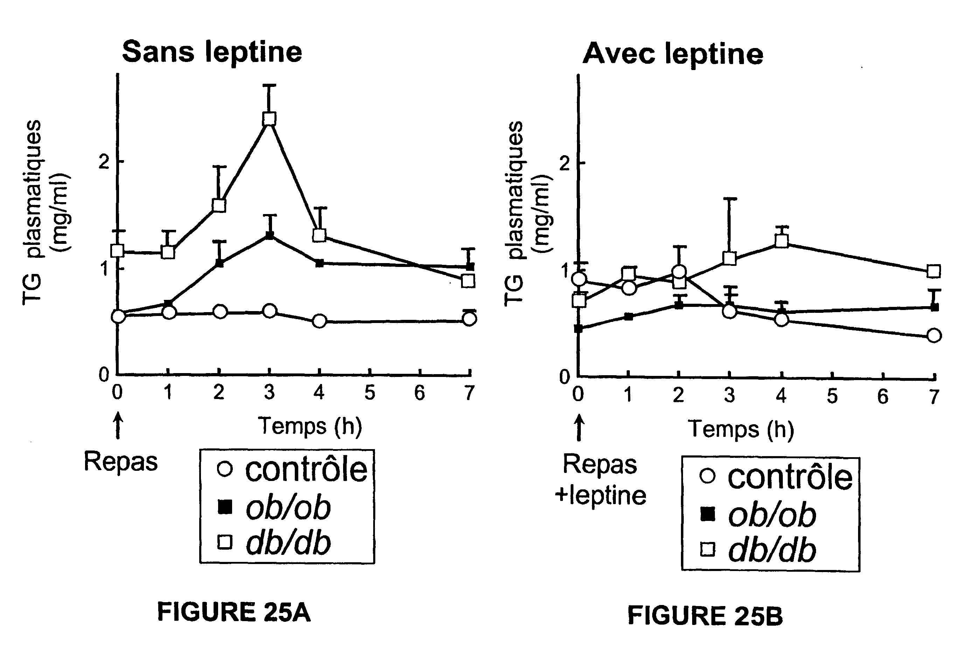

**FIGURE 25** : Effet de la leptine sur la réponse lipémique post-prandiale chez des souris témoins (○), *ob*/*ob* (■) et *db*/*db* (□), reflétée par l'évolution de la concentration plasmatique en triglycérides (TG) après ingestion d'un repas riche en graisses, avec (B) et sans (A) une injection de leptine recombinante murine.

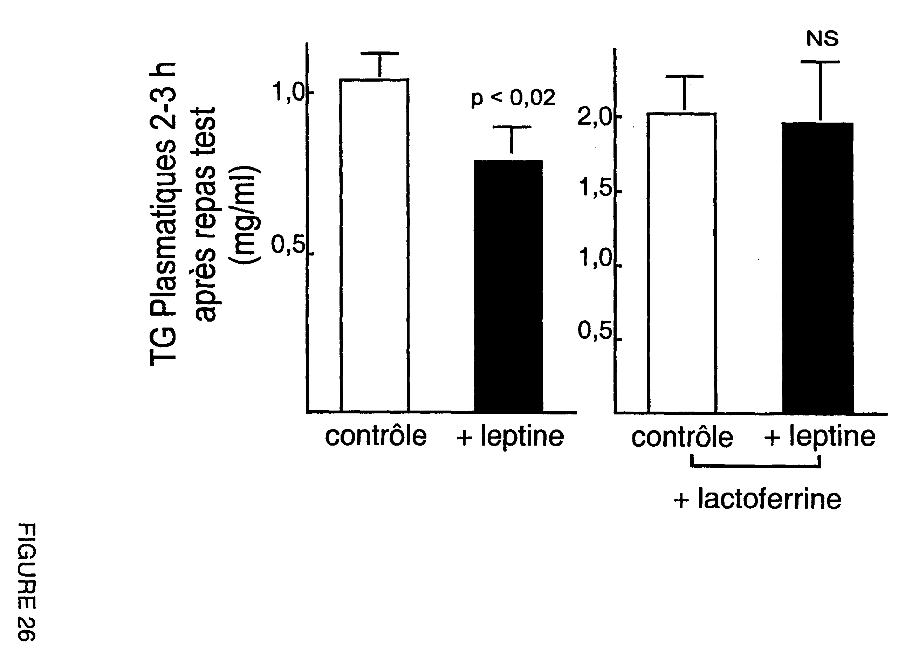

**FIGURE 26** : Effet de la leptine, en présence et en absence de lactoferrine, sur la réponse lipémique post-prandiale de souris *ob*/*ob*, exprimée par la mesure de la concentration plasmatique en triglycérides (TG) après ingestion d'un repas riche en graisses.

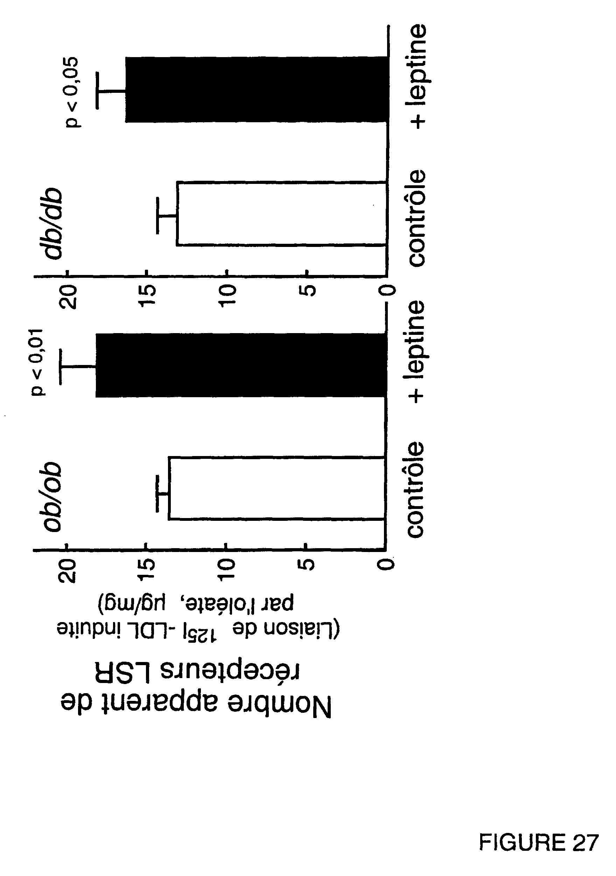

**FIGURE 27** : Effet de l'injection de leptine sur le nombre apparent de récepteurs LSR exprimés dans le foie de souris *ob*/*ob* et *db*/*db*.

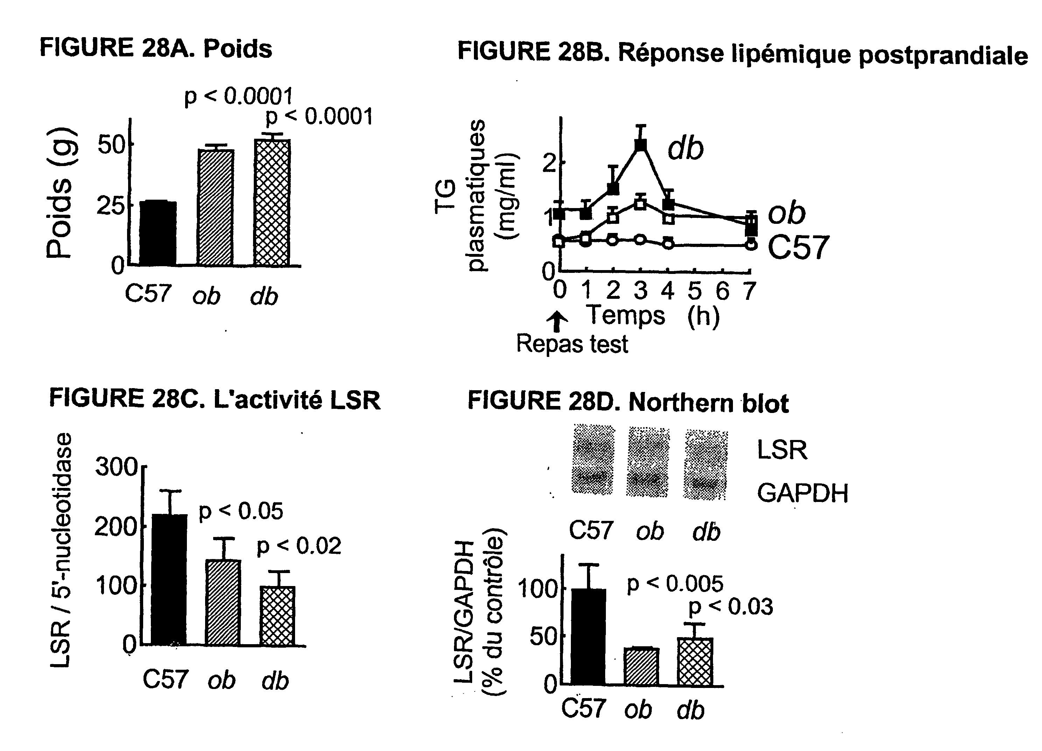

**FIGURE 28** : Réponse lipémique post-prandiale et activité LSR chez les souris témoin (C57BL6), *ob*/*ob* et *db*/*db*.
   **A-** Poids des souris mâle témoin, *ob*/*ob* et *db*/*db*
   **B-** Réponse lipémique post-prandiale de souris témoin, *ob*/*ob* et *db*/*db*.
   **C-** Nombre apparent de récepteur LSR estimé par la mesure de fixation de LDL et exprimé en unité arbitraire par comparaison avec l'activité 5'-nucléotidase dans chaque préparation de membrane plasmique.
   **D-** Northern blot sur un extrait d'ARN totaux de foie. Le GAPDH est utilisé comme contrôle.

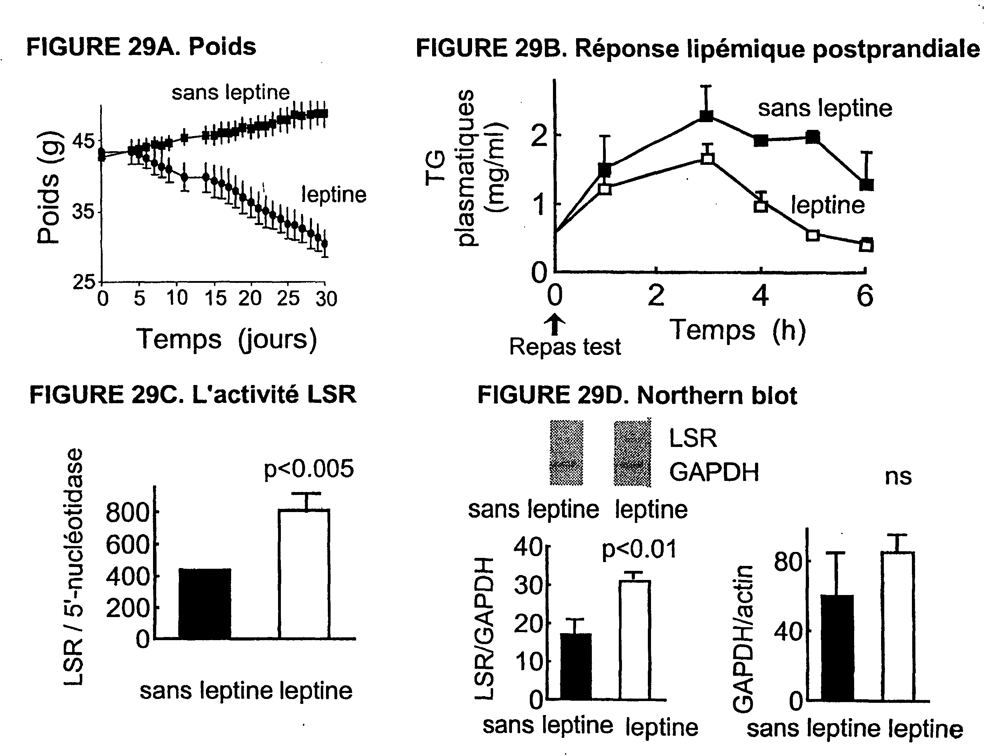

**FIGURE 29** : Effet d'un traitement à long terme par la leptine sur des souris *ob*/*ob*.
   **A-** Changement de poids sur 30 jours
   **B-** Réponse lipémique post-prandiale au 29^{ème} jour de traitement
   **C-** Nombre apparent de récepteurs LSR au jour 30, estimé par la mesure de fixation de LDL, et exprimé en unité arbitraire par comparaison avec l'activité 5'-nucléotidase dans chaque préparation de membrane plasmique
   **D-** Analyse en Nothern blot de l'expression du LSR établie sur un extrait total d'ARN de foie. GAPDH et actine sont utilisés comme contrôles.

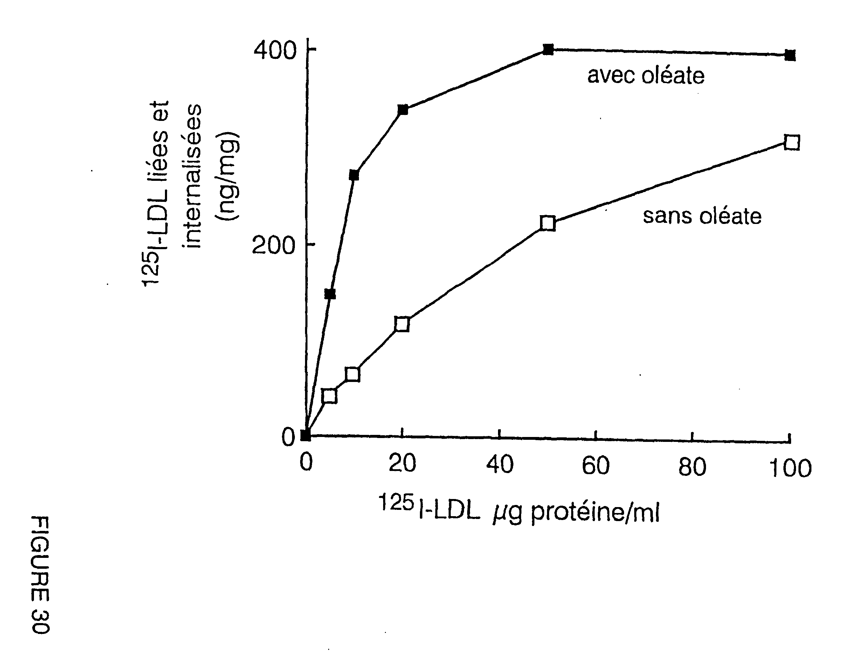

**FIGURE 30** : Effet des oléates sur la fixation et l'internalisation des ¹²⁵I-LDL dans des fibroblastes humains normaux, en conditions normales.

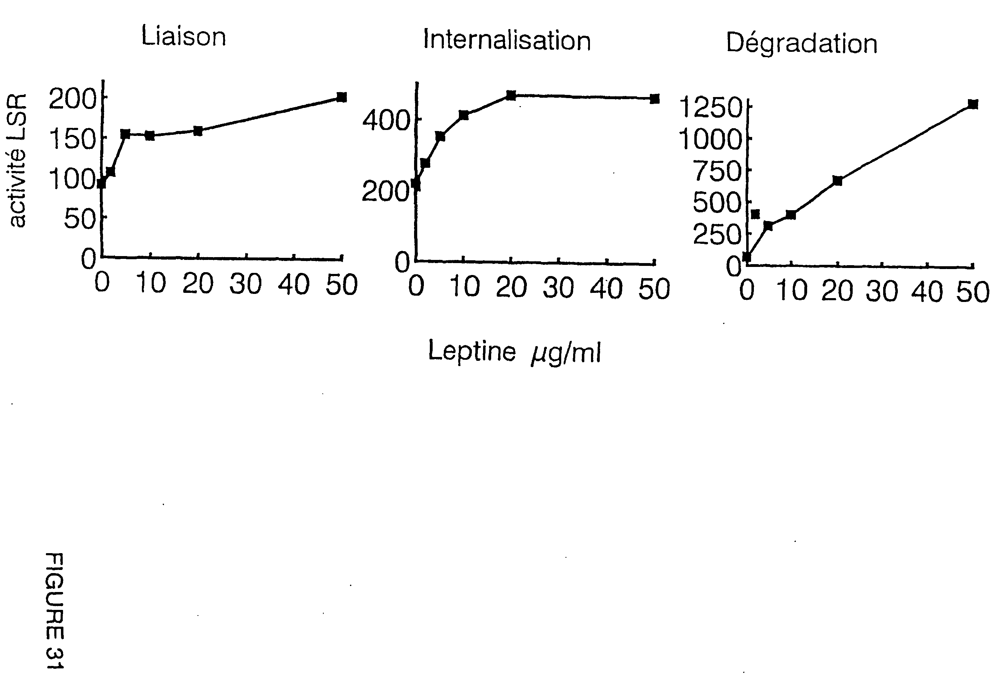

**FIGURE 31** : Effet de concentrations croissantes de leptine sur l'activité LSR de fibroblastes humains FH (hypercholestérolémie familiale).

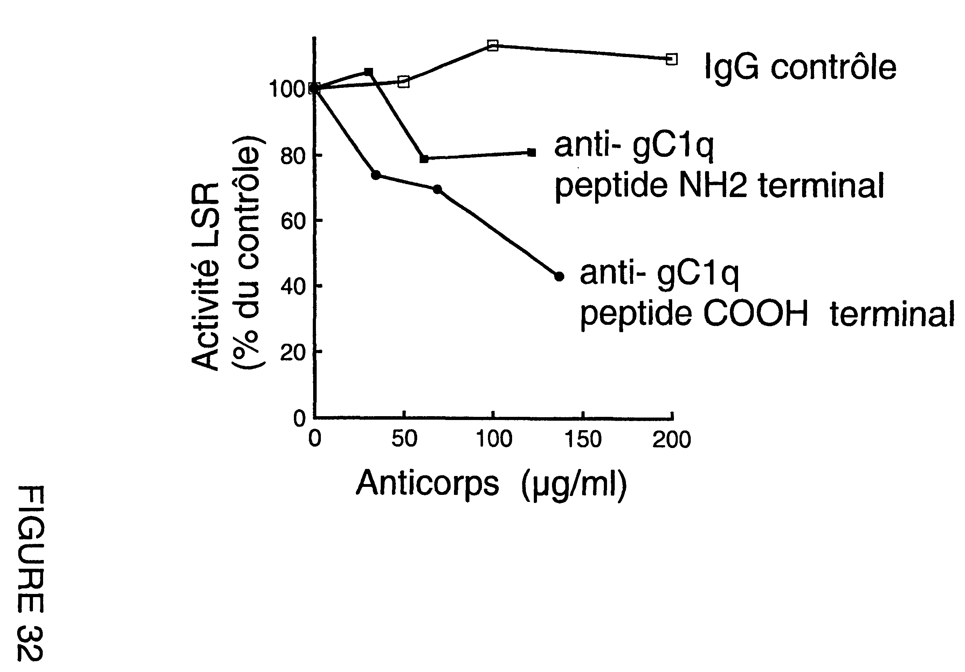

**FIGURE 32** : Effet inhibiteur d'anticorps dirigés contre un peptide NH₂-terminal ( ■), ou COOH-terminal (○) de gC1qR, ou d'anticorps contrôles (□) sur l'activité du LSR de membranes plasmiques d'hépatocytes de rat, exprimée en pourcentage de la quantité de ¹²⁵I-LDL fixée en l'absence d'anticorps.

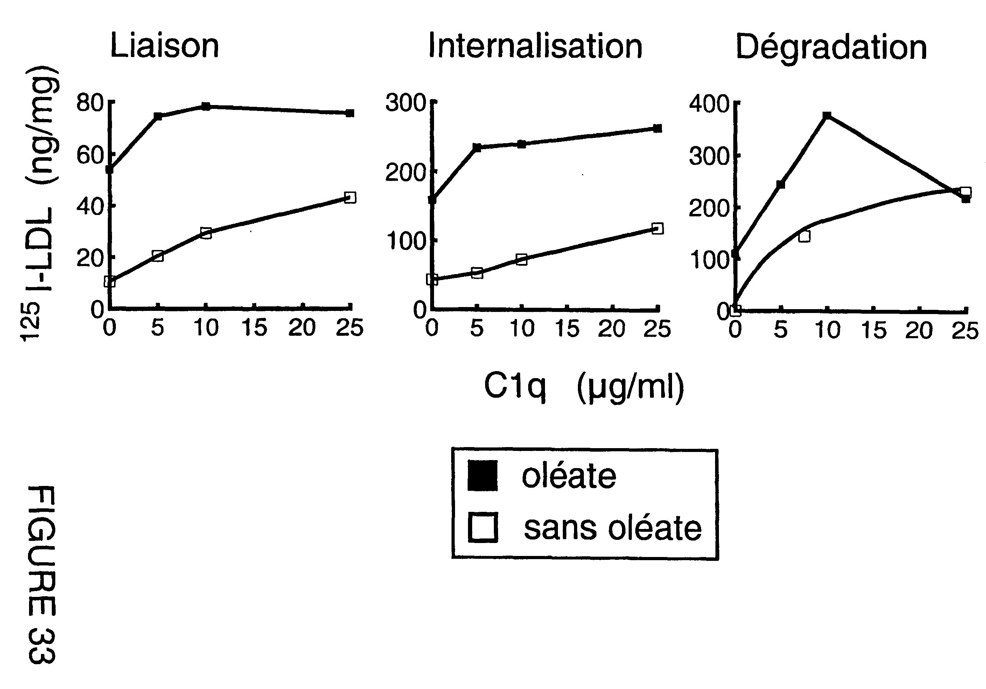

**FIGURE 33** : Effet de concentrations croissantes de C1q sur la fixation, l'intemalisation et la dégradation de ¹²⁵I-LDL sur les hépatocytes de rat en culture primaire, en présence (■) ou en absence (□) d'oléate.

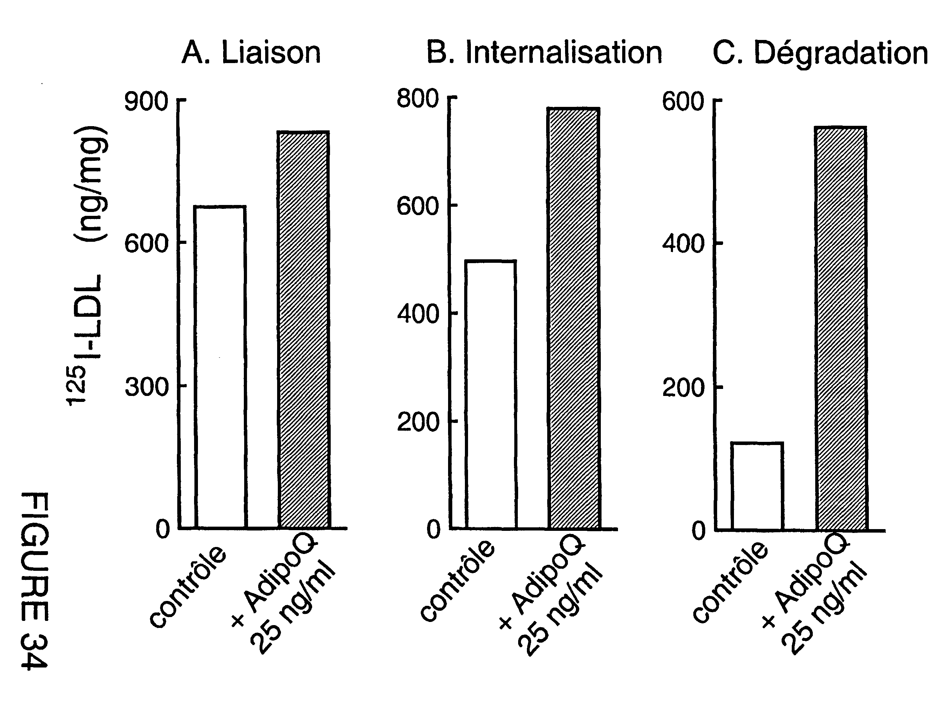

**FIGURE 34** : Effet de 25 ng/ml d'AdipoQ recombinante sur l'activité LSR dans une culture primaire d'hépatocytes de rat.

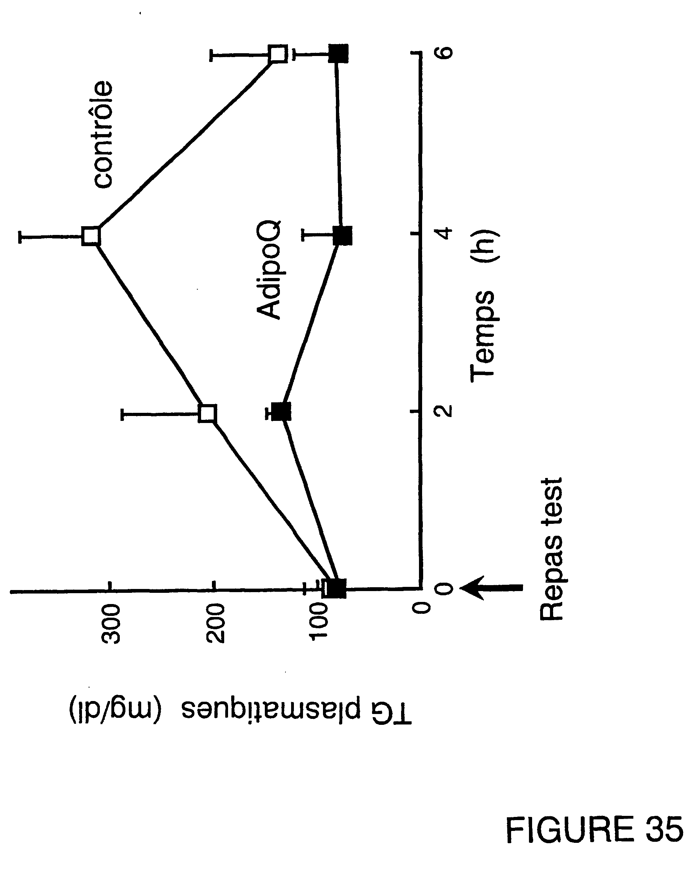

**FIGURE 35** : Effet de deux injections successives de 1 mg d'AdipoQ sur la réponse lipémique postprandiale chez le rat après ingestion d'un repas riche en graisses.

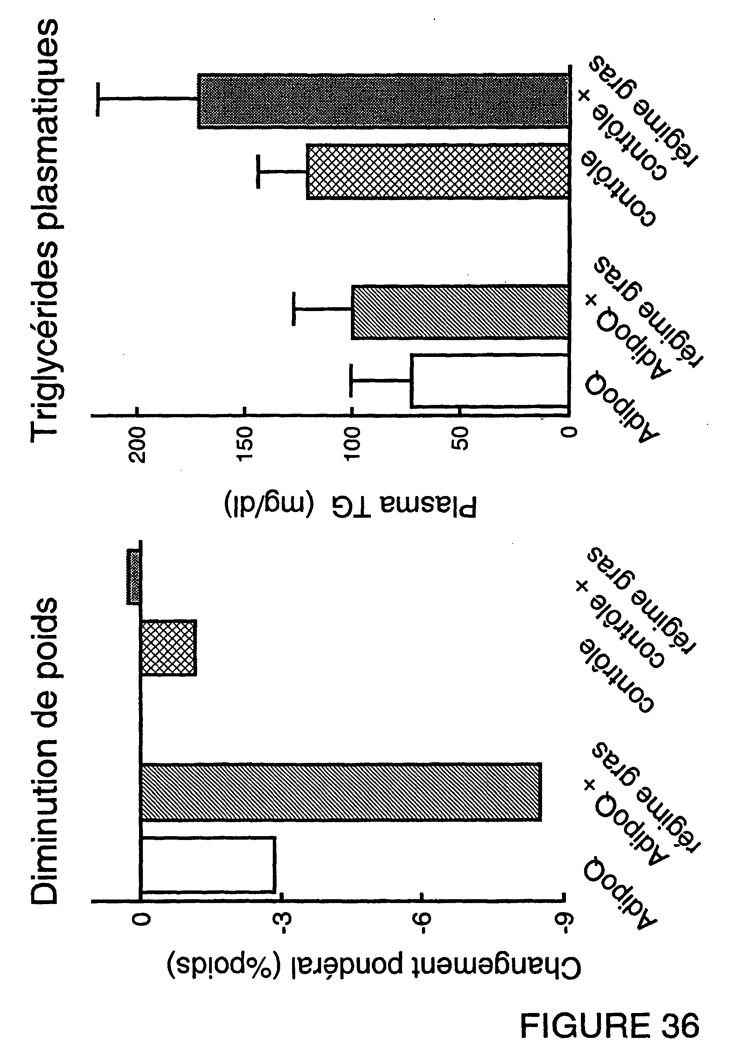

**FIGURE 36** : Effet d'une administration intrapéritonéale d'AdipoQ pendant 3 jours sur le poids et les concentrations en triglycérides plasmatiques de rats sous régime normal ou régime gras.

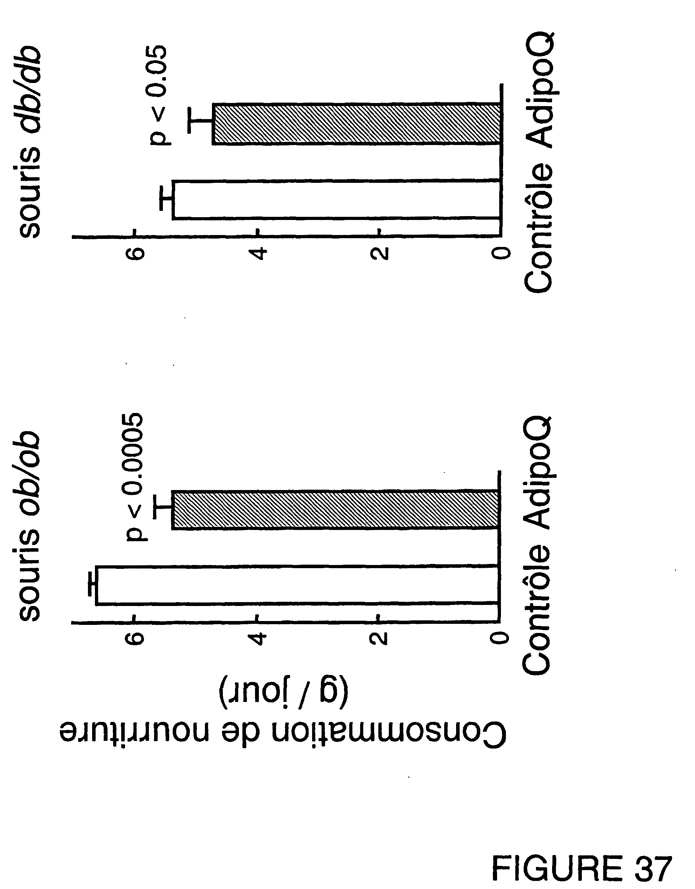

**FIGURE 37** : Effet d'une injection de quotidienne de 100 µg d'AdipoQ sur 5 jours, sur la prise de nourriture chez des souris obèses *ob*/*ob* et *db*/*db*.

### EXEMPLES

### Procédures expérimentales

### Matériel

Na¹²⁵I est fourni par Amersham (Les Ulis, France). L'acide oléique, l'albumine bovine sérique (A 2153) (BSA)et le Triton X-100 proviennent de chez Sigma (St Quentin Fallavier, France). La lactoferrine humaine (Serva) et l'héparine sodique sont fournies par Biowhittaker (Fontenay sous Bois, France) et les laboratoires Choay (Gentilly, France)respectivement. Les kits enzymatiques pour la détermination des triglycérides (TG) proviennent de chez Boehringer Mannheim (Meylan, France). Le suramine sodium est obtenu de CBC Chemicals (Woodburg, CT). Le milieu Dulbecco, Eagle modifié (DMEM), la trypsine et le sérum de veau foetal sont fournis par Life Technologies, Inc. (Eragny, France).

### Animaux

Les souris C57BL/6J type sauvage, C57BL/6J ob/ob, C57BL/Ks type sauvage et C57BL/Ks db/db sont obtenues de R. Janvier Breeding Center (Le Genest St Isle, France).

### Cellules

Les fibroblastes normaux (GMO8333) et FH (GM00486A, GM007001B, GM00488C) sont fournis par le NIGMS human genetic mutant cell repository (Camden, NJ). Les cellules sont étalées dans des boîtes de Petri de 36 mm comme décrit précédemment (300.000 fibroblastes normaux par puits, 150.000 fibroblastes FH par puits), et sont cultivées dans une étuve à CO₂ humidifiée, en milieu DMEM contenant 10% (fibroblaste normaux) ou 20% (fibroblastes FH) de sérum de veau foetal, 2mM glutamine, 100 u/ml de pénicilline et 100 u/ml de streptomycine.

Les hépatocytes en culture primaire sont obtenus suivant le protocole décrit précédemment (Mann et al., 1995). Les cellules sont ensuite étalées à 900.000 cellules par puits ou 22x10⁶ cellules par fiole de 165 cm². Les cellules sont utilisées pour les études après 48 heures en culture.

### Préparation et radiomarquage des lipoprotéines

Les VLDL (d < 1,006 g/ml) et LDL (1,025 < d < 1,055 g/ml) sont isolées par ultracentrifugation séquentielle de plasma frais de volontaires (Bihain et Yen, 1992 ; Goldstein, et al., 1983) et utilisées avant 2 semaines. Les lipoprotéines sont radioiodinées (Bilheimer et al., 1972) et utilisées moins d'une semaine après le marquage. ¹²⁵I-LDL et ¹²⁵I-VLDL sont filtrés (membrane 0,22 µm, Gelman) immédiatement avant utilisation.

### Préparation et radiomarquage de la leptine recombinante de souris

L'ADNc de leptine est obtenu à partir de l'ARNm de tissu adipeux de souris C57BL/6J par PCR. L'amorce 5' de PCR introduit un codon d'initiation après la séquence signale qui est délétée et une séquence codant pour une terminaison hexahistidine. La séquence modifiée codant pour la leptine murine est clonée dans un vecteur d'expression pSE280 (Invitrogen, France) et exprimée dans E.Coli. Le séquençage de l'ADN du plasmide confirme la séquence codante. Les bactéries sont cultivées à 37° C et la synthèse de la protéine est induite par 1 mM d'isopropyl-β-D-thiogalactopyranoside. Les bactéries, récupérées après centrifugation douce, sont lysées par congélation-décongélation et l'ADN est digéré par une déoxyribonucléase I. Les membranes cellulaires sont extraites à l'aide de détergent et les corps d'inclusions sont séparés après centrifugation. Après 3 lavages dans 1% de deoxycholate de sodium en PBS, les corps d'inclusion sont solubilisés dans une solution de guanidine-HCl 6M. La renaturation de la protéine recombinante est réalisée par dilution au 1/100 dans du PBS. La protéine renaturée est ensuite purifiée et concentrée sur colonne de chromatographie d'affinité métal chélate au Nickel (Probond, Invitrogen). L'élution est effectuée par de l'imidazol. On contrôle la pureté de la leptine recombinante par électrophorèse SDS-PAGE et son activité par l'évaluation de la satiété chez des souris C57BL/6J ob/ob après injection intrapéritonale de 25 µg de leptine. La leptine recombinante est ensuite radiomarquée en utilisant des lodo-Beads (Pierce) et selon la méthode préconisée par le fabricant.

### Clonage de l'ARNm d'AdipoQ. Production et purification de protéines AdipoQ recombinante

### Clonage de l'ADNc dans un vecteur d'expression

Du tissu adipeux de souris est obtenu à partir de souris C57BI/6J et l'ARNm est extrait à l'aide de polydT fixés sur des billes magnétiques (Dynabeads, Dynal, France). Une banque d'ADNc est construite à partir du tissu adipeux de souris par transcription inverse à 40°C en utilisant un kit commercial (Superscript Life Technologies) en suivant les instructions du vendeur. L'ADNc spécifique d'AdipoQ est amplifié en utilisant les deux amorces suivantes :

Le produit d'amplification est ensuite digéré par les enzymes de restriction BamH1 et Xho1 et inséré dans un vecteur d'expression pTRC HisB (Invitrogen, France) aux sites correspondants. La version B de pTRC permet l'expression de séquences hétérogènes en aval d'un peptide hexahistidine qui porte un site de reconnaissance pour une Enterokinase et un épitope pour l'anticorps anti-Xpress.

### Transfection bactérienne et vérification de la construction

Le plasmide ainsi obtenu est transfecté dans *E. coli* DII5 α. De plus, l'ADN du plasmide est extrait et l'insert hétérologue est séquencé.

### Culture cellulaire, extraction et purification de la protéine recombinante

Les cellules bactériennes recombinantes sont cultivées à 37°C dans un milieu LB contenant des antibiotiques jusqu'à ce que la DO à 600 nm atteigne 0,2. La production de protéine recombinante est alors induite en ajoutant 1 mM d'isopropyl-β-D-thiogalactopyranoside au milieu de culture. La culture bactérienne se poursuit pendant 16 h à 37°C. Les cellules sont récupérées par centrifugation. La lyse des cellules est effectuée en utilisant du lysozyme dans un tampon Tris pH 7,4 en présence de NaCl, PMSF et deoxycholate de sodium. L'ADN est dégradé par sonication. Après centrifugation, la protéine recombinante est séparée du surnageant par une colonne Probond (Invitrogen, France). Cette colonne présente du nickel chargé qui a une affinité pour les peptides hexahistidine. L'élution est effectuée en présence d'imidazole. La concentration protéique est estimée par la méthode de Lowry après avoir dialysé le produit de l'élution. La pureté de la protéine obtenue est testée par électrophorèse SDS PAGE, qui révèle une bande unique.

### Exemple 1 : Identification du complexe protéique responsable de l'activité LSR : purification partielle, et caractérisation au moyen d'anticorps polyclonaux

La technique des ligand blotting a été utilisée pour identifier le complexe protéique responsable de l'activité LSR. Cette technique, décrite en détail par Mann et al., 1995, est détaillée ci dessous.

### Ligand blotting

La technique consiste à isoler par centrifugation différentielle (Belcher et al., 1987) les membranes de foie de rat, et à solubiliser les protéines membranaires dans une solution contenant 125 mM d'octylglucoside, 20mM Tris et 2mM EDTA pH8. Les protéines ainsi solubilisées sont séparées dans des conditions non dénaturantes sur un gel (épaisseur 5mm) SDS préparatif formé d'un gradient de 4 à 12 % de polyacrylamide (35-50 mg de protéine par gel). Pour une partie du gel, les protéines sont ensuite électrotransférées (transfert semi-sec, 21 V, 45 min, Biorad) sur une membrane de nitrocellulose. Après blocage des sites libres de cette membrane dans une solution de PBS contenant 3 % d'albumine, la membrane est incubée avec 40 µg/ml de ¹²⁵I-LDL en présence (Figure 8, ligne 2) ou absence (Figure 8, ligne 1) d'oléate à 0,8 mM. La membrane est ensuite lavée cinq fois 10 minutes dans du PBS contenant 0,5 % (v/v) de Triton x100, et exposée sur un écran de Phosphor Imager.

L'analyse de l'image obtenue en présence (Figure 8, ligne 2) ou absence (Figure 8, ligne 1) d'oléate met en évidence la présence de 3 bandes principales ayant fixé les LDL. Le PM apparent de la première bande est d'environ 240 kDa, celui de la seconde est de 115 kDa et celui de la troisième est de 90 kDa. Sur la base de ces observations, deux hypothèses sont avancées : d'une part l'activité LSR est liée à la présence de plusieurs protéines distinctes ; d'autre part le même type d'image peut s'expliquer par une organisation multimérique d'un complexe protéique.

Afin de vérifier cette hypothèse, les inventeurs ont entrepris la purification de la bande présentant le poids moléculaire apparent le plus élevé (240 kDa). La purification partielle de cette protéine désignée comme « bande A » est réalisée par électrophorèse préparative comme suit.

### Purification partielle du LSR

La technique consiste à isoler par centrifugation différentielle (Belcher et al., 1987) les membranes de foie de rat, et à solubiliser les protéines membranaires dans une solution contenant 125 mM d'octylglucoside, 20mM Tris et 2mM EDTA pH8. Les protéines ainsi solubilisées sont séparées dans des conditions non dénaturantes sur un gel (épaisseur 5mm) SDS préparatif formé d'un gradient de 4 à 12 % de polyacrylamide (35-50 mg par gel). Pour une partie du gel, les protéines sont ensuite électrotransférées (transfert semi-sec, 21 V, 45 min, Biorad) sur une membrane de nitrocellulose. Après blocage des sites libres de cette membrane dans une solution de PBS contenant 3 % d'albumine, la membrane est incubée avec 40 µg/ml de ¹²⁵I-LDL en présence (Figure 8, ligne 2) ou absence (Figure 8, ligne 1) d'oléate à 0.8 mM. La membrane est ensuite lavée cinq fois 10 minutes dans du PBS contenant 0.5 % (v/v) de Triton x100, et exposée sur un écran de Phosphor Imager. Les protéines d'intérêt sont électroéluées (Eletroeluter, Biorad).

Les protéines de membrane plasmique de foie de rat ont été préparées et séparées sur gel de polyacrylamide comme ci-dessus. La localisation précise de la bande A a été établie par ligand blotting réalisé après électrotransfert d'échantillon de gel préparatif prélevé à différents niveaux.

Les fragments de gel contenant la bande A sont ensuite prélevés, électroélués et concentrées (speedvac), puis testées pour leur capacité à fixer les LDL en présence d'oléate après électrophorèse et transfert sur membranes de nitrocellulose (Figure 8, ligne 3 ; 80 µg de protéine/tigne).

Les protéines ainsi obtenues ont aussi été utilisées pour produire des anticorps polyclonaux, dont la spécificité a été testée par Western blotting (Figure 8, ligne 4).

### Préparation d'anticorps polyclonaux

Les protéines LSR utilisées comme antigènes pour la production des anticorps anti-LSR ont été préparées comme indiqué ci-dessus.

La préparation d'antigène est injectée à un lapin en sous-cutané en présence d'adjuvant de Freund complet suivi d'un protocole classique d'immunisation. Le titre d'anticorps dirigé contre les protéines de rat est déterminé régulièrement (technique par dot blot). Lorsque celui-ci est jugé suffisant, la spécificité des anticorps obtenus est testée par Western blotting sur une préparation de protéines solubilisées de membranes de foie de rat telle que celles décrites ci-dessus, avec des anticorps de chèvre anti-IgG de lapin marqués à l'iode I¹²⁵ comme seconds anticorps.

Les résultats de Western blot après électrophorèse en condition non-réductrices indiquent que les anticorps produits à partir des protéines de la bande A se fixent sur 3 bandes protéiques principales (240 kDa, 115 kDa et 90 kDa) qui fixent les ¹²⁵I-LDL en présence d'oléate (Figure 8, ligne 4). Afin de vérifier le lien entre ces complexes protéiques et l'activité LSR, l'effet de ces anticorps polyclonaux sur l'activité LSR a été testé.

Les méthodes utilisées sont décrites en détails ci-dessous (Mann et al., 1995 ; froussard et al., 1995). L'activité du LSR est estimée par mesure de la fixation des lipoprotéines sur les membranes plasmiques et par mesure de la fixation, de l'internalisation et de la dégradation des lipoprotéines sur des cultures primaires d'hépatocytes de rat.

### Mesure de la fixation des lipoprotéines sur membranes plasmiques

L'activité du LSR est mesurée sur une préparation de membranes plasmiques de foie de rat (Bartles et Hubbard, 1990). Ces membranes présentent un enrichissement en 5-nucléotidase (marqueur spécifique des membranes plasmiques) de 10 à 15 fois. Des aliquots de 100µg de protéines sont incubés pendant 30 minutes à 37°C en présence ou en absence de 0,8 mM d'oléate dans un volume final de 250 µl complété par du PBS 100 mM, EDTA 2 mM, NaCl 350 mM, pH 8 (tampon A). L'oléate est ajouté dans un volume de 5 à 10 µl d'isopropanol. L'oléate en excès et non fixé est ensuite éliminé par 6 lavages. Les culots sont resuspendus par 250 µl de tampon d'incubation, soniqués 5 secondes, puissance 1,90% du cycle actif, puis centrifugés 15 min à 18 000 rpm. Les membranes activées sont incubées pendant 1 heure à 4 °C avec les différentes concentrations d'anticorps puis ensuite avec 5 µg/ml de ¹²⁵I-LDL (1 heure à 4°C). 25 µl de BSA 2% sont ajoutés au mélange d'incubation. La quantité de ¹²⁵I-LDL liées aux membranes est mesurée en sédimentant les membranes par centrifugation après avoir déposé 200 µl du mélange d'incubation sur une couche de 5% (P/V) de BSA dans le tampon A. Les surnageants sont éliminés par aspiration, les fonds des tubes sont coupés et la radioactivité est comptée dans un compteur γ.

L'effet inhibiteur d'anticorps anti-LSR sur l'activité LSR, comparé à celui d'une préparation quelconque d'immunoglobulines de lapin est montré dans la Figure 9 A. L'inhibition de l'activité LSR par les anticorps anti-LSR confirme que le complexe multimérique décrit ci-dessus est responsable de l'activité du récepteur et valide la technique de ligand blotting utilisée pour l'identifier. L'effet des anticorps anti-bande A a en outre été testé sur les autres étapes de l'activité du récepteur : l'internalisation et la dégradation des lipoprotéines par le LSR exprimé en surface d'hépatocytes en culture primaires.

### Mesure de la fixation, de l'internalisation et de la dégradation des lipoprotéines par les hépatocytes

L'activité LSR dans les cultures primaires d'hépatocytes de rat est mesurée par la fixation, l'internalisation et la dégradation de ¹²⁵I-LDL et de ¹²¹I-VLDL (LDL : lipoprotéine de faible densité ; VLDL : lipoprotéine de très faible densité), comme décrit dans Bihain et Yen, 1992 et Mann et al., 1995.

Pour la mesure de l'effet des anticorps anti-LSR sur la fixation, l'internalisation et la dégradation des LDL par le LSR, des cultures primaires d'hépatocytes de rat (48 h après étalement) sont incubées en présence de 20 ng de leptine/puits pendant 30 min à 37° C, suivi par l'addition d'anticorps anti-LSR en présence ou en absence d'oléate. Après incubation à température ambiante pendant 30 min, on ajoute de I'¹²⁵I-LDL (20 µg/ml), puis les cellules sont incubées pendant 4 h à 37° C. La fixation, l'incorporation et la dégradation de l'¹²⁵I-LDL sont mesurées comme décrit dans Bihain et Yen, 1992 et Mann, et al., 1995.

Les données de la Figure 9 B montrent que les anticorps anti-bande A inhibent la plus grande partie de l'activité de fixation des LDL aux LSR présents au niveau des hépatocytes. Cette inhibition induit une diminution dans les mêmes proportions de l'internalisation et de la dégradation protéolytique des lipoprotéines.

Les anticorps anti-bande A sont ainsi caractérisés comme anti-LSR. Leur spécificité relative a été définie par une méthode d'immunoprécipitation sélective. Des extraits d'hépatocytes en culture primaire sont immunoprécipités au moyen des anticorps anti-LSR décrits précédemment, selon le protocole décrit ci-dessous.

### Immunoprécipitation d'extraits d'hépatocytes en présence d'anticorps spécifiques

Des cultures primaires d'hépatocyte de rat (Oukka et al., 1997) sont incubées pendant 60 minutes à 2 heures en présence d'un mélange de ³⁵S méthionine et ³⁵S cystéine (Promix, Amersham). Ce milieu est ensuite enlevé et les cellules sont lavées, puis incubées dans du PBS contenant 1% de Triton X100. Ce lysat cellulaire est ensuite incubé en présence d'anticorps non-spécifiques puis de protéine A. L'équivalent de 40 µg d'anticorps spécifiques anti-LSR est ensuite ajouté et les complexes LSR-anticorps sont précipités à l'aide d'une seconde préparation de protéine A. Après lavage, les complexes sont dissociés en présence de 1% SDS supplémenté ou non par 5% de β-mercaptoéthanol, incubés à 100°C pendant 5-10 minutes, et séparés sur un gel de 10% acrylamide. Les gels sont séchés et exposés sur un écran de Phosphor Imager. Chacune des lignes contient l'équivalent d'un flacon de 165 cm² soit 22 X 10⁶ cellules.

L'analyse des résultats de l'immunoprécipitation indique qu'en conditions non réduites (Figure 10, lignes 2 - sans incubation à 100°C - et 3 - avec incubation à 100°C -), les anticorps révèlent 3 bandes protéiques principales : 2 de poids moléculaire apparent 240 kDa et 180 kDa, 1 de poids moléculaire apparent de 68 kDa. On note également la présence de 2 bandes d'intensité plus faible correspondant à un poids moléculaire de 115 kDa et 90 kDa. Cette approche expérimentale met donc en évidence essentiellement les mêmes éléments protéiques que ceux identifiés par la méthode de ligand blotting. On observe par ailleurs qu'en conditions réduites (Figure 10, lignes 1 et 4), les éléments de haut poids moléculaire se dissocient en 3 éléments de poids moléculaires apparents respectifs de 68 kDa, 56 kDa et 35 kDa.

L'intensité relative des bandes de 68kDa et 56kDa est proche alors que celle de la bande de 35kDa est d'environ ¼ de celle des deux autres.

### Exemple 2 : Clonage du c-DNA codant pour le LSR α et β

Le criblage d'une banque d'expression au moyen des anticorps anti-LSR décrits précédemment a été réalisé comme indiqué ci-dessous.

### Criblage d'une banque d'expression

Après infection des bactéries par des bactériophages lambda GT11 contenant de l'ADNc de foie de rat (obtenu commercialement de Clontech Laboratories Inc (5' Strech Plus c-DNA Library), les cellules sont étalées sur milieu LB MgSO4. Après 4 heures de cultures à 42°C, une membrane de nitrocellulose préalablement incubée dans une solution d'IPTG 10mM, est déposée dans les boites de pétri. Quatre heures plus tard, la première membrane est retirée et une seconde appliquée sur la boite de pétri.

Chaque membrane est immergée dans une boite de pétri contenant du tampon bloquant sous agitation pendant une heure. Ensuite, l'anticorps est ajouté à une concentration finale de 10 µg/ml de tampon bloquant (Huynh et al., 1984 ; Young et Davis, 1983a et 1983b). Les membranes sont ensuite lavées trois fois 10 minutes avec du TNT (Tris 10 mM, NaCl 150 mM, Tween 20 à 0,05 %).

Les membranes sont incubées en présence de l'anticorps secondaire (alkaline phosphatase-conjugated affinipure F(ab') 2 Fragment Goat anti-rabbit IgG ; Immunotech) à une concentration finale de 0,08 µg/ml de tampon bloquant (TNT + 5% lait écrémé en poudre, marque Pâturage).

Après lavage des membranes dans le TNT, celles-ci sont incubées en présence de BCIP (5-bromo-4-chloro-3-indolyl phosphate) et de NBT (nitro-blue-tetrazolium) jusqu'à obtention d'une coloration.

Les clones positifs sont ensuite récupérés sur les boites, titrés et soumis à la même procédure d'immunocriblage afin de confirmer qu'il s'agit de vrai positifs (criblage secondaire). Eventuellement, un criblage tertiaire peut être conduit. L'ADN phagique des clones retenus, isolé à partir d'un lysat bactérien (protocole Clontech), et digéré par l'enzyme de restriction EcoR1 est inséré au site EcoRI du plasmide pBluescript SK+.

Deux clones contenant un insert de 1.8 kb ont ainsi été obtenus, et se sont révélés de séquences identiques. L'hybridation d'ARNm de foie de rat ( 2µg d'ARNm polyA+ ) avec une sonde correspondant au fragment BgIII-XbaI de cet insert a mis en évidence deux bandes de tailles respectives de 1,9 kb et 2,1 kb (Figure 11 A). L'analyse par Northern blotting, avec une sonde correspondant au fragment XbaI-XbaI de cet insert, de la distribution tissulaire des messagers correspondants a montré qu'ils sont exprimés préférentiellement dans le foie (Figure 11 B). Le Nothern blotting a été réalisé selon le protocole suivant.

### Northern blotting

Les membranes contenant les ARNm de différents tissus de rat (Clontech) ont été hybridées avec des fragments de l'ADNc du gène LSR et de l'ADNc de β-actine humaine (Clontech), marqués au dCTP[³³P], en tampon 5xSSPE, 10xDenhardt, 0,5% SDS, 100 µg/ml d'ADN de sperme de saumon, 50% formamide déionisée, à 42°C pendant 16 heures. Les membranes ont ensuite été lavées dans du 2xSSC, 0,5% SDS à température ambiante et dans du 1xSSC, 0.1% SDS à 65°C, puis exposées au Phospor Imager (Molecular Dynamics).

Un ADNc correspondant à la bande de 1,9kb a été reconstruit par 5'RACE PCR à partir du fragment de 1,8 kb et séquencé.

Afin d'élucider la présence de bandes multiples en Northem blotting, plusieurs couples d'amorces définissant des fragments d'une séquence d'ADNc de rat ont été synthétisés et utilisés comme amorces pour une amplification en PCR (Figure 11 C). Les séquences des oligonucléotides utilisés sont listées ci-dessous :

Alors que chaque couple d'amorces met en évidence un fragment unique, le couple bc' permet d'amplifier trois fragments de tailles différentes. L'analyse des séquences de ces fragments permet de reconstituer la séquence de trois ADNc complets de LSR de rat, ayant pour tailles respectives 2097 pb (SEQ ID 1), 2040 pb (SEQ ID 3) et 1893 pb (SEQ ID 5), et tous trois correspondant à un même messager précurseur par épissage alternatif.

Ces trois ADNc contiennent un cadre ouvert de lecture débutant par un codon AUG à la position 219 entouré d'une séquence consensus Kozak (Kozak, 1987 et 1990). Les poids moléculaires prédits des protéines codées par ces trois ADNc sont respectivement de 66 kDa, 64 kDa et 58 kDa.

Les deux ADNc codant respectivement pour les formes la plus longue et la plus courte du LSR de rat ont ensuite été traduites *in vitro* comme indiqué ci-dessous.

### Traduction in vitro

Les ADNc sont sous-clonés dans le plasmide pcDNA3 ; la transcription et la traduction *in vitro* sont conduites en utilisant le kit TNT de Promega. Les produits de traduction, marqués à la ³⁵S-méthionine et ³⁵S-cystéine sont visualisés après électrophorèse en gel gradient de polyacrylamide (10%) et exposition au Phospor Imager.

Les poids moléculaires des produits obtenus, soit 68 kDa et 56 kDa (Figure 12), correspondent de façon proche à ceux des sous-unités α et β du LSR.

Pour définir si les produits de ces ARNm sont responsables de l'activité du récepteur, trois approches expérimentales différentes ont été utilisées.

Premièrement, deux peptides correspondant aux résidus 169-186 (SAQDLDGNNEAYAELIVLGR : SEQ ID 29) du LSR produit de l'ARNm de taille 2097 pb et aux résidus 556-570 (EEGQYPPAPPPYSET : SEQ ID 30) ont été synthétisés. La séquence de ces peptides est commune aux trois protéines identifiées ci-dessus. Des anticorps dirigés contre ces peptides synthétiques ont été obtenus selon les protocoles indiqués ci-dessus. Les Figures 13 C et 14 C montrent que ces anticorps anti-peptide LSR présentent un effet inhibiteur sur la fixation des LDL aux LSR présents sur les membranes plasmiques de rat, mesurée selon le protocole décrit en exemple 1.

Deuxièmement, une purification partielle des sous-unités α et β a été obtenue par solubilisation sélective à l'aide de sarkosyl ; une étude en Western et ligand blotting a montré que les éléments α et β fixent les anticorps polyclonaux anti-LSR (Figure 13 B, ligne 1), les anticorps anti-peptide LSR (Figure 13 B, ligne 2 et Figure 14 B, ligne 2), et les LDL après incubation avec oléates (Figure 13 B, ligne 4). Le Ligand blotting a été réalisé selon le protocole décrit en exemple 1 ; le Western blotting a été réalisé comme indiqué ci-dessous.

### Western blotting

Des cultures primaires d'hépatocytes de rat sont préparées comme indiqués en « Procédures expérimentales ». Les cellules récoltées après 48 heures de culture sont lavées et lysées dans du PBS contenant 1% Triton X100. Les lysats sont déposés sur gel SDS-PAGE 10% dans des conditions réductrices (2% SDS, 5% β-mercaptoéthanol et 20 mM DTT, à 56°C pendant 1h). Après transfert sur une membrane de nitrocellulose, le Western blotting est réalisé avec des anticorps IgG dirigés contre le récepteur LSR.

Troisièmement, les protéines LSR 66 et 58 marquées obtenues par traduction *in vitro* à partir des ADNc LSR-Rn-2097 et LSR-Rn-1893 sont utilisées pour estimer l'effet de l'oléate sur la fixation des LDL selon le protocole détaillé ci-dessous.

### Fixation des LDL sur les protéines de LSR exprimées in vitro (« flotation »)

Les produits de traduction in vitro (17 µl) marqués à la ³⁵S-cystéine ou ³⁵S-méthionine sont incubés 1 heure à 37°C en présence de 100 µg/ml de LDL, 1 mM oléate en tampon A, dans un volume final de 400µl. Un volume égal de BSA 8%(p /v) est ajouté. La densité est ajustée à 1,21 g/ml (assumant une densité d'origine de 1,025 g/ml), avec de bromure de sodium. Les échantillons sont alors déposés sur une solution du bromure de sodium à 1,063 g/ml, puis centrifugés 20 heures à 4°C (rotor Beckman SW41). Un volume de 1 ml est recueilli en surface, dialysé contre du tampon d'élution d'électrophorèse, et la radioactivité est comptée (compteur β Beckman).

L'oléate augmente la fixation du LDL aux LSR 56 (respectivement LSR 68) par un facteur 2 (5 respectivement). On montre ainsi que les sous-unités α et β du LSR de rat, codées respectivement par les ADNc LSR-Rn-2097 et LSR-Rn-1893 (LSR 56 et LSR 68), fixent les LDL préférentiellement après incubation avec l'oléate.

L'ensemble de ces résultats indique que les ADNc LSR-Rn-2097 et LSR-Rn-2040 codent pour deux protéines indistinguables par électrophorèse et dont le poids moléculaire apparent est 68 kDa ; ces protéines correspondent à la bande comportant les sous-unités α et α' du LSR identifiée après immunoprécipitation en conditions réduites. La sous-unité β du LSR est vraisemblablement le produit de traduction de l'ADNc LSR-Rn-1893. Les analyses de stoechiométrie après immunoprécipitation indiquent que le complexe multimérique de poids moléculaire apparent 240 kDa est le résultat d'un assemblage d'une sous-unité α avec trois sous-unités β. L'analyse des différents domaines des protéines correspondant aux LSR α et β est compatible avec une fonction de récepteur lipoprotéique.

### Exemple 3 : Analyse de l'activité d'un récepteur LSR recombinant, et de ses sous-unités, en cellules transfectées

Les inventeurs ont également réalisé l'expression d'un récepteur LSR recombinant dans des cellules CHO selon le protocole suivant.

### Transfection par les séquences d'ADNc codant pour le récepteur LSR

Afin d'étudier l'activité de chacune des sous-unités recombinantes du LSR, ainsi que l'activité d'un récepteur reconstitué, les inventeurs ont utilisé le plasmide d'expression pcDNA3 (No et al., 1996) pour étudier l'expression, dans des cellules animales, soit de l'ADNc codant pour la sous-unité α (plasmide α), soit d'un ADNc codant pour la sous-unité β (plasmide β), du LSR de rat. Les cDNA du LSR ont été sous-clonés à l'intérieur du plasmide pcDNA3 (Invitrogen), en utilisant les sites de restriction EcoRI et/ou Notl. Ces constructions une fois obtenue sont utilisées pour transfecter les cellules animales CHO (cellule d'ovaire de hamster).

Après 48 heures de culture, des cellules CHO (Chinese hamster ovary) (CHO-K1, CCL-61, ATCC, Rockville, MD) ont été distribuées dans une plaque à 6 puits (Falcon) à 2,5-2,75 x 10⁵ cellules/puits. Après 24 h de culture dans un milieu Ham F-12 contenant 10 % (v/v) FBS, 2 mM glutamine and 100 unités/ml de pénicilline et de streptomycine, un maximum de 2 µg de plasmide/puits ont été transfectés en utilisant Superfect (Qiagen) selon les instructions du fournisseur (10 µl Superfect/puits, 2 h à 37°C dans un milieu Ham F-12 dénué de sérum). Les plaques ont alors été lavées dans du PBS pour enlever les réactifs de transfection, et les cellules ont ensuite été cultivées dans un milieu Ham F-12 contenant du sérum. L'activité LSR a été mesurée 48 h après la transfection selon les protocoles détaillés dans l'exemple 1.

Les inventeurs ont testé l'effet d'une co-transfection par les plasmides α et β par rapport à celui d'une transfection par le plasmide α seul, ou le plasmide β seul, sur les trois étapes de l'activité du récepteur LSR selon les protocoles détaillés ci-dessous. Les Figures 15 et 16 montrent les comparaisons entre les activités LSR obtenues sur les cellules recombinantes exprimant la sous-unité α seule, ou les deux sous-unités α et β ; des résultats similaires sont obtenus pour la comparaison β versus α + β, ce qui est compatible avec l'analyse comparée des séquences primaires de chacune des sous-unités (chacune d'entre elles portant également les sites de fixation potentiels des ligands lipoprotéiques et des acides gras, tels l'oléate).

### Effet d'une transfection avec le plasmide LSR (α) seul, ou d'une co-transfection avec les plasmides LSR (α) et LSR (β), sur la fixation, l'internalisation et la dégradation des LDL.

Les cellules CHO-K1 ont été transitoirement transfectées par des concentrations croissantes de plasmide α ou co-transfectées avec 0,4 µg de plasmide α et des concentrations croissantes de plasmide β. Après 48 h de culture, les cellules ont été lavées une fois avec du PBS et incubées 3 h à 37°C avec 20 µg/ml ¹²⁵I-LDL en présence ou en absence de 1 mM d'oléate dans du DMEM contenant 0,2% BSA, 5 mM Hepes, et 2 mM CaCl₂, pH 7,5. Ensuite, les cellules ont été lavées comme décrit précédemment et incubées à 4°C pendant 1 h avec 10 mM de suramine dans du PBS.

Pour la mesure de la fixation des LDL (Figure 15), le milieu a été récupéré et passé dans un compteur γ pour évaluer la quantité de ¹²⁵I-LDL liée. Les résultats sont des moyennes de deux mesures. Pour la mesure de l'internalisation et de la dégradation des LDL (Figure 16), la quantité de ¹²⁵I-LDL internatisée et dégradée a été mesurée selon les protocoles détaillés dans l'exemple 1.

La co-transfection avec des plasmides α et β permet d'établir les trois étapes de l'activité LSR (Figures 15 et 16).

Les inventeurs ont également observé que la co-transfection avec les deux plasmides α et β augmente l'activité LSR par rapport à une transfection par seulement un plasmide α. Les résultats suggérant une activité plus efficace du LSR lorsque le rapport ([β] / [α]) entre les concentrations de sous-unités β et α exprimées croît, est compatible avec l'observation que le récepteur LSR serait constitué de l'assemblage d'une sous-unité α (ou α'), et de plusieurs, probablement trois, sous-unités β.

Les résultats montrent que seule la cotransfection des sous-unités β et α permet la surexpression d'un récepteur LSR totalement fonctionnel, en ce sens qu'il permet la dégradation protéolytique complète de la protéine.

Afin de caractériser l'activité de dégradation des lipoprotéines obtenue ci-dessus en cellules transfectées par les ADNc de LSR, les inventeurs ont enfin testé la capacité d'anticorps anti-LSR à inhiber la fixation des LDL telle que mesurée ci-dessus, ainsi que la substrat-spécificité de celle-ci.

### Caractérisation de l'activité de dégradation des lipoprotéines obtenue en cellules transfectées exprimant un récepteur LSR recombinant

Les cellules CHO ont été transfectées avec les plasmides α et β, dans un rapport de concentrations de 1 à 3.

La Figure 17A montre que l'activité de fixation des LDL obtenue dans les cellules transfectées (exprimée par rapport à la même activité observée dans des cellules contrôles non transfectées), est inhibée de façon spécifique par les anticorps anti-LSR.

La Figure 17B montre l'activité de fixation des LDL obtenue dans les cellules transfectées, en présence de diverses lipoprotéines non marquées, agissant comme ligands compétiteurs. Les résultats montrent une spécificité de ligand similaire à celle observée pour l'activité LSR endogène chez le rat (Mann et al., 1995): les chylomicrons de rat sont les substrats préférés du LSR recombinant de rat ; viennent ensuite notamment, par ordre de spécificité décroissante, les VLDL puis les LDL.

### Exemple 4 : Implication du LSR dans la clairance des cytokines

L'analyse de la séquence de la sous-unité α du LSR met en évidence une région riche en cystéine, qui correspond à une signature de récepteur à cytokines de type Tumor Necrosis Factor. Le LSR se distingue cependant des récepteurs à cytokines par la présence de signaux permettant l'endocytose rapide du complexe récepteur/ligand (motif clathrine).

Les inventeurs ont émis l'hypothèse que ce récepteur pourrait servir à l'épuration des cytokines, et notamment de la leptine ; afin de vérifier cette hypothèse, ils ont analysé la dégradation de la leptine recombinante par des hépatocytes en culture primaire selon le protocole ci-dessous.

### Dégradation de la leptine par des hépatocytes en culture primaire

Les cellules primaires d'hépatocytes de rat sont incubées pendant 4 heures à 37°C avec 20 ng/ml de ¹²⁵I-leptine en absence ou en présence de 0,5 mM d'oléate, de 75 µg/ml de RAP, de 200 µg/ml d'anticorps non spécifiques ou anti-LSR spécifiques, ou de 50 µM de chloroquinine. On récupère ensuite le milieu et on mesure la quantité de ¹²⁵I-leptine dégradée.

Comme l'indique la Figure 18, la dégradation de la leptine par des hépatocytes en culture primaire est inhibée par :
a) les anticorps polyclonaux dirigés contre le LSR. Ces anticorps inhibent également dans les mêmes proportions l'activité LSR
b) la 39 kD Receptor Associated Protein (RAP) ; cette protéine bloque l'activité LSR *in vitro* et retarde la clairance des chylomicrons *in vivo* (Troussard et al. 1995 ; Willow et al., 1994)
c) la chloroquine ; ce poison cellulaire empêche l'acidification des vésicules d'endocytose et inhibe l'activité des protéases lysosomales
d) l'oléate ; cet acide gras libre induit le changement de conformation du LSR, qui démasque le site de fixation des lipoprotéines.

Ceci indique que la conformation FAF (Fatty Acid Free) du LSR est probablement seule compatible avec la fonction de fixation suivie de dégradation de la leptine. Les immunoglobulines non-spécifiques sont sans effet sur la dégradation de la leptine (Figure 18).

Afin de vérifier la fixation de la leptine au LSR, les protéines de membrane plasmique de foie de rat ont été déposées sur une colonne de chromatographie d'affinité contenant de la leptine recombinante, selon le protocole détaillé ci-dessous.

### Chromatographie d'affinité leptine

On utilise une colonne Hi-trap (Pharmacia) : 5mg de leptine sont fixés sur 1ml de colonne, selon les méthodes préconisées par le fabricant. Les protéines de membrane plasmique sont solubilisées à partir de foies de rat comme indiqué précédemment (Mann et al., 1995), puis dialysées une nuit contre du PBS pH 7,4, 0,1% Tween 20. La colonne est lavée dans le même tampon, et l'extrait protéique est déposé à un débit de 0,2 ml/minute. La colonne est lavée par 6 ml du même tampon. Elle est ensuite éluée par le même tampon supplémenté de 100mM glycine pH 3 ; 20 fractions de 500 µl sont alors neutralisées par 5 µl de PBS, 0,1% Tween 20 pH 8. 50 µl de chaque fraction sont déposés sur membrane de nitrocellulose pour analyse en Dot blot au moyen de l'anticorps anti-LSR. Les fractions positives (1, 3, 4, 7 et 8) sont dialysées contre du bicarbonate d'ammonium 24 mM, Tween 20 0,01 %, poolées et concentrées au speed vac en un volume final de 300 µl. 40 µl du produit final sont analysés en Western blot au moyen d'anticorps anti-LSR.

La Figure 19 montre que les anticorps anti-LSR reconnaissent spécifiquement la sous-unité α qui, après s'être fixée à la leptine, a été relarguée par le tampon glycine.

Des expériences de transfection stable de la sous-unité α permettront de mesurer l'affinité de la leptine pour ce nouveau récepteur.

L'ensemble de ces résultats suggèrent que le LSR représente une des voies de dégradation et d'élimination de la leptine. L'injection *in vivo* de leptine recombinante radiomarquée a montré, tant chez les souris obèses que chez les souris contrôles, une vitesse de clairance rapide et une captation préférentielle de la leptine par le foie et le rein : 50% de la dose injectée est retrouvée après 10 minutes dans ces deux organes. Afin d'analyser les mécanismes de captation sélective de la leptine, les inventeurs ont comparé les quantités de leptine et de β2 microglobuline (protéine soluble de poids moléculaire proche de celui de la leptine, choisie comme contrôle) présentes dans le rein et le foie de souris normales et de deux lignées de souris obèses 5 minutes après injection d'une même dose traceuse de ces deux protéines radio-marquées.

### Mesure de la clairance de la leptine chez les souris

Des souris (6-8 semaines) femelles témoins, *ob*/*ob*, ou *db*/*db*, à jeun, sont anesthésiées et reçoivent par la veine saphène une injection de 80 ng de ¹²⁵I-leptine recombinante murine ou de ¹²⁵I-β₂-microglobuline (Sigma, marquée par la méthode lodobeads, comme la leptine). Cinq minutes après, les animaux sont perfusés avec une solution saline physiologique (15 ml, à 4°C). Les tissus sont prélevés, et comptés pour leur radioactivité (compteur Gamma). Dans certains cas, un anti-corps anti-LSR ou une protéine contrôle sont injectés 30 minutes avant l'injection de ¹²⁵I-leptine. Il est important de noter que le marquage de la leptine avec l'¹²⁵I n'a pas d'effet sur son activité biologique.

Les résultats présentés en Figure 20 montrent que la quantité de leptine sélectivement captée par le foie est diminuée dans les souris obèses, par rapport aux souris contrôles ; par ailleurs, on ne trouve pas de différence entre les différentes lignées pour ce qui concerne la captation rénale de la leptine.

Les inventeurs ont ensuite mesuré le nombre de récepteurs LSR chez des souris témoins, *ob*/*ob* et *db*/*db* selon le protocole suivant.

### Mesure du nombre apparent de récepteurs LSR sur membranes plasmiques

Le nombre apparent de récepteurs LSR sur membranes plasmiques est mesuré comme décrit précédemment (Mann et al., 1995) par estimation de la quantité de LDL fixée sur une préparation de membrane plasmique. Les membranes plasmiques (100µg) sont incubées avec 1mM oléate ; elles sont ensuite lavées trois fois comme indiqué ci-dessus, puis incubées 1 heure à 37°C avec 40 µg/ml d'¹²⁵I-LDL. La quantité d'¹²⁵I-LDL fixés aux membranes plasmiques est ensuite déterminée par comptage. On établit la moyenne sur 3 mesures par animal pour 3 animaux différents dans chacun des groupes.

La Figure 21 montre que le nombre de récepteurs LSR chez des animaux obèses présentant soit un déficit en leptine (*ob*/*ob*), soit un déficit de l'ob récepteur (*db*/*db*), est diminué significativement. La diminution de la captation hépatique sélective de la leptine chez les souris obèses coïncide avec la diminution chez ces animaux du nombre apparent de récepteurs LSR.

Les inventeurs ont enfin testé, selon le protocole exposé ci-dessous, l'effet d'anticorps anti-LSR sur la distribution de la leptine entre foie et rein, 5 minutes après injection d'une dose traceuse.

### Mesure de la distribution de la leptine entre foie et rein en présence d'anticorps anti-LSR

On anesthésie des souris témoins puis on leur injecte par intraveineuse 1 mg d'anticorps IgG non spécifiques ou d'anticorps IgG anti-LSR. Après 30 minutes, on injecte 80 ng de ¹²⁵I-leptine et, après 5 minutes, une perfusion de solution saline physiologique à 4°C. On prélève immédiatement les tissus et on mesure la radioactivité. Les résultats représentent la moyenne et la déviation standard obtenues pour 3 animaux pour chacun des groupes.

Comme le montre la Figure 22, la captation hépatique de la leptine est diminuée, et la captation rénale est augmentée par les anticorps anti-LSR, en comparaison avec des immunoglobulines contrôles.

Ces résultats indiquent donc que le LSR est responsable de la captation hépatique sélective de la leptine et qu'une diminution du nombre de récepteurs est observée chez les animaux obèses. Une telle diminution peut expliquer le syndrome de résistance à la leptine et l'augmentation de la concentration plasmatique de la leptine qui est observée chez la plupart des sujets humains obèses.

Il est également possible que le récepteur LSR serve de voie de dégradation pour d'autres cytokines, notamment celles produites par le tissu adipeux. On retiendra particulièrement l'importance du Tumor Necrosis Factor α et du Nerve Growth Factor. Ces deux cytokines exercent un effet amaigrissant significatif lorsqu'elles sont injectées à des sujets humains (Cytokines and their receptors, 1996).

### Exemple 5 : Contrôle de l'activité LSR par les cytokines

La sous-unité α du récepteur LSR fixe la leptine et possède des sites potentiels de phosphorylation. Ceci en fait un récepteur qui non seulement médie l'endocytose mais pourrait également servir à la signalisation cellulaire.

Les inventeurs ont donc testé l'hypothèse selon laquelle la leptine module l'activité du LSR, comme décrit ci-dessous.

### Mesure de l'activité LSR de fixation, d'internalisation et de dégradation des lipoprotéines en présence de leptine

Les hépatocytes de rat en culture primaire sont incubés à 37°C pendant 30 min. avec une concentration croissante de leptine, puis incubés à 37°C pendant 4 heures avec soit 50 µg/ml de ¹²⁵I-LDL (activité spécifique : 209 cpm/ng) ou soit 50 µg/ml de ¹²⁵I-VLDL (activité spécifique : 157 cpm/ng) en absence ou en présence de 500 µM d'oléate. On lave ensuite les cellules et on mesure les quantités de ¹²⁵I-lipoprotéines fixées, incorporées et dégradées comme décrit précédemment dans l'exemple 1 (Bihain et Yen, 1992). Les résultats montrés Figure 23 représentent les différences obtenues entre les cellules incubées avec ou sans oléate. Chaque point représente la moyenne de 3 mesures. La déviation standard de chaque point est comprise dans le symbole.

L'ajout de concentrations croissantes de leptine à des hépatocytes en culture augmente la fixation, l'internalisation et la dégradation des VLDL et des LDL (Figure 23).

### Analyse de la capacité d'induction par la leptine de l'activité LSR

### Mesure, en présence de leptine, du nombre apparent de récepteurs LSR exprimés en surface d'hépatocytes de rats en culture primaire

Des cultures primaires d'hépatocytes de rat sont incubées pendant 30 min à 37°C en présence ou absence de 20 ng/ml de leptine, 10 min à 37°C en présence de 0,8 mM d'oléate. Les cellules sont lavées avec du tampon PBS pré-refroidi à 4°C, puis incubées 2 heures à 4°C en présence de concentrations croissantes de ¹²⁵I-LDL. Les cellules sont ensuite lavées, lysées et on mesure la quantité de ¹²⁵I-LDL fixée.

### Effets comparés de la leptine en présence de cycloheximide, de colchicine et de cytochalasine B

Les conditions initiales sont identiques à celles décrites ci-dessus ; après incubation avec la leptine, on incube les cellules pendant 30 min à 37°C avec 5 µM de cycloheximide, 5 µM de colchicine ou 2,5 µM de cytochalasine B. Les cellules sont ensuite incubées 10 min à 37°C en présence de 0,8 mM d'oléate. Les cellules sont alors lavées avec du tampon PBS pré-refroidi à 4°C, puis incubées 2 heures à 4°C en présence de 50 µg/ml de ¹²⁵I-LDL. 2 mesures sont effectuées, et les résultats moyens sont présentés.

On montre ainsi que l'augmentation de l'activité LSR par la leptine est obtenue à travers une augmentation du nombre apparent de récepteurs exprimés à la surface des hépatocytes (Figure 24 A). Cette augmentation résulte d'une part d'une augmentation de la synthèse protéique (elle est inhibée partiellement par la cycloheximide, inhibiteur de la synthèse protéique). Elle implique d'autre part la mobilisation des vésicules d'endocytose par le système des microtubules (elle est en effet inhibée par la cytochalasine B qui bloque le transport microtubulaire) (Figure 24 B).

Afin de vérifier *in vivo* l'effet de la leptine sur l'activité LSR, les inventeurs ont caractérisé la réponse triglycéridémique post-prandiale de souris contrôles, *ob*/*ob* et db/db, après un repas test de gavage selon les protocoles suivants.

### Mesure de la réponse lipémique postprandiale chez les souris

Des souris contrôles, *ob*/*ob* et *db*/*db* à jeun depuis la veille sont gavées avec un repas très riche en graisse [60% de graisse (acides gras saturés 37%, monoinsaturés 27% et polyinsaturés 36%), 20% de protéines et 20% d'hydrate de carbone] fournissant 56 kcal d'énergie / kg du poids de l'animal. Immédiatement après le repas (temps = 0 heure), on injecte par intraveineuse aux souris 200 µl de solution saline physiologique. A différents temps, on prélève dans des tubes contenant 90µg d'EDTA disodique, 20 µl de sang par la veine caudale, et après séparation du plasma par centrifugation, on détermine la concentration plasmatique en triglycérides à l'aide d'un kit de dosage enzymatique. Chaque point des courbes présentées correspond à la moyenne avec écart-type obtenue pour 3 mesures par animal et pour 3 animaux différents.

### Mesure de l'effet de la leptine sur la réponse lipémique post-prandiale chez les souris

La procédure est la même que ci-dessus, excepté qu'immédiatement après le repas (temps = 0 heure), on injecte par intraveineuse aux souris, soit 200 µl de solution saline physiologique, soit 200 µl d'une même solution contenant 50 µg de leptine recombinante murine.

### Mesure de la réponse lipémique post-prandiale chez les souris en présence de lactoferrine et/ou de leptine

Des souris *ob*/*ob*, à jeun depuis la veille, sont gavées avec un repas identique à celui décrit ci-dessus. Immédiatement après le repas (temps = 0 heure), on injecte aux souris par intraveineuse 200 µl de solution saline contenant soit aucun supplément, soit 0,5 µg de leptine, soit 2,5 mg de lactoferrine ou soit un mélange de 0,5 µg de leptine et de 2,5 mg de lactoferrine. Le sang est prélevé entre 2 et 3 heures après le repas et la concentration plasmatique en triglycérides (TG) est mesurée. Les valeurs obtenues représentent la moyenne avec écart-type obtenue pour 4 mesures par animal et pour 2 animaux différents [p < 0,02 (*ob*/*ob* comparé à *ob*/*ob* + leptine), p < 0,01 (*ob*/*ob* comparé à *ob*/*ob* + lactoferrine), NS (*ob*/*ob* + lactoferrine comparé à *ob*/*ob* + leptine + lactoferrine)].

En accord avec la réduction du nombre de récepteurs LSR observée chez les souris obèses, une amplification de la réponse lipémique post-prandiale est également observée chez les souris obèses non traitées. L'administration de leptine par voie intraveineuse en même temps que le repas test permet de réduire la réponse lipémique post-prandiale dans les deux lignées de souris obèses et chez les souris contrôles (Figure 25).

La diminution de la réponse lipémique induite par la leptine est supprimée par l'administration de lactoferrine (Figure 26), qui bloque l'activité du LSR (Yen et al., 1994 ; Mann et al., 1995). Ceci suggère fortement que la diminution de la réponse lipémique s'explique par une augmentation de l'activité LSR.

Enfin, *in vivo* aussi, l'administration de leptine induit une augmentation du nombre apparent de récepteurs LSR exprimés au niveau de la surface des hépatocytes. Cette augmentation est significative, tant chez les souris *ob*/*ob* que chez les souris *db*/*db* (Figure 27).

La leptine et vraisemblablement d'autres cytokines sont donc régulateurs de l'activité du LSR. Un syndrome de résistance à la leptine, ou à d'autres cytokines, peut entraîner une hypertriglycéridémie, soit permanente, soit limitée à la phase postprandiale.

### Exemple 6 : Effet de la leptine sur l'expression du LSR ; incidences thérapeutiques

Afin de renforcer la corrélation entre l'administration de leptine, la diminution de la réponse lipémique post-prandiale, et une expression ou activité accrue du récepteur LSR, et de mieux cerner les éventuelles implications thérapeutiques de l'induction de l'activité de clairance hépatique des lipoprotéines par la leptine, les inventeurs ont complété les analyses précédentes d'un suivi de l'évolution du poids, de l'activité LSR et de l'expression d'ARNm de LSR, chez les animaux contrôles ou obèses, traités ou non par la leptine.

### Réponse lipémique post-prandiale et activité LSR chez les souris témoins et obèses

Des souris mâles contrôles (C57BL6) (n=8), et obèses (ob/ob, n=8 - animaux déficients au niveau du gène de la leptine - et db/db, n=8 - animaux déficients au niveau du gène du récepteur à la leptine -) (âgés de 17 semaines) ont été pesées afin d'établir de façon quantitative les différences de poids entre lignées (Figure 28A). Les réponses lipémiques post-prandiales des animaux de chaque lignée ont été mesurées en l'absence d'un traitement par la leptine comme décrit ci-dessus. Le nombre apparent de récepteurs LSR exprimé à la surface des cellules hépatiques a été mesuré sur 4 animaux de chaque lignée, comme décrit plus haut, et exprimé en comparaison de l'activité 5'-nucléotidase (enzyme mesurée sélectivement au niveau des membranes plasmiques ; kit Sigma). Enfin un Northern blotting a permis d'estimer le niveau d'expression du récepteur LSR chez trois animaux de chaque lignée, suivant le protocole décrit précédemment.

La réponse lipémique postprandiale plus élevée chez les animaux obèses (Figure 28B) est en accord avec le plus faible nombre apparent de récepteurs LSR hépatiques chez ces mêmes animaux (Figure 28C). De plus, les résultats du Northern blotting (Figure 28D) indiquent que cette diminution du nombre apparent de récepteurs LSR chez les animaux obèses s'accompagne d'une diminution du taux d'expression dudit récepteur chez les mêmes animaux. Les inventeurs ont montré qu'en effet, on constate une diminution du nombre d'ARNm codant pour le récepteur LSR chez les souris obèses *ob*/*ob* et *db*/*db*.

Les inventeurs ont également étudié l'effet d'un traitement à long terme d'un traitement par la leptine sur des souris ob/ob (Figure 29).

### Effet d'un traitement à long terme par la leptine sur des souris ob/ob

Les souris obèses *ob*/*ob* reçoivent une injection quotidienne, soit de leptine, soit d'un volume équivalent de PBS stérile, pendant 30 jours. Les doses injectées sont de 50 µg/animal du jour 0 au jour 4, de 100 µg/animal du jour 5 au jour 17, et de 150 µg/animal du jour 18 au jour 30. Plusieurs paramètres indiqués ci-dessous sont mesurés :
- *le poids* (Figure 29 A) : le changement de poids est mesuré pour 6 animaux, sur la durée du traitement ;
- *la réponse lipémique post-prandiale* (Figure 29 B) : elle est mesurée selon le protocole détaillé dans l'exemple 5 sur trois animaux de chaque groupe, au jour 29.
- *le nombre apparent de récepteurs LSR* (Figure 29 C) : il est mesuré selon le protocole détaillé dans l'exemple 4 sur trois animaux de chaque groupe, au jour 30.
- *la quantité d'ARNm de LSR* (Figure 23 D) : elle est estimée par Northern blot comme indiqué dans le protocole de l'exemple 2.

Les inventeurs ont ainsi constaté une perte de poids très significative chez les souris obèses *ob*/*ob* traitées sur 30 jours avec la leptine. De plus, le traitement par la leptine provoque une diminution nette de la réponse lipémique postprandiale. Cette diminution de la réponse lipémique postprandiale est corrélée à une augmentation du nombre apparent de récepteurs LSR à la surface des cellules et à une augmentation de la quantité d'ARNm codant pour les sous-unités du récepteur LSR.

Ces résultats établissent *in vivo* que le LSR représente l'étape limitante de l'épuration des lipides alimentaires. De plus, le traitement de cette obésité induisant une perte de poids, provoque une augmentation de l'activité de dégradation hépatique des lipides alimentaires, et une diminution de la réponse lipémique post-prandiale.

### Exemple 7 : Caractérisation du récepteur LSR humain

### Analyse en Northern blot

Des sondes nucléiques de LSR de rat ont été utilisées pour réaliser des Northem blots avec une membrane (Human Multiple Tissue Northern Blot, Clontech #7760-1) comportant des ARNs poly A de Coeur, Cerveau, Placenta, Poumon, Foie, Muscle squelettique, Rein et Pancréas humains. Une bande d'environ 2 kpb est mise en évidence dans le foie et dans le rein. La quantification approximative des résultats d'hybridation indique que le récepteur LSR est exprimé dans le foie au moins 5 fois plus que dans le rein.

### Clonage du cDNA ; étude de la zone d'épissage

Des expériences de transcription inverse-PCR sur l'ARNm ont permis de déterminer avec plus de précision la taille de l'exon 1 du côté 5' et les sites d'épissage entre les exons 1 et 2. Toutefois, il n'est pas certain que cette extrémité constitue le début de cet exon. En outre, il existe un deuxième site d'initiation dans l'exon 1 qui se trouve plus en aval du premier et qui présente une probabilité plus grande que celui-ci. L'épissage entre les exons 1 et 2 était différent entre l'ARN humain et celui de rat.

L'amplification a été réalisée avec plusieurs couples d'amorces :

L'amplification réalisée avec le couple d'amorces ab a conduit à deux produits de taille 1,8 kb et 2 kb après séparation sur gel d'électrophorèse. Les tailles de ces deux produits pouvant s'expliquer par un épissage alternatif similaire à celui décrit chez le rat, les autres amorces d'amplification ont été dessinées. Ces amorces ont permis de mettre en évidence les trois formes de cDNA résultant de l'épissage alternatif de l'ARN.

Le premier ADNc qui contient la totalité des dix exons est appelé LSR-Hs-2062 et correspond à la SEQ ID 7. II correspond à l'ADNc de rat LSR-Rn-2097. Le deuxième ADNc contient les exons 1, 2, 3, 5, 6, 7, 8, 9 et 10, et est appelé LSR-Hs-2005. II correspond à la SEQ ID 9. Cet ADNc correspond à l'ADNc de rat LSR-Rn-2040. Enfin, l'ADNc contenant les exons 1, 2, 3, 6, 7, 8, 9 et 10 est appelé LSR-Hs-1858 et sa séquence est listée en SEQ ID 11. II correspond à l'ADNc de rat LSR-Rn-1893.

Il est à noter qu'un glissement de site d'épissage a pu être mis en évidence à la frontière de l'exon 8. Ce glissement, du triplet TAG en position 19953-19955 de SEQ ID 19 au triplet contigü AAG en position 19956-19958 de SEQ ID 19, résulte en la perte du résidu Glu en position 386 de l'ADNc de SEQ ID 8.

Les séquences des protéines codées par les ADNc LSR-Hs-2062, LSR-Hs-2005 et LSR-Hs-1858 correspondent respectivement aux SEQ ID 8, 10 et 12. Les séquences protéiques biologiques peuvent commencer au premier codon ATG observé dans la phase de lecture (position 35 de la séquence protéique). Cependant, le codon d'initiation de la traduction préféré se trouve plus en aval en position 83 de la séquence protéique. De plus, il est tout à fait possible que ce codon initiateur soit plus en amont dans la région 5' de l'exon 1 non encore déterminé ou dans un éventuel exon précédent celui-ci.

Enfin, la Figure 3 présente une représentation schématique des différentes formes de protéines mises en évidence chez l'homme en indiquant les motifs conservés.

Cette analyse permet de conclure à l'existence, chez l'homme, de trois sous-unités α, α' et β du LSR équivalentes aux formes LSR 66, LSR 64 et LSR 58 du rat.

### Identification et isolement de la séquence génomique du LSR humain

Un criblage des banques de données de séquences nucléiques publiques (Genebank, version : 101) aussi bien avec la séquence de lisch7 de souris (N° Accession : U49507) qu'avec celle du LSR-2097 de rat isolée par les inventeurs a permis d'isoler deux séquences d'ADN génomique humain. Il s'agit des cosmides dont les numéros d'accession sont AC002128 et AD000684, de tailles respectives 45.328 pb et 41.936 pb. Ces deux cosmides se recouvrent partiellement. L'extrémité 3' du cosmide AC002128 chevauche sur 12 838 pb l'extrémité 5' du cosmide AD000684. Sur la portion commune de 12 838 pb, les séquences sont identiques à 100%, mises à part deux délétions en positions 822 et 3170 du cosmide AD00684. Le gène du LSR humain est réparti sur les deux cosmides. Pour faciliter l'étude de cette région, une séquence génomique complète a été reconstituée : les 45 328 pb du cosmide AC002128 ont été rajoutées à la séquence du cosmide AD000684 comprise entre la base 12 839 et la base 41 936. L'ensemble constitue une séquence de 74 426 pb. Une séquence génomique couvrant le gène du LSR a été extraite (SEQ ID 19).

Les exons putatifs du gène LSR ont été déterminés après alignement de la séquence ci-dessus décrite avec les séquences des ARNs de Lisch7 de souris et de LSR de rat. La validité des sites d'épissage de part et d'autre des exons putatifs a été vérifiée.

Par ailleurs, une banque génomique humaine constituée de BACs a été criblée par les méthodes décrites dans Chumakov et al., 1995 ; les clones ainsi isolés ont été contigués, et sous-clonés puis séquencés afin d'obtenir la séquence génomique humaine codant pour le LSR (SEQ ID 41 ).

Les deux séquences ainsi obtenues (SEQ ID 19 et 41) portent des divergences mineures, mentionnées dans les listings ci-joints.

### Exemple 8 : Activité LSR chez l'homme

Des cultures primaires de fibroblastes humains isolés à partir de sujets présentant une délétion affectant le promoteur et le premier exon du gène du récepteur LDL ont été obtenues.

L'incubation de ces cellules en présence et en absence d'oléate montre que celui-ci induit une activité de fixation, d'internalisation et de dégradation des LDL qui suit une cinétique de saturation (Bihain et Yen, 1992). L'affinité de ce récepteur induit par l'oléate est maximale pour les particules riches en triglycérides (VLDL et chylomycrons) ainsi que pour des particules de trioléine et phosphatidylcholine supplémentées avec de l'apoprotéine E recombinante. L'affinité des LDL pour le récepteur est plus faible que celle des VLDL et des chylomicrons mais toutefois plus élevée que celles de particules de trioléine, phosphatidylcholine ne contenant pas d'ApoE, ou que celles de VLDL isolés à partir d'un sujet présentant une hyperlipidémie de type III et le phénotype E2/2 de l'Apo E (Yen et al., 1994).

L'activité du LSR a également pu être mesurée dans les fibroblastes de sujets humains normaux (Figure 30), suivant le protocole ci-dessous.

### Mesure de la fixation, de l'internalisaion et de la dégradation des LDL par des fibroblastes.

Les fibroblastes sont préalablement cultivés durant une semaine comme décrit précédemment, à l'exception que le milieu contient 20% de sérum bovin foetal (Goldstein et al., 1983). Ensuite, ils sont incubés avec des concentrations croissantes de ¹²⁵I-LDL en absence ou en présence de 1mM oléate. Les cellules sont ensuite lavées, lysées, et comptées pour leur radio-activité.

### Exemple 9 : Effet de la leptine sur l'activité LSR chez l'homme

L'activité LSR de fibroblastes humains FH (familial hypercholesterolemia) est également augmentée après incubation avec de la leptine (Figure 31 ), suggérant que tout comme chez le rat, le LSR participe chez l'homme à la clairance des cytokines, et voit son activité modulée par celles-ci. Les mesures correspondantes ont été effectuées comme indiqué ci-après.

### Effet de la leptine sur l'activité LSR sur des fibroblastes humains

Les fibroblastes FH sont incubés 30 minutes à 37°C avec des concentrations croissantes de leptine, puis 2 heures à 37°C avec 50µg/ml de ¹²⁵I-LDL, en présence de 500µM d'oléate. La fixation, l'internalisation et la dégradation des LDL sont mesurées comme indiqués dans l'exemple 1.

### Exemple 10 : Clonage de l'ADNc du LSR de souris ; analyse des produits de l'épissage alternatif.

Le clonage de l'ADNc du LSR de souris a été réalisé à partir d'une banque d'ARNm de foie de souris. La méthode de clonage utilisée est la même que pour l'ADNc du LSR humain. Les ARNm ont été purifiés et une amplification par transcription inverse par PCR avec les amorces d'ADN spécifiques a été réalisée. Le fragment d'amplification a été cloné dans un vecteur de clonage TA (Introgene).

Une étude des produits de l'épissage alternatif avec des amorces situées dans l'exon 2 et dans l'exon 9 a également été réalisée de manière similaire à celle effectuée pour le LSR humain.

Trois produits d'épissage alternatif ont été observés : LSR-Mm-1886, LSR-Mm-1829 et LSR-Mm-1682. LSR-Mm-1886 contient tous les exons de 1 à 10. LSR-Mm-1829 et LSR-Mm-1682 sont dénués des exons 4 et des exons 4 et 5, respectivement. Ces trois formes biologiques d'ADNc correspondent bien à ce qui a été observé chez l'homme et le rat. Les séquences nucléotidiques des ADNc LSR-Mm-1886, LSR-Mm-1829 et LSR-Mm-1682 sont illustrées dans les SEQ ID 13, 14 et 15, respectivement. Les séquences protéiques codées par les ADNc LSR-Mm-1886, LSR-Mm-1829 et LSR-Mm-1682 sont illustrées dans les SEQ ID 16,17 et 18.

### Exemple 11 : Identification de la sous-unité y du LSR

Les sous-unités α et β du LSR ont été identifiées comme indiqué ci-dessus. L'analyse des produits de traduction des ARNs codant pour ces deux sous-unités ne permet pas d'expliquer la présence d'une troisième sous-unité de poids moléculaire = 35 kDa. Cette dernière n'est détectée qu'après réduction du complexe LSR (Figure 10, ligne 4).

Nous avons purifié et obtenu la séquence NH₂ terminale de cette sous-unité γ.

La purification a été réalisée par chromatographie d'immunoaffinité selon la procédure suivante.

### Purification de la sous-unité γdu LSR

Des anticorps anti-LSR (bande A) sont couplés à une résine [2,5 mg d'IgG pour 3,5 ml de résine affi-gel Hz immunoaffinity kit (Biorad 153-6060)] qui est ensuite incubée avec des protéines solubilisées à partir de membranes totales de foie de rat (tampon Tris 20 mM, EDTA 2mM, Octylglucoside 0,125 M (5 X CMC), inhibiteur cocktail 1%, PH 7.4 : 160 mg de protéines membranaires donnent 41,3 mg de protéines solubilisées (PS) dans un volume de 17 ml.
- L'incubation est réalisée pendant 12 heures : 17 ml complétés à 50 ml avec du tampon Tris 20 mM, EDTA 2 mM, pH 7,4 avec les 3,5 ml de résine, sous agitation rotative, à température ambiante. La résine est lavée avec 40 ml de tampon Tris 20 mM, EDTA 2mM, pH 7,4 puis éluée avec du tampon Tris 20 mM, EDTA 2 mM, glycine 200 mM, pH 2,5 en 30 fractions de 500 µl. Le pH de chaque fraction est neutralisé avec 100 µl par tube de tampon Tris 1 M, EDTA 2mM, pH 9. 50 µl de chaque fraction sont déposés sur une membrane de nitrocellulose pour analyse en dot blot : incubation avec l'anticorps anti-LSR, puis avec un second anticorps couplé à la phosphatase alcaline.
- Les fractions positives de 7 à 28 sont poolées 2 par 2 et concentrées 2,5 fois au Speedvac. Un Western blot sur les fractions poolées concentrées et séparées, sur un gel PAGE-SDS 10% est réalisé. Des bandes sont observées dans les fractions 7 à 14 (les fractions sont poolées).
- Les deux pools sont dialysés contre du bicarbonate d'ammonium 24 mM, puis lyophilisés au Speedvac. La poudre est reprise dans 80 µl de tampon Tris 20 mM, EDTA 2 mM, SDS 2 %, Urée 3%, pH 7,4 réduite en présence de 5% β-mercaptoéthanol 30 minutes à 100°C.
- Après migration et transfert humide en Tris 50 mM, Borate 50 mM sur une membrane de séquençage (PVDF) à 30 mA, la membrane est colorée à l'Amido black.

Une bande de PM apparent d'environ 35 kDa a ainsi été identifiée et envoyée pour séquençage selon la méthode Edman.

La séquence obtenue est LHTGDKAFVEFLTDEIKEE. Cette séquence correspond à l'identique à celle d'une protéine de poids moléculaire 33 kDa identifiée précédemment comme une protéine de la surface cellulaire qui fixe les têtes globulaires du C1q (gC1q-R)(Ghebrehiwet et al., 1994). Une observation plus récente indique que ce récepteur potentiel du C1q est localisé également dans des vésicules situées sous la surface cellulaires (van den Berg et al., 1997). Cette protéine correspond également à une protéine précédemment identifiée comme p34, et qui s'associe à un récepteur de la lamine. Ce récepteur possède un long segment NH₂ terminal orienté vers l'intérieur du noyau cellulaire ainsi que 8 domaines transmembranaires. Ce récepteur se fixe à la lamine d'une façon qui dépend du degré de phosphorylation. Enfin gC1q-R s'associe avec le «splicing factor 2 » (Honoré et al., 1993). Le récepteur de la lamine et le « splicing factor 2 » présente en commun la caractéristique de contenir une séquence répétée de sérine et d'arginine (RSRS) située au niveau du segment NH2 terminal dans le cas du récepteur de la lamine et au niveau carboxy terminal dans le cas du SF2.

Il est remarquable de constater que tant LSR α que LSR β présentent des segments répétés riches en sérine et arginine (Figure 1). Notre hypothèse est que la protéine LSR γ représente un chaperon moléculaire qui s'associe aux sous-unités α et β du LSR via leur domaine RSRS.

Afin de vérifier cette hypothèse, nous avons obtenu des anticorps polyclonaux dirigés contre deux peptides synthétiques, dont la séquence était située à l'extrémité carboxy ou NH₂ terminale de la protéine gC1q-R :

La Figure 32 montre que ces anticorps inhibent spécifiquement l'activité du LSR. L'anticorps dirigé contre l'extrémité COOH terminale semble le plus efficace.
Ces résultats indiquent que gC1q-R, ou un de ses homologues structurellement proche, représente un chaperon moléculaire associé de façon non covalente au complexe multimérique LSR.

### Exemple 12 : Régulation de l'activité LSR par le C1q et ses homologues

Il a été montré que gC1q-R pouvait fixer la tête globulaire du facteur 1 du Complément. Nous avons cherché à utiliser cette propriété du C1q pour déplacer gC1q-R associé au LSR, et avons mesuré l'effet de doses croissantes de C1q sur la fixation, l'internalisation et la dégradation des LDLs par des hépatocytes en culture primaire. La Figure 33 montre une augmentation de la captation et dégradation des LDLs induite par le C1 q humain, même en l'absence d'oléate.

Une augmentation moins importante mais néanmoins significative est également observée en présence d'oléate. Toutefois dans ces conditions, l'effet maximal est obtenu pour des concentrations plus faibles de C1q.

Il apparaît donc que le gC1q-R exerce à l'égard du LSR un effet inhibiteur qui est comparable à celui induit par la 39 kD RAP à l'égard de la LRP, du LDL-récepteur, et du LSR (Troussard et al., 1995). Le déplacement du chaperon gC1q-R en utilisant sa capacité à se fixer au complément C1q permet la levée de l'effet inhibiteur. L'analyse de la séquence de gC1q-R fait apparaître qu'il ne peut s'agir d'un récepteur membranaire typique. En effet, la protéine ne possède pas de séquence hydrophobe susceptible de traverser la bicouche phospholipidique.

L'effet de complément C1q sur l'activité du LSR ouvre des perspectives importantes dans le cadre de la génétique de l'obésité. Il est possible en effet que des mutations affectant soit le gène du C1q, celui du gC1q-R, soit encore celui de leurs analogues comme par exemple AdipoQ , la cérébelline, le collagène alpha 1-10, SPA et SPD (protéines du surfactant pulmonaire), la protéine se liant au mannane, et le récepteur scavenger ou son homologue la LRP (Hu et al., 1996 ; Drickamer et al., 1986 ; Krieger et Herz, 1994 ; Elomaa et al., 1995) modulent l'activité du LSR, tant vis-à-vis de la clairance des lipoprotéines, que vis-à-vis de celle de la leptine.

Plusieurs protéines peuvent interagir avec gC1q-R car elles présentent des homologies avec le complément C1q. En particulier deux protéines isolées chez la souris, AdipoQ (Hu et al., 1996) et acrp30 (Scherer et al., 1995), et une protéine humaine APM1 (Maeda et al., 1996) présentent des homologies marquantes. Ces trois protéines, comme les éléments du complément C1q (C1q A, B, C) sont des protéines sécrétées ; elles ont une extrémité NH₂-terminale ressemblant au collagène (répétition de motifs Gly-X-Y) et une extrémité COOH-terminale correspondant au domaine globulaire du complément C1q. Ces trois protéines sont préférentiellement exprimées dans le tissu adipeux. Il n'y a que 3 acides aminés de différence entre AdipoQ et acrp30. APM1, protéine dont le messager a été caractérisé comme étant très exprimé dans des adipocytes, présente 79,7% d'identité en acide nucléique et 80,6% en acide aminé avec AdipoQ. APM1 est donc certainement l'homologue humaine de AdipoQ.

### Exemple 13 : Criblage de composés modifiant l'activité du récepteur LSR

Comme décrit précédemment, les inventeurs ont posé l'hypothèse que la « bande γ » du LSR, protéine très homologue à gC1qR, interagirait avec le récepteur LSR comme un chaperon moléculaire et formerait ainsi un « complexe LSR », comprenant les sous-unités α ou α' et β du récepteur LSR et une molécule de type gC1qR. gC1qR a précédemment été identifiée comme une protéine de la surface cellulaire qui fixe les têtes globulaires du facteur de complément C1q. Outre C1q, plusieurs protéines présentant des homologies avec les protéines C1q, notamment AdipoQ et acrp30 chez la souris et APM1 chez l'homme, sont susceptibles d'interagir avec la protéine homologue à gC1qR dans le complexe LSR et de modifier l'activité LSR.

### Paramètres du criblage

Le criblage d'un composé tel que C1 q ou AdipoQ a été réalisé à travers la mesure de différents paramètres dont le principal est la mesure de l'effet du composé sur l'activité du récepteur LSR. Les différents paramètres sont les suivants :
- le changement de poids
- la prise de nourriture
- la réponse lipémique post-prandiale
- la fixation, l'internalisation et/ou la dégradation des lipoprotéines telles que les LDL.

### Le changement de poids

Des pompes osmotiques ont été insérées chirurgicalement dans la cavités abdominales de 12 rats mâles Sprague-Drawley de 400-450 g. Les pompes osmotiques contenaient soit 2 ml de PBS (phosphate buffer saline), pH 7,4 (contrôle, 6 rats), soit 2 ml de protéine AdipoQ recombinante (5 mg/ml PBS, 6 rats). Ces pompes ont été conçues pour délivrer 10 µl/h (50 µg AdipoQ/h). Les animaux sont pesés et logés individuellement dans des cages métaboliques. 3 animaux de chaque groupe sont soumis ad libitum soit à un régime normal soit à un régime gras (jour 0). Le régime gras consiste en un régime normal supplémenté avec 2% (p/w) de cholestérol, 10% (p/w) d'acide gras saturé sous forme de végétaline, % (p/w) d'huile de tournesol et 15% (p/w) de sucrose. Au jour 3, les animaux sont pesés et des prélèvements de sang sont obtenus par la veine caudale. La quantité de triglycérides plasmatiques a été mesurée en utilisant un kit enzymatique.

### La prise de nourriture

La protéine AdipoQ recombinante (100 µg) ou du PBS seul ont été injectés quotidiennement pendant 5 jours par la veine caudale de souris ob/ob ou db/db gardées en cage métabolique. Les souris sont pesées chaque jour et la quantité de nourriture consommée a aussi été mesurée. Les résultats correspondent à une prise de nourriture moyenne et une déviation standard pour 4 souris dans chaque groupe.

### La réponse lipémique post-prandiale

Des rats Sprague-Drawley mâles (400-450 g) à jeun depuis la veille ont été gavés avec un repas très riche en graisse (t=0) (60% d'acide gras dont 37 % saturé, 27% mono-insaturé et 36% poly-insaturé, 20% de protéine et 20% d'hydrate de carbone, le total fournissant 56 kcal/kg de poids corporel) et ont reçu immédiatement après une injection intraveineuse (veine fémorale) de soit 300 µl de PBS seul soit d'un même volume contenant 1 mg de protéine AdipoQ recombinante de souris. Des échantillons de sang ont été prélevés à différents temps (0, 2, 4 et 6 h). La quantité de triglycérides plasmatiques a été mesurée en utilisant un kit enzymatique. Les résultats sont présentés comme des moyennes et des déviations standard sur 3 animaux.

### L'activité LSR ou fixation, internalisation et dégradation des lipoprotéines

Des cultures primaires d'hépatocytes de rat ont été préparées et distribuées dans des plaques à 6 puits (9000 000 cellules/puit). Après 48 h, les cellules ont été lavées une fois avec du PBS (2 ml/puit) et incubées 30 min à 37°C avec 20 ng/ml de leptine murine recombinante. Les cellules ont été ensuite incubées 4 h à 37°C avec des concentrations croissantes de protéines AdipoQ murines recombinantes et 20 µg/ml ¹²⁵I-LDL en présence ou absence de 0,5 mM d'oléate. La fixation, l'internalisation et la dégradation des lipoprotéines ont été mesurées comme indiqué dans l'exemple 1.

### C1q

Le composé C1q a été testé pour sa capacité à moduler l'activité du récepteur LSR (fixation, internalisation et dégradation des lipoprotéines). La figure 33 montre que le composé C1q présente la propriété d'augmenter l'activité en présence et en absence d'oléate. Ainsi ce composé C1q a pu être sélectionné comme modulateur de l'activité LSR à travers le test d'activité décrit ci-dessus.

### AdipoQ

Le composé AdipoQ a été testé selon les quatre paramètres exposés ci-dessus.

La figure 34 montre que le composé AdipoQ module l'activité LSR en présence d'oléate. En effet,à la concentration de 25 ng/ml, il augmente l'activité LSR.

La figure 35 montre que l'administration d'AdipoQ permet de réduire de façon massive la réponse lipémique postprandiale.

La figure 36 montre qu'un traitement ip infusion de 3 jours par AdipoQ provoque une perte de poids qui est beaucoup plus forte lorsque le rat est soumis à un régime gras. De plus, les inventeurs ont constaté que le taux de triglycérides plasmatiques est réduit chez les animaux traités avec AdipoQ.

La figure 37 montre qu'une injection d'AdipoQ diminue la prise de nourriture chez des animaux obèses.

L'augmentation de l'activité LSR induite par 25 ng/ml d'AdipoQ peut expliquer la diminution de la réponse lipémique post-prandiale et la perte de poids.

Ainsi, la protéine AdipoQ est un composé très intéressant qui pourrait être utilisé notamment dans le traitement de l'obésité. La sélection de cette protéine comme molécule candidate au traitement de l'obésité valide les paramètres de criblage de composé d'intérêt modulant l'activité LSR, le paramètre le plus important consistant en la mesure de l'activité LSR.

### REFERENCES

Aalto-Setälä, K., Fisher, E.A., Chen, X., Chajek-shaul, T., Hayek, T., Zechner, R., Walsh, A., Ramakrishnan, R., Ginsberg, H.N., and Breslow, J.L.. *J.Clin.Invest*. **90** : 1889-1900, 1992.
Banner, D.W., D'Arcy, A., Janes, W., Gentz, R., Schoenfeld, H.-J., Broger, C., Loetscher, H., and Lesslauer, W.. *Cell* **73**: 431-445, 1993.
Bartles, J.R., and Hubbard, A.L. *Methods Enzymol*. **191** : 825-841, 1990.
Belcher, J.D., Hamilton, R.L., Brady, S.E., Hornick, C.A., Jaeckle, S., Schneider, W.J., and
Havel, R.J.. *Proc. Natl. Acad. Sci.* **84** : 6785-6789, 1987.
Bihain, B.E., and Yen, F.T. *Biochemistry* **31** :4628-4636, 1992.
Bilheimer, D.W., Eisenberg, S., and Levy, R.I. *Biochim. Biophys. Acta* **260** : 212-221, 1972.
Bodansky M., Principles of peptide synthesis, (1984).
Brendel, V., Bucher, P., Nourbakhsh, I., Blaisdell, B.E., and Karlin, S. *Proc. Natl. Acad. Sci. USA* **89:** 2002-2006, 1992.
Buckholz, R.G.. *Curr. Op. Biotechnology* **4** : 538-542, 1993.
Busch et al. *J. Chromatogr.* **777** 311-328 (1997)
Carter, B.J. *Curr. Op. Biotechnology* **3** : 533-539, 1993.
Chen, W.-J., Goldstein, J.-L., and Brown, M.S. *J. Biol. Chem*. **263:** 3116-3123, 1990.
Chen, H., Charlat, O., Targlia, L.A., et al. *Cell* **84** : 491-495, 1996.
Cherif D., Julier, C., Delattre, O., Derré, J., Lathrop, G.M., and Berger, R. *Proc. Natl. Acad. Sci. USA*. **87** : 6639-6643, 1990.
Chumakov, I., Rigault, P., Guillou, S., Ougen, P., Billault, A., Guasconi, G., Gervy, P., Le Gall, I., Soularue, P., Grinas, P., et al. *Nature* **359** : 380-386, 1992.
Chumakov, I.M., Rigault, P., Le Gall, I., et al. *Nature* **377** : 175-183, 1995.
Compton, J. *Nature* **350:** 91-92, 1991.
Cytokines and Their Receptors (Nicola, N.A., ed.). Oxford University Press, Oxford. 1996.
Davis, C.G., Lehrman, M.A., Russell, D.W., Anderson, R.G.W., Brown, M.S., and Goldstein, J.L. *Cell* **45** : 15-24, 1986.
Edwards, C. P., and Aruffo, A. Curr. *Op. Biotechnology* **4** : 558-563, 1993.
Elomaa, O., Kangas, M., Sahlberg, C., Tuukkanen, J., Sormunen, R., Liakka, A., Thesleff, I., Kraal, G., and Tryggvason, K. *Cell* **80** (4) : 603-609, 1995.
Epstein, A. *Médecine*/*Sciences* **8** : 902-911, 1992.
Fan, J.L., Mccormick, S.P.A., Krauss, R.M., Taylor, S., Quan, R., Taylor, J.M., and Young, S.G. *Arterioscler.Thromb.Vasc.Biol* **15** : 1889-1899, 1995.
Goldstein, J.L., Basu, S.K., and Brown, M.S. *Methods Enzymol*. **98** : 241-260, 1983.
Goldstein, J.L., Hobbs, H.H., Brown, M.S. Familial Hypercholesterolemia In The Metabolic and Molecular Bases of Inherited Disease, Volume II, 7th Edition (Scriver, C.R., Beaudet, A.L., Sly, W.S., Valle, D., ed). Mc Graw-Hill, New-York. pp.1981-2030, 1995.
Guatelli J.C. et al. *Proc. Natl. Acad. Sci. USA* **87**: 1874-1878, 1990.
Gura T. *Science* **275** : 751-753, 1997.
Heldin, C.H. *Cell* **80** : 213-223, 1995.
Herz, J., Hamann, U., Rogne, S., Myklebost, O., Gausepohl, H., and Stanley, K.K. *EMBO J.* **7** : 4119-4127, 1988.
Herz, J., Qiu, S.-Q., Oesterle, A., DeSilva, H.V., Shafi, S., and Havel, R.J. *Proc.Natl.Acad.Sci.USA* **92** : 4611-4615, 1995.
Homanics, G.E., de Silva, H.V., Osada, J., Zhang, S.H., Wong, H., Borensztajn, J., and Maeda, N. *J.Biol.Chem*. **270** : 2974-2980, 1995.
Honoré, B., Madsen, P., Rasmussen, H.H., Vandekerckhove, J., Celis, J.E., and Leffers, H. *Gene* **134 :** 283-287, 1993.
Huang, Y.D., Schwendner, S.W., Rail, S.C., and Mahley, R.W. *J.Biol Chem.* **271** : 29146-29151,1996.
Huynh, T.U., Young R.A. and Davis R.W. DNA cloning techniques: A practical approach, ed Glover D. (IRL Press, Oxford), 1984.
lida, M., Murakami, T., Ishida, K., Mizuno, A., Kuwajima, M., and Shima, K. *Biochem. Biophys. Res. Commun.* **224** : 597-604, 1996.
Ishibashi, S., Brown, M.S., Goldstein, J.L., Gerard, R.D., Hammer, R.E., and Herz, J. *J. Clin. Invest*. **92** : 883-893, 1993.
Ito, Y., Azrolan, N., O'Connell, A., Walsh, A., and Breslow, J.L *Science* **249:** 790-793, 1990.
Kleyn, P.W., Fan, W., Kovats, S.G., et al. *Cell* **85** : 281-290, 1996.
Kobayashi, J., Applebaum-Bowden, D., Dugi, K.A., Brown, D.R., Kashyap, V.S., Parrott, C., Duarte, C., Maeda, N., and Santamarina-Fojo, S. *J.Biol.Chem.* **271** : 26296-26301, 1996.
Köhler et Milstein. *Nature* **256,** 495-497, 1975.
Kosak M. *Nucleic Acids Res*. **15**: 8125-8148, 1987.
Kosak M. Proc. *Natl. Acad. Sci. USA* **87**: 8301-8305, 1990.
Krainer, A.R., Mayeda, A., Kozak, D., and Binns, G. *Cell* **66** : 383-394, 1991.
Krieger, M., and Herz, J. *Ann. Rev. Biochem.* **63**: 601-637, 1994.
Landegren U., Kaiser R., Sanders J. & Hood L. *Science* **241**: 1077-1080, 1988.
Lee, M.G-S., Bihain, B.E., Russell, D.G., Deckelbaum, R.J., and Van Der Ploeg, L.H.T. *Molec. Cell. Biol.* **10** : 4506-4517, 1990.
Letoumeur, F., and Klausner, R.D. *Cell* **69** : 1143-1157, 1992.
Lockhart et al. *Nature Biotechnology* **14**: 1675-1680, 1996
Lu, D., Willard, D., Patel, I.R., et al. *Nature* **371** : 799-802, 1994.
Luckow, V.A. *Curr.* Op. *Biotechnology* **4** : 564-572, 1993.
Maeda, N., Li, H., Lee, D., Oliver, P., Quarfordt, S.H., and Osada, J. *J.Biol.Chem*. **269** : 23610-23616, 1994.
Mann, C.J., Khallou, J., Chevreuil, O., Troussard, A.A., Guermani, L.M., Launay, K., Delplanque, B., Yen, F.T., and Bihain, B.E. *Biochemistry* **34** : 10421-10431, 1995.
Manne, J., Argeson, A.C., Siracusa, L.D. *Proc. Natl. Acad. Sci. USA* **92** : 4721-4724, 1995.
Montague, C.T., Farooqi, I.S., Whitehead, J.P., Soos, M.A., Rau, H., Wareham, N.J., Sewter, C.P., Digby, J.E., Mohammed, S.N., Hurst, J.A., Cheetham, C.H., Earley, A.R., Barnett, A.H., Prins, J.B., and O'Rahilly, S.O. *Nature* **387** :903-908, 1997.
No D., Yao T.P. and Evans R.M. *Proc. Natl. Acad. Sci. USA*, **93**: 3346-3351,1996.
Nobben-Trauth, K., Naggert, J.K., North, M.A., and Nishina, P.M. *Nature* **380** : 534-538, 1996.
Olins, P.O., and Lee, S.C. *Curr. Op. Biotechnology* **4** : 520-525, 1993.
Oukka, M., André, P., Turmel, P., Besnard, N., Angevin, V., Karlsson, L., Trans, PL., Charron, D., Bihain, B., Kosmatopoulos, K., Lotteau, V. *Eur. J. Immunol*. **27** : 855-859, 1997.
Parra-Lopez, C.A., Lindner, R., Vidavsky, I., Gross, M., and Unanue, E.R. *J. Immunol*. **158:** 2670-2679, 1997.
Perricaudet, M., Stratford-Perricaudet, L. and Briand, P. *La Recherche* **23** : 471-473, 1992.
Pietu et al. *Genome Research* **6**:492-503, 1996
Plump, A.S., Smith, J.D., Hayek, T., Aalto-Setälä, K., Walsh, A., Verstuyft, J.G., Rubin, E.M., and Breslow, J.L. *Cell* **71** : 343-353, 1992.
Purcellhuynh, D.A., Farese, R.V., Johnson, D.F., Flynn, L.M., Pierotti, V., Newland, D.L., Linton, M.F., Sanan, D.A., and Young, S.G. *J.Clin.Invest*. **95** : 2246-2257, 1995.
Rohlmann, A., Gotthardt, M., Willnow, T.E., Hammer, R.E., and Herz, J. *Nature Biotech.* **14** : 1562-1565, 1996.
Sambrook, J., Fritsch, E.F., and Maniatis, T. Molecular cloning : a laboratory manual. Sec. Ed. Cold Spring Harbor Lab., Cold Spring Harbor, New York.
Schena et al. *Science* **270**:467-470, 1995
Simos, G., Georgatos, S.D. *FEBS Letters* **346** : 225-228, 1994.
Sosnowski RG, et al., *Proc Natl Acad Sci U S A* 1997;**94**:1119-1123
Stewart J.M. et Yound J.D., solid phase peptides synthesis, Pierce Chem. Company, Rockford, 111, 2ème éd., (1984).
Suggs S.V., Wallace R.B., Hirose T., Kawashima E.H. and Itakura K. *PNAS* **78:** 6613-6617,1981.
Szabo A. et al. *Curr Opin Struct Biol* **5**, 699-705 (1995)
Temin, H.M. Retrovirus vectors for gene transfer. In Kucherlapati R., ed. Gene Transfer, New York, Plenum Press, 149-187, 1986.
Troussard, A.A., Khallou, J., Mann, C.J., André, P., Strickland, D.K., Bihain, B.E., and Yen, F.T. *J. Biol. Chem.* **270** : 17068-17071, 1995.
Verhey, K.J., and Bimbaum, M.J. *J. Biol. Chem.* **269** : 2353-2356, 1994.
Walker G.T., Fraiser M.S., Schram J.L., Little M.C., Nadeau J.G., & Malinowski D.P. *Nucleic Acids Res.* **20** : 1691-1696, 1992.
Wang et al. *Chromatographia,* **44** 205-208 (1997)
West, D.B., Boozer, C.N., Moody, D.L., and Atkinson, R.L. *Am. J. Physiol.* **262** : R1025-R1032, 1992.
Willow, T.E., Sheng, Z., Ishibashi, S., Herz, J. *Science,* **264** : 1471-1474, 1994.
Woo S.L.C. *Methods Enzymol*. **68**: 389, 1979.
Yen, F.T., Mann, C.J., Guermani, L.M., Hannouche, N.F., Hubert, N., Hornick, C.A., Bordeau, V., Agnani, G., and Bihain, B.E. *Blochemistry* **33** : 1172-1180, 1994.
Young R.A. and Davis R.W. *PNAS* **80**: 1194-1198, 1983a.
Young R.A. and Davis R.W. Science **222** : 778-782, 1983b.
Zhang, S.H., Reddick, R.L., Piedrahit, J.A., and Maeda, N. Science **258** : 468-471, 1992.
Zhang, Y., Proenca, R., Maffei, M., Barone, M., Leopold, L., Friedman, J.M. *Nature,* **372** : 4425-4432, 1994.
Zhong, G., Romagnoli, P., and Germain, R.N. *J. Exp. Med.* **185 :** 429-438, 1997.

## Revendications

1. Polypeptide purifié ou recombinant du récepteur LSR de séquence choisie parmi les séquences SEQ ID Nos. 2, 4, 6, 8, 10, 12, 16, 17, et 18, les séquences présentant au moins 90 % d'identité avec les séquences SEQ ID Nos. 2, 4, 6, 8, 10, 12, 16, 17, et 18, ou parmi les fragments biologiquement actifs d'au moins cinq acides aminés consécutifs des polypeptides de séquence SEQ ID Nos. 2, 4, 6, 8, 10, 12, 16, 17, et 18.

2. Polypeptide purifié ou recombinant selon la revendication 1, **caractérisé en ce que** ledit fragment biologiquement actif est choisi parmi un site de fixation des acides gras, un site de fixation de la clathrine, un site de fixation d'une cytokine, un site de fixation de ligands apoprotéiques, un motif LI/LL, un motif RSRS et une région hydrophobe.

3. Anticorps **caractérisés en ce qu'**ils sont capables de reconnaître spécifiquement un polypeptide selon l'une des revendications 1 et 2.

4. Hydridome **caractérisé en ce qu'**il exprime des anticorps monoclonaux capables de reconnaître spécifiquement un polypeptide selon l'une des revendications 1 et 2.

5. Acide nucléique purifié, isolé ou recombinant, **caractérisé en ce qu'**il code une séquence d'un polypeptide selon la revendication 1 ou 2.

6. Acide nucléique purifié, isolé ou recombinant, **caractérisé en ce qu'**il comprend la séquence complémentaire d'un acide nucléique selon la revendication 5.

7. Vecteur recombinant, **caractérisé en ce qu'**il comprend un acide nucléique selon l'une des revendications 5 à 6.

8. Cellule recombinante **caractérisée en ce qu'**elle exprime un polypeptide recombinant selon l'une des revendications 1 et 2.

9. Cellule recombinante, **caractérisée en ce qu'**elle comprend un acide nucléique recombinant selon l'une des revendications 5 et 6.

10. Cellule recombinante, **caractérisée en ce qu'**elle comprend un vecteur recombinant selon la revendication 7.

11. Mammifère, excepté l'homme, **caractérisé en ce qu'**il comprend un acide nucléique recombinant selon la revendication 5 ou 6, ou un vecteur recombinant selon la revendication 7.

12. Méthode de détection de l'expression d'un polypeptide du récepteur LSR **caractérisée en ce qu'**elle comprend :
a) la mise en contact d'un échantillon biologique avec un anticorps capable de reconnaître spécifiquement un polypeptide selon l'une des revendications 1 et 2 ; et
b) la détection de complexes immunologiques entre ledit anticorps et ledit polypeptide comme moyen de détection de ladite expression.

13. Méthode de diagnostic de désordre ou de maladie lié à l'obésité **caractérisée en ce qu'**elle comprend :
a) la mise en contact d'un échantillon biologique avec un anticorps capable de reconnaître spécifiquement un polypeptide selon l'une des revendications 1 et 2 ; et
b) la détection de complexes immunologiques entre ledit anticorps et ledit polypeptide comme moyen de diagnostic dudit désordre ou maladie.

14. Méthode de diagnostic de désordre ou de maladie lié à l'obésité **caractérisée en ce qu'**elle comprend :
a) la mise en contact d'un échantillon biologique avec un acide nucléique selon l'une des revendications 5 et 6 ; et
b) la détection de mutations dans un acide nucléique dudit échantillon biologique comme moyen de diagnostic dudit désordre ou maladie.

15. Méthode de diagnostic de désordre ou de maladie lié à l'obésité **caractérisée en ce qu'**elle comprend :
a) la mise en contact d'un échantillon biologique avec un acide nucléique comprenant au moins 15 nucléotides consécutifs de la SEQ ID No. 19 ; et
b) la détection de mutations dans un acide nucléique dudit échantillon biologique comme moyen de diagnostic dudit désordre ou maladie.

16. Méthode selon l'une des revendications 12 à 15, **caractérisée en ce que** ledit échantillon biologique provient d'un mammifère.

17. Méthode selon la revendication 16, **caractérisée en ce que** ledit mammifère est un homme.

18. Méthode de sélection d'un composé utile pour le traitement ou la prévention d'un désordre ou maladie lié à l'obésité, **caractérisée en ce qu'**elle comprend :
a) la mise en contact d'un polypeptide selon l'une des revendications 1 et 2 avec un composé candidat ;
b) la détection d'un complexe entre ledit composé candidat et ledit polypeptide comme moyen de sélection dudit composé utile pour le traitement ou la prévention dudit désordre ou maladie lié à l'obésité.

19. Méthode de sélection d'un composé utile pour le traitement ou la prévention d'un désordre ou maladie lié à l'obésité, **caractérisée en ce qu'**elle comprend :
a) la mise en contact d'une cellule recombinante selon la revendication 8 ou 9 avec un composé candidat ;
b) la détection d'un résultat choisi parmi une interaction dudit composé candidat avec ladite cellule recombinante, une modulation de l'activité du récepteur LSR, et une modulation de l'expression du récepteur LSR comme moyen de sélection dudit composé utile pour le traitement ou la prévention dudit désordre ou maladie lié à l'obésité.

20. Méthode selon la revendication 18 ou 19, **caractérisée en ce que** ladite mise en contact se fait en présence d'un ligand dudit récepteur LSR.

21. Méthode selon la revendication 20, **caractérisée en ce que** ledit ligand est choisi parmi le groupe consistant en une cytokine, l'oléate, le LDL, et gC1qR.

22. Méthode selon la revendication 21, **caractérisée en ce que** ladite cytokine est la leptine.

23. Méthode selon la revendication 19, **caractérisée en ce que** ladite activité est choisie parmi la fixation des lipoprotéines, leur intemalisation et leur dégradation, et la fixation de la leptine, son intemalisation et sa dégradation.

24. Méthode selon la revendication 18 ou 19, **caractérisée en ce que** ledit composé candidat est choisi parmi les peptides, les acides gras, les lipoprotéines, des médicaments et petites molécules.

25. Méthode selon l'une des revendications 13, 15, 18 et 19, **caractérisée en ce que** ledit désordre ou maladie lié à l'obésité est sélectionné parmi le groupe consistant en l'obésité, l'anorexie, la cachexie, l'insuffisance cardiaque, l'insuffisance coronarienne, les accidents vasculaires cérébraux, la maladie athéromateuse, l'athérosclérose, l'hypertension artérielle, le diabète non insuline-dépendant, l'hyperlipidémie et l'hyperuricémie.

## Patentansprüche

1. Gereinigtes oder rekombinantes Polypeptid des LSR Rezeptors mit einer Sequenz, die ausgewählt ist aus den Sequenzen mit SEQ ID NO: 2, 4, 6, 8, 10, 12, 16, 17 und 18, Sequenzen, die zumindest 90% Identität zu den Sequenzen SEQ ID NO: 2, 4, 6, 8, 10, 12, 16, 17 and 18 aufweisen, oder biologisch aktiven Fragmenten, die zumindest fünf aufeinanderfolgende Aminosäuren der Polypeptide der Sequenzen mit SEQ ID NO: 2, 4, 6, 8, 10, 12, 16, 17, and 18 aufweisen.

2. Gereinigtes oder rekombinantes Polypeptid nach Anspruch 1, **dadurch gekennzeichnet, dass** das biologisch active Fragment ausgewählt is aus einer Fettsäure-Bindungsstelle, einer Clathrin-Bindungsstelle, einer Zytokin-Bindungsstelle, einer Apoprotein-Liganden-Bindungsstelle, einem LI/LL Motiv, einem RSRS Motiv und einer hydrophoben Region.

3. Antikörper, **dadurch gekennzeichnet, dass** sie dazu fähig sind, ein Polypeptid nach einem der Ansprüche 1 oder 2 spezifisch zu erkennen.

4. Hybridom, **dadurch gekennzeichnet, dass** es monoklonale Antikörper exprimiert, die ein Polypeptid nach einem der Ansprüche 1 oder 2 spezifisch erkennen.

5. Gereinigte, isolierte oder rekombinante Nukleinsäure, **dadurch gekennzeichnet, dass** sie für ein Polypeptid mit einer Sequenz nach einem der Ansprüche 1 oder 2 kodiert.

6. Gereinigte, isolierte oder rekombinante Nukleinsäure, **dadurch gekennzeichnet, dass** sie die komplementäre Sequenz einer Nukleinsäure nach Anspruch 5 aufweist.

7. Rekombinanter Vektor, **dadurch gekennzeichnet, dass** er eine Nukteinsäure nach einem der Ansprüche 5 oder 6 aufweist.

8. Rekombinante Zelle, **dadurch gekennzeichnet, dass** sie ein rekombinantes Polypeptid nach einem der Ansprüche 1 oder 2 enhält.

9. Rekombinante Zelle, **dadurch gekennzeichnet, dass** sie eine rekombinante Nukleinsäure nach einem der Ansprüche 5 und 6 aufweist.

10. Rekombinante Zelle, **dadurch gekennzeichnet, dass** sie einen rekombinanten Vektor nach Anspruch 7 aufweist.

11. Säugetier, mit Ausnahme eines Menschen, **dadurch gekennzeichnet, dass** es eine rekombinante Nukleinsäure nach einem der Ansprüche 5 oder 6 oder einen rekombinanten Vektor nach Anspruch 7 aufweist.

12. Verfahren zum Nachweis der Expression eines Polypeptids des LSR Rezeptors, **dadurch gekennzeichnet, dass** es folgendes aufweist:
a) In-Kontakt-bringen einer biologischen Probe mit einem Antikörper, der dazu fähig ist, ein Polypeptid nach einem der Ansprüche 1 und 2 spezifisch zu erkennen; und
b) Nachweis von immunologischen Komplexen zwischen dem Antikörper und dem Polypeptid as Mittel zum Nachweis der Expression.

13. Verfahren zur Diagnose einer mit Fettleibigkeit verbundenen Störung oder Krankheit, **dadurch gekennzeichnet, dass** es folgendes aufweist:
a) In-Kontakt-bringen einer biologischen Probe mit einem Antikörper, der dazu fähig ist, ein Polypeptid nach einem der Ansprüche 1 und 2 spezifisch zu erkennen; und
b) Nachweis von immunologischen Komplexen zwischen dem Antikörper und dem Polypeptid als Mittel zur Diagnose der Störung oder Krankheit.

14. Verfahren zur Diagnose einer mit Fettleibigkeit verbundenen Störung oder Krankheit, **dadurch gekennzeichnet, dass** es folgendes aufweist:
a) In-Kontakt-bringen einer biologischen Probe mit einer Nukleinsäure nach einem der Ansprüche 5 oder 6; und
b) Nachweis von Mutationen in einer Nukleinsäure der biologischen Probe als Mittel zur Diagnose der Störung oder Krankheit.

15. Verfahren zur Diagnose einer mit Fettleibigkeit verbundenen Störung oder Krankheit, **dadurch gekennzeichnet, dass** es folgendes aufweist:
a) In-Kontakt-bringen einer biologischen Probe mit einer Nukleinsäure die zumindest 15 aufeinanderfolgende Nukleotide der SEQ ID NO: 19 aufweist; und
b) Nachweis von Mutationen in einer Nukleinsäure der biologischen Probe als Mittel zur Diagnose der Störung oder Krankheit.

16. Verfahren nach einem der Ansprüche 12 bis 15, **dadurch gekennzeichnet, dass** die biologische Probe vom einem Säugetier stammt.

17. Verfarhren nach Anspruch 16, **dadurch gekennzeichnet, dass** das Säugetier ein Mensch ist.

18. Verfahren zur Selektion einer Verbindung, die zur Behandlung oder Prävention einer mit Fettleibigkeit verbundenen Störung oder Krankheit brauchbar ist, **dadurch gekennzeichnet, dass** es folgendes aufweist:
a) In-Kontakt-bringen eines Polypeptids nach einem der Ansprüche 1 oder 2 mit einer Kandidatenverbindung; und
b) Nachweis eines Komplexes zwischen der Kandidatenverbindung und dem Polypeptid als Mittel zur Selektion der Verbindung, die zur Behandlung oder Prävention einer mit Fettleibigkeit verbundenen Störung oder Krankheit brauchbar ist.

19. Verfahren zur Selektion einer Verbindung, die zur Behandlung oder Prävention einer mit Fettleibigkeit verbundenen Störung oder Krankheit brauchbar ist, **dadurch gekennzeichnet, dass** es folgendes aufweist:
a) In-Kontakt-bringen einer rekombinanten Zelle nach einem der Ansprüche 8 oder 9 mit einer Kandidatenverbindung; und
b) Nachweis eines Ergebnisses, das ausgewählt ist aus der Interaktion einer Kandidatenverbindung mit der rekombinanten Zelle, Modulierung der Aktivität des LSR Rezeptors, und Modulierung der Expression des LSR Rezeptors als Mittel zur Selektion der Verbindung, die zur Behandlung oder Prävention einer mit Fettleibigkeit verbundenen Störung oder Krankheit brauchbar ist.

20. Verfahren nach Anspruch 18 oder 19, **dadurch gekennzeichnet, dass** das In-Kontakt-Bringen in Anwesenheit eines Liganden des LSR Rezeptors durchgeführt wird.

21. Verfahren nach Anspruch 20, **dadurch gekennzeichnet, dass** der Ligand ausgewählt ist aus der Gruppe bestehend aus einem Zytokin, Oleat, LDL und gC1qR.

22. Verfahren nach Anspruch 21, **dadurch gekennzeichnet, dass** das Zytokin Leptin ist.

23. Verfahren nach Anspruch 19, **dadurch gekennzeichnet, dass** die Aktivität ausgewählt ist aus Lipoprotein-Bindung, -Internalisierung und -Degradierung, und Leptin-Bindung, -Internalisierung und -Degradierung.

24. Verfahren nach Anspruch 18 oder 19, **dadurch gekennzeichnet, dass** die Kandidatenverbindung ausgewählt ist aus Peptiden, Fettsäuren, Lipoproteinen, Medikamenten und niedermolekularen Verbindungen.

25. Verfahren nach einem der Ansprüche 13, 15, 18 and 19, **dadurch gekennzeichnet, dass** die mit Fettleibigkeit verbundene Störung oder Krankheit ausgewählt ist aus der Gruppe bestehend aus Fettleibigkeit, Anorexie, Cachexie, Herzinsuffizienz, Koronarinsuffizienz, Apoplexie, atheromatösen Krankheiten, Arteriosklerose, arterieller Hypertension, Insulin-unabhängiger Diabetes, Hyperlipidämie, und Hyperurikämie.

## Claims

1. Purified or recombinant polypeptide of the LSR receptor having a sequence selected from the sequences SEQ ID NO: 2, 4, 6, 8, 10, 12, 16, 17 and 18, sequences having at least 90% identity with the sequences SEQ ID NO: 2, 4, 6, 8, 10, 12, 16, 17 and 18, or biologically active fragments having at least five consecutive amino acids of the polypeptides of sequences SEQ ID NO: 2, 4, 6, 8, 10, 12, 16, 17, and 18.

2. Purified or recombinant polypeptide according to claim 1, **characterized in that** said biologically active fragment is selected from a fatty acid binding site, a clathrine binding site, a cytokine binding site, an apoprotein-ligand binding site, a LI/LL motif, a RSRS motif and a hydrophobic region.

3. Antibodies, **characterized in that** they are capable of specifically recognizing a polypeptide according to one of claims 1 and 2.

4. Hybridoma, **characterized in that** it expresses monoclonal antibodies capable of specifically recognizing a polypeptide according to one of claims 1 and 2.

5. Purified, isolated or recombinant nucleic acid, **characterized in that** it codes for a polypeptide having a sequence according to claim 1 or 2.

6. Purified, isolated or recombinant nucleic acid, **characterized in that** it comprises the complementary sequence of a nucleic acid according to claim 5.

7. Recombinant vector, **characterized in that** it comprises a nucleic acid according to one of claims 5 or 6.

8. Recombinant cell, **characterized in that** it expresses a recombinant polypeptide according to one of claims 1 and 2.

9. Recombinant cell, **characterized in that** it comprises a recombinant nucleic acid according to one of claims 5 and 6.

10. Recombinant cell, **characterized in that** it comprises a recombinant vector according to claim 7.

11. Mammal, except of a human being, **characterized in that** it comprises a recombinant nucleic acid according to claim 5 or 6, or a recombinant vector according to claim 7.

12. Method of detection of the expression of an LSR receptor polypeptide, **characterized in that** it comprises:
a) Contacting a biological sample with an antibody capable of specifically recognizing a polypeptide according to one of claims 1 and 2; and
b) Detecting immunological complexes between said antibody and said polypeptide as means of detection of said expression.

13. Method of diagnosing an obesity-related disorder or disease, **characterized in that** it comprises:
a) Contacting a biological sample with an antibody capable of specifically recognizing a polypeptide according to one of claims 1 and 2; and
b) Detecting immunological complexes between said antibody and said polypeptide as means of diagnosis of said disorder or disease.

14. Method of diagnosing an obesity-related disorder or disease, **characterized in that** it comprises:
a) Contacting a biological sample with a nucleic acid according to one of claims 5 and 6; and
b) Detecting mutations in a nucleic acid of said biological sample as means of diagnosis of said disorder or disease.

15. Method of diagnosing an obesity-related disorder or disease, **characterized in that** it comprises:
a) Contacting a biological sample with a nucleic acid comprising at least 15 consecutive nucleotides of SEQ ID NO: 19; and
b) Detecting mutations in a nucleic acid of said biological sample as means of diagnosis of said disorder or disease.

16. Method according to one of claims 12 to 15, **characterized in that** said biological sample is derived from a mammal.

17. Method according to claim 16, **characterized in that** said mammal is a human being.

18. Method of selection of a compound useful for treatment or prevention of an obesity-related disorder or disease, **characterized in that** it comprises:
a) Contacting a polypeptide according to one of claims 1 or 2 with a candidate compound; and
b) Detection of a complex between said candidate compound and said polypeptide as means of selection of said compound useful for treatment or prevention of said obesity-related disorder or disease.

19. Method of selection of a compound useful for treatment or prevention of an obesity-related disorder or disease, **characterized in that** it comprises:
a) Contacting a recombinant cell according to claims 8 or 9 with a candidate compound; and
b) Detection of a result selected from interaction of said candidate compound with said recombinant cell, modulation of the activity of the LSR receptor, and modulation of the expression of the LSR receptor as means of selection of said compound useful for treatment or prevention of said obesity-related disorder or disease.

20. Method according to claim 18 or 19, **characterized in that** said contacting is done in the presence of a ligand of said LSR receptor.

21. Method according to claim 20, **characterized in that** said ligand is selected from the group consisting of a cytokine, oleate, LDL, and gC1qR.

22. Method according to claim 21, **characterized in that** said cytokine is leptine.

23. Method according to claim 19, **characterized in that** said activity is selected from lipoprotein binding, intemalization and degradation, and leptin binding, internalization and degradation.

24. Method according to claim 18 or 19, **characterized in that** said candidate compound is selected from peptides, fatty acids, lipoproteins, medicaments and small molecular weight compounds.

25. Method according to one of claims 13, 15, 18 and 19, **characterized in that** said obesity-related disorder or disease is selected from the group consisting of obesity, anorexia, cachexia, cardiac insufficiency, coronary insufficiency, cerebral vascular accidents, atheromatous diseases, atherosclerosis, arterial hypertension, non-insulin dependent diabetes, hyperlipidemia and hyperuricemia.


### SEQUENCE LISTING

(1) GENERAL INFORMATION:
   (i) APPLICANT:
      (A) NAME : Genset SA
      (B) STREET : 24, RUE ROYALE
      (C) CITY : PARIS
      (E) COUNTRY : France
      (F) POSTAL CODE (ZIP) : 75008
   (ii) TITLE OF INVENTION: Recepteur LSR.
   (iii) NUMBER OF SEQUENCES: 41
   (v) COMPUTER READABLE FORM:
      (A) MEDIUM TYPE: Floppy Disk
      (B) COMPUTER: IBM PC compatible
      (C) OPERATING SYSTEM: Win95
      (D) SOFTWARE: Word
(2) INFORMATION FOR SEQ ID NO: 1:
   (i) SEQUENCE CHARACTERISTICS:
      (A) LENGTH: 2097 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 1:
   ACCGCTCACC AGGTCAGTTG TCCCCGGAAA GCCGAAGGCA TGAGCTTCGC CCAAGTTCTT 60
   TTTATGGGTT AGAACTCCTC CAGAGCGGGG GAAAAAGGAC TTGGAATAGG GGCGGGACGG 120
   AGCACGCACC CTTCTCCGCC TTGGTTCTCG CCGCGCCCCC TACTCTCGGG ATACTTGGGA 180
   GGGGACGCGC GGGCACCGTC GCTGCTAGAC GGCCGCG ATG GCG CCG GCG GCC GGC 235
   Met Ala Pro Ala Ala Gly
   1 5
   GCG TGT GCT GGG GCG CCT GAC TCC CAC CCA GCT ACC GTG GTC TTC GTG 283
   Ala Cys Ala Gly Ala Pro Asp Ser His Pro Ala Thr Val Val Phe Val
   10 15 20
   TGT CTC TTT CTC ATC ATT TTC TGC CCA GAC CCT GCC AGT GCC ATC CAG 331
   Cys Leu Phe Leu Ile Ile Phe Cys Pro Asp Pro Ala Ser Ala Ile Gln
   25 30 35
   GTG ACT GTG TCT GAC CCC TAC CAC GTA GTG ATC CTG TTC CAG CCA GTG 379
   Val Thr Val Ser Asp Pro Tyr His Val Val Ile Leu Phe Gln Pro Val
   40 45 50
   ACC CTG CCC TGC ACC TAT CAG ATG AGC AAC ACT CTC ACA GTC CCC ATC 427
   Thr Leu Pro Cys Thr Tyr Gln Met Ser Asn Thr Leu Thr Val Pro Ile
   55 60 65 70
   GTG ATC TGG AAG TAC AAG TCA TTC TGC CGG GAC CGT ATT GCC GAT GCC 475
   Val Ile Trp Lys Tyr Lys Ser Phe Cys Arg Asp Arg Ile Ala Asp Ala
   75 80 85
   TTC TCT CCT GCC AGT GTG GAC AAC CAG CTA AAT GCC CAG TTG GCA GCT 523
   Phe Ser Pro Ala Ser Val Asp Asn Gln Leu Asn Ala Gln Leu Ala Ala
   90 95 100
   GGC AAC CCC GGC TAC AAC CCC TAT GTG GAG TGC CAG GAC AGT GTA CGC 571
   Gly Asn Pro Gly Tyr Asn Pro Tyr Val Glu Cys Gln Asp Ser Val Arg
   105 110 115
   ACT GTC AGG GTG GTG GCC ACC AAA CAG GGC AAT GCG GTG ACC CTG GGA 619
   Thr Val Arg Val Val Ala Thr Lys Gln Gly Asn Ala Val Thr Leu Gly
   120 125 130
   GAC TAC TAC CAA GGC AGG AGG ATC ACC ATA ACA GGA AAT GCT GAC CTG 667
   Asp Tyr Tyr Gln Gly Arg Arg Ile Thr Ile Thr Gly Asn Ala Asp Leu
   135 140 145 150
   ACC TTC GAG CAG ACA GCC TGG GGA GAC AGT GGA GTG TAT TAC TGC TCT 715
   Thr Phe Glu Gln Thr Ala Trp Gly Asp Ser Gly Val Tyr Tyr Cys Ser
   155 160 165
   GTG GTC TCG GCC CAA GAT CTG GAT GGA AAC AAC GAG GCG TAC GCA GAG 763
   Val Val Ser Ala Gln Asp Leu Asp Gly Asn Asn Glu Ala Tyr Ala Glu
   170 175 180
   CTC ATC GTC CTT GGC AGG ACC TCA GAG GCC CCT GAG CTC CTA CCT GGT 811
   Leu Ile Val Leu Gly Arg Thr Ser Glu Ala Pro Glu Leu Leu Pro Gly
   185 190 195
   TTT CGG GCG GGG CCC TTG GAA GAT TGG CTC TTT GTG GTC GTG GTC TGC 859
   Phe Arg Ala Gly Pro Leu Glu Asp Trp Leu Phe Val Val Val Val Cys
   200 205 210
   CTG GCG AGC CTC CTC CTC TTC CTC CTC CTG GGC ATC TGC TGG TGC CAG 907
   Leu Ala Ser Leu Leu Leu Phe Leu Leu Leu Gly Ile Cys Trp Cys Gln
   215 220 225 230
   TGC TGT CCT CAC ACC TGC TGC TGC TAT GTC CGA TGT CCC TGC TGC CCA 955
   Cys Cys Pro His Thr Cys Cys Cys Tyr Val Arg Cys Pro Cys Cys Pro
   235 240 245
   GAC AAG TGC TGT TGC CCT GAG GCT CTT TAT GCT GCT GGC AAA GCA GCC 1003
   Asp Lys Cys Cys Cys Pro Glu Ala Leu Tyr Ala Ala Gly Lys Ala Ala
   250 255 260
   ACC TCA GGT GTC CCG AGC ATC TAT GCC CCC AGC ATC TAT ACC CAC CTC 1051
   Thr Ser Gly Val Pro Ser Ile Tyr Ala Pro Ser Ile Tyr Thr His Leu
   265 270 275
   TCA CCT GCC AAG ACC CCA CCA CCT CCG CCT GCC ATG ATT CCC ATG GGC 1099
   Ser Pro Ala Lys Thr Pro Pro Pro Pro Pro Ala Met Ile Pro Met Gly
       280                  285                  290
   CCT CCC TAT GGG TAC CCT GGA GAC TTT GAC AGA CAT AGC TCA GTT GGT      1147
   Pro Pro Tyr Gly Tyr Pro Gly Asp Phe Asp Arg His Ser Ser Val Gly
   295                 300                  305                  310
   GGC CAC AGC TCC CAA GTA CCC CTG CTG CGT GAC GTG GAT GGC AGT GTA      1195
   Gly His Ser Ser Gln Val Pro Leu Leu Arg Asp Val Asp Gly Ser Val
                   315                  320                  325
   TCT TCA GAA GTA CGA AGT GGC TAC AGG ATC CAG GCT AAC CAG CAA GAT      1243
   Ser Ser Glu Val Arg Ser Gly Tyr Arg Ile Gln Ala Asn Gln Gln Asp
               330                  335                  340
   GAC TCC ATG AGG GTC CTA TAC TAT ATG GAG AAA GAG CTA GCC AAC TTT      1291
   Asp Ser Met Arg Val Leu Tyr Tyr Met Glu Lys Glu Leu Ala Asn Phe
           345                  350                  355
   GAC CCT TCC CGA CCT GGC CCT CCC AAT GGC AGA GTG GAA CGG GCC ATG      1339
   Asp Pro Ser Arg Pro Gly Pro Pro Asn Gly Arg Val Glu Arg Ala Met
       360                  365                  370
   AGT GAA GTA ACC TCC CTC CAT GAA GAT GAC TGG CGA TCG AGG CCT TCC      1387
   Ser Glu Val Thr Ser Leu His Glu Asp Asp Trp Arg Ser Arg Pro Ser
   375                 380                  385                  390
   AGG GCT CCT GCC CTC ACC CCC ATC AGG GAT GAG GAG TGG AAT CGC CAC      1435
   Arg Ala Pro Ala Leu Thr Pro Ile Arg Asp Glu Glu Trp Asn Arg His
                   395                  400                  405
   TCC CCA CAG AGT CCC AGA ACA TGG GAG CAG GAA CCC CTT CAA GAA CAA      1483
   Ser Pro Gln Ser Pro Arg Thr Trp Glu Gln Glu Pro Leu Gln Glu Gln
               410                  415                  420
   CCA AGG GGT GGT TGG GGG TCT GGA CGC CCT CGG GCC CGC TCT GTG GAT      1531
   Pro Arg Gly Gly Trp Gly Ser Gly Arg Pro Arg Ala Arg Ser Val Asp
           425                  430                  435
   GCT CTA GAT GAT ATC AAC CGG CCT GGC TCC ACT GAA TCA GGA CGG TCT      1579
   Ala Leu Asp Asp Ile Asn Arg Pro Gly Ser Thr Glu Ser Gly Arg Ser
       440                 445                 450
   TCT CCC CCA AGT AGT GGA CGG AGA GGA CGG GCC TAT GCA CCT CCA AGA      1627
   Ser Pro Pro Ser Ser Gly Arg Arg Gly Arg Ala Tyr Ala Pro Pro Arg
   455                 460                 465                 470
   AGT CGC AGC CGG GAT GAC CTC TAT GAC CCG GAC GAT CCT AGG GAC TTG      1675
   Ser Arg Ser Arg Asp Asp Leu Tyr Asp Pro Asp Asp Pro Arg Asp Leu
                   475                 480                 485
   CCA CAT TCC CGA GAT CCC CAC TAT TAT GAC GAC ATC AGG TCT AGA GAT      1723
   Pro His Ser Arg Asp Pro His Tyr Tyr Asp Asp Ile Arg Ser Arg Asp
               490                 495                 500
   CCA CGT GCT GAC CCC AGA TCC CGT CAG CGA TCC CGA GAT CCT CGG GAT      1771
   Pro Arg Ala Asp Pro Arg Ser Arg Gln Arg Ser Arg Asp Pro Arg Asp
           505                 510                 515
   GCT GGC TTC AGG TCA AGG GAC CCT CAG TAT GAT GGG CGA CTA TTA GAA      1819
   Ala Gly Phe Arg Ser Arg Asp Pro Gln Tyr Asp Gly Arg Leu Leu Glu
       520                 525                 530
   GAG GCT TTA AAG AAA AAG GGG TCG GGC GAG AGA AGG AGG GTT TAC AGG      1867
   Glu Ala Leu Lys Lys Lys Gly Ser Gly Glu Arg Arg Arg Val Tyr Arg
   535                 540                 545                 550
   GAG GAA GAA GAG GAA GAG GAG GGC CAA TAC CCC CCA GCA CCT CCA CCT      1915
   Glu Glu Glu Glu Glu Glu Glu Gly Gln Tyr Pro Pro Ala Pro Pro Pro
                   555                 560                 565
   TAC TCA GAG ACT GAC TCG CAG GCC TCA CGG GAG AGG AGG CTG AAA AAG      1963
   Tyr Ser Glu Thr Asp Ser Gln Ala Ser Arg Glu Arg Arg Leu Lys Lys
               570                 575                 580
   AAT TTG GCC CTG AGT CGG GAA AGT TTA GTC GTC TGA TCCACGTTTT          2009
   Asn Leu Ala Leu Ser Arg Glu Ser Leu Val Val  *
           585                 590
   GTATGTAGCT TTTGTACTTT TTTTTTAATT GGAATCAATA TTGATGAAAC TTCAAGCCTA   2069
   ATAAAATGTC TAATCACAAA AAAAAAAA                                      2097
(2) INFORMATION FOR SEQ ID NO: 2:
   (i) SEQUENCE CHARACTERISTICS:
      (A) LENGTH: 593 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Rattus norvegicus
   (xi) SEQUENCE DESCRIPTION: SEQ ID NO: 2:
   Met Ala Pro Ala Ala Gly Ala Cys Ala Gly Ala Pro Asp Ser His Pro
   1               5                   10                  15
   Ala Thr Val Val Phe Val Cys Leu Phe Leu Ile Ile Phe Cys Pro Asp
               20                  25                  30
   Pro Ala Ser Ala Ile Gln Val Thr Val Ser Asp Pro Tyr His Val Val
           35                  40                  45
   Ile Leu Phe Gln Pro Val Thr Leu Pro Cys Thr Tyr Gln Met Ser Asn
       50                  55                  60
   Thr Leu Thr Val Pro Ile Val Ile Trp Lys Tyr Lys Ser Phe Cys Arg
   65                  70                  75                  80
   Asp Arg Ile Ala Asp Ala Phe Ser Pro Ala Ser Val Asp Asn Gln Leu
   85 90 95
   Asn Ala Gln Leu Ala Ala Gly Asn Pro Gly Tyr Asn Pro Tyr Val Glu
   100 105 110
   Cys Gln Asp Ser Val Arg Thr Val Arg Val Val Ala Thr Lys Gln Gly
   115 120 125
   Asn Ala Val Thr Leu Gly Asp Tyr Tyr Gln Gly Arg Arg Ile Thr Ile
   130 135 140
   Thr Gly Asn Ala Asp Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Gly Arg Thr Ser Glu Ala
   180 185 190
   Pro Glu Leu Leu Pro Gly Phe Arg Ala Gly Pro Leu Glu Asp Trp Leu
   195 200 205
   Phe Val Val Val Val Cys Leu Ala Ser Leu Leu Leu Phe Leu Leu Leu
   210 215 220
   Gly Ile Cys Trp Cys Gln Cys Cys Pro His Thr Cys Cys Cys Tyr Val
   225 230 235 240
   Arg Cys Pro Cys Cys Pro Asp Lys Cys Cys Cys Pro Glu Ala Leu Tyr
   245 250 255
   Ala Ala Gly Lys Ala Ala Thr Ser Gly Val Pro Ser Ile Tyr Ala Pro
   260 265 270
   Ser Ile Tyr Thr His Leu Ser Pro Ala Lys Thr Pro Pro Pro Pro Pro
   275 280 285
   Ala Met Ile Pro Met Gly Pro Pro Tyr Gly Tyr Pro Gly Asp Phe Asp
   290 295 300
   Arg His Ser Ser Val Gly Gly His Ser Ser Gln Val Pro Leu Leu Arg
   305 310 315 320
   Asp Val Asp Gly Ser Val Ser Ser Glu Val Arg Ser Gly Tyr Arg Ile
   325 330 335
   Gln Ala Asn Gln Gln Asp Asp Ser Met Arg Val Leu Tyr Tyr Met Glu
   340 345 350
   Lys Glu Leu Ala Asn Phe Asp Pro Ser Arg Pro Gly Pro Pro Asn Gly
   355 360 365
   Arg Val Glu Arg Ala Met Ser Glu Val Thr Ser Leu His Glu Asp Asp
   370 375 380
   Trp Arg Ser Arg Pro Ser Arg Ala Pro Ala Leu Thr Pro Ile Arg Asp
   385 390 395 400
   Glu Glu Trp Asn Arg His Ser Pro Gln Ser Pro Arg Thr Trp Glu Gln
   405 410 415
   Glu Pro Leu Gln Glu Gln Pro Arg Gly Gly Trp Gly Ser Gly Arg Pro
   420 425 430
   Arg Ala Arg Ser Val Asp Ala Leu Asp Asp Ile Asn Arg Pro Gly Ser
   435 440 445
   Thr Glu Ser Gly Arg Ser Ser Pro Pro Ser Ser Gly Arg Arg Gly Arg
   450 455 460
   Ala Tyr Ala Pro Pro Arg Ser Arg Ser Arg Asp Asp Leu Tyr Asp Pro
   465 470 475 480
   Asp Asp Pro Arg Asp Leu Pro His Ser Arg Asp Pro His Tyr Tyr Asp
   485 490 495
   Asp Ile Arg Ser Arg Asp Pro Arg Ala Asp Pro Arg Ser Arg Gln Arg
   500 505 510
   Ser Arg Asp Pro Arg Asp Ala Gly Phe Arg Ser Arg Asp Pro Gln Tyr
   515 520 525
   Asp Gly Arg Leu Leu Glu Glu Ala Leu Lys Lys Lys Gly Ser Gly Glu
   530 535 540
   Arg Arg Arg Val Tyr Arg Glu Glu Glu Glu Glu Glu Glu Gly Gln Tyr
   545 550 555 560
   Pro Pro Ala Pro Pro Pro Tyr Ser Glu Thr Asp Ser Gln Ala Ser Arg
   565 570 575
   Glu Arg Arg Leu Lys Lys Asn Leu Ala Leu Ser Arg Glu Ser Leu Val
   580 585 590
   Val
(2) INFORMATION FOR SEQ ID NO: 3:
   (i) SEQUENCE CHARACTERISTICS:
      (A) LENGTH: 2040 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 3:
   ACCGCTCACC AGGTCAGTTG TCCCCGGAAA GCCGAAGGCA TGAGCTTCGC CCAAGTTCTT 60
   TTTATGGGTT AGAACTCCTC CAGAGCGGGG GAAAAAGGAC TTGGAATAGG GGCGGGACGG     120
   AGCACGCACC CTTCTCCGCC TTGGTTCTCG CCGCGCCCCC TACTCTCGGG ATACTTGGGA     180
   GGGGACGCGC GGGCACCGTC GCTGCTAGAC GGCCGCG ATG GCG CCG GCG GCC GGC      235
                                            Met Ala Pro Ala Ala Gly
                                            1               5
   GCG TGT GCT GGG GCG CCT GAC TCC CAC CCA GCT ACC GTG GTC TTC GTG       283
   Ala Cys Ala Gly Ala Pro Asp Ser His Pro Ala Thr Val Val Phe Val
               10                  15                  20
   TGT CTC TTT CTC ATC ATT TTC TGC CCA GAC CCT GCC AGT GCC ATC CAG       331
   Cys Leu Phe Leu Ile Ile Phe Cys Pro Asp Pro Ala Ser Ala Ile Gln
           25                  30                  35
   GTG ACT GTG TCT GAC CCC TAC CAC GTA GTG ATC CTG TTC CAG CCA GTG       379
   Val Thr Val Ser Asp Pro Tyr His Val Val Ile Leu Phe Gln Pro Val
       40                  45                  50
   ACC CTG CCC TGC ACC TAT CAG ATG AGC AAC ACT CTC ACA GTC CCC ATC       427
   Thr Leu Pro Cys Thr Tyr Gln Met Ser Asn Thr Leu Thr Val Pro Ile
   55                  60                  65                  70
   GTG ATC TGG AAG TAC AAG TCA TTC TGC CGG GAC CGT ATT GCC GAT GCC       475
   Val Ile Trp Lys Tyr Lys Ser Phe Cys Arg Asp Arg Ile Ala Asp Ala
                   75                  80                  85
   TTC TCT CCT GCC AGT GTG GAC AAC CAG CTA AAT GCC CAG TTG GCA GCT       523
   Phe Ser Pro Ala Ser Val Asp Asn Gln Leu Asn Ala Gln Leu Ala Ala
               90                  95                  100
   GGC AAC CCC GGC TAC AAC CCC TAT GTG GAG TGC CAG GAC AGT GTA CGC       571
   Gly Asn Pro Gly Tyr Asn Pro Tyr Val Glu Cys Gln Asp Ser Val Arg
           105                 110                 115
   ACT GTC AGG GTG GTG GCC ACC AAA CAG GGC AAT GCG GTG ACC CTG GGA       619
   Thr Val Arg Val Val Ala Thr Lys Gln Gly Asn Ala Val Thr Leu Gly
       120                 125                 130
   GAC TAC TAC CAA GGC AGG AGG ATC ACC ATA ACA GGA AAT GCT GAC CTG 667
   Asp Tyr Tyr Gln Gly Arg Arg Ile Thr Ile Thr Gly Asn Ala Asp Leu
   135 140 145 150
   ACC TTC GAG CAG ACA GCC TGG GGA GAC AGT GGA GTG TAT TAC TGC TCT 715
   Thr Phe Glu Gln Thr Ala Trp Gly Asp Ser Gly Val Tyr Tyr Cys Ser
   155 160 165
   GTG GTC TCG GCC CAA GAT CTG GAT GGA AAC AAC GAG GCG TAC GCA GAG 763
   Val Val Ser Ala Gln Asp Leu Asp Gly Asn Asn Glu Ala Tyr Ala Glu
   170 175 180
   CTC ATC GTC CTT GAT TGG CTC TTT GTG GTC GTG GTC TGC CTG GCG AGC 811
   Leu Ile Val Leu Asp Trp Leu Phe Val Val Val Val Cys Leu Ala Ser
   185 190 195
   CTC CTC CTC TTC CTC CTC CTG GGC ATC TGC TGG TGC CAG TGC TGT CCT 859
   Leu Leu Leu Phe Leu Leu Leu Gly Ile Cys Trp Cys Gln Cys Cys Pro
   200 205 210
   CAC ACC TGC TGC TGC TAT GTC CGA TGT CCC TGC TGC CCA GAC AAG TGC 907
   His Thr Cys Cys Cys Tyr Val Arg Cys Pro Cys Cys Pro Asp Lys Cys
   215 220 225 230
   TGT TGC CCT GAG GCT CTT TAT GCT GCT GGC AAA GCA GCC ACC TCA GGT 955
   Cys Cys Pro Glu Ala Leu Tyr Ala Ala Gly Lys Ala Ala Thr Ser Gly
   235 240 245
   GTC CCG AGC ATC TAT GCC CCC AGC ATC TAT ACC CAC CTC TCA CCT GCC 1003
   Val Pro Ser Ile Tyr Ala Pro Ser Ile Tyr Thr His Leu Ser Pro Ala
   250 255 260
   AAG ACC CCA CCA CCT CCG CCT GCC ATG ATT CCC ATG GGC CCT CCC TAT 1051
   Lys Thr Pro Pro Pro Pro Pro Ala Met Ile Pro Met Gly Pro Pro Tyr
   265 270 275
   GGG TAC CCT GGA GAC TTT GAC AGA CAT AGC TCA GTT GGT GGC CAC AGC 1099
   Gly Tyr Pro Gly Asp Phe Asp Arg His Ser Ser Val Gly Gly His Ser
       280                 285                 290
   TCC CAA GTA CCC CTG CTG CGT GAC GTG GAT GGC AGT GTA TCT TCA GAA     1147
   Ser Gln Val Pro Leu Leu Arg Asp Val Asp Gly Ser Val Ser Ser Glu
   295                 300                 305                 310
   GTA CGA AGT GGC TAC AGG ATC CAG GCT AAC CAG CAA GAT GAC TCC ATG     1195
   Val Arg Ser Gly Tyr Arg Ile Gln Ala Asn Gln Gln Asp Asp Ser Met
                   315                 320                 325
   AGG GTC CTA TAC TAT ATG GAG AAA GAG CTA GCC AAC TTT GAC CCT TCC     1243
   Arg Val Leu Tyr Tyr Met Glu Lys Glu Leu Ala Asn Phe Asp Pro Ser
               330                 335                 340
   CGA CCT GGC CCT CCC AAT GGC AGA GTG GAA CGG GCC ATG AGT GAA GTA     1291
   Arg Pro Gly Pro Pro Asn Gly Arg Val Glu Arg Ala Met Ser Glu Val
           345                 350                 355
   ACC TCC CTC CAT GAA GAT GAC TGG CGA TCG AGG CCT TCC AGG GCT CCT     1339
   Thr Ser Leu His Glu Asp Asp Trp Arg Ser Arg Pro Ser Arg Ala Pro
       360                 365                 370
   GCC CTC ACC CCC ATC AGG GAT GAG GAG TGG AAT CGC CAC TCC CCA CAG     1387
   Ala Leu Thr Pro Ile Arg Asp Glu Glu Trp Asn Arg His Ser Pro Gln
   375                 380                 385                 390
   AGT CCC AGA ACA TGG GAG CAG GAA CCC CTT CAA GAA CAA CCA AGG GGT     1435
   Ser Pro Arg Thr Trp Glu Gln Glu Pro Leu Gln Glu Gln Pro Arg Gly
                   395                 400                 405
   GGT TGG GGG TCT GGA CGC CCT CGG GCC CGC TCT GTG GAT GCT CTA GAT     1483
   Gly Trp Gly Ser Gly Arg Pro Arg Ala Arg Ser Val Asp Ala Leu Asp
               410                 415                 420
   GAT ATC AAC CGG CCT GGC TCC ACT GAA TCA GGA CGG TCT TCT CCC CCA     1531
   Asp Ile Asn Arg Pro Gly Ser Thr Glu Ser Gly Arg Ser Ser Pro Pro
           425                 430                 435
   AGT AGT GGA CGG AGA GGA CGG GCC TAT GCA CCT CCA AGA AGT CGC AGC     1579
   Ser Ser Gly Arg Arg Gly Arg Ala Tyr Ala Pro Pro Arg Ser Arg Ser
       440                 445                 450
   CGG GAT GAC CTC TAT GAC CCG GAC GAT CCT AGG GAC TTG CCA CAT TCC      1627
   Arg Asp Asp Leu Tyr Asp Pro Asp Asp Pro Arg Asp Leu Pro His Ser
   455                 460                 465                 470
   CGA GAT CCC CAC TAT TAT GAC GAC ATC AGG TCT AGA GAT CCA CGT GCT      1675
   Arg Asp Pro His Tyr Tyr Asp Asp Ile Arg Ser Arg Asp Pro Arg Ala
                   475                 480                 485
   GAC CCC AGA TCC CGT CAG CGA TCC CGA GAT CCT CGG GAT GCT GGC TTC      1723
   Asp Pro Arg Ser Arg Gln Arg Ser Arg Asp Pro Arg Asp Ala Gly Phe
               490                 495                 500
   AGG TCA AGG GAC CCT CAG TAT GAT GGG CGA CTA TTA GAA GAG GCT TTA      1771
   Arg Ser Arg Asp Pro Gln Tyr Asp Gly Arg Leu Leu Glu Glu Ala Leu
           505                 510                 515
   AAG AAA AAG GGG TCG GGC GAG AGA AGG AGG GTT TAC AGG GAG GAA GAA      1819
   Lys Lys Lys Gly Ser Gly Glu Arg Arg Arg Val Tyr Arg Glu Glu Glu
       520                 525                 530
   GAG GAA GAG GAG GGC CAA TAC CCC CCA GCA CCT CCA CCT TAC TCA GAG      1867
   Glu Glu Glu Glu Gly Gln Tyr Pro Pro Ala Pro Pro Pro Tyr Ser Glu
   535                 540                 545                 550
   ACT GAC TCG CAG GCC TCA CGG GAG AGG AGG CTG AAA AAG AAT TTG GCC      1915
   Thr Asp Ser Gln Ala Ser Arg Glu Arg Arg Leu Lys Lys Asn Leu Ala
                   555                 560                 565
   CTG AGT CGG GAA AGT TTA GTC GTC TGA TCCACGTTTT GTATGTAGCT            1962
   Leu Ser Arg Glu Ser Leu Val Val  *
               570                 575
   TTTGTACTTT TTTTTTAATT GGAATCAATA TTGATGAAAC TTCAAGCCTA ATAAAATGTC    2022
   TAATCACAAA AAAAAAAA                                                  2040
(2) INFORMATION FOR SEQ ID NO: 4:
   (i) SEQUENCE CHARACTERISTICS:
      (A) LENGTH: 574 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Rattus norvegicus
   (xi) SEQUENCE DESCRIPTION: SEQ ID NO: 4:
   Met Ala Pro Ala Ala Gly Ala Cys Ala Gly Ala Pro Asp Ser His Pro
   1               5                   10                  15
   Ala Thr Val Val Phe Val Cys Leu Phe Leu Ile Ile Phe Cys Pro Asp
               20                  25                  30
   Pro Ala Ser Ala Ile Gln Val Thr Val Ser Asp Pro Tyr His Val Val
           35                  40                  45
   Ile Leu Phe Gln Pro Val Thr Leu Pro Cys Thr Tyr Gln Met Ser Asn
       50                  55                  60
   Thr Leu Thr Val Pro Ile Val Ile Trp Lys Tyr Lys Ser Phe Cys Arg
   65                  70                  75                  80
   Asp Arg Ile Ala Asp Ala Phe Ser Pro Ala Ser Val Asp Asn Gln Leu
                   85                  90                  95
   Asn Ala Gln Leu Ala Ala Gly Asn Pro Gly Tyr Asn Pro Tyr Val Glu
               100                 105                 110
   Cys Gln Asp Ser Val Arg Thr Val Arg Val Val Ala Thr Lys Gln Gly
   115 120 125
   Asn Ala Val Thr Leu Gly Asp Tyr Tyr Gln Gly Arg Arg Ile Thr Ile
   130 135 140
   Thr Gly Asn Ala Asp Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Asp Trp Leu Phe Val Val
   180 185 190
   Val Val Cys Leu Ala Ser Leu Leu Leu Phe Leu Leu Leu Gly Ile Cys
   195 200 205
   Trp Cys Gln Cys Cys Pro His Thr Cys Cys Cys Tyr Val Arg Cys Pro
   210 215 220
   Cys Cys Pro Asp Lys Cys Cys Cys Pro Glu Ala Leu Tyr Ala Ala Gly
   225 230 235 240
   Lys Ala Ala Thr Ser Gly Val Pro Ser Ile Tyr Ala Pro Ser Ile Tyr
   245 250 255
   Thr His Leu Ser Pro Ala Lys Thr Pro Pro Pro Pro Pro Ala Met Ile
   260 265 270
   Pro Met Gly Pro Pro Tyr Gly Tyr Pro Gly Asp Phe Asp Arg His Ser
   275 280 285
   Ser Val Gly Gly His Ser Ser Gln Val Pro Leu Leu Arg Asp Val Asp
   290 295 300
   Gly Ser Val Ser Ser Glu Val Arg Ser Gly Tyr Arg Ile Gln Ala Asn
   305 310 315 320
   Gln Gln Asp Asp Ser Met Arg Val Leu Tyr Tyr Met Glu Lys Glu Leu
   325 330 335
   Ala Asn Phe Asp Pro Ser Arg Pro Gly Pro Pro Asn Gly Arg Val Glu
   340 345 350
   Arg Ala Met Ser Glu Val Thr Ser Leu His Glu Asp Asp Trp Arg Ser
   355 360 365
   Arg Pro Ser Arg Ala Pro Ala Leu Thr Pro Ile Arg Asp Glu Glu Trp
   370 375 380
   Asn Arg His Ser Pro Gln Ser Pro Arg Thr Trp Glu Gln Glu Pro Leu
   385 390 395 400
   Gln Glu Gln Pro Arg Gly Gly Trp Gly Ser Gly Arg Pro Arg Ala Arg
   405 410 415
   Ser Val Asp Ala Leu Asp Asp Ile Asn Arg Pro Gly Ser Thr Glu Ser
   420 425 430
   Gly Arg Ser Ser Pro Pro Ser Ser Gly Arg Arg Gly Arg Ala Tyr Ala
   435 440 445
   Pro Pro Arg Ser Arg Ser Arg Asp Asp Leu Tyr Asp Pro Asp Asp Pro
   450 455 460
   Arg Asp Leu Pro His Ser Arg Asp Pro His Tyr Tyr Asp Asp Ile Arg
   465 470 475 480
   Ser Arg Asp Pro Arg Ala Asp Pro Arg Ser Arg Gln Arg Ser Arg Asp
   485 490 495
   Pro Arg Asp Ala Gly Phe Arg Ser Arg Asp Pro Gln Tyr Asp Gly Arg
   500 505 510
   Leu Leu Glu Glu Ala Leu Lys Lys Lys Gly Ser Gly Glu Arg Arg Arg
   515 520 525
   Val Tyr Arg Glu Glu Glu Glu Glu Glu Glu Gly Gln Tyr Pro Pro Ala
       530                 535                 540
   Pro Pro Pro Tyr Ser Glu Thr Asp Ser Gln Ala Ser Arg Glu Arg Arg
   545                 550                 555                 560
   Leu Lys Lys Asn Leu Ala Leu Ser Arg Glu Ser Leu Val Val
                   565                 570
(2) INFORMATION FOR SEQ ID NO: 5:
   (i) SEQUENCE CHARACTERISTICS:
      (A) LENGTH: 1893 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 5:
   ACCGCTCACC AGGTCAGTTG TCCCCGGAAA GCCGAAGGCA TGAGCTTCGC CCAAGTTCTT      60
   TTTATGGGTT AGAACTCCTC CAGAGCGGGG GAAAAAGGAC TTGGAATAGG GGCGGGACGG     120
   AGCACGCACC CTTCTCCGCC TTGGTTCTCG CCGCGCCCCC TACTCTCGGG ATACTTGGGA     180
   GGGGACGCGC GGGCACCGTC GCTGCTAGAC GGCCGCG ATG GCG CCG GCG GCC GGC     235
                                            Met Ala Pro Ala Ala Gly
                                            1               5
   GCG TGT GCT GGG GCG CCT GAC TCC CAC CCA GCT ACC GTG GTC TTC GTG      283
   Ala Cys Ala Gly Ala Pro Asp Ser His Pro Ala Thr Val Val Phe Val
           10                  15                  20
   TGT CTC TTT CTC ATC ATT TTC TGC CCA GAC CCT GCC AGT GCC ATC CAG      331
   Cys Leu Phe Leu Ile Ile Phe Cys Pro Asp Pro Ala Ser Ala Ile Gln
           25                  30                  35
   GTG ACT GTG TCT GAC CCC TAC CAC GTA GTG ATC CTG TTC CAG CCA GTG      379
   Val Thr Val Ser Asp Pro Tyr His Val Val Ile Leu Phe Gln Pro Val
       40                  45                  50
   ACC CTG CCC TGC ACC TAT CAG ATG AGC AAC ACT CTC ACA GTC CCC ATC      427
   Thr Leu Pro Cys Thr Tyr Gln Met Ser Asn Thr Leu Thr Val Pro Ile
   55                  60                  65                  70
   GTG ATC TGG AAG TAC AAG TCA TTC TGC CGG GAC CGT ATT GCC GAT GCC      475
   Val Ile Trp Lys Tyr Lys Ser Phe Cys Arg Asp Arg Ile Ala Asp Ala
                   75                  80                  85
   TTC TCT CCT GCC AGT GTG GAC AAC CAG CTA AAT GCC CAG TTG GCA GCT      523
   Phe Ser Pro Ala Ser Val Asp Asn Gln Leu Asn Ala Gln Leu Ala Ala
               90                  95                  100
   GGC AAC CCC GGC TAC AAC CCC TAT GTG GAG TGC CAG GAC AGT GTA CGC      571
   Gly Asn Pro Gly Tyr Asn Pro Tyr Val Glu Cys Gln Asp Ser Val Arg
           105                 110                 115
   ACT GTC AGG GTG GTG GCC ACC AAA CAG GGC AAT GCG GTG ACC CTG GGA      619
   Thr Val Arg Val Val Ala Thr Lys Gln Gly Asn Ala Val Thr Leu Gly
       120                 125                 130
   GAC TAC TAC CAA GGC AGG AGG ATC ACC ATA ACA GGA AAT GCT GAC CTG      667
   Asp Tyr Tyr Gln Gly Arg Arg Ile Thr Ile Thr Gly Asn Ala Asp Leu
   135                 140                 145                 150
   ACC TTC GAG CAG ACA GCC TGG GGA GAC AGT GGA GTG TAT TAC TGC TCT      715
   Thr Phe Glu Gln Thr Ala Trp Gly Asp Ser Gly Val Tyr Tyr Cys Ser
                   155                 160                 165
   GTG GTC TCG GCC CAA GAT CTG GAT GGA AAC AAC GAG GCG TAC GCA GAG      763
   Val Val Ser Ala Gln Asp Leu Asp Gly Asn Asn Glu Ala Tyr Ala Glu
               170                 175                 180
   CTC ATC GTC CTT GTT TAT GCT GCT GGC AAA GCA GCC ACC TCA GGT GTC      811
   Leu Ile Val Leu Val Tyr Ala Ala Gly Lys Ala Ala Thr Ser Gly Val
           185                 190                 195
   CCG AGC ATC TAT GCC CCC AGC ATC TAT ACC CAC CTC TCA CCT GCC AAG      859
   Pro Ser Ile Tyr Ala Pro Ser Ile Tyr Thr His Leu Ser Pro Ala Lys
       200                 205                 210
   ACC CCA CCA CCT CCG CCT GCC ATG ATT CCC ATG GGC CCT CCC TAT GGG      907
   Thr Pro Pro Pro Pro Pro Ala Met Ile Pro Met Gly Pro Pro Tyr Gly
   215                 220                 225                 230
   TAC CCT GGA GAC TTT GAC AGA CAT AGC TCA GTT GGT GGC CAC AGC TCC      955
   Tyr Pro Gly Asp Phe Asp Arg His Ser Ser Val Gly Gly His Ser Ser
                   235                 240                 245
   CAA GTA CCC CTG CTG CGT GAC GTG GAT GGC AGT GTA TCT TCA GAA GTA     1003
   Gln Val Pro Leu Leu Arg Asp Val Asp Gly Ser Val Ser Ser Glu Val
               250                 255                 260
   CGA AGT GGC TAC AGG ATC CAG GCT AAC CAG CAA GAT GAC TCC ATG AGG     1051
   Arg Ser Gly Tyr Arg Ile Gln Ala Asn Gln Gln Asp Asp Ser Met Arg
           265                 270                 275
   GTC CTA TAC TAT ATG GAG AAA GAG CTA GCC AAC TTT GAC CCT TCC CGA     1099
   Val Leu Tyr Tyr Met Glu Lys Glu Leu Ala Asn Phe Asp Pro Ser Arg
       280                 285                 290
   CCT GGC CCT CCC AAT GGC AGA GTG GAA CGG GCC ATG AGT GAA GTA ACC     1147
   Pro Gly Pro Pro Asn Gly Arg Val Glu Arg Ala Met Ser Glu Val Thr
   295                 300                 305                 310
   TCC CTC CAT GAA GAT GAC TGG CGA TCG AGG CCT TCC AGG GCT CCT GCC     1195
   Ser Leu His Glu Asp Asp Trp Arg Ser Arg Pro Ser Arg Ala Pro Ala
                   315                 320                 325
   CTC ACC CCC ATC AGG GAT GAG GAG TGG AAT CGC CAC TCC CCA CAG AGT      1243
   Leu Thr Pro Ile Arg Asp Glu Glu Trp Asn Arg His Ser Pro Gln Ser
               330                 335                 340
   CCC AGA ACA TGG GAG CAG GAA CCC CTT CAA GAA CAA CCA AGG GGT GGT      1291
   Pro Arg Thr Trp Glu Gln Glu Pro Leu Gln Glu Gln Pro Arg Gly Gly
           345                 350                 355
   TGG GGG TCT GGA CGC CCT CGG GCC CGC TCT GTG GAT GCT CTA GAT GAT      1339
   Trp Gly Ser Gly Arg Pro Arg Ala Arg Ser Val Asp Ala Leu Asp Asp
       360                 365                 370
   ATC AAC CGG CCT GGC TCC ACT GAA TCA GGA CGG TCT TCT CCC CCA AGT      1387
   Ile Asn Arg Pro Gly Ser Thr Glu Ser Gly Arg Ser Ser Pro Pro Ser
   375                 380                 385                 390
   AGT GGA CGG AGA GGA CGG GCC TAT GCA CCT CCA AGA AGT CGC AGC CGG      1435
   Ser Gly Arg Arg Gly Arg Ala Tyr Ala Pro Pro Arg Ser Arg Ser Arg
                   395                 400                 405
   GAT GAC CTC TAT GAC CCG GAC GAT CCT AGG GAC TTG CCA CAT TCC CGA      1483
   Asp Asp Leu Tyr Asp Pro Asp Asp Pro Arg Asp Leu Pro His Ser Arg
               410                 415                 420
   GAT CCC CAC TAT TAT GAC GAC ATC AGG TCT AGA GAT CCA CGT GCT GAC      1531
   Asp Pro His Tyr Tyr Asp Asp Ile Arg Ser Arg Asp Pro Arg Ala Asp
           425                 430                 435
   CCC AGA TCC CGT CAG CGA TCC CGA GAT CCT CGG GAT GCT GGC TTC AGG      1579
   Pro Arg Ser Arg Gln Arg Ser Arg Asp Pro Arg Asp Ala Gly Phe Arg
       440                 445                 450
   TCA AGG GAC CCT CAG TAT GAT GGG CGA CTA TTA GAA GAG GCT TTA AAG      1627
   Ser Arg Asp Pro Gln Tyr Asp Gly Arg Leu Leu Glu Glu Ala Leu Lys
   455                 460                 465                 470
   AAA AAG GGG TCG GGC GAG AGA AGG AGG GTT TAC AGG GAG GAA GAA GAG      1675
   Lys Lys Gly Ser Gly Glu Arg Arg Arg Val Tyr Arg Glu Glu Glu Glu
                   475                  480                  485
   GAA GAG GAG GGC CAA TAC CCC CCA GCA CCT CCA CCT TAC TCA GAG ACT     1723
   Glu Glu Glu Gly Gln Tyr Pro Pro Ala Pro Pro Pro Tyr Ser Glu Thr
               490                  495                  500
   GAC TCG CAG GCC TCA CGG GAG AGG AGG CTG AAA AAG AAT TTG GCC CTG     1771
   Asp Ser Gln Ala Ser Arg Glu Arg Arg Leu Lys Lys Asn Leu Ala Leu
           505                  510                  515
   AGT CGG GAA AGT TTA GTC GTC TGA TCCACGTTTT GTATGTAGCT TTTGTACTTT    1825
   Ser Arg Glu Ser Leu Val Val  *
       520                  525
   TTTTTTAATT GGAATCAATA TTGATGAAAC TTCAAGCCTA ATAAAATGTC TAATCACAAA   1885
   AAAAAAAA                                                            1893
(2) INFORMATION FOR SEQ ID NO: 6:
   (i) SEQUENCE CHARACTERISTICS:
      (A) LENGTH: 525 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Rattus norvegicus
   (xi) SEQUENCE DESCRIPTION: SEQ ID NO: 6:
   Met Ala Pro Ala Ala Gly Ala Cys Ala Gly Ala Pro Asp Ser His Pro
   1               5                   10                  15
   Ala Thr Val Val Phe Val Cys Leu Phe Leu Ile Ile Phe Cys Pro Asp
   20 25 30
   Pro Ala Ser Ala Ile Gln Val Thr Val Ser Asp Pro Tyr His Val Val
   35 40 45
   Ile Leu Phe Gln Pro Val Thr Leu Pro Cys Thr Tyr Gln Met Ser Asn
   50 55 60
   Thr Leu Thr Val Pro Ile Val Ile Trp Lys Tyr Lys Ser Phe Cys Arg
   65 70 75 80
   Asp Arg Ile Ala Asp Ala Phe Ser Pro Ala Ser Val Asp Asn Gln Leu
   85 90 95
   Asn Ala Gln Leu Ala Ala Gly Asn Pro Gly Tyr Asn Pro Tyr Val Glu
   100 105 110
   Cys Gln Asp Ser Val Arg Thr Val Arg Val Val Ala Thr Lys Gln Gly
   115 120 125
   Asn Ala Val Thr Leu Gly Asp Tyr Tyr Gln Gly Arg Arg Ile Thr Ile
   130 135 140
   Thr Gly Asn Ala Asp Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Val Tyr Ala Ala Gly Lys
   180 185 190
   Ala Ala Thr Ser Gly Val Pro Ser Ile Tyr Ala Pro Ser Ile Tyr Thr
   195 200 205
   His Leu Ser Pro Ala Lys Thr Pro Pro Pro Pro Pro Ala Met Ile Pro
   210 215 220
   Met Gly Pro Pro Tyr Gly Tyr Pro Gly Asp Phe Asp Arg His Ser Ser
   225 230 235 240
   Val Gly Gly His Ser Ser Gln Val Pro Leu Leu Arg Asp Val Asp Gly
   245 250 255
   Ser Val Ser Ser Glu Val Arg Ser Gly Tyr Arg Ile Gln Ala Asn Gln
   260 265 270
   Gln Asp Asp Ser Met Arg Val Leu Tyr Tyr Met Glu Lys Glu Leu Ala
   275 280 285
   Asn Phe Asp Pro Ser Arg Pro Gly Pro Pro Asn Gly Arg Val Glu Arg
   290 295 300
   Ala Met Ser Glu Val Thr Ser Leu His Glu Asp Asp Trp Arg Ser Arg
   305 310 315 320
   Pro Ser Arg Ala Pro Ala Leu Thr Pro Ile Arg Asp Glu Glu Trp Asn
   325 330 335
   Arg His Ser Pro Gln Ser Pro Arg Thr Trp Glu Gln Glu Pro Leu Gln
   340 345 350
   Glu Gln Pro Arg Gly Gly Trp Gly Ser Gly Arg Pro Arg Ala Arg Ser
   355 360 365
   Val Asp Ala Leu Asp Asp Ile Asn Arg Pro Gly Ser Thr Glu Ser Gly
   370 375 380
   Arg Ser Ser Pro Pro Ser Ser Gly Arg Arg Gly Arg Ala Tyr Ala Pro
   385 390 395 400
   Pro Arg Ser Arg Ser Arg Asp Asp Leu Tyr Asp Pro Asp Asp Pro Arg
   405 410 415
   Asp Leu Pro His Ser Arg Asp Pro His Tyr Tyr Asp Asp Ile Arg Ser
   420 425 430
   Arg Asp Pro Arg Ala Asp Pro Arg Ser Arg Gln Arg Ser Arg Asp Pro
   435 440 445
   Arg Asp Ala Gly Phe Arg Ser Arg Asp Pro Gln Tyr Asp Gly Arg Leu
   450 455 460
   Leu Glu Glu Ala Leu Lys Lys Lys Gly Ser Gly Glu Arg Arg Arg Val
   465 470 475 480
   Tyr Arg Glu Glu Glu Glu Glu Glu Glu Gly Gln Tyr Pro Pro Ala Pro
   485 490 495
   Pro Pro Tyr Ser Glu Thr Asp Ser Gln Ala Ser Arg Glu Arg Arg Leu
   500 505 510
   Lys Lys Asn Leu Ala Leu Ser Arg Glu Ser Leu Val Val
   515 520 525
(2) INFORMATION FOR SEQ ID NO: 7:
   (i) SEQUENCE CHARACTERISTICS:
      (A) LENGTH: 2158 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (ix) FEATURE:
      (A) NAME/KEY: Potential splicing site AAG
      (B) LOCATION: 1259..1261
   (ix) FEATURE:
      (A) NAME/KEY: Potential insertion of a AGG
      (B) LOCATION: 1657
   (xi) SEQUENCE DESCRIPTION: SEQ ID NO: 7:
   TGGAGTGTGG CTCGGAGGAC CGCGGCGGGT CAAGCACCTT TCTCCCCCAT ATCTGAAAGC      60
   ATGCCCTTTG TCCACGTCGT TTACGCTCAT TAAAACTTCC AGA ATG CAA CAG GAC       115
                                                   Met Gln Gln Asp
                                                   1
   GGA CTT GGA GTA GGG ACA AGG AAC GGA AGT GGG AAG GGG AGG AGC GTG       163
   Gly Leu Gly Val Gly Thr Arg Asn Gly Ser Gly Lys Gly Arg Ser Val
   5                   10                  15                  20
   CAC CCC TCC TGG CCT TGG TGC GCG CCG CGC CCC CTA AGG TAC TTT GGA       211
   His Pro Ser Trp Pro Trp Cys Ala Pro Arg Pro Leu Arg Tyr Phe Gly
                   25                  30                  35
   AGG GAC GCG CGG GCC AGA CGC GCC CAG ACG GCC GCG ATG GCG CTG TTG       259
   Arg Asp Ala Arg Ala Arg Arg Ala Gln Thr Ala Ala Met Ala Leu Leu
               40                  45                  50
   GCC GGC GGG CTC TCC AGA GGG CTG GGC TCC CAC CCG GCC GCC GCA GGC       307
   Ala Gly Gly Leu Ser Arg Gly Leu Gly Ser His Pro Ala Ala Ala Gly
           55                  60                  65
   CGG GAC GCG GTC GTC TTC GTG TGG CTT CTG CTT AGC ACC TGG TGC ACA       355
   Arg Asp Ala Val Val Phe Val Trp Leu Leu Leu Ser Thr Trp Cys Thr
       70                  75                  80
   GCT CCT GCC AGG GCC ATC CAG GTG ACC GTG TCC AAC CCC TAC CAC GTG       403
   Ala Pro Ala Arg Ala Ile Gln Val Thr Val Ser Asn Pro Tyr His Val
   85                  90                  95                  100
   GTG ATC CTC TTC CAG CCT GTG ACC CTG CCC TGT ACC TAC CAG ATG ACC       451
   Val Ile Leu Phe Gln Pro Val Thr Leu Pro Cys Thr Tyr Gln Met Thr
                   105                 110                 115
   TCG ACC CCC ACG CAA CCC ATC GTC ATC TGG AAG TAC AAG TCT TTC TGC      499
   Ser Thr Pro Thr Gln Pro Ile Val Ile Trp Lys Tyr Lys Ser Phe Cys
               120                 125                 130
   CGG GAC CGC ATC GCC GAT GCC TTC TCC CCG GCC AGC GTC GAC AAC CAG      547
   Arg Asp Arg Ile Ala Asp Ala Phe Ser Pro Ala Ser Val Asp Asn Gln
           135                 140                 145
   CTC AAT GCC CAG CTG GCA GCC GGG AAC CCA GGC TAC AAC CCC TAC GTT      595
   Leu Asn Ala Gln Leu Ala Ala Gly Asn Pro Gly Tyr Asn Pro Tyr Val
       150                 155                 160
   GAG TGC CAG GAC AGC GTG CGC ACC GTC AGG GTC GTG GCC ACC AAG CAG      643
   Glu Cys Gln Asp Ser Val Arg Thr Val Arg Val Val Ala Thr Lys Gln
   165                 170                 175                 180
   GGC AAC GCT GTG ACC CTG GGA GAT TAC TAC CAG GGC CGG AGG ATT ACC      691
   Gly Asn Ala Val Thr Leu Gly Asp Tyr Tyr Gln Gly Arg Arg Ile Thr
                   185                 190                 195
   ATC ACC GGA AAT GCT GAC CTG ACC TTT GAC CAG ACG GCG TGG GGG GAC      739
   Ile Thr Gly Asn Ala Asp Leu Thr Phe Asp Gln Thr Ala Trp Gly Asp
               200                 205                 210
   AGT GGT GTG TAT TAC TGC TCC GTG GTC TCA GCC CAG GAC CTC CAG GGG      787
   Ser Gly Val Tyr Tyr Cys Ser Val Val Ser Ala Gln Asp Leu Gln Gly
           215                 220                 225
   AAC AAT GAG GCC TAC GCA GAG CTC ATC GTC CTT GGG AGG ACC TCA GGG      835
   Asn Asn Glu Ala Tyr Ala Glu Leu Ile Val Leu Gly Arg Thr Ser Gly
       230                 235                 240
   GTG GCT GAG CTC TTA CCT GGT TTT CAG GCG GGG CCC ATA GAA GAC TGG      883
   Val Ala Glu Leu Leu Pro Gly Phe Gln Ala Gly Pro Ile Glu Asp Trp
   245                 250                 255                 260
   CTC TTC GTG GTT GTG GTA TGC CTG GCT GCC TTC CTC ATC TTC CTC CTC      931
   Leu Phe Val Val Val Val Cys Leu Ala Ala Phe Leu Ile Phe Leu Leu
                   265                 270                 275
   CTG GGC ATC TGC TGG TGC CAG TGC TGC CCG CAC ACT TGC TGC TGC TAC      979
   Leu Gly Ile Cys Trp Cys Gln Cys Cys Pro His Thr Cys Cys Cys Tyr
               280                 285                 290
   GTC AGG TGC CCC TGC TGC CCA GAC AAG TGC TGC TGC CCC GAG GCC CTG     1027
   Val Arg Cys Pro Cys Cys Pro Asp Lys Cys Cys Cys Pro Glu Ala Leu
           295                 300                 305
   TAT GCC GCC GGC AAA GCA GCC ACC TCA GGT GTT CCC AGC ATT TAT GCC     1075
   Tyr Ala Ala Gly Lys Ala Ala Thr Ser Gly Val Pro Ser Ile Tyr Ala
       310                 315                 320
   CCC AGC ACC TAT GCC CAC CTG TCT CCC GCC AAG ACC CCA CCC CCA CCA     1123
   Pro Ser Thr Tyr Ala His Leu Ser Pro Ala Lys Thr Pro Pro Pro Pro
   325                 330                 335                 340
   GCT ATG ATT CCC ATG GGC CCT GCC TAC AAC GGG TAC CCT GGA GGA TAC     1171
   Ala Met Ile Pro Met Gly Pro Ala Tyr Asn Gly Tyr Pro Gly Gly Tyr
                   345                 350                 355
   CCT GGA GAC GTT GAC AGG AGT AGC TCA GCT GGT GGC CAA GGC TCC TAT     1219
   Pro Gly Asp Val Asp Arg Ser Ser Ser Ala Gly Gly Gln Gly Ser Tyr
               360                 365                 370
   GTA CCC CTG CTT CGG GAC ACG GAC AGC AGT GTG GCC TCT GAA GTC CGC     1267
   Val Pro Leu Leu Arg Asp Thr Asp Ser Ser Val Ala Ser Glu Val Arg
           375                 380                 385
   AGT GGC TAC AGG ATT CAG GCC AGC CAG CAG GAC GAC TCC ATG CGG GTC     1315
   Ser Gly Tyr Arg Ile Gln Ala Ser Gln Gln Asp Asp Ser Met Arg Val
       390                 395                 400
   CTG TAC TAC ATG GAG AAG GAG CTG GCC AAC TTC GAC CCT TCT CGA CCT     1363
   Leu Tyr Tyr Met Glu Lys Glu Leu Ala Asn Phe Asp Pro Ser Arg Pro
   405                 410                 415                 420
   GGC CCC CCC AGT GGC CGT GTG GAG CGG GCC ATG AGT GAA GTC ACC TCC      1411
   Gly Pro Pro Ser Gly Arg Val Glu Arg Ala Met Ser Glu Val Thr Ser
                   425                 430                 435
   CTC CAC GAG GAC GAC TGG CGA TCT CGG CCT TCC CGG GGC CCT GCC CTC      1459
   Leu His Glu Asp Asp Trp Arg Ser Arg Pro Ser Arg Gly Pro Ala Leu
               440                 445                 450
   ACC CCG ATC CGG GAT GAG GAG TGG GGT GGC CAC TCC CCC CGG AGT CCC      1507
   Thr Pro Ile Arg Asp Glu Glu Trp Gly Gly His Ser Pro Arg Ser Pro
           455                 460                 465
   AGG GGA TGG GAC CAG GAG CCC GCC AGG GAG CAG GCA GGC GGG GGC TGG      1555
   Arg Gly Trp Asp Gln Glu Pro Ala Arg Glu Gln Ala Gly Gly Gly Trp
       470                 475                 480
   CGG GCC AGG CGG CCC CGG GCC CGC TCC GTG GAC GCC CTG GAC GAC CTC      1603
   Arg Ala Arg Arg Pro Arg Ala Arg Ser Val Asp Ala Leu Asp Asp Leu
   485                 490                 495                 500
   ACC CCG CCG AGC ACC GCC GAG TCA GGG AGC AGG TCT CCC ACG AGT AAT      1651
   Thr Pro Pro Ser Thr Ala Glu Ser Gly Ser Arg Ser Pro Thr Ser Asn
                   505                 510                 515
   GGT GGG AGA AGC CGG GCC TAC ATG CCC CCG CGG AGC CGC AGC CGG GAC      1699
   Gly Gly Arg Ser Arg Ala Tyr Met Pro Pro Arg Ser Arg Ser Arg Asp
               520                 525                 530
   GAC CTC TAT GAC CAA GAC GAC TCG AGG GAC TTC CCA CGC TCC CGG GAC      1747
   Asp Leu Tyr Asp Gln Asp Asp Ser Arg Asp Phe Pro Arg Ser Arg Asp
           535                 540                 545
   CCC CAC TAC GAC GAC TTC AGG TCT CGG GAG CGC CCT CCT GCC GAC CCC      1795
   Pro His Tyr Asp Asp Phe Arg Ser Arg Glu Arg Pro Pro Ala Asp Pro
       550                 555                 560
   AGG TCC CAC CAC CAC CGT ACC CGG GAC CCT CGG GAC AAC GGC TCC AGG      1843
   Arg Ser His His His Arg Thr Arg Asp Pro Arg Asp Asn Gly Ser Arg
   565                 570                 575                 580
   TCC GGG GAC CTC CCC TAT GAT GGG CGG CTA CTG GAG GAG GCT GTG AGG      1891
   Ser Gly Asp Leu Pro Tyr Asp Gly Arg Leu Leu Glu Glu Ala Val Arg
                   585                 590                 595
   AAG AAG GGG TCG GAG GAG AGG AGG AGA CCC CAC AAG GAG GAG GAG GAA      1939
   Lys Lys Gly Ser Glu Glu Arg Arg Arg Pro His Lys Glu Glu Glu Glu
               600                 605                 610
   GAG GCC TAC TAC CCG CCC GCG CCG CCC CCG TAC TCG GAG ACC GAC TCG      1987
   Glu Ala Tyr Tyr Pro Pro Ala Pro Pro Pro Tyr Ser Glu Thr Asp Ser
           615                 620                 625
   CAG GCG TCC CGA GAG CGC AGG CTC AAG AAG AAC TTG GCC CTG AGT CGG      2035
   Gln Ala Ser Arg Glu Arg Arg Leu Lys Lys Asn Leu Ala Leu Ser Arg
       630                 635                 640
   GAA AGT TTA GTC GTC TGA TCTGACGTTT TCTACGTAGC TTTTGTATTT             2083
   Glu Ser Leu Val Val  *
   645                 650
   TTTTTTTTAA TTTGAAGGAA CACTGATGAA GCCCTGCCAT ACCCCTCCCG AGTCTAATAA    2143
   AACGTATAAT CACAA                                                     2158
(2) INFORMATION FOR SEQ ID NO: 8:
   (i) SEQUENCE CHARACTERISTICS:
      (A) LENGTH: 649 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Homo sapiens
   (ix) FEATURE:
      (A) NAME/KEY: Potential deletion of a Glu
      (B) LOCATION: 386
   (ix) FEATURE:
      (A) NAME/KEY: Potential insertion of a Arg
      (B) LOCATION: 518
   (xi) SEQUENCE DESCRIPTION: SEQ ID NO: 8:
   Met Gln Gln Asp Gly Leu Gly Val Gly Thr Arg Asn Gly Ser Gly Lys
   1               5                   10                  15
   Gly Arg Ser Val His Pro Ser Trp Pro Trp Cys Ala Pro Arg Pro Leu
               20                  25                  30
   Arg Tyr Phe Gly Arg Asp Ala Arg Ala Arg Arg Ala Gln Thr Ala Ala
           35                  40                  45
   Met Ala Leu Leu Ala Gly Gly Leu Ser Arg Gly Leu Gly Ser His Pro
       50                  55                  60
   Ala Ala Ala Gly Arg Asp Ala Val Val Phe Val Trp Leu Leu Leu Ser
   65                  70                  75                  80
   Thr Trp Cys Thr Ala Pro Ala Arg Ala Ile Gln Val Thr Val Ser Asn
                   85                  90                  95
   Pro Tyr His Val Val Ile Leu Phe Gln Pro Val Thr Leu Pro Cys Thr
               100                 105                 110
   Tyr Gln Met Thr Ser Thr Pro Thr Gln Pro Ile Val Ile Trp Lys Tyr
           115                 120                 125
   Lys Ser Phe Cys Arg Asp Arg Ile Ala Asp Ala Phe Ser Pro Ala Ser
   130 135 140
   Val Asp Asn Gln Leu Asn Ala Gln Leu Ala Ala Gly Asn Pro Gly Tyr
   145 150 155 160
   Asn Pro Tyr Val Glu Cys Gln Asp Ser Val Arg Thr Val Arg Val Val
   165 170 175
   Ala Thr Lys Gln Gly Asn Ala Val Thr Leu Gly Asp Tyr Tyr Gln Gly
   180 185 190
   Arg Arg Ile Thr Ile Thr Gly Asn Ala Asp Leu Thr Phe Asp Gln Thr
   195 200 205
   Ala Trp Gly Asp Ser Gly Val Tyr Tyr Cys Ser Val Val Ser Ala Gln
   210 215 220
   Asp Leu Gln Gly Asn Asn Glu Ala Tyr Ala Glu Leu Ile Val Leu Gly
   225 230 235 240
   Arg Thr Ser Gly Val Ala Glu Leu Leu Pro Gly Phe Gln Ala Gly Pro
   245 250 255
   Ile Glu Asp Trp Leu Phe Val Val Val Val Cys Leu Ala Ala Phe Leu
   260 265 270
   Ile Phe Leu Leu Leu Gly Ile Cys Trp Cys Gln Cys Cys Pro His Thr
   275 280 285
   Cys Cys Cys Tyr Val Arg Cys Pro Cys Cys Pro Asp Lys Cys Cys Cys
   290 295 300
   Pro Glu Ala Leu Tyr Ala Ala Gly Lys Ala Ala Thr Ser Gly Val Pro
   305 310 315 320
   Ser Ile Tyr Ala Pro Ser Thr Tyr Ala His Leu Ser Pro Ala Lys Thr
   325 330 335
   Pro Pro Pro Pro Ala Met Ile Pro Met Gly Pro Ala Tyr Asn Gly Tyr
   340 345 350
   Pro Gly Gly Tyr Pro Gly Asp Val Asp Arg Ser Ser Ser Ala Gly Gly
   355 360 365
   Gln Gly Ser Tyr Val Pro Leu Leu Arg Asp Thr Asp Ser Ser Val Ala
   370 375 380
   Ser Glu Val Arg Ser Gly Tyr Arg Ile Gln Ala Ser Gln Gln Asp Asp
   385 390 395 400
   Ser Met Arg Val Leu Tyr Tyr Met Glu Lys Glu Leu Ala Asn Phe Asp
   405 410 415
   Pro Ser Arg Pro Gly Pro Pro Ser Gly Arg Val Glu Arg Ala Met Ser
   420 425 430
   Glu Val Thr Ser Leu His Glu Asp Asp Trp Arg Ser Arg Pro Ser Arg
   435 440 445
   Gly Pro Ala Leu Thr Pro Ile Arg Asp Glu Glu Trp Gly Gly His Ser
   450 455 460
   Pro Arg Ser Pro Arg Gly Trp Asp Gln Glu Pro Ala Arg Glu Gln Ala
   465 470 475 480
   Gly Gly Gly Trp Arg Ala Arg Arg Pro Arg Ala Arg Ser Val Asp Ala
   485 490 495
   Leu Asp Asp Leu Thr Pro Pro Ser Thr Ala Glu Ser Gly Ser Arg Ser
   500 505 510
   Pro Thr Ser Asn Gly Gly Arg Ser Arg Ala Tyr Met Pro Pro Arg Ser
   515 520 525
   Arg Ser Arg Asp Asp Leu Tyr Asp Gln Asp Asp Ser Arg Asp Phe Pro
   530 535 540
   Arg Ser Arg Asp Pro His Tyr Asp Asp Phe Arg Ser Arg Glu Arg Pro
   545                 550                 555                 560
   Pro Ala Asp Pro Arg Ser His His His Arg Thr Arg Asp Pro Arg Asp
                   565                 570                 575
   Asn Gly Ser Arg Ser Gly Asp Leu Pro Tyr Asp Gly Arg Leu Leu Glu
               580                 585                 590
   Glu Ala Val Arg Lys Lys Gly Ser Glu Glu Arg Arg Arg Pro His Lys
           595                 600                 605
   Glu Glu Glu Glu Glu Ala Tyr Tyr Pro Pro Ala Pro Pro Pro Tyr Ser
       610                 615                 620
   Glu Thr Asp Ser Gln Ala Ser Arg Glu Arg Arg Leu Lys Lys Asn Leu
   625                 630                 635                 640
   Ala Leu Ser Arg Glu Ser Leu Val Val
                   645
(2) INFORMATION FOR SEQ ID NO: 9:
   (i) SEQUENCE CHARACTERISTICS:
      (A) LENGTH: 2101 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 9:
   TGGAGTGTGG CTCGGAGGAC CGCGGCGGGT CAAGCACCTT TCTCCCCCAT ATCTGAAAGC      60
   ATGCCCTTTG TCCACGTCGT TTACGCTCAT TAAAACTTCC AGA ATG CAA CAG GAC       115
                                                   Met Gln Gln Asp
                                                   1
   GGA CTT GGA GTA GGG ACA AGG AAC GGA AGT GGG AAG GGG AGG AGC GTG       163
   Gly Leu Gly Val Gly Thr Arg Asn Gly Ser Gly Lys Gly Arg Ser Val
   5                   10                  15                  20
   CAC CCC TCC TGG CCT TGG TGC GCG CCG CGC CCC CTA AGG TAC TTT GGA       211
   His Pro Ser Trp Pro Trp Cys Ala Pro Arg Pro Leu Arg Tyr Phe Gly
                   25                  30                  35
   AGG GAC GCG CGG GCC AGA CGC GCC CAG ACG GCC GCG ATG GCG CTG TTG       259
   Arg Asp Ala Arg Ala Arg Arg Ala Gln Thr Ala Ala Met Ala Leu Leu
               40                  45                  50
   GCC GGC GGG CTC TCC AGA GGG CTG GGC TCC CAC CCG GCC GCC GCA GGC       307
   Ala Gly Gly Leu Ser Arg Gly Leu Gly Ser His Pro Ala Ala Ala Gly
           55                  60                  65
   CGG GAC GCG GTC GTC TTC GTG TGG CTT CTG CTT AGC ACC TGG TGC ACA       355
   Arg Asp Ala Val Val Phe Val Trp Leu Leu Leu Ser Thr Trp Cys Thr
       70                  75                  80
   GCT CCT GCC AGG GCC ATC CAG GTG ACC GTG TCC AAC CCC TAC CAC GTG       403
   Ala Pro Ala Arg Ala Ile Gln Val Thr Val Ser Asn Pro Tyr His Val
   85                  90                  95                  100
   GTG ATC CTC TTC CAG CCT GTG ACC CTG CCC TGT ACC TAC CAG ATG ACC       451
   Val Ile Leu Phe Gln Pro Val Thr Leu Pro Cys Thr Tyr Gln Met Thr
                   105                 110                 115
   TCG ACC CCC ACG CAA CCC ATC GTC ATC TGG AAG TAC AAG TCT TTC TGC       499
   Ser Thr Pro Thr Gln Pro Ile Val Ile Trp Lys Tyr Lys Ser Phe Cys
               120                 125                 130
   CGG GAC CGC ATC GCC GAT GCC TTC TCC CCG GCC AGC GTC GAC AAC CAG       547
   Arg Asp Arg Ile Ala Asp Ala Phe Ser Pro Ala Ser Val Asp Asn Gln
           135                 140                 145
   CTC AAT GCC CAG CTG GCA GCC GGG AAC CCA GGC TAC AAC CCC TAC GTT      595
   Leu Asn Ala Gln Leu Ala Ala Gly Asn Pro Gly Tyr Asn Pro Tyr Val
       150                 155                 160
   GAG TGC CAG GAC AGC GTG CGC ACC GTC AGG GTC GTG GCC ACC AAG CAG      643
   Glu Cys Gln Asp Ser Val Arg Thr Val Arg Val Val Ala Thr Lys Gln
   165                 170                 175                 180
   GGC AAC GCT GTG ACC CTG GGA GAT TAC TAC CAG GGC CGG AGG ATT ACC      691
   Gly Asn Ala Val Thr Leu Gly Asp Tyr Tyr Gln Gly Arg Arg Ile Thr
                   185                 190                 195
   ATC ACC GGA AAT GCT GAC CTG ACC TTT GAC CAG ACG GCG TGG GGG GAC      739
   Ile Thr Gly Asn Ala Asp Leu Thr Phe Asp Gln Thr Ala Trp Gly Asp
               200                 205                 210
   AGT GGT GTG TAT TAC TGC TCC GTG GTC TCA GCC CAG GAC CTC CAG GGG      787
   Ser Gly Val Tyr Tyr Cys Ser Val Val Ser Ala Gln Asp Leu Gln Gly
           215                 220                 225
   AAC AAT GAG GCC TAC GCA GAG CTC ATC GTC CTT GAC TGG CTC TTC GTG      835
   Asn Asn Glu Ala Tyr Ala Glu Leu Ile Val Leu Asp Trp Leu Phe Val
       230                 235                 240
   GTT GTG GTA TGC CTG GCT GCC TTC CTC ATC TTC CTC CTC CTG GGC ATC      883
   Val Val Val Cys Leu Ala Ala Phe Leu Ile Phe Leu Leu Leu Gly Ile
   245                 250                 255                 260
   TGC TGG TGC CAG TGC TGC CCG CAC ACT TGC TGC TGC TAC GTC AGG TGC      931
   Cys Trp Cys Gln Cys Cys Pro His Thr Cys Cys Cys Tyr Val Arg Cys
                   265                 270                 275
   CCC TGC TGC CCA GAC AAG TGC TGC TGC CCC GAG GCC CTG TAT GCC GCC      979
   Pro Cys Cys Pro Asp Lys Cys Cys Cys Pro Glu Ala Leu Tyr Ala Ala
               280                 285                 290
   GGC AAA GCA GCC ACC TCA GGT GTT CCC AGC ATT TAT GCC CCC AGC ACC     1027
   Gly Lys Ala Ala Thr Ser Gly Val Pro Ser Ile Tyr Ala Pro Ser Thr
           295                 300                 305
   TAT GCC CAC CTG TCT CCC GCC AAG ACC CCA CCC CCA CCA GCT ATG ATT     1075
   Tyr Ala His Leu Ser Pro Ala Lys Thr Pro Pro Pro Pro Ala Met Ile
       310                 315                 320
   CCC ATG GGC CCT GCC TAC AAC GGG TAC CCT GGA GGA TAC CCT GGA GAC     1123
   Pro Met Gly Pro Ala Tyr Asn Gly Tyr Pro Gly Gly Tyr Pro Gly Asp
   325                 330                 335                 340
   GTT GAC AGG AGT AGC TCA GCT GGT GGC CAA GGC TCC TAT GTA CCC CTG     1171
   Val Asp Arg Ser Ser Ser Ala Gly Gly Gln Gly Ser Tyr Val Pro Leu
                   345                 350                 355
   CTT CGG GAC ACG GAC AGC AGT GTG GCC TCT GAA GTC CGC AGT GGC TAC     1219
   Leu Arg Asp Thr Asp Ser Ser Val Ala Ser Glu Val Arg Ser Gly Tyr
               360                 365                 370
   AGG ATT CAG GCC AGC CAG CAG GAC GAC TCC ATG CGG GTC CTG TAC TAC     1267
   Arg Ile Gln Ala Ser Gln Gln Asp Asp Ser Met Arg Val Leu Tyr Tyr
           375                 380                 385
   ATG GAG AAG GAG CTG GCC AAC TTC GAC CCT TCT CGA CCT GGC CCC CCC     1315
   Met Glu Lys Glu Leu Ala Asn Phe Asp Pro Ser Arg Pro Gly Pro Pro
       390                 395                 400
   AGT GGC CGT GTG GAG CGG GCC ATG AGT GAA GTC ACC TCC CTC CAC GAG     1363
   Ser Gly Arg Val Glu Arg Ala Met Ser Glu Val Thr Ser Leu His Glu
   405                 410                 415                 420
   GAC GAC TGG CGA TCT CGG CCT TCC CGG GGC CCT GCC CTC ACC CCG ATC     1411
   Asp Asp Trp Arg Ser Arg Pro Ser Arg Gly Pro Ala Leu Thr Pro Ile
                   425                 430                 435
   CGG GAT GAG GAG TGG GGT GGC CAC TCC CCC CGG AGT CCC AGG GGA TGG     1459
   Arg Asp Glu Glu Trp Gly Gly His Ser Pro Arg Ser Pro Arg Gly Trp
   440 445 450
   GAC CAG GAG CCC GCC AGG GAG CAG GCA GGC GGG GGC TGG CGG GCC AGG 1507
   Asp Gln Glu Pro Ala Arg Glu Gln Ala Gly Gly Gly Trp Arg Ala Arg
   455 460 465
   CGG CCC CGG GCC CGC TCC GTG GAC GCC CTG GAC GAC CTC ACC CCG CCG 1555
   Arg Pro Arg Ala Arg Ser Val Asp Ala Leu Asp Asp Leu Thr Pro Pro
   470 475 480
   AGC ACC GCC GAG TCA GGG AGC AGG TCT CCC ACG AGT AAT GGT GGG AGA 1603
   Ser Thr Ala Glu Ser Gly Ser Arg Ser Pro Thr Ser Asn Gly Gly Arg
   485 490 495 500
   AGC CGG GCC TAC ATG CCC CCG CGG AGC CGC AGC CGG GAC GAC CTC TAT 1651
   Ser Arg Ala Tyr Met Pro Pro Arg Ser Arg Ser Arg Asp Asp Leu Tyr
   505 510 515
   GAC CAA GAC GAC TCG AGG GAC TTC CCA CGC TCC CGG GAC CCC CAC TAC 1699
   Asp Gln Asp Asp Ser Arg Asp Phe Pro Arg Ser Arg Asp Pro His Tyr
   520 525 530
   GAC GAC TTC AGG TCT CGG GAG CGC CCT CCT GCC GAC CCC AGG TCC CAC 1747
   Asp Asp Phe Arg Ser Arg Glu Arg Pro Pro Ala Asp Pro Arg Ser His
   535 540 545
   CAC CAC CGT ACC CGG GAC CCT CGG GAC AAC GGC TCC AGG TCC GGG GAC 1795
   His His Arg Thr Arg Asp Pro Arg Asp Asn Gly Ser Arg Ser Gly Asp
   550 555 560
   CTC CCC TAT GAT GGG CGG CTA CTG GAG GAG GCT GTG AGG AAG AAG GGG 1843
   Leu Pro Tyr Asp Gly Arg Leu Leu Glu Glu Ala Val Arg Lys Lys Gly
   565 570 575 580
   TCG GAG GAG AGG AGG AGA CCC CAC AAG GAG GAG GAG GAA GAG GCC TAC 1891
   Ser Glu Glu Arg Arg Arg Pro His Lys Glu Glu Glu Glu Glu Ala Tyr
   585 590 595
   TAC CCG CCC GCG CCG CCC CCG TAC TCG GAG ACC GAC TCG CAG GCG TCC     1939
   Tyr Pro Pro Ala Pro Pro Pro Tyr Ser Glu Thr Asp Ser Gln Ala Ser
               600                 605                 610
   CGA GAG CGC AGG CTC AAG AAG AAC TTG GCC CTG AGT CGG GAA AGT TTA     1987
   Arg Glu Arg Arg Leu Lys Lys Asn Leu Ala Leu Ser Arg Glu Ser Leu
           615                 620                 625
   GTC GTC TGA TCTGACGTTT TCTACGTAGC TTTTGTATTT TTTTTTTTAA             2036
   Val Val  *
       630
   TTTGAAGGAA CACTGATGAA GCCCTGCCAT ACCCCTCCCG AGTCTAATAA AACGTATAAT   2096
   CACAA                                                               2101
(2) INFORMATION FOR SEQ ID NO: 10:
   (i) SEQUENCE CHARACTERISTICS:
      (A) LENGTH: 630 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Homo sapiens
   (xi) SEQUENCE DESCRIPTION: SEQ ID NO: 10:
   Met Gln Gln Asp Gly Leu Gly Val Gly Thr Arg Asn Gly Ser Gly Lys
   1               5                   10                  15
   Gly Arg Ser Val His Pro Ser Trp Pro Trp Cys Ala Pro Arg Pro Leu
               20                  25                  30
   Arg Tyr Phe Gly Arg Asp Ala Arg Ala Arg Arg Ala Gln Thr Ala Ala
   35 40 45
   Met Ala Leu Leu Ala Gly Gly Leu Ser Arg Gly Leu Gly Ser His Pro
   50 55 60
   Ala Ala Ala Gly Arg Asp Ala Val Val Phe Val Trp Leu Leu Leu Ser
   65 70 75 80
   Thr Trp Cys Thr Ala Pro Ala Arg Ala Ile Gln Val Thr Val Ser Asn
   85 90 95
   Pro Tyr His Val Val Ile Leu Phe Gln Pro Val Thr Leu Pro Cys Thr
   100 105 110
   Tyr Gln Met Thr Ser Thr Pro Thr Gln Pro Ile Val Ile Trp Lys Tyr
   115 120 125
   Lys Ser Phe Cys Arg Asp Arg Ile Ala Asp Ala Phe Ser Pro Ala Ser
   130 135 140
   Val Asp Asn Gln Leu Asn Ala Gln Leu Ala Ala Gly Asn Pro Gly Tyr
   145 150 155 160
   Asn Pro Tyr Val Glu Cys Gln Asp Ser Val Arg Thr Val Arg Val Val
   165 170 175
   Ala Thr Lys Gln Gly Asn Ala Val Thr Leu Gly Asp Tyr Tyr Gln Gly
   180 185 190
   Arg Arg Ile Thr Ile Thr Gly Asn Ala Asp Leu Thr Phe Asp Gln Thr
   195 200 205
   Ala Trp Gly Asp Ser Gly Val Tyr Tyr Cys Ser Val Val Ser Ala Gln
   210 215 220
   Asp Leu Gln Gly Asn Asn Glu Ala Tyr Ala Glu Leu Ile Val Leu Asp
   225 230 235 240
   Trp Leu Phe Val Val Val Val Cys Leu Ala Ala Phe Leu Ile Phe Leu
   245 250 255
   Leu Leu Gly Ile Cys Trp Cys Gln Cys Cys Pro His Thr Cys Cys Cys
   260 265 270
   Tyr Val Arg Cys Pro Cys Cys Pro Asp Lys Cys Cys Cys Pro Glu Ala
   275 280 285
   Leu Tyr Ala Ala Gly Lys Ala Ala Thr Ser Gly Val Pro Ser Ile Tyr
   290 295 300
   Ala Pro Ser Thr Tyr Ala His Leu Ser Pro Ala Lys Thr Pro Pro Pro
   305 310 315 320
   Pro Ala Met Ile Pro Met Gly Pro Ala Tyr Asn Gly Tyr Pro Gly Gly
   325 330 335
   Tyr Pro Gly Asp Val Asp Arg Ser Ser Ser Ala Gly Gly Gln Gly Ser
   340 345 350
   Tyr Val Pro Leu Leu Arg Asp Thr Asp Ser Ser Val Ala Ser Glu Val
   355 360 365
   Arg Ser Gly Tyr Arg Ile Gln Ala Ser Gln Gln Asp Asp Ser Met Arg
   370 375 380
   Val Leu Tyr Tyr Met Glu Lys Glu Leu Ala Asn Phe Asp Pro Ser Arg
   385 390 395 400
   Pro Gly Pro Pro Ser Gly Arg Val Glu Arg Ala Met Ser Glu Val Thr
   405 410 415
   Ser Leu His Glu Asp Asp Trp Arg Ser Arg Pro Ser Arg Gly Pro Ala
   420 425 430
   Leu Thr Pro Ile Arg Asp Glu Glu Trp Gly Gly His Ser Pro Arg Ser
   435 440 445
   Pro Arg Gly Trp Asp Gln Glu Pro Ala Arg Glu Gln Ala Gly Gly Gly
   450 455 460
   Trp Arg Ala Arg Arg Pro Arg Ala Arg Ser Val Asp Ala Leu Asp Asp
   465 470 475 480
   Leu Thr Pro Pro Ser Thr Ala Glu Ser Gly Ser Arg Ser Pro Thr Ser
   485 490 495
   Asn Gly Gly Arg Ser Arg Ala Tyr Met Pro Pro Arg Ser Arg Ser Arg
   500 505 510
   Asp Asp Leu Tyr Asp Gln Asp Asp Ser Arg Asp Phe Pro Arg Ser Arg
   515 520 525
   Asp Pro His Tyr Asp Asp Phe Arg Ser Arg Glu Arg Pro Pro Ala Asp
   530 535 540
   Pro Arg Ser His His His Arg Thr Arg Asp Pro Arg Asp Asn Gly Ser
   545 550 555 560
   Arg Ser Gly Asp Leu Pro Tyr Asp Gly Arg Leu Leu Glu Glu Ala Val
   565 570 575
   Arg Lys Lys Gly Ser Glu Glu Arg Arg Arg Pro His Lys Glu Glu Glu
   580 585 590
   Glu Glu Ala Tyr Tyr Pro Pro Ala Pro Pro Pro Tyr Ser Glu Thr Asp
   595 600 605
   Ser Gln Ala Ser Arg Glu Arg Arg Leu Lys Lys Asn Leu Ala Leu Ser
   610 615 620
   Arg Glu Ser Leu Val Val
   625 630
(2) INFORMATION FOR SEQ ID NO: 11:
   (i) SEQUENCE CHARACTERISTICS:
      (A) LENGTH: 1954 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 11:
   TGGAGTGTGG CTCGGAGGAC CGCGGCGGGT CAAGCACCTT TCTCCCCCAT ATCTGAAAGC       60
   ATGCCCTTTG TCCACGTCGT TTACGCTCAT TAAAACTTCC AGA ATG CAA CAG GAC      115
                                                   Met Gln Gln Asp
                                                   1
   GGA CTT GGA GTA GGG ACA AGG AAC GGA AGT GGG AAG GGG AGG AGC GTG      163
   Gly Leu Gly Val Gly Thr Arg Asn Gly Ser Gly Lys Gly Arg Ser Val
   5                   10                  15                  20
   CAC CCC TCC TGG CCT TGG TGC GCG CCG CGC CCC CTA AGG TAC TTT GGA      211
   His Pro Ser Trp Pro Trp Cys Ala Pro Arg Pro Leu Arg Tyr Phe Gly
                   25                  30                  35
   AGG GAC GCG CGG GCC AGA CGC GCC CAG ACG GCC GCG ATG GCG CTG TTG      259
   Arg Asp Ala Arg Ala Arg Arg Ala Gln Thr Ala Ala Met Ala Leu Leu
               40                  45                  50
   GCC GGC GGG CTC TCC AGA GGG CTG GGC TCC CAC CCG GCC GCC GCA GGC      307
   Ala Gly Gly Leu Ser Arg Gly Leu Gly Ser His Pro Ala Ala Ala Gly
           55                  60                  65
   CGG GAC GCG GTC GTC TTC GTG TGG CTT CTG CTT AGC ACC TGG TGC ACA      355
   Arg Asp Ala Val Val Phe Val Trp Leu Leu Leu Ser Thr Trp Cys Thr
        70                  75                  80
   GCT CCT GCC AGG GCC ATC CAG GTG ACC GTG TCC AAC CCC TAC CAC GTG       403
   Ala Pro Ala Arg Ala Ile Gln Val Thr Val Ser Asn Pro Tyr His Val
   85                  90                  95                  100
   GTG ATC CTC TTC CAG CCT GTG ACC CTG CCC TGT ACC TAC CAG ATG ACC       451
   Val Ile Leu Phe Gln Pro Val Thr Leu Pro Cys Thr Tyr Gln Met Thr
                   105                 110                 115
   TCG ACC CCC ACG CAA CCC ATC GTC ATC TGG AAG TAC AAG TCT TTC TGC       499
   Ser Thr Pro Thr Gln Pro Ile Val Ile Trp Lys Tyr Lys Ser Phe Cys
               120                 125                 130
   CGG GAC CGC ATC GCC GAT GCC TTC TCC CCG GCC AGC GTC GAC AAC CAG       547
   Arg Asp Arg Ile Ala Asp Ala Phe Ser Pro Ala Ser Val Asp Asn Gln
           135                 140                 145
   CTC AAT GCC CAG CTG GCA GCC GGG AAC CCA GGC TAC AAC CCC TAC GTT       595
   Leu Asn Ala Gln Leu Ala Ala Gly Asn Pro Gly Tyr Asn Pro Tyr Val
       150                 155                 160
   GAG TGC CAG GAC AGC GTG CGC ACC GTC AGG GTC GTG GCC ACC AAG CAG       643
   Glu Cys Gln Asp Ser Val Arg Thr Val Arg Val Val Ala Thr Lys Gln
   165                 170                 175                 180
   GGC AAC GCT GTG ACC CTG GGA GAT TAC TAC CAG GGC CGG AGG ATT ACC       691
   Gly Asn Ala Val Thr Leu Gly Asp Tyr Tyr Gln Gly Arg Arg Ile Thr
                   185                 190                 195
   ATC ACC GGA AAT GCT GAC CTG ACC TTT GAC CAG ACG GCG TGG GGG GAC       739
   Ile Thr Gly Asn Ala Asp Leu Thr Phe Asp Gln Thr Ala Trp Gly Asp
               200                 205                 210
   AGT GGT GTG TAT TAC TGC TCC GTG GTC TCA GCC CAG GAC CTC CAG GGG       787
   Ser Gly Val Tyr Tyr Cys Ser Val Val Ser Ala Gln Asp Leu Gln Gly
           215                 220                 225
   AAC AAT GAG GCC TAC GCA GAG CTC ATC GTC CTT GTG TAT GCC GCC GGC      835
   Asn Asn Glu Ala Tyr Ala Glu Leu Ile Val Leu Val Tyr Ala Ala Gly
       230                 235                 240
   AAA GCA GCC ACC TCA GGT GTT CCC AGC ATT TAT GCC CCC AGC ACC TAT      883
   Lys Ala Ala Thr Ser Gly Val Pro Ser Ile Tyr Ala Pro Ser Thr Tyr
   245                 250                 255                 260
   GCC CAC CTG TCT CCC GCC AAG ACC CCA CCC CCA CCA GCT ATG ATT CCC      931
   Ala His Leu Ser Pro Ala Lys Thr Pro Pro Pro Pro Ala Met Ile Pro
                   265                 270                 275
   ATG GGC CCT GCC TAC AAC GGG TAC CCT GGA GGA TAC CCT GGA GAC GTT      979
   Met Gly Pro Ala Tyr Asn Gly Tyr Pro Gly Gly Tyr Pro Gly Asp Val
               280                 285                 290
   GAC AGG AGT AGC TCA GCT GGT GGC CAA GGC TCC TAT GTA CCC CTG CTT     1027
   Asp Arg Ser Ser Ser Ala Gly Gly Gln Gly Ser Tyr Val Pro Leu Leu
           295                 300                 305
   CGG GAC ACG GAC AGC AGT GTG GCC TCT GAA GTC CGC AGT GGC TAC AGG     1075
   Arg Asp Thr Asp Ser Ser Val Ala Ser Glu Val Arg Ser Gly Tyr Arg
       310                 315                 320
   ATT CAG GCC AGC CAG CAG GAC GAC TCC ATG CGG GTC CTG TAC TAC ATG     1123
   Ile Gln Ala Ser Gln Gln Asp Asp Ser Met Arg Val Leu Tyr Tyr Met
   325                 330                 335                 340
   GAG AAG GAG CTG GCC AAC TTC GAC CCT TCT CGA CCT GGC CCC CCC AGT     1171
   Glu Lys Glu Leu Ala Asn Phe Asp Pro Ser Arg Pro Gly Pro Pro Ser
                   345                 350                 355
   GGC CGT GTG GAG CGG GCC ATG AGT GAA GTC ACC TCC CTC CAC GAG GAC     1219
   Gly Arg Val Glu Arg Ala Met Ser Glu Val Thr Ser Leu His Glu Asp
               360                 365                 370
   GAC TGG CGA TCT CGG CCT TCC CGG GGC CCT GCC CTC ACC CCG ATC CGG     1267
   Asp Trp Arg Ser Arg Pro Ser Arg Gly Pro Ala Leu Thr Pro Ile Arg
           375                 380                 385
   GAT GAG GAG TGG GGT GGC CAC TCC CCC CGG AGT CCC AGG GGA TGG GAC      1315
   Asp Glu Glu Trp Gly Gly His Ser Pro Arg Ser Pro Arg Gly Trp Asp
       390                 395                 400
   CAG GAG CCC GCC AGG GAG CAG GCA GGC GGG GGC TGG CGG GCC AGG CGG      1363
   Gln Glu Pro Ala Arg Glu Gln Ala Gly Gly Gly Trp Arg Ala Arg Arg
   405                 410                 415                 420
   CCC CGG GCC CGC TCC GTG GAC GCC CTG GAC GAC CTC ACC CCG CCG AGC      1411
   Pro Arg Ala Arg Ser Val Asp Ala Leu Asp Asp Leu Thr Pro Pro Ser
                   425                 430                 435
   ACC GCC GAG TCA GGG AGC AGG TCT CCC ACG AGT AAT GGT GGG AGA AGC      1459
   Thr Ala Glu Ser Gly Ser Arg Ser Pro Thr Ser Asn Gly Gly Arg Ser
               440                 445                 450
   CGG GCC TAC ATG CCC CCG CGG AGC CGC AGC CGG GAC GAC CTC TAT GAC      1507
   Arg Ala Tyr Met Pro Pro Arg Ser Arg Ser Arg Asp Asp Leu Tyr Asp
           455                 460                 465
   CAA GAC GAC TCG AGG GAC TTC CCA CGC TCC CGG GAC CCC CAC TAC GAC      1555
   Gln Asp Asp Ser Arg Asp Phe Pro Arg Ser Arg Asp Pro His Tyr Asp
       470                 475                 480
   GAC TTC AGG TCT CGG GAG CGC CCT CCT GCC GAC CCC AGG TCC CAC CAC      1603
   Asp Phe Arg Ser Arg Glu Arg Pro Pro Ala Asp Pro Arg Ser His His
   485                 490                 495                 500
   CAC CGT ACC CGG GAC CCT CGG GAC AAC GGC TCC AGG TCC GGG GAC CTC      1651
   His Arg Thr Arg Asp Pro Arg Asp Asn Gly Ser Arg Ser Gly Asp Leu
                   505                 510                 515
   CCC TAT GAT GGG CGG CTA CTG GAG GAG GCT GTG AGG AAG AAG GGG TCG      1699
   Pro Tyr Asp Gly Arg Leu Leu Glu Glu Ala Val Arg Lys Lys Gly Ser
               520                 525                 530
   GAG GAG AGG AGG AGA CCC CAC AAG GAG GAG GAG GAA GAG GCC TAC TAC      1747
   Glu Glu Arg Arg Arg Pro His Lys Glu Glu Glu Glu Glu Ala Tyr Tyr
             535                 540                 545
   CCG CCC GCG CCG CCC CCG TAC TCG GAG ACC GAC TCG CAG GCG TCC CGA      1795
   Pro Pro Ala Pro Pro Pro Tyr Ser Glu Thr Asp Ser Gln Ala Ser Arg
       550                 555                 560
   GAG CGC AGG CTC AAG AAG AAC TTG GCC CTG AGT CGG GAA AGT TTA GTC      1843
   Glu Arg Arg Leu Lys Lys Asn Leu Ala Leu Ser Arg Glu Ser Leu Val
   565                 570                 575                 580
   GTC TGA TCTGACGTTT TCTACGTAGC TTTTGTATTT TTTTTTTTAA TTTGAAGGAA       1899
   Val  *
   CACTGATGAA GCCCTGCCAT ACCCCTCCCG AGTCTAATAA AACGTATAAT CACAA         1954
(2) INFORMATION FOR SEQ ID NO: 12:
   (i) SEQUENCE CHARACTERISTICS:
      (A) LENGTH: 581 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Homo sapiens
   (xi) SEQUENCE DESCRIPTION: SEQ ID NO: 12:
   Met Gln Gln Asp Gly Leu Gly Val Gly Thr Arg Asn Gly Ser Gly Lys
   1               5                   10                  15
   Gly Arg Ser Val His Pro Ser Trp Pro Trp Cys Ala Pro Arg Pro Leu
               20                  25                  30
   Arg Tyr Phe Gly Arg Asp Ala Arg Ala Arg Arg Ala Gln Thr Ala Ala
   35 40 45
   Met Ala Leu Leu Ala Gly Gly Leu Ser Arg Gly Leu Gly Ser His Pro
   50 55 60
   Ala Ala Ala Gly Arg Asp Ala Val Val Phe Val Trp Leu Leu Leu Ser
   65 70 75 80
   Thr Trp Cys Thr Ala Pro Ala Arg Ala Ile Gln Val Thr Val Ser Asn
   85 90 95
   Pro Tyr His Val Val Ile Leu Phe Gln Pro Val Thr Leu Pro Cys Thr
   100 105 110
   Tyr Gln Met Thr Ser Thr Pro Thr Gln Pro Ile Val Ile Trp Lys Tyr
   115 120 125
   Lys Ser Phe Cys Arg Asp Arg Ile Ala Asp Ala Phe Ser Pro Ala Ser
   130 135 140
   Val Asp Asn Gln Leu Asn Ala Gln Leu Ala Ala Gly Asn Pro Gly Tyr
   145 150 155 160
   Asn Pro Tyr Val Glu Cys Gln Asp Ser Val Arg Thr Val Arg Val Val
   165 170 175
   Ala Thr Lys Gln Gly Asn Ala Val Thr Leu Gly Asp Tyr Tyr Gln Gly
   180 185 190
   Arg Arg Ile Thr Ile Thr Gly Asn Ala Asp Leu Thr Phe Asp Gln Thr
   195 200 205
   Ala Trp Gly Asp Ser Gly Val Tyr Tyr Cys Ser Val Val Ser Ala Gln
   210 215 220
   Asp Leu Gln Gly Asn Asn Glu Ala Tyr Ala Glu Leu Ile Val Leu Val
   225 230 235 240
   Tyr Ala Ala Gly Lys Ala Ala Thr Ser Gly Val Pro Ser Ile Tyr Ala
   245 250 255
   Pro Ser Thr Tyr Ala His Leu Ser Pro Ala Lys Thr Pro Pro Pro Pro
   260 265 270
   Ala Met Ile Pro Met Gly Pro Ala Tyr Asn Gly Tyr Pro Gly Gly Tyr
   275 280 285
   Pro Gly Asp Val Asp Arg Ser Ser Ser Ala Gly Gly Gln Gly Ser Tyr
   290 295 300
   Val Pro Leu Leu Arg Asp Thr Asp Ser Ser Val Ala Ser Glu Val Arg
   305 310 315 320
   Ser Gly Tyr Arg Ile Gln Ala Ser Gln Gln Asp Asp Ser Met Arg Val
   325 330 335
   Leu Tyr Tyr Met Glu Lys Glu Leu Ala Asn Phe Asp Pro Ser Arg Pro
   340 345 350
   Gly Pro Pro Ser Gly Arg Val Glu Arg Ala Met Ser Glu Val Thr Ser
   355 360 365
   Leu His Glu Asp Asp Trp Arg Ser Arg Pro Ser Arg Gly Pro Ala Leu
   370 375 380
   Thr Pro Ile Arg Asp Glu Glu Trp Gly Gly His Ser Pro Arg Ser Pro
   385 390 395 400
   Arg Gly Trp Asp Gln Glu Pro Ala Arg Glu Gln Ala Gly Gly Gly Trp
   405 410 415
   Arg Ala Arg Arg Pro Arg Ala Arg Ser Val Asp Ala Leu Asp Asp Leu
   420 425 430
   Thr Pro Pro Ser Thr Ala Glu Ser Gly Ser Arg Ser Pro Thr Ser Asn
   435 440 445
   Gly Gly Arg Ser Arg Ala Tyr Met Pro Pro Arg Ser Arg Ser Arg Asp
   450 455 460
   Asp Leu Tyr Asp Gln Asp Asp Ser Arg Asp Phe Pro Arg Ser Arg Asp
   465 470 475 480
   Pro His Tyr Asp Asp Phe Arg Ser Arg Glu Arg Pro Pro Ala Asp Pro
   485 490 495
   Arg Ser His His His Arg Thr Arg Asp Pro Arg Asp Asn Gly Ser Arg
   500 505 510
   Ser Gly Asp Leu Pro Tyr Asp Gly Arg Leu Leu Glu Glu Ala Val Arg
   515 520 525
   Lys Lys Gly Ser Glu Glu Arg Arg Arg Pro His Lys Glu Glu Glu Glu
   530 535 540
   Glu Ala Tyr Tyr Pro Pro Ala Pro Pro Pro Tyr Ser Glu Thr Asp Ser
   545 550 555 560
   Gln Ala Ser Arg Glu Arg Arg Leu Lys Lys Asn Leu Ala Leu Ser Arg
   565 570 575
   Glu Ser Leu Val Val
   580
(2) INFORMATION FOR SEQ ID NO: 13:
   (i) SEQUENCE CHARACTERISTICS:
      (A) LENGTH: 1886 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Mus musculus
   (xi) SEQUENCE DESCRIPTION: SEQ ID NO: 13:
   GCACCGTCGC TGCTAGACGG CCGCG ATG GCG CCG GCG GCC AGC GCG TGT GCT     52
                               Met Ala Pro Ala Ala Ser Ala Cys Ala
                               1               5
   GGG GCG CCT GGC TCC CAC CCG GCC ACC ACG ATC TTC GTG TGT CTT TTT    100
   Gly Ala Pro Gly Ser His Pro Ala Thr Thr Ile Phe Val Cys Leu Phe
   10                  15                  20                  25
   CTC ATC ATT TAC TGC CCA GAC CGT GCC AGT GCC ATC CAG GTG ACC GTG    148
   Leu Ile Ile Tyr Cys Pro Asp Arg Ala Ser Ala Ile Gln Val Thr Val
                   30                  35                  40
   CCT GAC CCC TAC CAC GTA GTG ATC CTG TTC CAG CCA GTG ACA CTA CAC    196
   Pro Asp Pro Tyr His Val Val Ile Leu Phe Gln Pro Val Thr Leu His
               45                  50                  55
   TGC ACC TAC CAG ATG AGC AAT ACC CTC ACA GCC CCT ATC GTG ATC TGG    244
   Cys Thr Tyr Gln Met Ser Asn Thr Leu Thr Ala Pro Ile Val Ile Trp
           60                  65                  70
   AAG TAT AAG TCG TTC TGT CGG GAC CGT GTT GCC GAC GCC TTC TCC CCT    292
   Lys Tyr Lys Ser Phe Cys Arg Asp Arg Val Ala Asp Ala Phe Ser Pro
       75                  80                  85
   GCC AGC GTG GAC AAC CAG CTC AAC GCC CAG CTG GCG GCT GGC AAC CCC    340
   Ala Ser Val Asp Asn Gln Leu Asn Ala Gln Leu Ala Ala Gly Asn Pro
   90                  95                  100                 105
   GGC TAC AAC CCC TAT GTG GAG TGC CAG GAC AGC GTA CGC ACT GTC AGG    388
   Gly Tyr Asn Pro Tyr Val Glu Cys Gln Asp Ser Val Arg Thr Val Arg
                   110                 115                 120
   GTG GTG GCC ACC AAA CAG GGC AAT GCT GTG ACC CTG GGA GAC TAC TAC      436
   Val Val Ala Thr Lys Gln Gly Asn Ala Val Thr Leu Gly Asp Tyr Tyr
               125                 130                 135
   CAG GGC AGG AGA ATC ACC ATC ACA GGA AAT GCT GGC CTG ACC TTC GAG      484
   Gln Gly Arg Arg Ile Thr Ile Thr Gly Asn Ala Gly Leu Thr Phe Glu
           140                 145                 150
   CAG ACG GCC TGG GGA GAC AGT GGA GTG TAT TAC TGC TCC GTG GTC TCA      532
   Gln Thr Ala Trp Gly Asp Ser Gly Val Tyr Tyr Cys Ser Val Val Ser
       155                 160                 165
   GCC CAA GAT CTG GAT GGG AAC AAC GAG GCG TAC GCA GAG CTC ATT GTC      580
   Ala Gln Asp Leu Asp Gly Asn Asn Glu Ala Tyr Ala Glu Leu Ile Val
   170                 175                 180                 185
   CTT GGC AGG ACC TCA GAA GCC CCT GAG CTC CTA CCT GGT TTT CGG GCG      628
   Leu Gly Arg Thr Ser Glu Ala Pro Glu Leu Leu Pro Gly Phe Arg Ala
                   190                 195                 200
   GGG CCC TTG GAA GAT TGG CTC TTT GTG GTC GTG GTC TGC CTG GCA AGC      676
   Gly Pro Leu Glu Asp Trp Leu Phe Val Val Val Val Cys Leu Ala Ser
               205                 210                 215
   CTC CTC TTC TTC CTC CTC CTG GGC ATC TGC TGG TGC CAG TGC TGT CCC      724
   Leu Leu Phe Phe Leu Leu Leu Gly Ile Cys Trp Cys Gln Cys Cys Pro
           220                 225                 230
   CAC ACC TGC TGC TGC TAT GTC AGA TGT CCC TGC TGC CCA GAC AAG TGC      772
   His Thr Cys Cys Cys Tyr Val Arg Cys Pro Cys Cys Pro Asp Lys Cys
       235                 240                 245
   TGT TGC CCT GAG GCC CTT TAT GCT GCT GGC AAA GCA GCC ACC TCA GGT      820
   Cys Cys Pro Glu Ala Leu Tyr Ala Ala Gly Lys Ala Ala Thr Ser Gly
   250                 255                 260                 265
   GTG CCA AGC ATC TAT GCC CCC AGC ATC TAT ACC CAC CTC TCT CCT GCC      868
   Val Pro Ser Ile Tyr Ala Pro Ser Ile Tyr Thr His Leu Ser Pro Ala
                   270                 275                 280
   AAG ACT CCG CCA CCT CCG CCT GCC ATG ATT CCC ATG CGT CCT CCC TAT      916
   Lys Thr Pro Pro Pro Pro Pro Ala Met Ile Pro Met Arg Pro Pro Tyr
               285                     290                     295
   GGG TAC CCT GGA GAC TTT GAC AGG ACC AGC TCA GTT GGT GGC CAC AGC      964
   Gly Tyr Pro Gly Asp Phe Asp Arg Thr Ser Ser Val Gly Gly His Ser
           300                     305                     310
   TCC CAG GTG CCC CTG CTG CGT GAA GTG GAT GGG AGC GTA TCT TCA GAA     1012
   Ser Gln Val Pro Leu Leu Arg Glu Val Asp Gly Ser Val Ser Ser Glu
       315                     320                     325
   GTA CGA AGT GGC TAC AGG ATC CAG GCT AAC CAG CAA GAT GAC TCC ATG     1060
   Val Arg Ser Gly Tyr Arg Ile Gln Ala Asn Gln Gln Asp Asp Ser Met
   330                     335                     340                 345
   AGG GTC CTA TAC TAT ATG GAG AAG GAG CTA GCC AAC TTC GAT CCT TCC     1108
   Arg Val Leu Tyr Tyr Met Glu Lys Glu Leu Ala Asn Phe Asp Pro Ser
                   350                     355                     360
   CGG CCT GGC CCT CCC AAT GGC CGA GTG GAA CGG GCC ATG AGT GAA GTA     1156
   Arg Pro Gly Pro Pro Asn Gly Arg Val Glu Arg Ala Met Ser Glu Val
               365                     370                     375
   ACC TCC CTC CAT GAA GAT GAC TGG CGA TCT CGG CCT TCC AGG GCT CCT     1204
   Thr Ser Leu His Glu Asp Asp Trp Arg Ser Arg Pro Ser Arg Ala Pro
           380                     385                     390
   GCC CTC ACA CCC ATC AGG GAT GAG GAG TGG AAT CGC CAC TCC CCT CGG     1252
   Ala Leu Thr Pro Ile Arg Asp Glu Glu Trp Asn Arg His Ser Pro Arg
       395                     400                     405
   AGT CCC AGA ACA TGG GAG CAG GAA CCC CTT CAA GAA CAG CCA AGG GGT     1300
   Ser Pro Arg Thr Trp Glu Gln Glu Pro Leu Gln Glu Gln Pro Arg Gly
   410                     415                     420                 425
   GGT TGG GGG TCT GGG CGG CCT CGG GCC CGC TCT GTG GAT GCT CTA GAT     1348
   Gly Trp Gly Ser Gly Arg Pro Arg Ala Arg Ser Val Asp Ala Leu Asp
   430 435 440
   GAC ATC AAC CGG CCT GGC TCC ACT GAA TCA GGA AGG TCT TCT CCC CCA 1396
   Asp Ile Asn Arg Pro Gly Ser Thr Glu Ser Gly Arg Ser Ser Pro Pro
   445 450 455
   AGT AGT GGA CGG AGA GGG CGG GCC TAT GCA CCT CCG AGA AGT CGC AGC 1444
   Ser Ser Gly Arg Arg Gly Arg Ala Tyr Ala Pro Pro Arg Ser Arg Ser
   460 465 470
   CGG GAT GAC CTC TAT GAC CCC GAC GAT CCT AGA GAC TTG CCA CAT TCC 1492
   Arg Asp Asp Leu Tyr Asp Pro Asp Asp Pro Arg Asp Leu Pro His Ser
   475 480 485
   CGA GAT CCC CAC TAT TAT GAT GAT TTG AGG TCT AGG GAT CCA CGT GCT 1540
   Arg Asp Pro His Tyr Tyr Asp Asp Leu Arg Ser Arg Asp Pro Arg Ala
   490 495 500 505
   GAC CCC AGA TCC CGT CAG CGA TCC CAC GAT CCT CGG GAT GCT GGC TTC 1588
   Asp Pro Arg Ser Arg Gln Arg Ser His Asp Pro Arg Asp Ala Gly Phe
   510 515 520
   AGG TCA CGG GAC CCT CAG TAT GAT GGG CGA CTC TTA GAA GAG GCT TTA 1636
   Arg Ser Arg Asp Pro Gln Tyr Asp Gly Arg Leu Leu Glu Glu Ala Leu
   525 530 535
   AAG AAA AAA GGG GCT GGG GAG AGA AGA CGC GTT TAC AGG GAG GAA GAA 1684
   Lys Lys Lys Gly Ala Gly Glu Arg Arg Arg Val Tyr Arg Glu Glu Glu
   540 545 550
   GAA GAA GAA GAG GAG GGC CAC TAT CCC CCA GCA CCT CCG CCT TAC TCT 1732
   Glu Glu Glu Glu Glu Gly His Tyr Pro Pro Ala Pro Pro Pro Tyr Ser
   555 560 565
   GAG ACT GAC TCG CAG GCC TCG AGG GAG CGG AGG ATG AAA AAG AAT TTG 1780
   Glu Thr Asp Ser Gln Ala Ser Arg Glu Arg Arg Met Lys Lys Asn Leu
   570 575 580 585
   GCC CTG AGT CGG GAA AGT TTA GTC GTC TGA TCCCACGTTT TGTTATGTAG 1830
   Ala Leu Ser Arg Glu Ser Leu Val Val  *
                   590                 595
   CTTTTATACT TTTTTAATTG GAATATTGAT GAAACTCTTC ACCAAGCCTA ATAAAA       1886
(2) INFORMATION FOR SEQ ID NO: 14:
   (i) SEQUENCE CHARACTERISTICS:
      (A) LENGTH: 1829 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Mus musculus
   (xi) SEQUENCE DESCRIPTION: SEQ ID NO: 14:
   GCACCGTCGC TGCTAGACGG CCGCG ATG GCG CCG GCG GCC AGC GCG TGT GCT       52
                               Met Ala Pro Ala Ala Ser Ala Cys Ala
                               1               5
   GGG GCG CCT GGC TCC CAC CCG GCC ACC ACG ATC TTC GTG TGT CTT TTT      100
   Gly Ala Pro Gly Ser His Pro Ala Thr Thr Ile Phe Val Cys Leu Phe
   10                  15                  20                  25
   CTC ATC ATT TAC TGC CCA GAC CGT GCC AGT GCC ATC CAG GTG ACC GTG      148
   Leu Ile Ile Tyr Cys Pro Asp Arg Ala Ser Ala Ile Gln Val Thr Val
                   30                  35                  40
   CCT GAC CCC TAC CAC GTA GTG ATC CTG TTC CAG CCA GTG ACA CTA CAC      196
   Pro Asp Pro Tyr His Val Val Ile Leu Phe Gln Pro Val Thr Leu His
               45                  50                  55
   TGC ACC TAC CAG ATG AGC AAT ACC CTC ACA GCC CCT ATC GTG ATC TGG      244
   Cys Thr Tyr Gln Met Ser Asn Thr Leu Thr Ala Pro Ile Val Ile Trp
           60                  65                  70
   AAG TAT AAG TCG TTC TGT CGG GAC CGT GTT GCC GAC GCC TTC TCC CCT      292
   Lys Tyr Lys Ser Phe Cys Arg Asp Arg Val Ala Asp Ala Phe Ser Pro
       75                  80                  85
   GCC AGC GTG GAC AAC CAG CTC AAC GCC CAG CTG GCG GCT GGC AAC CCC      340
   Ala Ser Val Asp Asn Gln Leu Asn Ala Gln Leu Ala Ala Gly Asn Pro
   90                  95                  100                 105
   GGC TAC AAC CCC TAT GTG GAG TGC CAG GAC AGC GTA CGC ACT GTC AGG      388
   Gly Tyr Asn Pro Tyr Val Glu Cys Gln Asp Ser Val Arg Thr Val Arg
                   110                 115                 120
   GTG GTG GCC ACC AAA CAG GGC AAT GCT GTG ACC CTG GGA GAC TAC TAC      436
   Val Val Ala Thr Lys Gln Gly Asn Ala Val Thr Leu Gly Asp Tyr Tyr
               125                 130                 135
   CAG GGC AGG AGA ATC ACC ATC ACA GGA AAT GCT GGC CTG ACC TTC GAG      484
   Gln Gly Arg Arg Ile Thr Ile Thr Gly Asn Ala Gly Leu Thr Phe Glu
           140                 145                 150
   CAG ACG GCC TGG GGA GAC AGT GGA GTG TAT TAC TGC TCC GTG GTC TCA      532
   Gln Thr Ala Trp Gly Asp Ser Gly Val Tyr Tyr Cys Ser Val Val Ser
       155                 160                 165
   GCC CAA GAT CTG GAT GGG AAC AAC GAG GCG TAC GCA GAG CTC ATT GTC      580
   Ala Gln Asp Leu Asp Gly Asn Asn Glu Ala Tyr Ala Glu Leu Ile Val
   170                 175                 180                 185
   CTT GAT TGG CTC TTT GTG GTC GTG GTC TGC CTG GCA AGC CTC CTC TTC      628
   Leu Asp Trp Leu Phe Val Val Val Val Cys Leu Ala Ser Leu Leu Phe
                   190                 195                 200
   TTC CTC CTC CTG GGC ATC TGC TGG TGC CAG TGC TGT CCC CAC ACC TGC      676
   Phe Leu Leu Leu Gly Ile Cys Trp Cys Gln Cys Cys Pro His Thr Cys
               205                 210                 215
   TGC TGC TAT GTC AGA TGT CCC TGC TGC CCA GAC AAG TGC TGT TGC CCT       724
   Cys Cys Tyr Val Arg Cys Pro Cys Cys Pro Asp Lys Cys Cys Cys Pro
           220                 225                 230
   GAG GCC CTT TAT GCT GCT GGC AAA GCA GCC ACC TCA GGT GTG CCA AGC       772
   Glu Ala Leu Tyr Ala Ala Gly Lys Ala Ala Thr Ser Gly Val Pro Ser
       235                 240                 245
   ATC TAT GCC CCC AGC ATC TAT ACC CAC CTC TCT CCT GCC AAG ACT CCG       820
   Ile Tyr Ala Pro Ser Ile Tyr Thr His Leu Ser Pro Ala Lys Thr Pro
   250                 255                 260                 265
   CCA CCT CCG CCT GCC ATG ATT CCC ATG CGT CCT CCC TAT GGG TAC CCT       868
   Pro Pro Pro Pro Ala Met Ile Pro Met Arg Pro Pro Tyr Gly Tyr Pro
                   270                 275                 280
   GGA GAC TTT GAC AGG ACC AGC TCA GTT GGT GGC CAC AGC TCC CAG GTG       916
   Gly Asp Phe Asp Arg Thr Ser Ser Val Gly Gly His Ser Ser Gln Val
               285                 290                 295
   CCC CTG CTG CGT GAA GTG GAT GGG AGC GTA TCT TCA GAA GTA CGA AGT       964
   Pro Leu Leu Arg Glu Val Asp Gly Ser Val Ser Ser Glu Val Arg Ser
           300                 305                 310
   GGC TAC AGG ATC CAG GCT AAC CAG CAA GAT GAC TCC ATG AGG GTC CTA      1012
   Gly Tyr Arg Ile Gln Ala Asn Gln Gln Asp Asp Ser Met Arg Val Leu
       315                 320                 325
   TAC TAT ATG GAG AAG GAG CTA GCC AAC TTC GAT CCT TCC CGG CCT GGC      1060
   Tyr Tyr Met Glu Lys Glu Leu Ala Asn Phe Asp Pro Ser Arg Pro Gly
   330                 335                 340                 345
   CCT CCC AAT GGC CGA GTG GAA CGG GCC ATG AGT GAA GTA ACC TCC CTC      1108
   Pro Pro Asn Gly Arg Val Glu Arg Ala Met Ser Glu Val Thr Ser Leu
                   350                 355                 360
   CAT GAA GAT GAC TGG CGA TCT CGG CCT TCC AGG GCT CCT GCC CTC ACA      1156
   His Glu Asp Asp Trp Arg Ser Arg Pro Ser Arg Ala Pro Ala Leu Thr
               365                 370                 375
   CCC ATC AGG GAT GAG GAG TGG AAT CGC CAC TCC CCT CGG AGT CCC AGA      1204
   Pro Ile Arg Asp Glu Glu Trp Asn Arg His Ser Pro Arg Ser Pro Arg
           380                 385                 390
   ACA TGG GAG CAG GAA CCC CTT CAA GAA CAG CCA AGG GGT GGT TGG GGG      1252
   Thr Trp Glu Gln Glu Pro Leu Gln Glu Gln Pro Arg Gly Gly Trp Gly
       395                 400                 405
   TCT GGG CGG CCT CGG GCC CGC TCT GTG GAT GCT CTA GAT GAC ATC AAC      1300
   Ser Gly Arg Pro Arg Ala Arg Ser Val Asp Ala Leu Asp Asp Ile Asn
   410                 415                 420                 425
   CGG CCT GGC TCC ACT GAA TCA GGA AGG TCT TCT CCC CCA AGT AGT GGA      1348
   Arg Pro Gly Ser Thr Glu Ser Gly Arg Ser Ser Pro Pro Ser Ser Gly
                   430                 435                 440
   CGG AGA GGG CGG GCC TAT GCA CCT CCG AGA AGT CGC AGC CGG GAT GAC      1396
   Arg Arg Gly Arg Ala Tyr Ala Pro Pro Arg Ser Arg Ser Arg Asp Asp
               445                 450                 455
   CTC TAT GAC CCC GAC GAT CCT AGA GAC TTG CCA CAT TCC CGA GAT CCC      1444
   Leu Tyr Asp Pro Asp Asp Pro Arg Asp Leu Pro His Ser Arg Asp Pro
           460                 465                 470
   CAC TAT TAT GAT GAT TTG AGG TCT AGG GAT CCA CGT GCT GAC CCC AGA      1492
   His Tyr Tyr Asp Asp Leu Arg Ser Arg Asp Pro Arg Ala Asp Pro Arg
       475                 480                 485
   TCC CGT CAG CGA TCC CAC GAT CCT CGG GAT GCT GGC TTC AGG TCA CGG      1540
   Ser Arg Gln Arg Ser His Asp Pro Arg Asp Ala Gly Phe Arg Ser Arg
   490                 495                 500                 505
   GAC CCT CAG TAT GAT GGG CGA CTC TTA GAA GAG GCT TTA AAG AAA AAA      1588
   Asp Pro Gln Tyr Asp Gly Arg Leu Leu Glu Glu Ala Leu Lys Lys Lys
                   510                 515                 520
   GGG GCT GGG GAG AGA AGA CGC GTT TAC AGG GAG GAA GAA GAA GAA GAA     1636
   Gly Ala Gly Glu Arg Arg Arg Val Tyr Arg Glu Glu Glu Glu Glu Glu
               525                 530                 535
   GAG GAG GGC CAC TAT CCC CCA GCA CCT CCG CCT TAC TCT GAG ACT GAC     1684
   Glu Glu Gly His Tyr Pro Pro Ala Pro Pro Pro Tyr Ser Glu Thr Asp
           540                 545                 550
   TCG CAG GCC TCG AGG GAG CGG AGG ATG AAA AAG AAT TTG GCC CTG AGT     1732
   Ser Gln Ala Ser Arg Glu Arg Arg Met Lys Lys Asn Leu Ala Leu Ser
       555                 560                 565
   CGG GAA AGT TTA GTC GTC TGA TCCCACGTTT TGTTATGTAG CTTTTATACT        1783
   Arg Glu Ser Leu Val Val  *
   570                 575
   TTTTTAATTG GAATATTGAT GAAACTCTTC ACCAAGCCTA ATAAAA                  1829
(2) INFORMATION FOR SEQ ID NO: 15:
   (i) SEQUENCE CHARACTERISTICS:
      (A) LENGTH: 1682 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Mus musculus
   (xi) SEQUENCE DESCRIPTION: SEQ ID NO: 15:
   GCACCGTCGC TGCTAGACGG CCGCG ATG GCG CCG GCG GCC AGC GCG TGT GCT       52
                               Met Ala Pro Ala Ala Ser Ala Cys Ala
                                           1              5
   GGG GCG CCT GGC TCC CAC CCG GCC ACC ACG ATC TTC GTG TGT CTT TTT      100
   Gly Ala Pro Gly Ser His Pro Ala Thr Thr Ile Phe Val Cys Leu Phe
   10                  15                  20                  25
   CTC ATC ATT TAC TGC CCA GAC CGT GCC AGT GCC ATC CAG GTG ACC GTG      148
   Leu Ile Ile Tyr Cys Pro Asp Arg Ala Ser Ala Ile Gln Val Thr Val
                   30                  35                  40
   CCT GAC CCC TAC CAC GTA GTG ATC CTG TTC CAG CCA GTG ACA CTA CAC      196
   Pro Asp Pro Tyr His Val Val Ile Leu Phe Gln Pro Val Thr Leu His
               45                  50                  55
   TGC ACC TAC CAG ATG AGC AAT ACC CTC ACA GCC CCT ATC GTG ATC TGG      244
   Cys Thr Tyr Gln Met Ser Asn Thr Leu Thr Ala Pro Ile Val Ile Trp
           60                  65                  70
   AAG TAT AAG TCG TTC TGT CGG GAC CGT GTT GCC GAC GCC TTC TCC CCT      292
   Lys Tyr Lys Ser Phe Cys Arg Asp Arg Val Ala Asp Ala Phe Ser Pro
       75                  80                  85
   GCC AGC GTG GAC AAC CAG CTC AAC GCC CAG CTG GCG GCT GGC AAC CCC      340
   Ala Ser Val Asp Asn Gln Leu Asn Ala Gln Leu Ala Ala Gly Asn Pro
   90                  95                  100                 105
   GGC TAC AAC CCC TAT GTG GAG TGC CAG GAC AGC GTA CGC ACT GTC AGG      388
   Gly Tyr Asn Pro Tyr Val Glu Cys Gln Asp Ser Val Arg Thr Val Arg
                   110                 115                 120
   GTG GTG GCC ACC AAA CAG GGC AAT GCT GTG ACC CTG GGA GAC TAC TAC      436
   Val Val Ala Thr Lys Gln Gly Asn Ala Val Thr Leu Gly Asp Tyr Tyr
               125                 130                 135
   CAG GGC AGG AGA ATC ACC ATC ACA GGA AAT GCT GGC CTG ACC TTC GAG      484
   Gln Gly Arg Arg Ile Thr Ile Thr Gly Asn Ala Gly Leu Thr Phe Glu
           140                 145                 150
   CAG ACG GCC TGG GGA GAC AGT GGA GTG TAT TAC TGC TCC GTG GTC TCA      532
   Gln Thr Ala Trp Gly Asp Ser Gly Val Tyr Tyr Cys Ser Val Val Ser
       155                 160                 165
   GCC CAA GAT CTG GAT GGG AAC AAC GAG GCG TAC GCA GAG CTC ATT GTC      580
   Ala Gln Asp Leu Asp Gly Asn Asn Glu Ala Tyr Ala Glu Leu Ile Val
   170                 175                 180                 185
   CTT GTT TAT GCT GCT GGC AAA GCA GCC ACC TCA GGT GTG CCA AGC ATC      628
   Leu Val Tyr Ala Ala Gly Lys Ala Ala Thr Ser Gly Val Pro Ser Ile
                   190                 195                 200
   TAT GCC CCC AGC ATC TAT ACC CAC CTC TCT CCT GCC AAG ACT CCG CCA      676
   Tyr Ala Pro Ser Ile Tyr Thr His Leu Ser Pro Ala Lys Thr Pro Pro
               205                 210                 215
   CCT CCG CCT GCC ATG ATT CCC ATG CGT CCT CCC TAT GGG TAC CCT GGA      724
   Pro Pro Pro Ala Met Ile Pro Met Arg Pro Pro Tyr Gly Tyr Pro Gly
           220                 225                 230
   GAC TTT GAC AGG ACC AGC TCA GTT GGT GGC CAC AGC TCC CAG GTG CCC      772
   Asp Phe Asp Arg Thr Ser Ser Val Gly Gly His Ser Ser Gln Val Pro
       235                 240                 245
   CTG CTG CGT GAA GTG GAT GGG AGC GTA TCT TCA GAA GTA CGA AGT GGC      820
   Leu Leu Arg Glu Val Asp Gly Ser Val Ser Ser Glu Val Arg Ser Gly
   250                 255                 260                 265
   TAC AGG ATC CAG GCT AAC CAG CAA GAT GAC TCC ATG AGG GTC CTA TAC      868
   Tyr Arg Ile Gln Ala Asn Gln Gln Asp Asp Ser Met Arg Val Leu Tyr
                   270                 275                 280
   TAT ATG GAG AAG GAG CTA GCC AAC TTC GAT CCT TCC CGG CCT GGC CCT      916
   Tyr Met Glu Lys Glu Leu Ala Asn Phe Asp Pro Ser Arg Pro Gly Pro
               285                 290                 295
   CCC AAT GGC CGA GTG GAA CGG GCC ATG AGT GAA GTA ACC TCC CTC CAT      964
   Pro Asn Gly Arg Val Glu Arg Ala Met Ser Glu Val Thr Ser Leu His
           300                 305                 310
   GAA GAT GAC TGG CGA TCT CGG CCT TCC AGG GCT CCT GCC CTC ACA CCC 1012
   Glu Asp Asp Trp Arg Ser Arg Pro Ser Arg Ala Pro Ala Leu Thr Pro
   315 320 325
   ATC AGG GAT GAG GAG TGG AAT CGC CAC TCC CCT CGG AGT CCC AGA ACA 1060
   Ile Arg Asp Glu Glu Trp Asn Arg His Ser Pro Arg Ser Pro Arg Thr
   330 335 340 345
   TGG GAG CAG GAA CCC CTT CAA GAA CAG CCA AGG GGT GGT TGG GGG TCT 1108
   Trp Glu Gln Glu Pro Leu Gln Glu Gln Pro Arg Gly Gly Trp Gly Ser
   350 355 360
   GGG CGG CCT CGG GCC CGC TCT GTG GAT GCT CTA GAT GAC ATC AAC CGG 1156
   Gly Arg Pro Arg Ala Arg Ser Val Asp Ala Leu Asp Asp Ile Asn Arg
   365 370 375
   CCT GGC TCC ACT GAA TCA GGA AGG TCT TCT CCC CCA AGT AGT GGA CGG 1204
   Pro Gly Ser Thr Glu Ser Gly Arg Ser Ser Pro Pro Ser Ser Gly Arg
   380 385 390
   AGA GGG CGG GCC TAT GCA CCT CCG AGA AGT CGC AGC CGG GAT GAC CTC 1252
   Arg Gly Arg Ala Tyr Ala Pro Pro Arg Ser Arg Ser Arg Asp Asp Leu
   395 400 405
   TAT GAC CCC GAC GAT CCT AGA GAC TTG CCA CAT TCC CGA GAT CCC CAC 1300
   Tyr Asp Pro Asp Asp Pro Arg Asp Leu Pro His Ser Arg Asp Pro His
   410 415 420 425
   TAT TAT GAT GAT TTG AGG TCT AGG GAT CCA CGT GCT GAC CCC AGA TCC 1348
   Tyr Tyr Asp Asp Leu Arg Ser Arg Asp Pro Arg Ala Asp Pro Arg Ser
   430 435 440
   CGT CAG CGA TCC CAC GAT CCT CGG GAT GCT GGC TTC AGG TCA CGG GAC 1396
   Arg Gln Arg Ser His Asp Pro Arg Asp Ala Gly Phe Arg Ser Arg Asp
   445 450 455
   CCT CAG TAT GAT GGG CGA CTC TTA GAA GAG GCT TTA AAG AAA AAA GGG 1444
   Pro Gln Tyr Asp Gly Arg Leu Leu Glu Glu Ala Leu Lys Lys Lys Gly
   460 465 470
   GCT GGG GAG AGA AGA CGC GTT TAC AGG GAG GAA GAA GAA GAA GAA GAG      1492
   Ala Gly Glu Arg Arg Arg Val Tyr Arg Glu Glu Glu Glu Glu Glu Glu
       475                 480                 485
   GAG GGC CAC TAT CCC CCA GCA CCT CCG CCT TAC TCT GAG ACT GAC TCG      1540
   Glu Gly His Tyr Pro Pro Ala Pro Pro Pro Tyr Ser Glu Thr Asp Ser
   490                 495                 500                 505
   CAG GCC TCG AGG GAG CGG AGG ATG AAA AAG AAT TTG GCC CTG AGT CGG      1588
   Gln Ala Ser Arg Glu Arg Arg Met Lys Lys Asn Leu Ala Leu Ser Arg
                   510                 515                 520
   GAA AGT TTA GTC GTC TGA TCCCACGTTT TGTTATGTAG CTTTTATACT             1636
   Glu Ser Leu Val Val  *
               525
   TTTTTAATTG GAATATTGAT GAAACTCTTC ACCAAGCCTA ATAAAA                   1682
(2) INFORMATION FOR SEQ ID NO: 16:
   (i) SEQUENCE CHARACTERISTICS:
      (A) LENGTH: 594 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Mus musculus
   (xi) SEQUENCE DESCRIPTION: SEQ ID NO: 16:
   Met Ala Pro Ala Ala Ser Ala Cys Ala Gly Ala Pro Gly Ser His Pro
   1               5                   10                  15
   Ala Thr Thr Ile Phe Val Cys Leu Phe Leu Ile Ile Tyr Cys Pro Asp
   20 25 30
   Arg Ala Ser Ala Ile Gln Val Thr Val Pro Asp Pro Tyr His Val Val
   35 40 45
   Ile Leu Phe Gln Pro Val Thr Leu His Cys Thr Tyr Gln Met Ser Asn
   50 55 60
   Thr Leu Thr Ala Pro Ile Val Ile Trp Lys Tyr Lys Ser Phe Cys Arg
   65 70 75 80
   Asp Arg Val Ala Asp Ala Phe Ser Pro Ala Ser Val Asp Asn Gln Leu
   85 90 95
   Asn Ala Gln Leu Ala Ala Gly Asn Pro Gly Tyr Asn Pro Tyr Val Glu
   100 105 110
   Cys Gln Asp Ser Val Arg Thr Val Arg Val Val Ala Thr Lys Gln Gly
   115 120 125
   Asn Ala Val Thr Leu Gly Asp Tyr Tyr Gln Gly Arg Arg Ile Thr Ile
   130 135 140
   Thr Gly Asn Ala Gly Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Gly Arg Thr Ser Glu Ala
   180 185 190
   Pro Glu Leu Leu Pro Gly Phe Arg Ala Gly Pro Leu Glu Asp Trp Leu
   195 200 205
   Phe Val Val Val Val Cys Leu Ala Ser Leu Leu Phe Phe Leu Leu Leu
   210 215 220
   Gly Ile Cys Trp Cys Gln Cys Cys Pro His Thr Cys Cys Cys Tyr Val
   225 230 235 240
   Arg Cys Pro Cys Cys Pro Asp Lys Cys Cys Cys Pro Glu Ala Leu Tyr
   245 250 255
   Ala Ala Gly Lys Ala Ala Thr Ser Gly Val Pro Ser Ile Tyr Ala Pro
   260 265 270
   Ser Ile Tyr Thr His Leu Ser Pro Ala Lys Thr Pro Pro Pro Pro Pro
   275 280 285
   Ala Met Ile Pro Met Arg Pro Pro Tyr Gly Tyr Pro Gly Asp Phe Asp
   290 295 300
   Arg Thr Ser Ser Val Gly Gly His Ser Ser Gln Val Pro Leu Leu Arg
   305 310 315 320
   Glu Val Asp Gly Ser Val Ser Ser Glu Val Arg Ser Gly Tyr Arg Ile
   325 330 335
   Gln Ala Asn Gln Gln Asp Asp Ser Met Arg Val Leu Tyr Tyr Met Glu
   340 345 350
   Lys Glu Leu Ala Asn Phe Asp Pro Ser Arg Pro Gly Pro Pro Asn Gly
   355 360 365
   Arg Val Glu Arg Ala Met Ser Glu Val Thr Ser Leu His Glu Asp Asp
   370 375 380
   Trp Arg Ser Arg Pro Ser Arg Ala Pro Ala Leu Thr Pro Ile Arg Asp
   385 390 395 400
   Glu Glu Trp Asn Arg His Ser Pro Arg Ser Pro Arg Thr Trp Glu Gln
   405 410 415
   Glu Pro Leu Gln Glu Gln Pro Arg Gly Gly Trp Gly Ser Gly Arg Pro
   420 425 430
   Arg Ala Arg Ser Val Asp Ala Leu Asp Asp Ile Asn Arg Pro Gly Ser
           435                 440                 445
   Thr Glu Ser Gly Arg Ser Ser Pro Pro Ser Ser Gly Arg Arg Gly Arg
       450                 455                 460
   Ala Tyr Ala Pro Pro Arg Ser Arg Ser Arg Asp Asp Leu Tyr Asp Pro
   465                 470                 475                 480
   Asp Asp Pro Arg Asp Leu Pro His Ser Arg Asp Pro His Tyr Tyr Asp
                   485                 490                 495
   Asp Leu Arg Ser Arg Asp Pro Arg Ala Asp Pro Arg Ser Arg Gln Arg
               500                 505                 510
   Ser His Asp Pro Arg Asp Ala Gly Phe Arg Ser Arg Asp Pro Gln Tyr
           515                 520                 525
   Asp Gly Arg Leu Leu Glu Glu Ala Leu Lys Lys Lys Gly Ala Gly Glu
       530                 535                 540
   Arg Arg Arg Val Tyr Arg Glu Glu Glu Glu Glu Glu Glu Glu Gly His
   545                 550                 555                 560
   Tyr Pro Pro Ala Pro Pro Pro Tyr Ser Glu Thr Asp Ser Gln Ala Ser
                   565                 570                 575
   Arg Glu Arg Arg Met Lys Lys Asn Leu Ala Leu Ser Arg Glu Ser Leu
               580                 585                 590
   Val Val
(2) INFORMATION FOR SEQ ID NO: 17:
   (i) SEQUENCE CHARACTERISTICS:
      (A) LENGTH: 575 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Mus musculus
   (xi) SEQUENCE DESCRIPTION: SEQ ID NO: 17:
   Met Ala Pro Ala Ala Ser Ala Cys Ala Gly Ala Pro Gly Ser His Pro
   1               5                   10                  15
   Ala Thr Thr Ile Phe Val Cys Leu Phe Leu Ile Ile Tyr Cys Pro Asp
               20                  25                  30
   Arg Ala Ser Ala Ile Gln Val Thr Val Pro Asp Pro Tyr His Val Val
           35                  40                  45
   Ile Leu Phe Gln Pro Val Thr Leu His Cys Thr Tyr Gln Met Ser Asn
       50                  55                  60
   Thr Leu Thr Ala Pro Ile Val Ile Trp Lys Tyr Lys Ser Phe Cys Arg
   65                  70                  75                  80
   Asp Arg Val Ala Asp Ala Phe Ser Pro Ala Ser Val Asp Asn Gln Leu
                   85                  90                  95
   Asn Ala Gln Leu Ala Ala Gly Asn Pro Gly Tyr Asn Pro Tyr Val Glu
               100                 105                 110
   Cys Gln Asp Ser Val Arg Thr Val Arg Val Val Ala Thr Lys Gln Gly
           115                 120                 125
   Asn Ala Val Thr Leu Gly Asp Tyr Tyr Gln Gly Arg Arg Ile Thr Ile
       130                 135                 140
   Thr Gly Asn Ala Gly Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Asp Trp Leu Phe Val Val
   180 185 190
   Val Val Cys Leu Ala Ser Leu Leu Phe Phe Leu Leu Leu Gly Ile Cys
   195 200 205
   Trp Cys Gln Cys Cys Pro His Thr Cys Cys Cys Tyr Val Arg Cys Pro
   210 215 220
   Cys Cys Pro Asp Lys Cys Cys Cys Pro Glu Ala Leu Tyr Ala Ala Gly
   225 230 235 240
   Lys Ala Ala Thr Ser Gly Val Pro Ser Ile Tyr Ala Pro Ser Ile Tyr
   245 250 255
   Thr His Leu Ser Pro Ala Lys Thr Pro Pro Pro Pro Pro Ala Met Ile
   260 265 270
   Pro Met Arg Pro Pro Tyr Gly Tyr Pro Gly Asp Phe Asp Arg Thr Ser
   275 280 285
   Ser Val Gly Gly His Ser Ser Gln Val Pro Leu Leu Arg Glu Val Asp
   290 295 300
   Gly Ser Val Ser Ser Glu Val Arg Ser Gly Tyr Arg Ile Gln Ala Asn
   305 310 315 320
   Gln Gln Asp Asp Ser Met Arg Val Leu Tyr Tyr Met Glu Lys Glu Leu
   325 330 335
   Ala Asn Phe Asp Pro Ser Arg Pro Gly Pro Pro Asn Gly Arg Val Glu
   340 345 350
   Arg Ala Met Ser Glu Val Thr Ser Leu His Glu Asp Asp Trp Arg Ser
   355 360 365
   Arg Pro Ser Arg Ala Pro Ala Leu Thr Pro Ile Arg Asp Glu Glu Trp
   370 375 380
   Asn Arg His Ser Pro Arg Ser Pro Arg Thr Trp Glu Gln Glu Pro Leu
   385 390 395 400
   Gln Glu Gln Pro Arg Gly Gly Trp Gly Ser Gly Arg Pro Arg Ala Arg
   405 410 415
   Ser Val Asp Ala Leu Asp Asp Ile Asn Arg Pro Gly Ser Thr Glu Ser
   420 425 430
   Gly Arg Ser Ser Pro Pro Ser Ser Gly Arg Arg Gly Arg Ala Tyr Ala
   435 440 445
   Pro Pro Arg Ser Arg Ser Arg Asp Asp Leu Tyr Asp Pro Asp Asp Pro
   450 455 460
   Arg Asp Leu Pro His Ser Arg Asp Pro His Tyr Tyr Asp Asp Leu Arg
   465 470 475 480
   Ser Arg Asp Pro Arg Ala Asp Pro Arg Ser Arg Gln Arg Ser His Asp
   485 490 495
   Pro Arg Asp Ala Gly Phe Arg Ser Arg Asp Pro Gln Tyr Asp Gly Arg
   500 505 510
   Leu Leu Glu Glu Ala Leu Lys Lys Lys Gly Ala Gly Glu Arg Arg Arg
   515 520 525
   Val Tyr Arg Glu Glu Glu Glu Glu Glu Glu Glu Gly His Tyr Pro Pro
   530 535 540
   Ala Pro Pro Pro Tyr Ser Glu Thr Asp Ser Gln Ala Ser Arg Glu Arg
   545 550 555 560
   Arg Met Lys Lys Asn Leu Ala Leu Ser Arg Glu Ser Leu Val Val
   565 570 575
(2) INFORMATION FOR SEQ ID NO: 18:
   (i) SEQUENCE CHARACTERISTICS:
      (A) LENGTH: 526 amino acids
      (B) TYPE: AMINO ACID
      (C) STRANDEDNESS: SINGLE
      (D) TOPOLOGY: LINEAR
   (ii) MOLECULE TYPE: PROTEIN
   (vi) ORIGINAL SOURCE:
      (A) ORGANISM: Mus musculus
   (xi) SEQUENCE DESCRIPTION: SEQ ID NO: 18:
   Met Ala Pro Ala Ala Ser Ala Cys Ala Gly Ala Pro Gly Ser His Pro
   1 5 10 15
   Ala Thr Thr Ile Phe Val Cys Leu Phe Leu Ile Ile Tyr Cys Pro Asp
   20 25 30
   Arg Ala Ser Ala Ile Gln Val Thr Val Pro Asp Pro Tyr His Val Val
   35 40 45
   Ile Leu Phe Gln Pro Val Thr Leu His Cys Thr Tyr Gln Met Ser Asn
   50 55 60
   Thr Leu Thr Ala Pro Ile Val Ile Trp Lys Tyr Lys Ser Phe Cys Arg
   65 70 75 80
   Asp Arg Val Ala Asp Ala Phe Ser Pro Ala Ser Val Asp Asn Gln Leu
   85 90 95
   Asn Ala Gln Leu Ala Ala Gly Asn Pro Gly Tyr Asn Pro Tyr Val Glu
   100 105 110
   Cys Gln Asp Ser Val Arg Thr Val Arg Val Val Ala Thr Lys Gln Gly
   115 120 125
   Asn Ala Val Thr Leu Gly Asp Tyr Tyr Gln Gly Arg Arg Ile Thr Ile
   130 135 140
   Thr Gly Asn Ala Gly Leu Thr Phe Glu Gln Thr Ala Trp Gly Asp Ser
   145 150 155 160
   Gly Val Tyr Tyr Cys Ser Val Val Ser Ala Gln Asp Leu Asp Gly Asn
   165 170 175
   Asn Glu Ala Tyr Ala Glu Leu Ile Val Leu Val Tyr Ala Ala Gly Lys
   180 185 190
   Ala Ala Thr Ser Gly Val Pro Ser Ile Tyr Ala Pro Ser Ile Tyr Thr
   195 200 205
   His Leu Ser Pro Ala Lys Thr Pro Pro Pro Pro Pro Ala Met Ile Pro
   210 215 220
   Met Arg Pro Pro Tyr Gly Tyr Pro Gly Asp Phe Asp Arg Thr Ser Ser
   225 230 235 240
   Val Gly Gly His Ser Ser Gln Val Pro Leu Leu Arg Glu Val Asp Gly
   245 250 255
   Ser Val Ser Ser Glu Val Arg Ser Gly Tyr Arg Ile Gln Ala Asn Gln
   260 265 270
   Gln Asp Asp Ser Met Arg Val Leu Tyr Tyr Met Glu Lys Glu Leu Ala
   275 280 285
   Asn Phe Asp Pro Ser Arg Pro Gly Pro Pro Asn Gly Arg Val Glu Arg
   290 295 300
   Ala Met Ser Glu Val Thr Ser Leu His Glu Asp Asp Trp Arg Ser Arg
   305 310 315 320
   Pro Ser Arg Ala Pro Ala Leu Thr Pro Ile Arg Asp Glu Glu Trp Asn
   325 330 335
   Arg His Ser Pro Arg Ser Pro Arg Thr Trp Glu Gln Glu Pro Leu Gln
   340 345 350
   Glu Gln Pro Arg Gly Gly Trp Gly Ser Gly Arg Pro Arg Ala Arg Ser
   355 360 365
   Val Asp Ala Leu Asp Asp Ile Asn Arg Pro Gly Ser Thr Glu Ser Gly
   370 375 380
   Arg Ser Ser Pro Pro Ser Ser Gly Arg Arg Gly Arg Ala Tyr Ala Pro
   385 390 395 400
   Pro Arg Ser Arg Ser Arg Asp Asp Leu Tyr Asp Pro Asp Asp Pro Arg
   405 410 415
   Asp Leu Pro His Ser Arg Asp Pro His Tyr Tyr Asp Asp Leu Arg Ser
   420 425 430
   Arg Asp Pro Arg Ala Asp Pro Arg Ser Arg Gln Arg Ser His Asp Pro
   435 440 445
   Arg Asp Ala Gly Phe Arg Ser Arg Asp Pro Gln Tyr Asp Gly Arg Leu
   450 455 460
   Leu Glu Glu Ala Leu Lys Lys Lys Gly Ala Gly Glu Arg Arg Arg Val
   465 470 475 480
   Tyr Arg Glu Glu Glu Glu Glu Glu Glu Glu Gly His Tyr Pro Pro Ala
   485 490 495
   Pro Pro Pro Tyr Ser Glu Thr Asp Ser Gln Ala Ser Arg Glu Arg Arg
   500 505 510
   Met Lys Lys Asn Leu Ala Leu Ser Arg Glu Ser Leu Val Val
               515                 520                 525
(2) INFORMATION FOR SEQ ID NO: 19:
   (i) SEQUENCE CHARACTERISTICS:
      (A) LENGTH: 22976 base pairs
      (B) TYPE: NUCLEIC ACID
      (C) STRANDEDNESS: DOUBLE
      (D) TOPOLOGY: LINEAR
   (ii) MOLECULE TYPE: DNA
   (vi) ORIGINAL SOURCE:
      (A) ORGANISM: Homo sapiens
   (ix) FEATURE:
      (A) NAME/KEY: exon1
      (B) LOCATION: 1898..2253
   (ix) FEATURE:
      (A) NAME/KEY: exon2
      (B) LOCATION: 3437..3781
   (ix) FEATURE:
      (A) NAME/KEY: exon3
      (B) LOCATION: 12065..12184
   (ix) FEATURE:
      (A) NAME/KEY: exon4
      (B) LOCATION: 15045..15101
   (ix) FEATURE:
      (A) NAME/KEY: exon5
      (B) LOCATION: 15666..15812
   (ix) FEATURE:
      (A) NAME/KEY: exon6
      (B) LOCATION: 19479..19652
   (ix) FEATURE:
      (A) NAME/KEY: exon7
      (B) LOCATION: 19799..19858
   (ix) FEATURE:
      (A) NAME/KEY: exon8
      (B) LOCATION: 19956..20087
   (ix) FEATURE:
      (A) NAME/KEY: exon9
      (B) LOCATION: 20229..20854
   (ix) FEATURE:
      (A) NAME/KEY: exon10
      (B) LOCATION: 20944..21094
   (ix) FEATURE:
      (A) NAME/KEY: Potential variant splicing site AAG
      (B) LOCATION: 19956..19958
   (xi) SEQUENCE DESCRIPTION: SEQ ID NO: 19:
   AACAGTTTGG CAGTTCCTCA AAAGGTTAAA AATAGAACTA CCAAGTCACC CAGCAATTCC     60
   ATTCTTAGGC ATATATTCAA AAGAAATGAA AGCAGATATT TGTACACCAG TGTTCACAGC    120
   TGCACTATTT ACAATAGTCA AAAGGTAGAA ACAACCTAGG TCCATCCACA AATGAATGGA    180
   TAAATAAAAC GTAGCATATA CATACAATGG TACACTAGTC CGCTGTAAAA AGAAATTTTG    240
   ATCTTACTGC ATGCTACATG GCTTCGACAT ACTACAACAT GGATGGACCT TGAAAACATT    300
   ATTCTTTGTG AAATAAACTA GACACAGGAC AAATGTTAGA CGATTCCACT TATATGAGGC    360
   ACCTAGAATG GGCAATTTGG TAAGCAAAGT AGAATAGAAA TTACTAGGGG CACAGGTAGC    420
   AGGGAATGGG GAGTTACTGT TTAATGGTCA CAGAGTTTAT GTTGGGGATG ATGAAACAGT 480
   TTCGGGGATA AAGAGTGGTG ATTGGTACAC GACATTGTGA ATATACTTAA TGCCACTGAA 540
   TTTTACACTT GAAGTGGTTA AAGCGATAAA TATTATAGTT TGCATATTTT ATCATAAAAA 600
   TATTTTTTTA AACGATGAAG GGACGTGAAC GGGTTGAAAT TTTATAAAAA GTGGCCAGGG 660
   AAGGTGTCAC TGCAATGGTG TCCTACAGGA GGAGGAAGAT CATGTGGACA TCTGCGGGAA 720
   GGGTGTTCTG GCAGAGGGAG TAGCACGGGC GATGGCTCTG AGGACTGTGA GAAGTATAGT 780
   TGGAAACAGC GAGGAGGCCA GGGTGTCCGA AGCTGAGTAA GCCAGAGAGA GTGGGAGGAG 840
   GTGAGATAAG AGGGGGAAGG TCAGTTTCTG CTGAGAGTGA GGAGGAGCCA CAGGAGGGCT 900
   GTGAGCAGGT GGACGTGATC TGGCTTGAGT TTTAACAGGG CCAGTAGAAC AAAGCACGCC 960
   TGGGTACCGA AACCAGCCAC TGGCCAGTTG GCAACCTGGG GGAGTCTAAC GCGAGGAAGC 1020
   GCCCAGGGTT CCCCCAGGAT GCGCTTTCCC TCGCCGCCAC CTGGAGACAG CAGAGTCACG 1080
   CCCAGCGCTG CGCAGGCTGA TCGCCGCGCC GCGCCCCCGC CCTCGGTCGC AGGTGGCTCG 1140
   TTCCGGGAAT TCCTAAGCGG AAACCGGTCC CAAGCCCCGC GCCTTCGCTC GGCCCCTTTA 1200
   AGAGCCAGAA TTTCCGGAGG GCTGACCCGG GGGCTAGGGA TGCCCAGGGG CCGAACCACA 1260
   AGTTGGGAAC GGGTGGGGGA GGTGGCGAAA ACTTCCGAAG TGGAATTCCA ACTTTTCCTG 1320
   GCCCTGATTC CCCTTGGGCA TCCCTGAGGG GGCAGAGCTT CCCTTCCGGG GACTTTAGAG 1380
   GGTTCCTCAG GTCATCTAAC TGGGAGACAC AGGAGGCCCG AAGCGCCCCC CCTCCACCCG 1440
   GTCCGGAGGA ACCCCAGTGG AAGTGGAGAA GTCAGGCGCC ACCAACAAGC CTCTCCCAGC 1500
   CAGGACTTTG CTTAGACTCG CTCCTCCCGG CAGGGCGCAC CTAGGCGGGT CCATCGCCAG 1560
   CCGGGGAGAG GGGTTTGGGC AGGGAGGGAA CAGGTGCGCG GCGGGACCCG CCCTATCTCA 1620
   ACAGGTGAAT CGCTCCAAGT GGGTCTCGGT TGCATGGATC TCGGTGCGCT TGGTTTGGCC 1680
   GGAGCAGATG GGGGCCGGAA GGGACCTGTG GTCCGCAGGC GCCCTCCCAG CGGGCCAGTC 1740
   ACTTGGTTCG GGCCCTGGGG GACGGAGCGC ACCTGGGTCA GCCCACTTCC GGGGAGGGAG 1800
   GCAGAGGAAC CCCTCCCCGC CGCTCACCCC TAAGCCCAGC CCTCGGCTCC CACCCTTGTG 1860
   TACCTGGGCC GAACCATTCA CCGGAGCGCG CAGCGGGTGG AGTGTGGCTC GGAGGACCGC 1920
   GGCGGGTCAA GCACCTTTCT CCCCCATATC TGAAAGCATG CCCTTTGTCC ACGTCGTTTA 1980
   CGCTCATTAA AACTTCCAGA ATGCAACAGG ACGGACTTGG AGTAGGGACA AGGAACGGAA 2040
   GTGGGAAGGG GAGGAGCGTG CACCCCTCCT GGCCTTGGTG CGCGCCGCGC CCCCTAAGGT 2100
   ACTTTGGAAG GGACGCGCGG GCCAGACGCG CCCAGACGGC CGCGATGGCG CTGTTGGCCG 2160
   GCGGGCTCTC CAGAGGGCTG GGCTCCCACC CGGCCGCCGC AGGCCGGGAC GCGGTCGTCT 2220
   TCGTGTGGCT TCTGCTTAGC ACCTGGTGCA CAGGTACGGG GCACGGGGCC TCTGACGCTG 2280
   CGGAACGCCG GAGGGAACTG TAGAGGGGGA TGGATGGAGT TGGAGGCGGC GGGAAGCGGG 2340
   AAGCGGGGGT CTCAGAGGCT GGGACCTTCC GATCCCCTGG GTCTTGGGCG ATCTGTTGCG 2400
   CGCGGGAGTG AGAGGAATTC CCCATTTGTG CCGGGGAGCG CTCCCCGCGC CCTTATCTGG 2460
   AAGATAGCAG GAAGTGAAAC TCCCTGGACG GTGAGACCCG GAGCGGCAGG GAGAATGGAA 2520
   CTCTTTGTGG GGAGGGAGTG GAAGACCGCC CGATCTCTGG GAAAAGAAAA GCCGGGATGG 2580
   GACTTGGGCG CACCCGGGGA TTTCTAAGTT TTGGAGTAAC GGGGAGAGGG CACGGGAGGG 2640
   CTGGATCAGA CGCTTCCTAG AGGGACAGAG ACGAAGGAAC AATGCCTAGG CCTCGGGTGG 2700
   GTGTGGGACT GGGGACTCCC CATCCCCCGC ACCCCACCCA CCTCCCGCGG GCTCCGGATT 2760
   ATACGTGCGT AAGAGTCTGG TGGGATGGAT TTACGGACTT GAAACCGACT TCTGCTGGCA 2820
   GGCTTTCACC TGGATGGGAT ATTTGGGTGG TGATGAGGTC TTTCCCGAGA CACTTTTGGT 2880
   TCAGTCATTT GAAATGACTT TAGAGTAGGG TGAGGTGGTG GGAGGCTGAT GGAGATATTG 2940
   TGGGGGCTTT AGTCCCTCCA TGGCAAAGCA GTTCAGGCAA ACAACTCCAT GGTTTTCCCT 3000
   CCAAATTCAA AAGGCCCCGG GTAACCTGGA ATCCTTCGTA GTCGGTTTTG AAGTGGGGCC 3060
   TTGGGCGCTG GGGGCATCAA CATGGCCATC TGGGCTTGCC TGCCCAGGCC ACACAGAGGC 3120
   CCCTTGTTGT GGGTGAATGG CAAAGGGAAG AGGGGACTGG TGTGGTTCAG AGGCCACAGG 3180
   CTGGGAAGAG GGATGGCGGG CGAGTCCAAG GAAACTGGCC GTGTCACCGT GCACCTGCCA 3240
   CTTCAGCCCC ACGGGTCTAT AAAATGGGCA TGATTATCGT GGCTACCTCA CTGGTCCTGG 3300
   CAATTAAGGA ACAATGTGTG CCAGGCACTC TGTAAACCAC ATACTTGCGA GTGTCAAGCT 3360
   GGTGACAGGT GGCGTTCCTG TTGAAGCACC TCCCTGAGCT CACAGCAACC CTTGCTGTCT 3420
   CTCCTCTTGC CCTCAGCTCC TGCCAGGGCC ATCCAGGTGA CCGTGTCCAA CCCCTACCAC 3480
   GTGGTGATCC TCTTCCAGCC TGTGACCCTG CCCTGTACCT ACCAGATGAC CTCGACCCCC 3540
   ACGCAACCCA TCGTCATCTG GAAGTACAAG TCTTTCTGCC GGGACCGCAT CGCCGATGCC 3600
   TTCTCCCCGG CCAGCGTCGA CAACCAGCTC AATGCCCAGC TGGCAGCCGG GAACCCAGGC 3660
   TACAACCCCT ACGTTGAGTG CCAGGACAGC GTGCGCACCG TCAGGGTCGT GGCCACCAAG 3720
   CAGGGCAACG CTGTGACCCT GGGAGATTAC TACCAGGGCC GGAGGATTAC CATCACCGGA 3780
   AGTATGTTGG GCAGGGCAGG GGGATGAGGC TGGGCTTGCC CGGGTGGTGG GACTGGCGTC 3840
   CTTGTGCGGG ACCTGGAGTC CCCATCTGAA AGCTCTTGAG TGCCAGTGTC TGAAAGGACC 3900
   ATTGAAGGGA GCAATTCTTT TTTTTTTTTT TTTTGAAGAT GGAGTCTTGC TCTGGACTCC 3960
   AGGCTGGAGT GCAGTGGTGC GATCTCAGCT CACTGCAACC TCCACCTCCC AGGTTCAAGC 4020
   AATTCTCTTG CCTCAGCCTC CCGAGTAGCT GGGACTCCAG GTGCGTGCCA CCACGCCCAG 4080
   TTAATTTTTG TATTTTTAGT AGAGATGGGG TTTCACCATG TTGGCCAGGC TGGTCTCAAA 4140
   CTCCTGACCT CAAATGATCT GCCCGCCTTG GCCTCGCAAA GTGCTGAGAG ACACCATACC 4200
   CAGCCTAAAG GGAGCGATTC TATTCTACTA TTCTTCCTTC TGCTAATCCT TCCATTCTTT 4260
   AATTTAATAA CGAAGATTTT TTGAGTACCT GTCATATACC AGGTGCTGTT CTGGGCCCTG 4320
   GGAATACAGC TGTTAACAAA ATCATCAAAC CACTTCCCTC GTGGAGCCCA CATTGCAGTG 4380
   AGAGAGACAA ACACGACACA CACTCTCAAG TCCTTGAAGA TAAAGAAAAC TGGGTAACGG 4440
   AGAGAAGAGG CCAGGGTTTG TTCTATAATC ATTAATAACA CGAGCAGTAA GAAGTAAAAT 4500
   TTATCTAAGT AACAACTTAT AAAGGGTCTA CTGTGTGCTA AGCTCTCATC CAGGTTCCCA 4560
   AGGATTAACT CAGACCACAC AGTAATTGAA TAGATTCTAT CATTGTCATC TTACAGAGGC 4620
   CCAGAGAGAG AAAGTGACTT GCCTAGTGTC ATAGCTGGTA ACGGGGCTGG GATTCTAACT 4680
   CAGCCACTTT GGGTCTAGTG GCCAAGCTCC TAATCCCTTT GCTTGCCTAG GGTGGTCCGC 4740
   AGAGGACTCA CAGAGGAGAT GGCAGGAGTG AACTGCAGGG GCAAGAGAGC TTAATGGAGA 4800
   AAGCCTGTGA CATGCCAGGA ACTGCACACA TATTCTCCCA TTGAGTCCTC TCCTCTACCC 4860
   TCCTGACAGC TGAGGCACAG AGAGGTTACC TTGTTCAAAT GGGTGCATAG GAAGTCAAAG 4920
   TCTGGAGCTG GGGTTTGAAC CCAGGCAGCC CTGAGAACCT TGTTCTTTTT TTTTGAGACG 4980
   GAGTCTCGCT CTGTCGCCCA GGCTGGAGTG CAGTGGCGGG ATCTCGGCTC ACTGCAACTC 5040
   CGCCTCCCGG GTTCACGCCA TTCTCCTGCC TCAGCCTCCC AAGTAGCTGG GACTACAGGC 5100
   GCCCGCCACT ACGCCCGGCT AATTTTTTGT ATTTTTAGTA GAGACGGGGT TTCACCGTTT 5160
   TAGCCGGGAT GGTCTCGATC TCCTGACCTC GTGATCCGCC CGCCTCGGCC TCCCAAAGTG 5220
   CTGGGATTAC AGGCGTGAGC CACCGCGCCC GGCCCCTTGT TCTTAACTGT AATGCTGCCT 5280
   CCTGATAGGA TGTGCCTGTT GGGACTAAGT AAGGGGCAGT CATTCATTCA TTCATTTGGT 5340
   ATTTATCAAG CATCGACTAT GTGTCGTTGG TGCTGGGGAT AGAGGTGATT GGGATGGCTG 5400
   AAGTTTCTGT CGTCAAGGAG ATGACATTCT GGTGGAGTGA GACTGGCAGT AAATAAGCAG 5460
   ATAAAGAAAG AGTATGAGAA TTTCAAAGTC TGGGCACGGT GGCTCACGTC TGTAATCTCA 5520
   GCACTTTGGG AGGCCAAGGT GGGTGGATCA CCTGAGGTCA GGAGTTCCAG ACCAGCCTGG 5580
   CCAACATGGT GAAACCCCGT CTCTACTAAA AATACAAAGA TTAGCCAGGC ATGGTGGCAC 5640
   ATGCCTGTAA TCCCAGCTAC TCAGGAGGCT GAGGCATGAG AATCGCTTGA ACCCAGGAGG 5700
   CAGAGGTTGC AGTGAGCTGA GATCGCACCA CTGTACTGCA GTCTGGGCGA CAGAGTGAGA 5760
   CTCTGTCTCA AAAAAAAAAA AAAAAAAAAA GACTCCGTCA AGGTATAAGA ATGTCAGAGA 5820
   GTACTAAGTG TTGCAAAGAA AATAACACCA GGCTGGGTGC ATTGGCTCAT GCCTGTAAAT 5880
   TTCAGCACTT TGGGAGGCCA AGGCAGGAGG ATCACTTGAG CCTAGGAGTT TGAGACCAGC 5940
   CTGGACAACA AAATGAGACC CCATGTCTAC AAAAATTTTA AAAATTTAAA AATTAGCTGG 6000
   GCATGGTGGC ATGTGCCTGT GGTCCCGGCT GCTCAGGAGG CTGAGGTGGG AGGATTGCTT 6060
   GGGCTTGAGA GGTCAAGGCT TCAGTGAGTC ATGATCGTGC CACTGCATTC CAGCCTGGGT 6120
   GACAGAGTGA GACCCTGTCT TGAAATGAAA AGAAAATAGG CTGGGCGCAG TGGCTCACAC 6180
   CTGTAATCCC AGCACTTTGG GAGGCCGAGG TGGGTGGATC ACCTGAGGTC AGGAGATCGA 6240
   GACCAGCCTG GCCAACATGG TGAAATCCCA TCTCTACTAA AAATACAAAA TTTAGCCGGG 6300
   CGTGGTGGTG GGCGCCTGTA ATCCCAGCTA CTCGGGAGGC TGAGGCAGGA GAATCGCTTG 6360
   AACCTGGGAG GCGAAGGTTG CGGTGCGCCA AGATTGCGCC ACTGCACTCT AGCCTGGGAA 6420
   ACAGTGAGAC TCCGTCTTAA AAAAAAAAGA AAAAAGAAAA TAGCACTGGG TGATGTGCTA 6480
   CATGGAATGA CTTGGGCTGT GAATATGATT TGAGGAGGGC CTGGGCCTGG GCCTTACAGA 6540
   ACCTAGAAGG CAGAGAGGAA GGGGAGGGGC AGGGTGCCAG GGATGAAGGC TCACGTACCT 6600
   CATGTCTTAG TGTGTGTTCA CTGTCTTAAA CAAGAATTTA AAGTTGGGCA TGGGGCAGAG 6660
   CGGGGAAGGG AGCATCCCTT TGCAGACCCC AAGAAGCCAG GAACTGGAGC ACATTCTGCT 6720
   AGAGGATCGA TGGGAAGCAG GGTTCCAGGG GCTGAGCCTA TGTCAGTCCT GTTTCAGAGG 6780
   AGGCACCAGG CTTGCTTGCC CTGAATTTCT GTGGGCAGCT CAGCCATGAG CATCCTACTG 6840
   TTATTGAGGT CACAGGGCTG CTTAGGCCCC CTCCTCTCTA ACCCAGGGAT TGTGCCTGCC 6900
   TGGACCAGGC GTGACTGCTA AGCTTCTGCC AGGACAAGCC AAATACTGAG GGTGCTTCCT 6960
   CTGCTGGACG CAAAAGTCCA GGATGACCCC CCAGGCTCTG TCTCGGGGAA GGGGCCCTGC 7020
   ATGCTCCAGG GGCCTCACAG GCCTGGGTCT TTCAAACCAC CCCCACCTGG GCCTGTGTTT 7080
   GATCAAGGCC CTGAGTGTAA ACATCCATTG TGTGTGTCCT TTCAGGAAAT CCCATAGCCA 7140
   TAGGAGCTTC CTCTGTTTCA GCTTTGAGGA TGGGGAAAAG TGGACTCCCC GTGGTGTTCC 7200
   TAGGGTCACC CACTGTGCTG GGGTTTTTCT GTTGTTGTTG TTTTTTTTCT GTTGCCCAGG 7260
   CTGGAGTGCA GTGGTGCAAT CTCAGCTCAC TGCAACCTCT GCCTCGCAAG TTCAAGTGAT 7320
   TCTCCGCCTC AGCCTCCTGA GTAGCTGGGA TTACAGGTGC ACACCACCAC ACCTGGCTAA 7380
   TTTTTGTATC TTTTTGGTAG AGATGGGATT TCGCCATGTT GGCCAGGCTG GTCTCAAACT 7440
   CCTGACCTCA GGTGATCTGC CTGCCTTGGC CTCCCAAAGT TCTGGGATTA CAGATGTGAG 7500
   CCACCATGCC CGGCCTATCC TGGTTTCAAA AGTGAAAATA GTCCTGGATA AGGTAGAAGG 7560
   CTGTCCACTC CAGGCATCCC TCCGGTCCGG TGGCTCATTC CCTGCTTTGT CCTTCCATGC 7620
   TTTGGGTGAT GGACCAGCAC CTGGACAGGA GGCCCTGTTC CACCTCCTCG GGCTCCTTGG 7680
   GGTCCAAGTG CCCCCACCTC CAGCTGCACT GCAGCAGAGA GCCCATGGGA CCTCTGAAAT 7740
   CATGAAGGTC ACCTTTGCGG TGTATAAAGA AGGAACCAGA GGTTGGAGAT GTGGAGGAGG 7800
   CCTGGCTGCT GTTCCCACTG GAGACCTGGC ATCTTCTCCC CGACCTAAAA CAATGAAAGC 7860
   AGTGCTCAGC CCGGATGAGA TCACGGCCAG CCCAAGACCA GGAACAGGGT ACGCCCTGCA 7920
   GGAAGAAGGT GTGCCCAGAC CTTAGGATGG ATCAAAAGAA GCCGGAAAAC TATATTTTTT 7980
   GTGAGTTTTG AAAATGTCAG ACAGGTCAAA CAAAACACAG TGAGGTCCAG CCTCGGCCTA 8040
   CAAGATGCCA GATTTCAACC CCTGGCCTAT ATGATCTGTT TGCCATGGCA GGCGGTTCCT 8100
   GTCCACCTCT TTTGTTTATA GCAGGGACCA GCTCTTGAGC TCCAGTGTTG AAGAGGCACG 8160
   GTCAGGGTCT GATCTGAAGA CACTGGTGGC TCATGCCTGT AATCCCAGCA CTTCAGGAGG 8220
   CCGAGGCAGG AGGATTGCTT GAGGACAGGA GCTGGGAGAC CAGCCTGGGC AACACAGTGA 8280
   GACCCAGAGA CTACAAAAAA ATAAATTTAG CGGGGCATGA TGGCACACCC TGCTACTCTG 8340
   GAGATGGGAA GATTGCTTGA GCCTAGGAGT TCGAAGCTGC AGTGACCCAT GATCGCACCA 8400
   CTGCACTCCA GCCTGGGCGA CCAAGCTAGG CCCTCTCAAA AAAGATACAG GTGGAAAAAT 8460
   GATGGACGAA GAGGGCATTG TGGCAAACCT GGGGATTTAG GAGAACCTAG TTTGGAATTC 8520
   TATGAGGATT CAATGAAAGA ATGTGTGTAG AGGGGCCCAG CACATAGTAA GAGCTCAATA 8580
   AACGGTGGGG GCTAGGGGCG GTGGCTCATG CCTGTAATCC CAGCACTTTG GGAGGCTGAG 8640
   GCAGGTGGAT CACTTGAGCC CTGGAGTTCA AGATCAACCT GGACAACAAA GCAAGATCCC 8700
   ATCTCAAAAT TAAAAAACAA CACCAACAAC AAAAAAACAG TGGCTTAGAT GCCTGATCAT 8760
   TAGGGTAAGT CGTGTCCTCA ACCCCTTCAC ATCTGCTCTG AAGGTCACCA TATCCGGAAG 8820
   CCTTCCCTGG CCTCCTTGTT TAAAATGGCA CAGCCCCCAC TCCACGCCTG GCACTCTCTG 8880
   CTGTCCCTGA TTCGTTTTCT CCATACAGCT TATCTTTGTC TGATATGTGA CATAGTTAAC 8940
   ATTTTATATT TGTCTTTCTT TCCTAGTTAG AATCTGAACT CTAGAAGGGC AAGGGCAAGG 9000
   ATTTATAACT CAAAGGTTCC GGGCTTAGGC CTCTTTTATA TTCTTGATTT TGAGGTTAAT 9060
   TAAGAGCTCA GGCCTAGCGA GGTGGCTCAT GCCTGGAATC CCAGCACTTT GGGAGGCCCA 9120
   GGCGGGCAGA TCACTTGAGG TCAGGAGTTC CAGACCTGCC TGGCCAACAC AGTGAAAAAC 9180
   CTGTCTCTAC TAAAAATACA AAAATTAGCC AGTTATGTTG GCAGGCGCCT ATAATCCCAG 9240
   CTACTCAAGA GGCTGAGGCA GGAGAATCGC TTGAACCCAG GAGGCAGAGG CTGCAGTGAG 9300
   CCAAGATCGT GCCACTGCAC TCCAGCCTGG GCAACAGAGC GAGACTCCAT CTCAAAAAAA 9360
   AAAAAAAAAT TAAGAGCTCA AAGAGTTTGT TTTCATAGGC AGCAGAATGA GAAAAGTTTA 9420
   CAAAATAGTT TAAATGACAA TAAAGTCATT ATAGATTAAC ATAAATAAAA TACCTTTTAT 9480
   GAAAAAAATA ATCATTTTCT GAAATCAGAC AAAACATTGT GAATGAGAAG GTGGCATGGT 9540
   TTTATTTTTT TGCAAGTCTC CGAAGCCTGG CTGGATAGAA GAGCCTGGCT TCTCAGAGCT 9600
   GCTTCAGTCT GTTGTGATAT CTATTGTATG TCACGTAGCC TCTGGAAAAC TCCACAGTTA 9660
   GTATTGTTGG GAAAATAACT TTGACCTCAG GATCTCCTGA AAACGTCTTG GGGAACCCCA 9720
   GGGTCTAGAG GCTGCAGTTT GAGAACTGTT GCTGTGGTAT CCCAGGTGTC TCAAATACTG 9780
   CCTAGAACAT AGGTGGTACT CAGTAATTAT TGTTGAAGGA TGAATGAATG AATGAATGAA 9840
   TGAATGAATG AAAGAAAGAA AGAAATGTGT CTTTGAATCC AGCCATGTGC CCAGAATGAT 9900
   GAGACAGATG ACAAAAGCTA AGGGACTTTA GCATGAGGAG AGGGGGTTCG TTTCCTTTTT 9960
   TTTCTTTTTT TTTTGAGATG GAGTCTCACT CTACTGCCCA GGCTAGAGTG CAGTGGTGCA 10020
   ATCTCAGCTC ACTGCAATCT CTGCCTCCTG AGTTCAAGCA ATTCTCCTGC CTCAGCCTCC 10080
   AGGGTAGCTG GGACTACAGG TGCGTGCCAC CATGCCTAGC TAATTTTTTA CATTTTTGGT 10140
   AGAGATGGGG TTTTACCATG TTGGCCGGGC TGGTCTGGAA CTCCTGACCT CAAGTGATCC 10200
   ACCTGCCTCA GCCTCCCAAA GTGTTAGGAT TACAGGTGTG AGCCACCATG TCCGGCCAAG 10260
   AGGGTGTTCA TTTCTGCTCC TTGCCAGGTA TTGTGTCAGG CACTGGGGAC CCAGCAGTGG 10320
   CTGAGACAGA CAGGGCTCTG CCTCACGGAG CCCACATTTT CACCAGGCAA AGGATGGTCG 10380
   GCCCCTAAGC TGGGAGATAA GACTTCAGCA GTTGGGTGGG GGAGCCGTGG GAGAAGCCCA 10440
   GCCCACAGGG GGACAGTGCA AATCTAGAAC CAAGGCGATG GCAGGGGTGA GGCTGGCACG 10500
   GTAGCTAGAG ACCACGTCGT GCCAAGGGCC TTGGGGACCA TGGGACTATG GGACCTTAGG 10560
   GAAGGCGTCT GGAATGCTGT AGCCAGACAC TGTTGCAAGG AGGATTTTTC TGTAGACATG 10620
   AGGCCTTCCT TATGAAGAAA GCAAGGGTTC TTTCATTCCT GGGGGTGCCA GGTGCTGTGG 10680
   ACTGCAGCAC GCGTGGTTGC TGCCGTCACA GAGCTGTCAT GCAGGAGGGC AGCGCGTCCT 10740
   TGGGAAGGTG GCAGGCAGGT CAGGCTAGGA GGAAAGAGGC CGGGAAGCTG AGGGCATTTC 10800
   CTGCCCGAGA TGCCCAATGT AGCCTACTTC TGTCCCCAGT GGCTTAAGGC AGAGTTGCCT 10860
   GGTAGGTGCC CTGGTCCCAC CCTGGTGAAA GGCTGAAGGT ATTTAATTAG TGCCTGAGAA 10920
   GCAGAGAGGA AACAGGATGT GCCAAAACAC TTTGATGGAT GGTAGAGTTA ACAGGCTCCT 10980
   TGCCTGCAGC TGCTTCAGAC AAGAGCGTCC CCAAGCCCTG GGCCTGACCT GGAATGTGGG 11040
   GATGGAAGGG GAGGGGGAGG AACCAAGGCA CTGGGAGGGT AAGTCTCTCT CTCCCACATA 11100
   GACACACCCA CTCCTTATGG GTGCCTGGGC ATCTCCTGGT ACCTAGAATC TGGCCTGTTT 11160
   ATCTCCACAC CCATCCCTGG GGTCTACACT AGGCCCTGTG GGTGGCAGTT CACATCAGGG 11220
   GAGTTCTGAC TTTGGCTCTG AGAGGTGGTT CAGAGATGGC TGTAAGTTGA GAAGCACAGA 11280
   CTGCTGGGTG TGGTGGTTCA CGCCTGTAAT CCCAGCACTT TGGGAGGCTG AGGTGGGGGT 11340
   GGATCACCTG AGGTCTGGAG TTCAAAACCA ACTTGGTCAA CATGGCGAAA CTCCATCTCT 11400
   ACTAAAAATG CAAAAATTAG CCAGGTGTGG TGGCAGGTGC CTATAATCCC AGCTACATGG 11460
   GAGGCTGAGG CAGGAGAATT GCTTGAATCT GGGAGGCGAA GATTGTAGTG AGCCGAGATT 11520
   AGTTCGCACC ATTGCATGCC AGCCTGGGCA ACAAGAGTGA AACTCCGATT CAAACAAAAA 11580
   AAAAAAAAAG CTGGGCATGG TGGAGTGCCT GTAGTCCTAA CTACTCAGGT GGGAGGATTG 11640
   CTTGAGTCCA GGAGGTTGAA GTTGCAGTGG GCTATAATTA CACCACTGCA CTCCAGCCAG 11700
   GGCCACAGAG TGAGACCCTG TCTCTAAAGA AAGAAAAAAA AAAACAACCT CAGGCTCCGA 11760
   GGGCACCATT ACTGCTCTAT ACTGAAGAGC TGTGCAGCTT TTCCAGACCC GAAATGTCAT 11820
   CCACAAAACA GAAGTGATAA TGGTCCTGCC TCACAGACTT CTTGCAGTAG TCCAGGTGTT 11880
   TAGAACGGGG TGTAAAAGGC CGTGTGCCCT TGGTAGGAAT CTTTGCATAT GCATTTGATC 11940
   ATCTGCAGCC TGCCCAGCCC ACTGCTTGCC CCCTCCTGGG TGTGCTGGGA AGGGGTCTTT 12000
   GGCCCTCCAG GGGTTAGGTG CCCCAGCCTC CAAGGTGCCC TCACGCCTTT TCATCCCGAC 12060
   TCAGATGCTG ACCTGACCTT TGACCAGACG GCGTGGGGGG ACAGTGGTGT GTATTACTGC 12120
   TCCGTGGTCT CAGCCCAGGA CCTCCAGGGG AACAATGAGG CCTACGCAGA GCTCATCGTC 12180
   CTTGGTGAGT GGGCCTGGGA AGGGGGAGGC ATGGCCCTTC CTTTTGTCCG CTTCTGTTCT 12240
   GTCTGCCCTC CCCTGTGTCC GCCCTCTGCC CTCCAGCTTA CCCTCTGGGC TCTGTCGCCT 12300
   GCTCTGCTCT CCCCCAGGCT CTGCCAGTCA CTTAGGCTCC CCTGTGCCCT GCACCCCAGG 12360
   CAGGGACCAC TGGCCCACAG TGCCTCCAAT CACCCAAGCC AAACTAAGAG AAGAGTGGAG 12420
   ACAATTGGAG ACTCTGCCTT TTCAAAGTCT CATTTTTAAA AAAAATCCAG ACTTGGGGTC 12480
   CGGGTGCGGT AGTTCATGCC TGTAATCCCA GCACTTTGGG AGGCCGAGGC GGGTGGATCA 12540
   CTTGAGGCCA GGAGTTCGAG ACTAGCCTGG CCAACGTGGC AAAATCCCGT CTCTATAAAA 12600
   AATATAAAAG CCAGGCGTGG TGGTGCACAT GCCTGTAATC CCAGTTACTC AGAAGGCTGA 12660
   GGCATGAGGA TTGCTTGAAC CTGGGAGGCA GAGGATGCAG TAAGCCAAGA TCAAGCCACT 12720
   GCACTCCAGC CTGGGCGACA GAGTGAGACT CTGTCCAAAA AAAAAAAAAA TCCAGACGTG 12780
   GTCAGAGTCC ATGGGCAGTG AATGAGGACA GTTGATGGTG TGCAAAATCG ACCCACCTCT 12840
   TGCTACATCC CCAAGGCCTC ATCTCACCCG AGTCCCTCGC CAAAGCACAG CGGTTTTGCC 12900
   GTGTGCCCTG CTGGGATGGC GCTGCATGGC ACACACACTG TGTAAGTTTG AGTGCAGCTG 12960
   AAACGAAGCC GATTCCAGAC ACCCAGGGGC AGGGCGGGGT GTCCGTGTGG CTGGGAGGCC 13020
   TCCTTGTGTT AGGGGGATGT TGCCATCGGC CAGGTGCCCT GCTGTAAGCC AACACATGGA 13080
   GTCTTGTATG ACATGTGCTC TGCATGAGTG ATGCCGCTGG GCTGTACACT GCCATCTTCA 13140
   CATGTGTGAA TGAGCACGTG ACTGGGGGGT ACTTGGGCTG CAAGACAGAG TTCATGTGTG 13200
   GGGGATGGAA CACGTGCACC AGTGACCCAG GAACCTCTGC CTGTTCTTCG GTAAAATGCA 13260
   CCATTTGCAT CAGCAGTTCC CAAAATTAGT CTCCAGGTCT ATTTACACTC TAAAACATTA 13320
   TCGAGGGTCT CCAAGAGCTT TTGTTTGTTT CTGTGGGTTT TATGTCTATC TGTTGCTTAA 13380
   CATATTAGGA ATTAAAATGG GGAGATTTTC CTTTTTTTTT TTTTTTTTTG AGATGGAGTC 13440
   TCGTTCTGTC GCCCAGGCTG GAGTGCAGTG GCTCGATCTC GGCTCACTGC AAGCTTCACC 13500
   TCCTGGGTTC ACGCCATTCT CCTGCCTCAG CCTCCCAGGT AGCTGGGACT ACAGGCACCC 13560
   GCCACCACAC CCGGCTAATT TTTTTTGTAT TTTTAGTAGA GACTGGGTTT CACCATGTTA 13620
   GCCAGGATGG TCTCGATCTC CTGACCTCGT GATCCACCCA CCTGGGCCTC CCAAAGTGCT 13680
   GGGATTACAG GCATGAGCCA CTGCCCGGCC TTAAAATGGG GAGATTTTTC AAGCCCAAGA 13740
   TACACAAGGA AGACTGGGCA ACATGGCAAG ACCCTGACTC TACAAAAAAT TTTAAAATTA 13800
   ACCAGGCATG GTGGCATGCA CCTGTGAGCC CAGCTTCTTG GGAGGCTGAG GCAGGAGTAT 13860
   CGCTTGCACC CAGGAGGTCA AGGCTGCAGT GAGCCGTGAC TATGCTACTG CACTCTAGCA 13920
   TGAGTGACAG AGACCCTGGC TCAAGAAACA CAAACACACA CACACACACA CACACGCATA 13980
   TAGTCCATTA GGCATCAGGG CGATGATGGC ATCAGGGAGC CTGGGAAACT CTACTGGACA 14040
   TTCATGGGAG AACAAGTGAA AAAGGCAAAT AACATCTTAG TGTTATTCTA AAATTTCTTC 14100
   TTTTGGCCTT GTGGACAGGA CCACGCTTTG AGAGCTGTGA CTGACATGCC TCTGTCCTGT 14160
   TGCGAGGGCC TATAGTGCCA AGTGCATGAG CTCTGGGGAG GGCTTCGTGG GTGCAGAGCT 14220
   GGGCCTGTGG AGGCCCCTCA GACACAACAC TGGTGGGGCT CAGAGCTCCA GGGGCACTCG 14280
   AGGGAAGACA AGAACCGGCT CTGAGATGCG TGAATGTGAC AGTGCATGAG TAGAGATGGA 14340
   GACCTTGTGG GTCCCAGAAC CAGGACTGCA TATGACTTTC ATATGTGGGT ATTTTTGCCT 14400
   TCATGGGTCC CTTCCTGTTT TAAAAAAAAT GTGTGATTAT GTTGTCACAA AGAGTTTATT 14460
   CCTGTATATT GTGTTAATTT GTGTTCAGAT TTGTAAAGTA AAATTAAACC ATTTCAGCCA 14520
   GGTGTGGTGA CACATGCCTG TAGCCCTAGC TACTTACCCC AGAGGCTGAG GTGGGAGGAT 14580
   CGCCTGAGCC CACGAGGTTG AAGCTGCAGT GAGCCATGAT CACACCCCTG CACTCCAGAC 14640
   TGGGCGACAG AGCTGAGATC CTATTTCGTG GGCCCTAGGT CCCTGTGCCT GCTGGAACAG 14700
   GACATCCCTA TCACCGTGGT TGGAGCCCTT TGGGGTGCTA AGACCTATGA ATGAGGGAAA 14760
   CTTAGGGTGC CCAAGCTGAG GTAGAGCCCT CAGAACCCCC TGGGATTTGT ATTGGAGCCC 14820
   TCGTGGCATA ACACAGGTGG ATTATGCAAT GGGAGTTTCT TACCTATAAG CACCCACATG 14880
   TGGGCGGGTG GAGGGTAGGA GCCATGCACT AGGGCTTCAG CCCCCAGCCC CTTCCCGCTT 14940
   CAGGGCACAC CTTGCACTTG GCCAGCCTGG AGCTGGGCTT TCGGGGGTGG CACAGCCTGG 15000
   GCTGGCTCTG GCCAGCATAA TCTGTTTCTC TTTTGTCCCT CCAGGGAGGA CCTCAGGGGT 15060
   GGCTGAGCTC TTACCTGGTT TTCAGGCGGG GCCCATAGAA GGTACGGGGG GTGGATCCTG 15120
   AGTTGGGCTT CTCGGGAGCT CCCATACATC ACCTACTGCT TCTGACTCTA GTTAGTATCC 15180
   CCTTCCCCAC TAAACCCTGC TCACTGTGGA CCCCTCACTA ACCTGGCCTG ACTGTGGCTC 15240
   TGAGGCATCT AGTGGTCTGG CGCTGGGCCT AGGCTAGGCT GGGCTGAGGA GAGCCTGGGG 15300
   TGCAGGCCAG GGCTCTGTGA CTGGCACCTG CGGTGCTCTT GAGGGTGTGG CGTCTGGGCA 15360
   GCTGGCTCTC TCTTTGGTCT GGGGGCTGCA GTCTGTCTCC CTCTGTGCAG GCTGCCTCGT 15420
   TTTCTGCCTT GTGTTTTTTG CACCTGGGGG AGGGCCGTAA CTGGGGAATG GCCGGGATGG 15480
   TAGAATGGGG AGTGTGCTGT GCCCAGCCTC TGGCACAAAA AATCCAGCCA GGGCTGCAGG 15540
   TTCCTTGGTG AGCTTTGCAA ATCGTCCCCG ACCTCAGTGC TGGCTCCGCA CCATGTACCC 15600
   CTGCTGTGCC GTTAGCCCTG TTCCCTCCCA GGCCTCCGGG CTCAGGGCCT GTTGTCTTTC 15660
   TGCAGACTGG CTCTTCGTGG TTGTGGTATG CCTGGCTGCC TTCCTCATCT TCCTCCTCCT 15720
   GGGCATCTGC TGGTGCCAGT GCTGCCCGCA CACTTGCTGC TGCTACGTCA GGTGCCCCTG 15780
   CTGCCCAGAC AAGTGCTGCT GCCCCGAGGC CCGTAAGTGT CCCGCTCATG GCCACCCTGG 15840
   TTTGGGCAAC ATCCTGCATC CAAGGGAAGG AGGTGGCCAT CCACCTGCCC CCAGGACAGT 15900
   GGCGTTGGTC TGGAGGGTGT GAATTTAGCC AGTGGGGAGA AAGTAGGCTG AGGAGGGTCT 15960
   GCTGTTTAGA TTGTCGTTTA CTTCCTCCAA CTTTTAGTTT ATTTTTATTT ATGTTGTTCT 16020
   TTTCTTTTGT AAGTATAATC CATACACATG GTAAAAATGT CCAACAGTAC AAGATACTAG 16080
   TCACATGGAA GTAAAGCCCT CTAAAAAAAC CAAATCTTGG CTAGGCGCAG TGATTACGCC 16140
   TGTAATCCCA GCACTTTGGG AGGCCAAGAC GAGTGGATCA CTTGAGGTCA GGAGTTCCAG 16200
   ATCAGCCTGG CCAACATGGT AAAACCCAGT TCTCTACTAA AAATACAAAA ATTAGCTGGG 16260
   CATGGTGGTG ATCGCCTGTA ATCCCAGCTA CTCAGGAGAC TGAGGCATGA GAATCGCTTA 16320
   AACCCAAGAA GTGGAGGTTG CAGTGAGCTG AGATCACGCC ACTGCACTCC AGCCTGGGCG 16380
   ACAGAGTGAG ACTCTGTCTC AAAAAAAAAA GAAAAAAAAA TGTTAAGTGA AAAAGTTAAG 16440
   AAACCAAACA AGGTTTACAA CACTACATGA TTTAAGCAAA AAAAATTTTT TTTGTTTTAG 16500
   AGAAAGGGTC TCATTCTGTC ATCCAGGCAG TGCAGTGCGA TCATAGCTCT CTGCAGCCTC 16560
   AAACTCCCGG GTTCAAGCAG TCCTCCCGCC TCAGCCTCTG GAGCAGCTGG GACTGTAGGC 16620
   ACACACCACC ATGCCCAGCT AATTTTTTGA TTTTTGTTTT TTGTAGAGAC GGGGTCTCAG 16680
   TATGTTGCCC AGCCTGATCT CAAACTCCTG GCCTCAGGTG ATCCTCCCAA GTCAGCCTCC 16740
   CCAAAGTGCT GGGATTACAG GCATGTGCCA CCATGCTGGC CAATTTTTAA AAATTTTCTG 16800
   TAGAGACAGG GTCTTGCTAT GTTGCCCAGG CTGGTCTTGA ACTCTTGACC TCAAGTGATC 16860
   CTGCCTCAGG CTCCCAAAGT GATGGGATTA CAGGCATGAA CTACCACACC TGGCCTTAAA 16920
   CTTAAGCAAA TTTTTTTTTT TTTTTGGAGA CAGTTTCACT CTGTCGCCCA GGCTGGAGTA 16980
   AAGTGGCGTG ATCTCTGCTC ACTGCAACCT CCGCCCCCCG GGTTTAAGCT ATTCTCCTGC 17040
   CTCAGCCTCC CGAGTAGCTG GGATATAGGC GCCTGCCACC ACGCCTGACT AATTTTTGTA 17100
   TTTTTAGTAG AGACGGGGTT TTGCCATGTT GGCCAGGCTG GTCTCGAACT CCTGACCTCA 17160
   GGCAGTCCGC TCCCCCGCAC CCCTACCTTG GCCTCCCAAA GTGTTAGGAC TACAGGTGTG 17220
   AGCCACCATG CCTGGCCAAA TTTAAGCAAA TGTTTGAAAA CACATACCCA CAGGAATGCT 17280
   GCACATTTTA CCCAGCTACT ATGTCTAGGG TCGTATCTAG CACACCAGCA TGGCTACTGT 17340
   GGAGAGCTGG GACTGGATGT GAGATGAGAG CTAAAGGGGA AGTAAGCAAA CCAAGCAGGG 17400
   GAAGGTAAGA GAAGACAGAA GACAGAGAGA GAGGGACCTA ACTCTATGAG AGGAGTCAGA 17460
   CATGTGCAAT TGAAAAAGAC TTGCTCCTGT CTCTCTTCTG TGAATGTTTG TGAATATCCC 17520
   AACGGGACAC TTTCACAGAG GAGCTGATTG ACGTGGTCAC AGCCATCAGC CTTGGGACAC 17580
   CAGACCACAG TGTGTACACT AAGTGGCACT GATGGACACT TCAGCATCCC TCTAGCTGCT 17640
   GTCCCGTTTC CCCTCCTCGG GGACCACAGC TGTTGCCAGT CCTTGGTTTC CTTCAGGAGG 17700
   GTGTCTGGGT AGACCAGCCT GTGTGCACAC AGTCCAAGAT ACATGAACAG TGAAGTGCCA 17760
   GGCAATCCTT GCAAGCATGG GCAGGTGGAG AGCTGAGGCC TGCTTGACAC CTTCCTGCTC 17820
   AGAAGCCCAG TGAGCAGTTT CCCTCCCTAG GGCTCAGTGT CATCCCCTAT AAAATGGGGC 17880
   TTATGGCAGA GCTCACCACA CTGGGTGCAT CTGGGGATTT GGCGAGCTCA TGTGCACACC 17940
   ATTGAGCATG GGGCCCAACC TATATAAAAT ATTCTACGTC TGTCAGCTGC TGGGCACTGC 18000
   CACTATCAGC CTCAGTAGTG ACTGAGGGAC AGGGCACCAG TCAGAGCCCT GGTGCACACA 18060
   GAGTGACCCC AGAGAAGCAG CCTTCCCTCT CTGAGTCCTG TTTCCTTCTG TTAGGTCCTG 18120
   ACTTCATGGG TTGTTGTTAG CATTAAGGAA GTCGCTGGCT AATTTTATAG TCATTGAAGT 18180
   CAGTGGTGTG CAACCTGGTT CCTCAAAGGA TCACTTCCCT GAAAAAATTC CACTGCTCCC 18240
   TGGAGGCTTA TGCAGGCCAT CCCATCCCCT CCCTCTTGTT GTGTTCAGCT GACAGCTTTT 18300
   TGCTCAGTGA GTAAGTGTTA GGTCCATTTC ACAGATGGGC TGCAACCAAG TTTGCAGTGA 18360
   ACCCACTAAG ACCAGAGCTA GGGCCAGGAC TAAATGCTGG TCCCAATGCC ACATTCCCCT 18420
   GTCCCCACAC CACATTTCCT CCATCCGGAG ACCCTGTTAC CCCAACCCAG GGCCCCATTA 18480
   ACTCCCTGGC AGAGGCCCTG TTACATCTGC TGCTGCCACA GCCTCCGCCC ACCCTTCAGG 18540
   AGGCAGCAGG TCCCACTGCT GATGATAAAG TTGCAGGCTG CCTGAGCTAA TGAAGGGGCT 18600
   TCCTCTAGGC TGTGCACTTA GTCTTCTGCT TCCAAACCAA ATCAGAGGTG AGGCACCCTC 18660
   TCTGGGCCCA TCTCTCTCCT CCATTTTCCT GTTGGGGTCC CAGGGAGGAA GCCACTTGCC 18720
   TAGGGCCCAG GAATTTTGCA AGCCTCTTGC CCTAGGGAGG AAGGAAGGGA GGAGGATCTT 18780
   ACCTTGAACT GTCAAGCCTA GAGCCTGGTG GGGCAGGCAG AAATGGGTGC AGTCCATGAG 18840
   TTAGAAACAC TAGAGGAGAC ACTTTGCTGC TTGGCCGGGG CAGGCAAGTT AATTCCCGAG 18900
   GCTCCTGCCA CTGCATCTCA ATCTGGAAGG TGACCAGGTG GGCAGGACCC ACGTCTCCCA 18960
   GATGACTCAT TTTTTCTAGA ACAGGGGCTT GGCTGCCAAA GAGGATACTT GATTTCGGCT 19020
   TGTGGGGACA GTGGTGGACC CAGCATCTGG GCTTTATATA AAGGGCAGCT TTGTTGCCCT 19080
   GTAAACACAC AGACCATGGG TGGCCACTTC TTCCAGTAAG TTAGCTGGGG AGTTGGAAGT 19140
   TTAGGTAAAA CCTTTTGATT GACAAATGTT GGCGAATTAC CATGCTGTTA AATGAAACAT 19200
   TGTTCTGCCA CCCTGGGGCT GTGGGTGCCT GCGTGCACCC TCTGAAAAAT CACACAGGAA 19260
   GTGGGGTGGG GTCTCTGTGA AGCTGGTGTC CCCCAGCCTC AGGGATGCTG CAGAAATGGA 19320
   ATGAGGACCA ACAGGGACTC AGATGTCCAA GGAAGCTCTA CAGCGGAGAG GACGGCTTGG 19380
   GAAGGAGGTC CAGGCCCAGG TCCCTCCGGA ACCCAATGGG TATGGGGCAG CCTGGCTCCT 19440
   GCCTCATCCC CCTTCTCCTG TTGATTGTGT CCTCACAGTG TATGCCGCCG GCAAAGCAGC 19500
   CACCTCAGGT GTTCCCAGCA TTTATGCCCC CAGCACCTAT GCCCACCTGT CTCCCGCCAA 19560
   GACCCCACCC CCACCAGCTA TGATTCCCAT GGGCCCTGCC TACAACGGGT ACCCTGGAGG 19620
   ATACCCTGGA GACGTTGACA GGAGTAGCTC AGGTGAGGCC GGGGGAAGCA GGAACAGCTG 19680
   GTGGGAGTGT GCTGGGCATC TGGACACTGA GGGGCAGGGG CTGGAAGGAA GAGTGTCTTG 19740
   GGAGCCGAGG AGGGGCTCTG CTCCTGGTGC GCGGCCACTG ACAGCCACTC TCCCCCAGCT 19800
   GGTGGCCAAG GCTCCTATGT ACCCCTGCTT CGGGACACGG ACAGCAGTGT GGCCTCTGGT 19860
   GAGAATCCAT CGTCCCGAAG TTGGATGTGC CTGTAAGGGA GAGGGGTGGG CCAGGATCCA 19920
   TCCTCCCAAA CCGACCACCA CCCCCCTGTC CCTAGAAGTC CGCAGTGGCT ACAGGATTCA 19980
   GGCCAGCCAG CAGGACGACT CCATGCGGGT CCTGTACTAC ATGGAGAAGG AGCTGGCCAA 20040
   CTTCGACCCT TCTCGACCTG GCCCCCCCAG TGGCCGTGTG GAGCGGGGTA AGCAGGAGCC 20100
   TTGGGGTCTG AGGGCTTTTA AGGTGGGGGG GTGAAACATG TCTCCCTGAT ACCTGCCGCA 20160
   GGGACTCTTG GTGCAAACCC TGGACCCCGG GCTCCTCCAG CAGTCAGTGA CACCCCCCTT 20220
   CCCTGCAGCC ATGAGTGAAG TCACCTCCCT CCACGAGGAC GACTGGCGAT CTCGGCCTTC 20280
   CCGGGGCCCT GCCCTCACCC CGATCCGGGA TGAGGAGTGG GGTGGCCACT CCCCCCGGAG 20340
   TCCCAGGGGA TGGGACCAGG AGCCCGCCAG GGAGCAGGCA GGCGGGGGCT GGCGGGCCAG 20400
   GCGGCCCCGG GCCCGCTCCG TGGACGCCCT GGACGACCTC ACCCCGCCGA GCACCGCCGA 20460
   GTCAGGGAGC AGGTCTCCCA CGAGTAATGG TGGGAGAAGC CGGGCCTACA TGCCCCCGCG 20520
   GAGCCGCAGC CGGGACGACC TCTATGACCA AGACGACTCG AGGGACTTCC CACGCTCCCG 20580
   GGACCCCCAC TACGACGACT TCAGGTCTCG GGAGCGCCCT CCTGCCGACC CCAGGTCCCA 20640
   CCACCACCGT ACCCGGGACC CTCGGGACAA CGGCTCCAGG TCCGGGGACC TCCCCTATGA 20700
   TGGGCGGCTA CTGGAGGAGG CTGTGAGGAA GAAGGGGTCG GAGGAGAGGA GGAGACCCCA 20760
   CAAGGAGGAG GAGGAAGAGG CCTACTACCC GCCCGCGCCG CCCCCGTACT CGGAGACCGA 20820
   CTCGCAGGCG TCCCGAGAGC GCAGGCTCAA GAAGGTGAGG GCCGCCCTCC CTGGCGTCCA 20880
   GACCGTCCCT GGGCCCCCAG CCGGTCCCCG CGGCTCATAC CCTTCTTTCT TTCTCCCTTG 20940
   CAGAACTTGG CCCTGAGTCG GGAAAGTTTA GTCGTCTGAT CTGACGTTTT CTACGTAGCT 21000
   TTTGTATTTT TTTTTTTAAT TTGAAGGAAC ACTGATGAAG CCCTGCCATA CCCCTCCCGA 21060
   GTCTAATAAA ACGTATAATC ACAAGCTCTG GAGAGAACCA TTTGTTCGGC CGCGCGGGGC 21120
   GGGGGACCGG GGCTGCTCCC GTATGCGTCT GTAAAGCGCC GCGTCCCGGG GGCACCGGAG 21180
   TCCGGGGCCG GGAGGAAGAG ACCCAGCCTG GCCCGGCCCG CGCCCGCGCC GCCGGCCGGA 21240
   GAACGTGCCC CGCGCAGCCG CCGCCCGCCT GCGTGCGCGC CCCGGCCCCG CCCAGGCGTG 21300
   CGCATGCGCC CCGGCCCTCC GCCTTCGCGC ACCGCAGGCT GGCCGTCCGG GACGCGCGCG 21360
   CGCTCCTCTC CCCTTCCAGC CCATCCCCCC CAGCCCCCCA CCGACCTACT TTACTGTCTC 21420
   CAAACTCGGG CAGCCCACCT GGCCCCCGAC GACCCCAGCC CCTGCACCGG GTACCCCGAC 21480
   GTTCCATCCA GACCCGCGTT TCACCAGGGC GGCGCGCGGC GACCTCGCGC CCCGCGGAGC 21540
   CCCGGGCTCG CGCGCGCCCG CCCGCCCCCG GAGACAGACC AGCGCGCGCG CCCCGGGCCG 21600
   CCTCCCCCCA GCGCGCGTCC GCCCCGGGGC TCGCGCCGCC GCCGCCGCCG CCGCCGCGCG 21660
   CGCGCAGCTC AAGTAAAGGA GGAAAAAAAA AAGGGGGAAA AATAGAAAGC GGCGGCGGCT 21720
   GCAGCAGCGA TCCGCCGCCG GACTGGGCCA AGCCGGGCGG CGGCCGCGCG AGCCGGCGAT 21780
   CCAGGGCACT GGCGGCGGCC AGCCAGGGCG GGCCGTGTTC AAAAAAAAAA GTCGCGGCGG 21840
   CGGCGGCTGC TCAGGGAAGG AGGCCTGAGG GCCGCGTGCA GCGGGCGGGC AGCTGGGTGG 21900
   GCTGGGGGCG GCCGCGCGGC GTCCCGGAGC CTCGGGCCGC CCGGAGCCGG CGGGCGGGCG 21960
   GAGGCGGAGG CGGCGGCGGC TGCAGCGGCT GCAGGAGCGG CGGCGGCTGC GGCGGCGGCG 22020
   GCGGCATCTC CTCCTCACAT GACCCCACTG TTTGTCCCCG TGATCAGCGC GAGCGGCTCC 22080
   CGTATCTCCT CCGTCCCCTC CTGCCGCGCG GCGTGAGCGC CGGGCTCGGG GCCCCCCCGG 22140
   CCGCCCGCCC CCTCCCCTCC CTCCCTCCCC TCCCCTCCCC TCCCCCCCGG GCCCCGCGCC 22200
   CCCCCCGCCC CCGCCCCCCC CATGGACATG CTGGACCCGG GTCTGGATCC CGCTGCCTCG 22260
   GCCACCGCTG CTGCCGCCGC CAGGTAAGAT CCCCGGCCCG GCCGTGCCCC CGCGCCCCGG 22320
   CCCCGGCCCC GGCCCCGCGG CCTGCAGGCC GGGGCCGCCA TGATCCCGAG CGGCCGCGGG 22380
   CCCGGCTCAA AATGGAGGCC GCCGGCGCGG GGGGGACCTG GCGCCTCCCG CCCCCGGCCC 22440
   CCGGCCTCGG CGGCGCCCCC GGCCTCAGGC GCGGCCGGGT GGGACTGGGG CCCTGCAGCT 22500
   GGGCGCGGGG GCGGGGGCGC GGGCGCGGGC CGCGCTGACC CTGCTCCCTC CTGTGCCCCT 22560
   GGCAGCCACG ACAAGGGACC CGAGGCGGAG GAGGGCGTCG AGCTGCAGGA AGGTGAGTGC 22620
   TTGCCGGGCC GGCCGCGCCC GGGGAGGGCT GGGGGCGCTC GGCGCGGCCC TGACCGTGCC 22680
   CCGACCCTCC TCGGCCCCAG GCGGGGACGG CCCAGGAGCG GAGGAGCAGA CAGCGGTGGC 22740
   CATCACCAGC GTCCAGCAGG CGGCGTTCGG CGACCACAAC ATCCAGTACC AGTTCCGCAC 22800
   AGAGACAAAT GGAGGACAGG TGAGCGGCGG GCCGCGAGGG CGAACGGGCG GGCGGGCGGG 22860
   CGCGCCGGGA AGGCTCGGAC CTGGCCCCAG CGCCGGCCTC GCCGCTCTGC CGCCCCCTGC 22920
   AGGTGACATA CCGCGTAGTC CAGGTGACTG ATGGTCAGCT GGACGGCCAG GGCGAC 22976
(2) INFORMATION FOR SEQ ID NO: 20:
   (i) SEQUENCE CHARACTERISTICS:
      (A) LENGTH: 30 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 20:
   GTTACAGAAT TCGCCGCGAT GGCGCCGGCG 30
(2) INFORMATION FOR SEQ ID NO: 21:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 21:
   GCCAGGACAG TGTACGCACT 20
(2) INFORMATION FOR SEQ ID NO: 22:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 22:
   ACCTCAGGTG TCCCGAGCAT 20
(2) INFORMATION FOR SEQ ID NO: 23:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 23:
   GAAGATGACT GGCGATCGAG 20
(2) INFORMATION FOR SEQ ID NO: 24:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 24:
   ACCTCTATGA CCCGGACGAT 20
(2) INFORMATION FOR SEQ ID NO: 25:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 25:
   CACCACCCTG ACAGTGCGTA 20
(2) INFORMATION FOR SEQ ID NO: 26:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 26:
   CTGGGGGCAT AGATGCTCGG 20
(2) INFORMATION FOR SEQ ID NO: 27:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 27:
   GCCCTGGAAG GCCTCGATCG 20
(2) INFORMATION FOR SEQ ID NO: 28:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Rattus norvegicus
   (xi) SEQUENCE DESCRIPTION: SEQ ID NO: 28:
   CAAGTCCCTA GGATCGTCCG 20
(2) INFORMATION FOR SEQ ID NO: 29:
   (i) SEQUENCE CHARACTERISTICS:
      (A) LENGTH: 20 amino acids
      (B) TYPE: AMINO ACID
      (C) STRANDEDNESS: SINGLE
      (D) TOPOLOGY: LINEAR
   (ii) MOLECULE TYPE: PEPTIDE
   (vi) ORIGINAL SOURCE:
      (A) ORGANISM: Rattus norvegicus
   (ix) FEATURE:
      (A) NAME/KEY: Position in SEQID2
      (B) LOCATION: 169..188
   (xi) SEQUENCE DESCRIPTION: SEQ ID NO: 29:
   Ser Ala Gln Asp Leu Asp Gly Asn Asn Glu Ala Tyr Ala Glu Leu Ile
   1 5 10 15
   Val Leu Gly Arg
   20
(2) INFORMATION FOR SEQ ID NO: 30:
   (i) SEQUENCE CHARACTERISTICS:
      (A) LENGTH: 15 amino acids
      (B) TYPE: AMINO ACID
      (C) STRANDEDNESS: SINGLE
      (D) TOPOLOGY: LINEAR
   (ii) MOLECULE TYPE: PEPTIDE
   (vi) ORIGINAL SOURCE:
      (A) ORGANISM: Rattus norvegicus
   (ix) FEATURE:
      (A) NAME/KEY: Position in SEQID2
      (B) LOCATION: 556..570
   (xi) SEQUENCE DESCRIPTION: SEQ ID NO: 30:
   Glu Glu Gly Gln Tyr Pro Pro Ala Pro Pro Pro Tyr Ser Glu Thr
   1 5 10 15
(2) INFORMATION FOR SEQ ID NO: 31:
   (i) SEQUENCE CHARACTERISTICS:
      (A) LENGTH: 21 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 31:
   ATGCAACAGG ACGGACTTGG A 21
(2) INFORMATION FOR SEQ ID NO: 32:
   (i) SEQUENCE CHARACTERISTICS:
      (A) LENGTH: 28 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 32:
   TCAGACGACT AAACTTTCCC GACTCAGG 28
(2) INFORMATION FOR SEQ ID NO: 33:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 33:
   CTACAACCCC TACGTTGAGT 20
(2) INFORMATION FOR SEQ ID NO: 34:
   (i) SEQUENCE CHARACTERISTICS:
      (A) LENGTH: 24 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 34:
   TCGTGACCTG ACCTTTGACC AGAC 24
(2) INFORMATION FOR SEQ ID NO: 35:
   (i) SEQUENCE CHARACTERISTICS:
      (A) LENGTH: 24 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 35:
   CCTGAGCTAC TCCTGTCAAC GTCT 24
(2) INFORMATION FOR SEQ ID NO: 36:
   (i) SEQUENCE CHARACTERISTICS:
      (A) LENGTH: 20 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 36:
   AGGCCGAGAT CGCCAGTCGT 20
(2) INFORMATION FOR SEQ ID NO: 37:
   (i) SEQUENCE CHARACTERISTICS:
      (A) LENGTH: 27 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 37:
   CTACATGGAT CCAGTCATGC CGAAGAT 27
(2) INFORMATION FOR SEQ ID NO: 38:
   (i) SEQUENCE CHARACTERISTICS:
      (A) LENGTH: 28 base pairs
      (B) TYPE: NUCLEIC ACID
      (C) STRANDEDNESS: SINGLE
      (D) TOPOLOGY: LINEAR
   (ii) MOLECULE TYPE: DNA
   (vi) ORIGINAL SOURCE:
      (A) ORGANISM: Homo sapiens
   (xi) SEQUENCE DESCRIPTION: SEQ ID NO: 38:
   CGACAACTCG AGTCAGTTGG TATCATGG 28
(2) INFORMATION FOR SEQ ID NO: 39:
   (i) SEQUENCE CHARACTERISTICS:
      (A) LENGTH: 15 amino acids
      (B) TYPE: AMINO ACID
      (C) STRANDEDNESS: SINGLE
      (D) TOPOLOGY: LINEAR
   (ii) MOLECULE TYPE: PEPTIDE
   (vi) ORIGINAL SOURCE:
      (A) ORGANISM: Homo sapiens
   (ix) FEATURE:
      (A) NAME/KEY: Identical to 5 .. 18 in ref swissprot :Q07021
      (B) LOCATION: 1..14
   (xi) SEQUENCE DESCRIPTION: SEQ ID NO: 39:
   Leu Arg Cys Val Pro Arg Val Leu Gly Ser Ser Val Ala Gly Tyr
   1               5                   10                  15
(2) INFORMATION FOR SEQ ID NO: 40:
   (i) SEQUENCE CHARACTERISTICS:
      (A) LENGTH: 16 amino acids
      (B) TYPE: AMINO ACID
      (C) STRANDEDNESS: SINGLE
      (D) TOPOLOGY: LINEAR
   (ii) MOLECULE TYPE: PEPTIDE
   (vi) ORIGINAL SOURCE:
      (A) ORGANISM: Homo sapiens
   (ix) FEATURE:
      (A) NAME/KEY: Identical to 268 .. 282 in ref swissprot :Q07021
      (B) LOCATION: 2..15
   (xi) SEQUENCE DESCRIPTION: SEQ ID NO: 40:
   Cys Tyr Ile Thr Phe Leu Glu Asp Leu Lys Ser Phe Val Lys Ser Gln
   1               5                   10                  15
(2) INFORMATION FOR SEQ ID NO: 41:
   (i) SEQUENCE CHARACTERISTICS:
      (A) LENGTH: 21721 base pairs
      (B) TYPE: NUCLEIC ACID
      (C) STRANDEDNESS: DOUBLE
      (D) TOPOLOGY: LINEAR
   (ii) MOLECULE TYPE: DNA
   (vi) ORIGINAL SOURCE:
      (A) ORGANISM: Homo sapiens
   (ix) FEATURE:
      (A) NAME/KEY: exon1
      (B) LOCATION: 1898..2253
   (ix) FEATURE:
      (A) NAME/KEY: exon2
      (B) LOCATION: 3438..3782
   (ix) FEATURE:
      (A) NAME/KEY: exon3
      (B) LOCATION: 12064..12183
   (ix) FEATURE:
      (A) NAME/KEY: exon4
      (B) LOCATION: 15049..15105
   (ix) FEATURE:
      (A) NAME/KEY: exon5
      (B) LOCATION: 15670..15816
   (ix) FEATURE:
      (A) NAME/KEY: exon6
      (B) LOCATION: 19486..19659
   (ix) FEATURE:
      (A) NAME/KEY: exon7
      (B) LOCATION: 19806..19865
   (ix) FEATURE:
      (A) NAME/KEY: exon8
      (B) LOCATION: 19963..20094
   (ix) FEATURE:
      (A) NAME/KEY: exon9
      (B) LOCATION: 20236..20864
   (ix) FEATURE:
      (A) NAME/KEY: exon10
      (B) LOCATION: 20954..21094
   (ix) FEATURE:
      (A) NAME/KEY: diverging nucleotide, G in ref genbank:AC002128
      (B) LOCATION: 715
   (ix) FEATURE:
      (A) NAME/KEY: diverging insertion, G in ref genbank:AC002128
      (B) LOCATION: 1229
   (ix) FEATURE:
      (A) NAME/KEY: diverging nucleotide, T in ref genbank:AC002128
      (B) LOCATION: 3676
   (ix) FEATURE:
      (A) NAME/KEY: diverging deletion, G in ref genbank:AD000684
      (B) LOCATION: 5039
   (ix) FEATURE:
      (A) NAME/KEY: diverging nucleotide, C in ref genbank:AD000684
      (B) LOCATION: 5118
   (ix) FEATURE:
      (A) NAME/KEY: diverging deletion, C in ref genbank:AD000684
      (B) LOCATION: 7337
   (ix) FEATURE:
      (A) NAME/KEY: diverging nucleotide, G in ref genbank:AD000684
      (B) LOCATION: 8294
   (ix) FEATURE:
      (A) NAME/KEY: diverging nucleotide, C in ref genbank:AD000684
      (B) LOCATION: 8604
   (ix) FEATURE:
      (A) NAME/KEY: diverging nucleotide, A in ref genbank:AD000684
      (B) LOCATION: 8928
   (ix) FEATURE:
      (A) NAME/KEY: diverging nucleotide, G in ref genbank:AD000684
      (B) LOCATION: 9021
   (ix) FEATURE:
      (A) NAME/KEY: diverging insertion, GAATGAAA in ref genbank:AD000684
      (B) LOCATION: 9851
   (ix) FEATURE:
      (A) NAME/KEY: diverging nucleotide, C in ref genbank:AD000684
      (B) LOCATION: 9878
   (ix) FEATURE:
      (A) NAME/KEY: diverging nucleotide, T in ref genbank:AD000684
      (B) LOCATION: 11478
   (ix) FEATURE:
      (A) NAME/KEY: diverging deletion, C in ref genbank:AD000684
      (B) LOCATION: 11577
   (ix) FEATURE:
      (A) NAME/KEY: diverging nucleotide, T in ref genbank:AD000684
      (B) LOCATION: 11779
   (ix) FEATURE:
      (A) NAME/KEY: diverging deletion, T in ref genbank:AD000684
      (B) LOCATION: 13411
   (ix) FEATURE:
      (A) NAME/KEY: diverging nucleotide, G in ref genbank:AD000684
      (B) LOCATION: 13538
   (ix) FEATURE:
      (A) NAME/KEY: diverging nucleotide, G in ref genbank:AD000684
      (B) LOCATION: 13896
   (ix) FEATURE:
      (A) NAME/KEY: diverging nucleotide, A in ref genbank:AD000684
      (B) LOCATION: 14912
   (ix) FEATURE:
      (A) NAME/KEY: diverging nucleotide, C in ref genbank:AD000684
      (B) LOCATION: 16732
   (ix) FEATURE:
      (A) NAME/KEY: diverging nucleotide, G in ref genbank:AD000684
      (B) LOCATION: 17169
   (ix) FEATURE:
      (A) NAME/KEY: diverging deletion, G in ref genbank:AD000684
      (B) LOCATION: 18946
   (ix) FEATURE:
      (A) NAME/KEY: diverging nucleotide, G in ref genbank:AD000684
      (B) LOCATION: 19474
   (ix) FEATURE:
      (A) NAME/KEY: diverging deletion, G in ref genbank:AD000684
      (B) LOCATION: 20500
   (ix) FEATURE:
      (A) NAME/KEY: diverging deletion, G in ref genbank:AD000684
      (B) LOCATION: 20501
   (ix) FEATURE:
      (A) NAME/KEY: diverging deletion, A in ref genbank:AD000684
      (B) LOCATION: 20502
   (ix) FEATURE:
      (A) NAME/KEY: diverging nucleotide, G in ref genbank:AD000684
      (B) LOCATION: 21270
   (ix) FEATURE:
      (A) NAME/KEY: diverging insertion, T in ref genbank:AD000684
      (B) LOCATION: 21356
   (ix) FEATURE:
      (A) NAME/KEY: diverging nucleotide, A in ref genbank:AD000684
      (B) LOCATION: 21476
   (ix) FEATURE:
      (A) NAME/KEY: diverging insertion, C in ref genbank:AD000684
      (B) LOCATION: 21588
   (ix) FEATURE:
      (A) NAME/KEY: diverging deletion, T in ref genbank:AD000684
      (B) LOCATION: 21601
   (ix) FEATURE:
      (A) NAME/KEY: diverging insertion, G in ref genbank:AD000684
      (B) LOCATION: 21635
   (ix) FEATURE:
      (A) NAME/KEY: Potential variant splicing site AAG
      (B) LOCATION: 19963..19965
   (xi) SEQUENCE DESCRIPTION: SEQ ID NO: 41:
   AACAGTTTGG CAGTTCCTCA AAAGGTTAAA AATAGAACTA CCAAGTCACC CAGCAATTCC 60
   ATTCTTAGGC ATATATTCAA AAGAAATGAA AGCAGATATT TGTACACCAG TGTTCACAGC 120
   TGCACTATTT ACAATAGTCA AAAGGTAGAA ACAACCTAGG TCCATCCACA AATGAATGGA 180
   TAAATAAAAC GTAGCATATA CATACAATGG TACACTAGTC CGCTGTAAAA AGAAATTTTG 240
   ATCTTACTGC ATGCTACATG GCTTCGACAT ACTACAACAT GGATGGACCT TGAAAACATT 300
   ATTCTTTGTG AAATAAACTA GACACAGGAC AAATGTTAGA CGATTCCACT TATATGAGGC 360
   ACCTAGAATG GGCAATTTGG TAAGCAAAGT AGAATAGAAA TTACTAGGGG CACAGGTAGC 420
   AGGGAATGGG GAGTTACTGT TTAATGGTCA CAGAGTTTAT GTTGGGGATG ATGAAACAGT 480
   TTCGGGGATA AAGAGTGGTG ACTGGTACAC GACATTGTGA ATATACTTAA TGCCACTGAA 540
   TTTTACACTT GAAGTGGTTA AAGCGATAAA TATTATAGNT TTGCATATTT TATCATAAAA 600
   ATATTTTTTT AAACGATGAA GGGACGTGAA CGGGTTGAAA TTTTATAAAA AGTGGCCAGG 660
   GAAGGTGTCA CTGCAATGGT GTCCTACAGG AGGAGGAAGA TCATGTGGAC ATCTCCGGGA 720
   AGGGTGTTCT GGCAGAGGGA GTAGCACGGG CGATGGCTCT GAGGACTGTG AGAAGTATAG 780
   TTGGAAACAG CGAGGAGGCC AGGGTGTCCG AAGCTGAGTA AGCCAGAGAG AGTGGGAGGA 840
   GGTGAGATAA GAGGGGGAAG GTCAGTTTCT GCTGAGAGTG AGGAGGAGCC ACAGGAGGGC 900
   TGTGAGCAGG TGGACGTGAT CTGGCTTGAG TTTTAACAGG GCCAGTAGAA CAAAGCACGC 960
   CTGGGTACCG AAACCAGCCA CTGGCCAGTT GGCAACCTGG GGGAGTCTAA CGCGAGGAAG 1020
   CGCCCAGGGT TCCCCCAGGA TGCGCTTTCC CTCGCCGCCA CCTGGAGACA GCAGAGTCAC 1080
   GCCCAGCGCT GCGCAGGCTG ATCGCCGCGC CGCGCCCCCG CCCTCGGTCG CAGGTGGCTC 1140
   GTTCCGGGAA TTCCTAAGCG GAAACCGGTC CCAAGCCCCG CGCCTTCGCT CGGCCCCTTT 1200
   AAGAGCCAGA ATTTCCGGAG GGCTGACCCG GGGCTAGGGA TGCCCAGGGG CCGAACCACA 1260
   AGTTGGGAAC GGGTGGGGGA GGTGGCGAAA ACTTCCGAAG TGGAATTCCA ACTTTTCCTG 1320
   GCCCTGATTC CCCTTGGGCA TCCCTGANGG GGCAGAGCTT CCCTTCCGGG GACTTTAGAG 1380
   GGTTCCTCAG GTCATCTAAC TGGGAGACAC AGGAGGCCCG AAGCGCCCCC CCTCCACCCG 1440
   GTCCGGAGGA ACCCCAGTGG AAGTGGAGAA GTCAGGCGCC ACCAACAAGC CTCTCCCAGC 1500
   CAGGACTTTG CTTAGACTCG CTCCTCCCGG CAGGGCGCAC CTAGGCGGGT CCATCGCCAG 1560
   CCGGGGAGAG GGGTTTGGGC AGGGAGGGAA CAGGTGCGCG GCGGGACCCG CCCTATCTCA 1620
   ACAGGTGAAT CGCTCCAAGT GGGTCTCGGT TGCATGGATC TCGGTGCGCT TGGTTTGGCC 1680
   GGAGCAGATG GGGGCCGGAA GGGACCTGTG GTCCGCAGGC GCCCTCCCAG CGGGCCAGTC 1740
   ACTTGGTTCG GGCCCTGGGG GACGGAGCGC ACCTGGGTCA GCCCACTTCC GGGGAGGGAG 1800
   GCAGAGGAAC CCCTCCCCGC CGCTCACCCC TAAGCCCAGC CCTCGGCTCC CACCCTTGTG 1860
   TACCTGGGCC GAACCATTCA CCGGAGCGCG CAGCGGGTGG AGTGTGGCTC GGAGGACCGC 1920
   GGCGGGTCAA GCACCTTTCT CCCCCATATC TGAAAGCATG CCCTTTGTCC ACGTCGTTTA 1980
   CGCTCATTAA AACTTCCAGA ATGCAACAGG ACGGACTTGG AGTAGGGACA AGGAACGGAA 2040
   GTGGGAAGGG GAGGAGCGTG CACCCCTCCT GGCCTTGGTG CGCGCCGCGC CCCCTAAGGT 2100
   ACTTTGGAAG GGACGCGCGG GCCAGACGCG CCCAGACGGC CGCGATGGCG CTGTTGGCCG 2160
   GCGGGCTCTC CAGAGGGCTG GGCTCCCACC CGGCCGCCGC AGGCCGGGAC GCGGTCGTCT 2220
   TCGTGTGGCT TCTGCTTAGC ACCTGGTGCA CAGGTACGGG GCACGGGGCC TCTGACGCTG 2280
   CGGAACGCCG GAGGGAACTG TAGAGGGGGA TGGATGGAGT TGGAGGCGGC GGGAAGCGGG 2340
   AAGCGGGGGT CTCAGAGGCT GGGACCTTCC GATCCCCTGG GTCTTGGGCG ATCTGTTGCG 2400
   NCGCGGGAGT GAGAGGAATT CCCCATTTGT GCCGGGGAGC GCTCCCCGCG CCCTTATCTG 2460
   GAAGATAGCA GGAAGTGAAA CTCCCTGGAC GGTGAGACCC GGAGCGGCAG GGAGAATGGA 2520
   ACTCTTTGTG GGGAGGGAGT GGAAGACCGC CCGATCTCTG GGAAAAGAAA AGCCGGGATG 2580
   GGACTTGGGC GCACCCGGGG ATTTCTAAGT TTTGGAGTAA CGGGGAGAGG GCACGGGAGG 2640
   GCTGGATCAG ACGCTTCCTA GAGGGACAGA GACGAAGGAA CAATGCCTAG GCCTCGGGTG 2700
   GGTGTGGGAC TGGGGACTCC CCATCCCCCG CACCCCACCC ACCTCCCGCG GGCTCCGGAT 2760
   TATACGTGCG TAAGAGTCTG GTGGGATGGA TTTACGGACT TGAAACCGAC TTCTGCTGGC 2820
   AGGCTTTCAC CTGGATGGGA TATTTGGGTG GTGATGAGGT CTTTCCCGAG ACACTTTTGG 2880
   TTCAGTCATT TGAAATGACT TTAGAGTAGG GTGAGGTGGT GGGAGGCTGA TGGAGATATT 2940
   GTGGGGGCTT TAGTCCCTCC ATGGCAAAGC AGTTCAGGCA AACAACTCCA TGGTTTTCCC 3000
   TCCAAATTCA AAAGGCCCCG GGTAACCTGG AATCCTTCGT AGTCGGTTTT GAAGTGGGGC 3060
   CTTGGGCGCT GGGGGCATCA ACATGGCCAT CTGGGCTTGC CTGCCCAGGC CACACAGAGG 3120
   CCCCTTGTTG TGGGTGAATG GCAAAGGGAA GAGGGGACTG GTGTGGTTCA GAGGCCACAG 3180
   GCTGGGAAGA GGGATGGCGG GCGAGTCCAA GGAAACTGGC CGTGTCACCG TGCACCTGCC 3240
   ACTTCAGCCC CACGGGTCTA TAAAATGGGC ATGATTATCG TGGCTACCTC ACTGGTCCTG 3300
   GCAATTAAGG AACAATGTGT GCCAGGCACT CTGTAAACCA CATACTTGCG AGTGTCAAGC 3360
   TGGTGACAGG TGGCGTTCCT GTTGAAGCAC CTCCCTGAGC TCACAGCAAC CCTTGCTGTC 3420
   TCTCCTCTTG CCCTCAGCTC CTGCCAGGGC CATCCAGGTG ACCGTGTCCA ACCCCTACCA 3480
   CGTGGTGATC CTCTTCCAGC CTGTGACCCT GCCCTGTACC TACCAGATGA CCTCGACCCC 3540
   CACGCAACCC ATCGTCATCT GGAAGTACAA GTCTTTCTGC CGGGACCGCA TCGCCGATGC 3600
   CTTCTCCCCG GCCAGCGTCG ACAACCAGCT CAATGCCCAG CTGGCAGCCG GGAACCCAGG 3660
   CTACAACCCC TACGTCGAGT GCCAGGACAG CGTGCGCACC GTCAGGGTCG TGGCCACCAA 3720
   GCAGGGCAAC GCTGTGACCC TGGGAGATTA CTACCAGGGC CGGAGGATTA CCATCACCGG 3780
   AAGTATGTTG GGCAGGGCAG GGGGATGAGG CTGGGCTTGC CCGGGTGGTG GGACTGGCGT 3840
   CCTTGTGCGG GACCTGGAGT CCCCATCTGA AAGCTCTTGA GTGCCAGTGT CTGAAAGGAC 3900
   CATTGAAGGG AGCAATTCTT TTTTTTTTTT TTTTTGAAGA TGGAGTCTTG CTCTGGACTC 3960
   CAGGCTGGAG TGCAGTGGTG CGATCTCAGC TCACTGCAAC CTCCACCTCC CAGGTTCAAG 4020
   CAATTCTCTT GCCTCAGCCT CCCGAGTAGC TGGGACTCCA GGTGCGTGCC ACCACGCCCA 4080
   GTTAATTTTT GTATTTTTAG TAGAGATGGG GTTTCACCAT GTTGGCCAGG CTGGTCTCAA 4140
   ACTCCTGACC TCAAATGATC TGCCCGCCTT GGCCTCGCAA AGTGCTGAGA GACACCATAC 4200
   CCAGCCTAAA GGGAGCGATT CTATTCTACT ATTCTTCCTT CTGCTAATCC TTCCATTCTT 4260
   TAATTTAATA ACGAAGATTT TTTGAGTACC TGTCATATAC CAGGTGCTGT TCTGGGCCCT 4320
   GGGAATACAG CTGTTAACAA AATCATCAAA CCACTTCCCT CGTGGAGCCC ACATTGCAGT 4380
   GAGAGAGACA AACACGACAC ACACTCTCAA GTCCTTGAAG ATAAAGAAAA CTGGGTAACG 4440
   GAGAGAAGAG GCCAGGGTTT GTTCTATAAT CATTAATAAC ACGAGCAGTA AGAAGTAAAA 4500
   TTTATCTAAG TAACAACTTA TAAAGGGTCT ACTGTGTGCT AAGCTCTCAT CCAGGTTCCC 4560
   AAGGATTAAC TCAGACCACA CAGTAATTGA ATAGATTCTA TCATTGTCAT CTTACAGAGG 4620
   CCCAGAGAGA GAAAGTGACT TGCCTAGTGT CATAGCTGGT AACGGGGCTG GGATTCTAAC 4680
   TCAGCCACTT TGGGTCTAGT GGCCAAGCTC CTAATCCCTT TGCTTGCCTA GGGTGGTCCG 4740
   CAGAGGACTC ACAGAGGAGA TGGCAGGAGT GAACTGCAGG GGCAAGAGAG CTTAATGGAG 4800
   AAAGCCTGTG ACATGCCAGG AACTGCACAC ATATTCTCCC ATTGAGTCCT CTCCTCTACC 4860
   CTCCTGACAG CTGAGGCACA GAGAGGTTAC CTTGTTCAAA TGGGTGCATA GGAAGTCAAA 4920
   GTCTGGAGCT GGGGTTTGAA CCCAGGCAGC CCTGAGAACC TTGTTCTTTT TTTTTNANNC 4980
   GGAGTCTCGC TCTGTCGCCC AGGCTGGAGT GCAGTGGCGG GATCTCGGCT CACTGCAAGC 5040
   TCCGCCTCCC GGGTTCACGC CATTCTCCTG CCTCAGCCTC CCAAGTAGCT GGGACTACAG 5100
   GCGCCCGCCA CTACGCCTGG CTAATTTTTT GTATTTTTAG TAGAGACGGG GTTTCACCGT 5160
   TTTAGCCGGG ATGGTCTCGA TCTCCTGACC TCGTGATCCG CCCGCCTCGG CCTCCCAAAG 5220
   TGCTGGGATT ACAGGCGTGA GCCACCGCGC CCGGCCCCTT GTTCTTAACT GTAATGCTGC 5280
   CTCCTGATAG GATGTGCCTG TTGGGACTAA GTAAGGGGCA GTCATTCATT CATTCATTTG 5340
   GTATTTATCA AGCATCGACT ATGTGTCGTT GGTGCTGGGG ATAGAGGTGA TTGGGATGGC 5400
   TGAAGTTTCT GTCGTCAAGG AGATGACATT CTGGTGGAGT NAGACTGGCA GTAAATNAAG 5460
   CAGATAAAGA AAGAGTATGA GAATTTCAAA GTCTGGGCAC GGTGGCTCAC GTCTGTAATC 5520
   TCAGCACTTT GGGAGGCCAA GGTGGGTGGA TCACCTGAGG TCAGGAGTTC CAGACCAGCC 5580
   TGGCCAACAT GGTGAAACCC CGTCTCTACT AAAAATACAA AGATTAGCCA GGCATGGTGG 5640
   CACATGCCTG TAATCCCAGC TACTCAGGAG GCTGAGGCAT GAGAATCGCT TGAACCCAGG 5700
   AGGCAGAGGT TGCAGTGAGC TGAGATCGCA CCACTGTACT GCAGTNTGGG CGACAGAGTG 5760
   AGACTCTGTC TCAAAAAAAA AAAAAAAAAA AAAGACTCCG TCAAGGTATA AGAATGTCAG 5820
   AGAGTACTAA GTGTTGCAAA GAAAATAACA CCAGGCTGGG TGCATTGGCT CATGCCTGTA 5880
   AATTTCAGCA CTTTGGGAGG CCAAGGCAGG AGGATCACTT GAGCCTAGGA GTTTGAGACC 5940
   AGCCTGGACA ACAAAATGAG ACCCCATGTC TACAAAAATT TTAAAAATTT AAAAATTAGC 6000
   TGGGCATGGT GGCATGTGCC TGTGGTCCCG GCTGCTCAGG AGGCTGAGGT GGGAGGATTG 6060
   CTTGGGCTTG AGAGGTCAAG GCTTCAGTGA GTCATGATCG TGCCACTGCA TTCCAGCCTG 6120
   GGTGACAGAG TGAGACCCTG TCTTGAAATG AAAAGAAAAT AGGCTGGGCG CAGTGGCTCA 6180
   CACCTGTAAT CCCAGCACTT TGGGAGGCCG AGGTGGGTGG ATCACCTGAG GTCAGGAGAT 6240
   CGAGACCAGC CTGGCCAACA TGGTGAAATC CCATCTCTAC TAAAAATACA AAATTTAGCC 6300
   GGGCGTGGTG GTGGGCGCCT GTAATCCCAG CTACTCGGGA GGCTGAGGCA GGAGAATCGC 6360
   TTGAACCTGG GAGGCGAAGG TTGCGGTGCG CCAAGATTGC GCCACTGCAC TCTAGCCTGG 6420
   GAAACAGTGA GACTCCGTCT TAAAAAAAAA AGAAAAAAGA AAATAGCACT GGGTGATGTG 6480
   CTACATGGAA TGACTTGGGC TGTGAATATG ATTTGAGGAG GGCCTGGGCC TGGGCCTTAC 6540
   AGAACCTAGA AGGCAGAGAG GAAGGGGAGG GGCAGGGTGC CAGGGATGAA GGCTCACGTA 6600
   CCTCATGTCT TAGTGTGTGT TCACTGTCTT AAACAAGAAT TTAAAGTTGG GCATGGGGCA 6660
   GAGCGGGGAA GGGAGCATCC CTTTGCAGAC CCCAAGAAGC CAGGAACTGG AGCACATTCT 6720
   GCTAGAGGAT CGATGGGAAG CAGGGTTCCA GGGGCTGAGC CTATGTCAGT CCTGTTTCAG 6780
   AGGAGGCACC AGGCTTGCTT GCCCTGAATT TCTGTGGGCA GCTCAGCCAT GAGCATCCTA 6840
   CTGTTATTGA GGTCACAGGG CTGCTTAGGC CCCCTCCTCT CTAACCCAGG GATTGTGCCT 6900
   GCCTGGACCA GGCGTGACTG CTAAGCTTCT GCCAGGACAA GCCAAATACT GAGGGTGCTT 6960
   CCTCTGCTGG ACGCAAAAGT CCAGGATGAC CCCCCAGGCT CTGTCTCGGG GAAGGGGCCC 7020
   TGCATGCTCC AGGGGCCTCA CAGGCCTGGG TCTTTCAAAC CACCCCCACC TGGGCCTGTG 7080
   TTTGATCAAG GCCCTGAGTG TAAACATCCA TTGTGTGTGT CCTTTCAGGA AATCCCATAG 7140
   CCATAGGAGC TTCCTCTGTT TCAGCTTTGA GGATGGGGAA AAGTGGACTC CCCGTGGTGT 7200
   TCCTAGGGTC ACCCACTGTG CTGGGGTTTT TCTGTTGNNT GTTGTTTTTT TTCTGTTGCC 7260
   CAGGCTGGAG TGCAGTGGTG CAATCTCAGC TCACTGCAAC CTCTGCCTCG CAAGTTCAAG 7320
   TGATTCTCCC GCCTCAGCCT CCTGAGTAGC TGGGATTACA GGTGCACACC ACCACACCTG 7380
   GCTAATTTTT GTATCTTTTT GGTAGAGATG GGATTTCGCC ATGTTGGCCA GGCTGGTCTC 7440
   AAACTCCTGA CCTCAGGTGA TCTGCCTGCC TTGGCCTCCC AAAGTTCTGG GATTACAGAT 7500
   GTGAGCCACC ATGCCCGGCC TATCCTGGTT TCAAAAGTGA AAATAGTCCT GGATAAGGTA 7560
   GAAGGCTGTC CACTCCAGGC ATCCCTCCGG TCCGGTGGCT CATTCCCTGC TTTGTCCTTC 7620
   CATGCTTTGG GTGATGGACC AGCACCTGGA CAGGAGGCCC TGTTCCACCT CCTCGGGCTC 7680
   CTTGGGGTCC AAGTGCCCCC ACCTCCAGCT GCACTGCAGC AGAGAGCCCA TGGGACCTCT 7740
   GAAATCATGA AGGTCACCTT TGCGGTGTAT AAAGAAGGAA CCAGAGGTTG GAGATGTGGA 7800
   GGAGGCCTGG CTGCTGTTCC CACTGGAGAC CTGGCATCTT CTCCCCGACC TAAAACAATG 7860
   AAAGCAGTGC TCAGCCCGGA TGAGATCACG GCCAGCCCAA GACCAGGAAC AGGGTACGCC 7920
   CTGCAGGAAG AAGGTGTGCC CAGACCTTAG GATGGATCAA AAGAAGCCGG AAAACTATAT 7980
   TTTTTGTGAG TTTTGAAAAT GTCAGACAGG TCAAACAAAA CACAGTGAGG TCCAGCCTCG 8040
   GCCTACAAGA TGCCAGATTT CAACCCCTGG CCTATATGAT CTGTTTGCCA TGGCAGGCGG 8100
   TTCCTGTCCA CCTCTTTTGT TTATAGCAGG GACCAGCTCT TGAGCTCCAG TGTTGAAGAG 8160
   GCACGGTCAG GGTCTGATCT GAAGACACTG GTGGCTCATG CCTGTAATCC CAGCACTTCA 8220
   GGAGGCCGAG GCAGGAGGAT TGCTTGAGGA CAGGAGCTGG GAGACCAGCC TGGGCAACAC 8280
   AGTGAGACCC AGACACTACA AAAAAATAAA TTTAGCGGGG CATGATGGCA CACCCTGCTA 8340
   CTCTGGAGAT GGGAAGATTG CTTGAGCCTA GGAGTTCGAA GCTGCAGTGA CCCATGATCG 8400
   CACCACTGCA CTCCAGCCTG GGCGACCAAG CTAGGCCCTC TCAAAAAAGA TACAGGTGGA 8460
   AAAATGATGG ACGAAGAGGG CATTGTGGCA AACCTGGGGA TTTAGGAGAA CCTAGTTTGG 8520
   AATTCTATGA GGATTCAATG AAAGAATGTG TGTAGAGGGG CCCAGCACAT AGTAAGAGCT 8580
   CAATAAACGG TGGGGGCTAG GGGTGGTGGC TCATGCCTGT AATCCCAGCA CTTTGGGAGG 8640
   CTGAGGCAGG TGGATCACTT GAGCCCTGGA GTTCAAGATC AACCTGGACA ACAAAGCAAG 8700
   ATCCCATCTC AAAATTAAAA AACAACACCA ACAACAAAAA AACAGTGGCT TAGATGCCTG 8760
   ATCATTAGGG TAAGTCGTGT CCTCAACCCC TTCACATCTG CTCTGAAGGT CACCATATCC 8820
   GGAAGCCTTC CCTGGCCTCC TTGTTTAAAA TGGCACAGCC CCCACTCCAC GCCTGGCACT 8880
   CTCTGCTGTC CCTGATTCGT TTTCTCCATA CAGCTTATCT TTGTCTGGTA TGTGACATAG 8940
   TTAACATTTT ATATTTGTCT TTCTTTCCTA GTTAGAATCT GAACTCTAGA AGGGCAAGGG 9000
   CAAGGATTTA TAACTCAAAG ATTCCGGGCT TAGGCCTCTT TTATATTCTT GATTTTGAGG 9060
   TTAATTAAGA GCTCAGGCCT AGCGAGGTGG CTCATGCCTG GAATCCCAGC ACTTTGGGAG 9120
   GCCCAGGCGG GCAGATCACT TGAGGTCAGG AGTTCCAGAC CTGCCTGGCC AACACAGTGA 9180
   AAAACCTGTC TCTACTAAAA ATACAAAAAT TAGCCAGTTA TGTTGGCAGG CGCCTATAAT 9240
   CCCAGCTACT CAAGAGGCTG AGGCAGGAGA ATCGCTTGAA CCCAGGAGGC AGAGGCTGCA 9300
   GTGAGCCAAG ATCGTGCCAC TGCACTCCAG CCTGGGCAAC AGAGCGAGAC TCCATCTCAA 9360
   AAAAAAAAAA AAAATTAAGA GCTCAAAGAG TTTGTTTTCA TAGGCAGCAG AATGAGAAAA 9420
   GTTTACAAAA TAGTTTAAAT GACAATAAAG TCATTATAGA TTAACATAAA TAAAATACCT 9480
   TTTATGAAAA AAATAATCAT TTTCTGAAAT CAGACAAAAC ATTGTGAATG AGAAGGTGGC 9540
   ATGGTTTTAT TTTTTTGCAA GTCTCCGAAG CCTGGCTGGA TAGAAGAGCC TGGCTTCTCA 9600
   GAGCTGCTTC AGTCTGTTGT GATATCTATT GTATGTCACG TAGCCTCTGG AAAACTCCAC 9660
   AGTTAGTATT GTTGGGAAAA TAACTTTGAC CTCAGGATCT CCTGAAAACG TCTTGGGGAA 9720
   CCCCAGGGTC TAGAGGCTGC AGTTTGAGAA CTGTTGCTGT GGTATCCCAG GTGTCTCAAA 9780
   TACTGCCTAG AACATAGGTG GTACTCAGTA ATTATTGTTG NAAGGATGAA TGAATGAATG 9840
   AATGAATGAA TGAAAGAAAG AAATGTGTCT TTGAATCTAG CCATGTGCCC AGAATGATGA 9900
   GACAGATGAC AAAAGCTAAG GGACTTTAGC ATGAGGAGAG GGGGTTCGTT TCCTTTTTTT 9960
   TCTTTTTTTT TTGAGATGGA GTCTCACTCT ACTGCCCAGG CTAGAGTGCA GTGGTGCAAT 10020
   CTCAGCTCAC TGCAATCTCT GCCTCCTGAG TTCAAGCAAT TCTCCTGCCT CAGCCTCCAG 10080
   GGTAGCTGGG ACTACAGGTG CGTGCCACCA TGCCTAGCTA ATTTTTTACA TTTTTGGTAG 10140
   AGATGGGGTT TTACCATGTT GGCCGGGCTG GTCTGGAACT CCTGACCTCA AGTGATCCAC 10200
   CTGCCTCAGC CTCCCAAAGT GTTAGGATTA CAGGTGTGAG CCACCATGTC CGGCCAAGAG 10260
   GGTGTTCATT TCTGCTCCTT GCCAGGTATT GTGTCAGGCA CTGGGGACCC AGCAGTGGCT 10320
   GAGACAGACA GGGCTCTGCC TCACGGAGCC CACATTTTCA CCAGGCAAAG GATGGTCGGC 10380
   CCCTAAGCTG GGAGATAAGA CTTCAGCAGT TGGGTGGGGG AGCCGTGGGA GAAGCCCAGC 10440
   CCACAGGGGG ACAGTGCAAA TCTAGAACCA AGGCGATGGC AGGGGTGAGG CTGGCACGGT 10500
   AGCTAGAGAC CACGTCGTGC CAAGGGCCTT GGGGACCATG GGACTATGGG ACCTTAGGGA 10560
   AGGCGTCTGG AATGCTGTAG CCAGACACTG TTGCAAGGAG GATTTTTCTG TAGACATGAG 10620
   GCCTTCCTTA TGAAGAAAGC AAGGGTTCTT TCATTCCTGG GGGTGCCAGG TGCTGTGGAC 10680
   TGCAGCACGC GTGGTTGCTG CCGTCACAGA GCTGTCATGC AGGAGGGCAG CGCGTCCTTG 10740
   GGAAGGTGGC AGGCAGGTCA GGCTAGGAGG AAAGAGGCCG GGAAGCTGAG GGCATTTCCT 10800
   GCCCGAGATG CCCAATGTAG CCTACTTCTG TCCCCAGTGG CTTAAGGCAG AGTTGCCTGG 10860
   TAGGTGCCCT GGTCCCACCC TGGTGAAAGG CTGAAGGTAT TTAATTAGTG CCTGAGAAGC 10920
   AGAGAGGAAA CAGGATGTGC CAAAACACTT TGATGGATGG TAGAGTTAAC AGGCTCCTTG 10980
   CCTGCAGCTG CTTCAGACAA GAGCGTCCCC AAGCCCTGGG CCTGACCTGG AATGTGGGGA 11040
   TGGAAGGGGA GGGGGAGGAA CCAAGGCACT GGGAGGGTAA GTCTCTCTCT CCCACATAGA 11100
   CACACCCACT CCTTATGGGT GCCTGGGCAT CTCCTGGTAC CTAGAATCTG GCCTGTTTAT 11160
   CTCCACACCC ATCCCTGGGG TCTACACTAG GCCCTGTGGG TGGCAGTTCA CATCAGGGGA 11220
   GTTCTGACTT TGGCTCTGAG AGGTGGTTCA GAGATGGCTG TAAGTTGAGA AGCACAGACT 11280
   GCTGGGTGTG GTGGTTCACG CCTGTAATCC CAGCACTTTG GGAGGCTGAG GTGGGGGTGG 11340
   ATCACCTGAG GTCTGGAGTT CAAAACCAAC TTGGTCAACA TGGCGAAACT CCATCTCTAC 11400
   TAAAAATGCA AAAATTAGCC AGGTGTGGTG GCAGGTGCCT ATAATCCCAG CTACATGGGA 11460
   GGCTGAGGCA GGAGAATCGC TTGAATCTGG GAGGCGAAGA TTGTAGTGAG CCGAGATTAG 11520
   TTCGCACCAT TGCATGCCAG CCTGGGCAAC AAGAGTGAAA CTCCGATTCA AACAAACAAA 11580
   AAAAAAAAGC TGGGCATGGT GGAGTGCCTG TAGTCCTAAC TACTCAGGTG GGAGGATTGC 11640
   TTGAGTCCAG GAGGTTGAAG TTGCAGTGGG CTATAATTAC ACCACTGCAC TCCAGCCAGG 11700
   GCCACAGAGT GAGACCCTGT CTCTAAAGAA AGAAAAAAAA AAACAACCTC AGGCTCCGAG 11760
   GGCACCATTA CTGCTCTACA CTGAAGAGCT GTGCAGCTTT TCCAGACCCG AAATGTCATC 11820
   CACAAAACAG AAGTGATAAT GGTCCTGCCT CACAGACTTC TTGCAGTAGT CCAGGTGTTT 11880
   AGAACGGGGT GTAAAAGGCC GTGTGCCCTT GGTAGGAATC TTNGCATATG CATTTGATCA 11940
   TCTGCAGCCT GCCCAGCCCA CTGCTTGCCC CCTCCTGGGT GTGCTGGGAA GGGGTCTTTG 12000
   GCCCTCCAGG GGTTAGGTGC CCCAGCCTCC AAGGTGCCCT CACGCCTTTT CATCCCGACT 12060
   CAGATGCTGA CCTGACCTTT GACCAGACGG CGTGGGGGGA CAGTGGTGTG TATTACTGCT 12120
   CCGTGGTCTC AGCCCAGGAC CTCCAGGGGA ACAATGAGGC CTACGCAGAG CTCATCGTCC 12180
   TTGGTGAGTG GGCCTGGGAA GGGGGAGGCA TGGCCCTTCC TTTTGTCCGC TTCTGTTCTG 12240
   TCTGCCCTCC CCTGTGTCCG CCCTCTGCCC TCCAGCTTAC CCTCTGGGCT CTGTCGCCTG 12300
   CTCTGCTCTC CCCCAGGCTC TGCCAGTCAC TTAGGCTCCC CTGTGCCCTG CACCCCAGGC 12360
   AGGGACCACT GGCCCACAGT GCCTCCAATC ACCCAAGCCA AACTAAGAGA AGAGTGGAGA 12420
   CAATTGGAGA CTCTGCCTTT TCAAAGTCTC ATTTTTAAAA AAAATCCAGA CTTGGGGTCC 12480
   GGGTGCGGTA GTTCATGCCT GTAATCCCAG CACTTTGGGA GGCCGAGGCG GGTGGATCAC 12540
   TTGAGGCCAG GAGTTCGAGA CTAGCCTGGC CAACGTGGCA AAATCCCGTC TCTATAAAAA 12600
   ATATAAAAGC CAGGCGTGGT GGTGCACATG CCTGTAATCC CAGTTACTCA GAAGGCTGAG 12660
   GCATGAGGAT TGCTTGAACC TGGGAGGCAG AGGATGCAGT AAGCCAAGAT CAAGCCACTG 12720
   CACTCCAGCC TGGGCGACAG AGTGAGACTC TGTCCAAAAA AAAAAAAAAT CCAGACGTGG 12780
   TCAGAGTCCA TGGGCAGTGA ATGAGGACAG TTGATGGTGT GCAAAATCGA CCCACCTCTT 12840
   GCTACATCCC CAAGGCCTCA TCTCACCCGA GTCCCTCGCC AAAGCACAGC GGTTTTGCCG 12900
   TGTGCCCTGC TGGGATGGCG CTGCATGGCA CACACACTGT GTAAGTTTGA GTGCAGCTGA 12960
   AACGAAGCCG ATTCCAGACA CCCAGGGGCA GGGCGGGGTG TCCGTGTGGC TGGGAGGCCT 13020
   CCTTGTGTTA GGGGGATGTT GCCATCGGCC AGGTGCCCTG CTGTAAGCCA ACACATGGAG 13080
   TCTTGTATGA CATGTGCTCT GCATGAGTGA TGCCGCTGGG CTGTACACTG CCATCTTCAC 13140
   ATGTGTGAAT GAGCACGTGA CTGGGGGGTA CTTGGGCTGC AAGACAGAGT TCATGTGTGG 13200
   GGGATGGAAC ACGTGCACCA GTGACCCAGG AACCTCTGCC TGTTCTTCGG TAAAATGCAC 13260
   CATTTGCATC AGCAGTTCCC AAAATTAGTC TCCAGGTCTA TTTACACTCT AAAACATTAT 13320
   CGAGGGTCTC CAAGAGCTTT TGTTTGTTTC TGTGGGTTTT ATGTCTATCT GTTGCTTAAC 13380
   ATATTAGGAA TTAAAATGGG GAGATTTTCC TTTTTTTTTT TTTTTTTTTG AGATGGAGTC 13440
   TCGTTCTGTC GCCCAGGCTG GAGTGCAGTG GCTCGATCTC GGCTCACTGC AAGCTTCACC 13500
   TCCTGGGTTC ACGCCATTCT CCTGCCTCAG CCTCCCAAGT AGCTGGGACT ACAGGCACCC 13560
   GCCACCACAC CCGGCTAATT TTTTTTGTAT TTTTAGTAGA GACTGGGTTT CACCATGTTA 13620
   GCCAGGATGG TCTCGATCTC CTGACCTCGT GATCCACCCA CCTGGGCCTC CCAAAGTGCT 13680
   GGGATTACAG GCATGAGCCA CTGCCCGGCC TTAAAATGGG GAGATTTTTC AAGCCCAAGA 13740
   TACACAAGGA AGACTGGGCA ACATGGCAAG ACCCTGACTC TACAAAAAAT TTTAAAATTA 13800
   ACCAGGCATG GTGGCATGCA CCTGTGAGCC CAGCTTCTTG GGAGGCTGAG GCAGGAGTAT 13860
   CGCTTGCACC CAGGAGGTCA AGGCTGCAGT GAGCCATGAC TATGCTACTG CACTCTAGCA 13920
   TGAGTGACAG AGACCCTGGC TCAAGAAANN CANACAAACA CACACACACA CACACACACG 13980
   CATATAGTCC ATTAGGCATC AGGGCGATGA TGGCATCAGG GAGCCTGGGA AACTCTACTG 14040
   GACATTCATG GGAGAACAAG TGAAAAAGGC AAATAACATC TTAGTGTTAT TCTAAAATTT 14100
   CTTCTTTTGG CCTTGTGGAC AGGACCACGC TTTGAGAGCT GTGACTGACA TGCCTCTGTC 14160
   CTGTTGCGAG GGCCTATAGT GCCAAGTGCA TGAGCTCTGG GGAGGGCTTC GTGGGTGCAG 14220
   AGCTGGGCCT GTGGAGGCCC CTCAGACACA ACACTGGTGG GGCTCAGAGC TCCAGGGGCA 14280
   CTCGAGGGAA GACAAGAACC GGCTCTGAGA TGCGTGAATG TGACAGTGCA TGAGTAGAGA 14340
   TGGAGACCTT GTGGGTCCCA GAACCAGGAC TGCATATGAC TTTCATATGT GGGTATTTTT 14400
   GCCTTCATGG GTCCCTTCCT GTTTTAAAAA AAATGTGTGA TTATGTTGTC ACAAAGAGTT 14460
   TATTCCTGTA TATTGTGTTA ATTTGTGTTC AGATTTGTAA AGTAAAATTA AACCATTTCA 14520
   GCCAGGTGTG GTGACACATG CCTGTAGCCC TAGCTACTTA CCCCAGAGGC TGAGGTGGGA 14580
   GGATCGCCTG AGCCCACGAG GTTGAAGCTG CAGTGAGCCA TGATCACACC CCTGCACTCC 14640
   AGACTGGGCG ACAGAGCTGA GATCCTATTT CGTGGGCCCT AGGTCCCTGT GCCTGCTGGA 14700
   ACAGGACATC CCTATCACCG TGGTTGGAGC CCTTTGGGGT GCTAAGACCT ATGAATGAGG 14760
   GAAACTTAGG GTGCCCAAGC TGAGGTAGAG CCCTCAGAAC CCCCTGGGAT TTGTATTGGA 14820
   GCCCTCGTGG CATAACACAG GTGGATTATG CAATGGGAGT TTCTTACCTA TAAGCACCCA 14880
   CATGTGGGCG GGTGGAGGGT AGGAGCCATG CGCTAGGGCT TCAGCCCCCA GCCCCTTCCC 14940
   GCTTCAGGGC ACACCTTGCA CTTGGCCAGC CTGGAGCTGG GCTTTCGGGG GTGGCACAGC 15000
   CTGGGCTGGC TCTGGCCAGC ATAATCTGTT TCTCTTTTGT CCCTCCAGGG AGGACCTCAG 15060
   GGGTGGCTGA GCTCTTACCT GGTTTTCAGG CGGGGCCCAT AGAAGGTACG GGGGGTGGAT 15120
   CCTGAGTTGG GCTTCTCGGG AGCTCCCATA CATCACCTAC TGCTTCTGAC TCTAGTTAGT 15180
   ATCCCCTTCC CCACTAAACC CTGCTCACTG TGGACCCCTC ACTAACCTGG CCTGACTGTG 15240
   GCTCTGAGGC ATCTAGTGGT CTGGCGCTGG GCCTAGGCTA GGCTGGGCTG AGGAGAGCCT 15300
   GGGGTGCAGG CCAGGGCTCT GTGACTGGCA CCTGCGGTGC TCTTGAGGGT GTGGCGTCTG 15360
   GGCAGCTGGC TCTCTCTTTG GTCTGGGGGC TGCAGTCTGT CTCCCTCTGT GCAGGCTGCC 15420
   TCGTTTTCTG CCTTGTGTTT TTTGCACCTG GGGGAGGGCC GTAACTGGGG AATGGCCGGG 15480
   ATGGTAGAAT GGGGAGTGTG CTGTGCCCAG CCTCTGGCAC AAAAAATCCA GCCAGGGCTG 15540
   CAGGTTCCTT GGTGAGCTTT GCAAATCGTC CCCGACCTCA GTGCTGGCTC CGCACCATGT 15600
   ACCCCTGCTG TGCCGTTAGC CCTGTTCCCT CCCAGGCCTC CGGGCTCAGG GCCTGTTGTC 15660
   TTTCTGCAGA CTGGCTCTTC GTGGTTGTGG TATGCCTGGC TGCCTTCCTC ATCTTCCTCC 15720
   TCCTGGGCAT CTGCTGGTGC CAGTGCTGCC CGCACACTTG CTGCTGCTAC GTCAGGTGCC 15780
   CCTGCTGCCC AGACAAGTGC TGCTGCCCCG AGGCCCGTAA GTGTCCCGCT CATGGCCACC 15840
   CTGGTTTGGG CAACATCCTG CATCCAAGGG AAGGAGGTGG CCATCCACCT GCCCCCAGGA 15900
   CAGTGGCGTT GGTCTGGAGG GTGTGAATTT AGCCAGTGGG GAGAAAGTAG GCTGAGGAGG 15960
   GTCTGCTGTT TAGATTGTCG TTTACTTCCT CCAACTTTTA GTTTATTTTT ATTTATGTTG 16020
   TTCTTTTCTT TTGTAAGTAT AATCCATACA CATGGTAAAA ATGTCCAACA GTACAAGATA 16080
   CTAGTCACAT GGAAGTAAAG CCCTCTAAAA AAACCAAATC TTGGCTAGGC GCAGTGATTA 16140
   CGCCTGTAAT CCCAGCACTT TGGGAGGCCA AGACGAGTGG ATCACTTGAG GTCAGGAGTT 16200
   CCAGATCAGC CTGGCCAACA TGGTAAAACC CAGTTCTCTA CTAAAAATAC AAAAATTAGC 16260
   TGGGCATGGT GGTGATCGCC TGTAATCCCA GCTACTCAGG AGACTGAGGC ATGAGAATCG 16320
   CTTAAACCCA AGAAGTGGAG GTTGCAGTGA GCTGAGATCA CGCCACTGCA CTCCAGCCTG 16380
   GGCGACAGAG TGAGACTCTG TCTCAAAAAA AAAAGAAAAA AAAATGTTAA GTGAAAAAGT 16440
   TAAGAAACCA AACAAGGTTT ACAACACTAC ATGATTTAAG CAAAAAAAAT TTTTTTTGTT 16500
   TTAGAGAAAG GGTCTCATTC TGTCATCCAG GCAGTGCAGT GCGATCATAG CTCTCTGCAG 16560
   CCTCAAACTC CCGGGTTCAA GCAGTCCTCC CGCCTCAGCC TCTGGAGCAG CTGGGACTGT 16620
   AGGCACACAC CACCATGCCC AGCTAATTTT TTGATTTTTG TTTTTTGTAG AGACGGGGTC 16680
   TCAGTATGTT GCCCAGCCTG ATCTCAAACT CCTGGCCTCA GGTGATCCTC CGAAGTCAGC 16740
   CTCCCCAAAG TGCTGGGATT ACAGGCATGT GCCACCATGC TGGCCAATTT TTAAAAATTT 16800
   TCTGTAGAGA CAGGGTCTTG CTATGTTGCC CAGGCTGGTC TTGAACTCTT GACCTCAAGT 16860
   GATCCTGCCT CAGGCTCCCA AAGTGATGGG ATTACAGGCA TGAACTACCA CACCTGGCCT 16920
   TAAACTTAAG CAAATTTTTT TTTTTTTTTG GAGACAGTTT CACTCTGTCG CCCAGGCTGG 16980
   AGTAAAGTGG CGTGATCTCT GCTCACTGCA ACCTCCGCCC CCCGGGTTTA AGCTATTCTC 17040
   CTGCCTCAGC CTCCCGAGTA GCTGGGATAT AGGCGCCTGC CACCACGCCT GACTAATTTT 17100
   TGTATTTTTA GTAGAGACGG GGTTTTGCCA TGTTGGCCAG GCTGGTCTCG AACTCCTGAC 17160
   CTCAGGCAAT CCGCTCCCCC GCACCCCTAC CTTGGCCTCC CAAAGTGTTA GGACTACAGG 17220
   TGTGAGCCAC CATGCCTGGC CAAATTTAAG CAAATGTTTG AAAACACATA CCCACAGGAA 17280
   TGCTGCACAT TTTACCCAGC TACTATGTCT AGGGTCGTAT CTAGCACACC AGCATGGCTA 17340
   CTGTGGAGAG CTGGGACTGG ATGTGAGATG AGAGCTAAAG GGGAAGTAAG CAAACCAAGC 17400
   AGGGGAAGGT AAGAGAAGAC AGAAGACAGA GAGAGAGGGA CCTAACTCTA TGAGAGGAGT 17460
   CAGACATGTG CAATTGAAAA AGACTTGCTC CTGTCTCTCT TCTGTGAATG TTTGTGAATA 17520
   TCCCAACGGG ACACTTTCAC AGAGGAGCTG ATTGACGTGG TCACAGCCAT CAGCCTTGGG 17580
   ACACCAGACC ACAGTGTGTA CACTAAGTGG CACTGATGGA CACTTCAGCA TCCCTCTAGC 17640
   TGCTGTCCCG TTTCCCCTCC TCGGGGACCA CAGCTGTTGC CAGTCCTTGG TTTCCTTCAG 17700
   GAGGGTGTCT GGGTAGACCA GCCTGTGTGC ACACAGTCCA AGATACATGA ACAGTGAAGT 17760
   GCCAGGCAAT CCTTGCAAGC ATGGGCAGGT GGAGAGCTGA GGCCTGCTTG ACACCTTCCT 17820
   GCTCAGAAGC CCAGTGAGCA GTTTCCCTCC CTAGGGCTCA GTGTCATCCC CTATAAAATG 17880
   GGGCTTATGG CAGAGCTCAC CACACTGGGT GCATCTGGGG ATTTGGCGAG CTCATGTGCA 17940
   CACCATTGAG CATGGGGCCC AACCTATATA AAATATTCTA CGTCTGTCAG CTGCTGGGCA 18000
   CTGCCACTAT CAGCCTCAGT AGTGACTGAG GGACAGGGCA CCAGTCAGAG CCCTGGTGCA 18060
   CACAGAGTGA CCCCAGAGAA GCAGCCTTCC CTCTCTGAGT CCTGTTTCCT TCTGTTAGGT 18120
   CCTGACTTCA TGGGTTGTTG TTAGCATTAA GGAAGTCGCT GGCTAATTTT ATAGTCATTG 18180
   AAGTCAGTGG TGTGCAACCT GGTTCCTCAA AGGATCACTT CCCTGAAAAA ATTCCACTGC 18240
   TCCCTGGAGG CTTATGCAGG CCATCCCATC CCCTCCCTCT TGTTGTGTTC AGCTGACAGC 18300
   TTTTTGCTCA GTGAGTAAGT GTTAGGTCCA TTTCACAGAT GGGCTGCAAC CAAGTTTGCA 18360
   GTGAACCCAC TAAGACCAGA GCTAGGGCCA GGACTAAATG CTGGTCCCAA TGCCACATTC 18420
   CCCTGTCCCC ACACCACATT TCCTCCATCC GGAGACCCTG TTACCCCAAC CCAGGGCCCC 18480
   ATTAACTCCC TGGCAGAGGC CCTGTTACAT CTGCTGCTGC CACAGCCTCC GCCCACCCTT 18540
   CAGGAGGCAG CAGGTCCCAC TGCTGATGAT AAAGTTGCAG GCTGCCTGAG CTAATGAAGG 18600
   GGCTTCCTCT AGGCTGTGCA CTTAGTCTTC TGCTTCCAAA CCAAATCAGA GGTGAGGCAC 18660
   CCTCTCTGGG CCCATCTCTC TCCTCCATTT TCCTGTTGGG GTCCCAGGGA GGAAGCCACT 18720
   TGCCTAGGGC CCAGGAATTT TGCAAGCCTC TTGCCCTAGG GAGGAAGGAA GGGAGGAGGA 18780
   TCTTACCTTG AACTGTCAAG CCTAGAGCCT GGTGGGGCAG GCAGAAATGG GTGCAGTCCA 18840
   TGAGTTAGAA ACACTAGAGG AGACACTTTG CTGCTTGGNC CGGGGCAGGC AAGNTTAATT 18900
   CCCGAGGCTC CTGCCACTGC ATCTCAATCT GGAAGGTGAC CAGGTGGGGC AGGACCCACG 18960
   TCTCCCAGAT GACTCATTTT TTCTAGAACA GGGGCTTGGC TGCCAAAGAG GATACTTGAT 19020
   TTCGGCTTGT GGGGACAGTG GTGGACCCAG CATCTGGGCT TTATATAAAG GGCAGCTTTG 19080
   TTGCCCTGTA AACACACAGA CCATGGGTGG CCACTTCTTC CAGTAAGTTA GCTGGGGAGT 19140
   TGGAAGTTTA GGTAAAACCT TTTGATTGAC AAATGTTGGC GAATTACCAT GCTGTTAAAT 19200
   GAAACATTGT TCTGCCACCC TGGGGCTGTG GGTGCCTGCG TGCACCCTCT GAAAAATCAC 19260
   ACAGGAAGTG GGGTGGGGTC TCTGTGAAGC TGGTGTCCCC CAGCCTCAGG GATGCTGCAG 19320
   AAATGGAATG AGGACCAACA GGGACTCAGA TGTCCAAGGA AGCTCTACAG CGGAGAGGAC 19380
   GGCTTGGGAA GGAGGTCCAG GCCCAGGTCC CTCCGGAACC CAATGGGTAT GGGGCAGCCT 19440
   GGCTCCTGCC TCATCCCCCT TCTCCTGTTG ATTATGTCCT CACAGTGTAT GCCGCCGGCA 19500
   AAGCAGCCAC CTCAGGTGTT CCCAGCATTT ATGCCCCCAG CACCTATGCC CACCTGTCTC 19560
   CCGCCAAGAC CCCACCCCCA CCAGCTATGA TTCCCATGGG CCCTGCCTAC AACGGGTACC 19620
   CTGGAGGATA CCCTGGAGAC GTTGACAGGA GTAGCTCAGG TGAGGCCGGG GGAAGCAGGA 19680
   ACAGCTGGTG GGAGTGTGCT GGGCATCTGG ACACTGAGGG GCAGGGGCTG GAAGGAAGAG 19740
   TGTCTTGGGA GCCGAGGAGG GGCTCTGCTC CTGGTGCGCG GCCACTGACA GCCACTCTCC 19800
   CCCAGCTGGT GGCCAAGGCT CCTATGTACC CCTGCTTCGG GACACGGACA GCAGTGTGGC 19860
   CTCTGGTGAG AATCCATCGT CCCGAAGTTG GATGTGCCTG TAAGGGAGAG GGGTGGGCCA 19920
   GGATCCATCC TCCCAAACCG ACCACCACCC CCCTGTCCCT AGAAGTCCGC AGTGGCTACA 19980
   GGATTCAGGC CAGCCAGCAG GACGACTCCA TGCGGGTCCT GTACTACATG GAGAAGGAGC 20040
   TGGCCAACTT CGACCCTTCT CGACCTGGCC CCCCCAGTGG CCGTGTGGAG CGGGGTAAGC 20100
   AGGAGCCTTG GGGTCTGAGG GCTTTTAAGG TGGGGGGGTG AAACATGTCT CCCTGATACC 20160
   TGCCGCAGGG ACTCTTGGTG CAAACCCTGG ACCCCGGGCT CCTCCAGCAG TCAGTGACAC 20220
   CCCCCTTCCC TGCAGCCATG AGTGAAGTCA CCTCCCTCCA CGAGGACGAC TGGCGATCTC 20280
   GGCCTTCCCG GGGCCCTGCC CTCACCCCGA TCCGGGATGA GGAGTGGGGT GGCCACTCCC 20340
   CCCGGAGTCC CAGGGGATGG GACCAGGAGC CCGCCAGGGA GCAGGCAGGC GGGGGCTGGC 20400
   GGGCCAGGCG GCCCCGGGCC CGCTCCGTGG ACGCCCTGGA CGACCTCACC CCGCCGAGCA 20460
   CCGCCGAGTC AGGGAGCAGG TCTCCCACGA GTAATGGTGG GAGGAGAAGC CGGGCCTACA 20520
   TGCCCCCGCG GAGCCGCAGC CGGGACGACC TCTATGACCA AGACGACTCG AGGGACTTCC 20580
   CACGCTCCCG GGACCCCCAC TACGACGACT TCAGGTCTCG GGAGCGCCCT CCTGCCGACC 20640
   CCAGGTCCCA CCACCACCGT ACCCGGGACC CTCGGGACAA CGGCTCCAGG TCCGGGGACC 20700
   TCCCCTATGA TGGGCGGCTA CTGGAGGAGG CTGTGAGGAA GAAGGGGTCG GAGGAGAGGA 20760
   GGAGACCCCA CAAGGAGGAG GAGGAAGAGG CCTACTACCC GCCCGCGCCG CCCCCGTACT 20820
   CGGAGACCGA CTCGCAGGCG TCCCGAGAGC GCAGGCTCAA GAAGGTGAGG GCCGCCCTCC 20880
   CTGGCGTCCA GACCGTCCCT GGGCCCCCAG CCGGTCCCCG CGGCTCATAC CCTTCTTTCT 20940
   TTCTCCCTTG CAGAACTTGG CCCTGAGTCG GGAAAGTTTA GTCGTCTGAT CTGACGTTTT 21000
   CTACGTAGCT TTTGTATTTT TTTTTTTAAT TTGAAGGAAC ACTGATGAAG CCCTGCCATA 21060
   CCCCTCCCGA GTCTAATAAA ACGTATAATC ACAAGCTCTG GAGAGAACCA TTTGTTCGGC 21120
   CGCGCGGGGC GGGGGACCGG GGCTGCTCCC GTATGCGTCT GTAAAGCGCC GCGTCCCGGG 21180
   GGCACCGGAG TCCGGGGCCG GGAGGAAGAG ACCCAGCCTG GCCCGGCCCG CGCCCGCGCC 21240
   GCCGGCCGGA GAACGTGCCC CGCGCAGCCA CCGCCCGCCT GCGTGCGCGC CCCGGCCCCG 21300
   CCCAGGCGTG CGCATGCGCC CCGGCCCTCC GCCTTCGCGC ACCGCAGGCT GGCCGCCGGG 21360
   AGCGCGCGCG CGCTCCTCTC CCCTTCCAGC CCATCCCCCC CAGCCCCCCA CCGACCTACT 21420
   TTACTGTCTC CAAACTCGGG CAGCCCACCT GGCCCCCGAC GACCCCAGCC CCTGCTCCGG 21480
   GTACCCCGAC GTTCCATCCA GACCCGCGTT TCACCAGGGC GGCGCGCGGC GACCTCGCGC 21540
   CCCGCGGAGC CCCGGGCTCG CGCGCGCCCG CCCGCCCCCG GAGACAGACA GCGCGCGCGC 21600
   TCCCGGGCCG CCTCCCCCCA GCGCGCGTCC GCCCCGGGCT CGCGCCGCCG CCGCCGCCGC  21660
   CGCCGCGCGC GCGCAGCTCA AGTAAAGGAG GAAAAAAAAA AGGGGGAAAA ATAGAAAGCG  21720
   G                                                                  21721